# EUROPEAN PATENT APPLICATION

(11) **EP 4 455 129 A1**
(43) Date of publication of application: **30.10.2024**
(21) Application number: 22915167.5
(22) Date of filing: 30.12.2022
(51) Int. Cl.: C07D 401/04, C07D 401/14, C07D 491/048, C07D 495/04, C07D 498/04, C07D 517/04, A61K 31/4439, A61K 31/4353, A61K 31/4355, A61K 31/436, A61K 31/4365, A61K 31/437, A61P 35/00

(54) **DNA POLYMERASE THETA INHIBITOR AND USE THEREOF**

(30) Priority: 30.12.2021 CN 202111654169; 23.09.2022 CN 202211163698; 26.12.2022 CN 202211678161
(71) Applicant: Shanghai Apeiron Therapeutics Company Limited, Shanghai 201210 (CN)
(72) Inventor: GU, Xiaohui, Shanghai 201210 (CN)
(74) Representative: Bayramoglu et al.
(86) International application number: PCT/CN2022/143856
(87) International publication number: WO 2023/125918

(57) **Abstract**

The present disclosure discloses a DNA polymerase theta inhibitor and use thereof. The present disclosure specifically provides a compound of formula I or formula II, or a tautomer thereof, a stereoisomer thereof, a pharmaceutically acceptable salt of any of the above, or a solvate of any of the above, which has a better inhibitory effect on Polθ.

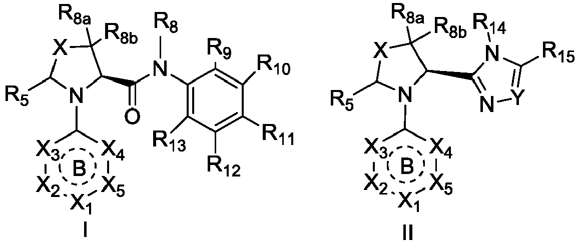

## Description

The present application claims priority to Chinese Patent Application No. 2021116541695, filed on Dec. 30, 2021; Chinese Patent Application No. 202211163698X, filed on Sep. 23, 2022; and Chinese Patent Application No. 2022116781617, filed on Dec. 26, 2022, the entire contents of which are incorporated herein by reference.

### Technical Field

The present disclosure relates to a DNA polymerase theta inhibitor and use thereof.

### Background Art

Mammalian cells have evolved multiple pathways to repair DNA double-strand breaks (DSBs) to ensure genome stability. DNA polymerase theta (Pοlθ) is a key component of the alternative non-homologous end joining (alt-NHEJ) pathway, also known as the microhomology-mediated end joining (MMEJ) pathway, involved in the repair of DNA double-strand breaks. MMEJ is another cell repair pathway besides non-homologous end joining (NHEJ) and homologous recombination (HR). MMEJ repair is driven by the annealing of microhomologous sequences flanking the ends of DNA, which was initially considered as a backup pathway for gene repair. With the development of research, it was also discovered that MMEJ is not a backup repair mechanism. Genetic, cell biological, and biochemical data have identified Polθ as a key protein in MMEJ.

When DNA end resection occurs, in the presence of BRCA2, BRCA2 not only recruits the recombinase RAD51 to DSB to promote HR,but also inhibits repair pathways such as alt-NHEJ. When homologous recombination-mediated repair is impaired (HR defect), such as BRCA1 or BRCA2 mutations, Polθ is highly expressed and directs DSB repair towards alt-EJ, initiating the DNA repair process of microhomology-mediated end joining (MMEJ). In the case of HR defect, inhibition of Polθ leads to cell death through accumulation of toxic RAD51 intermediates and inhibition of the alt-EJ repair pathway.

Polθ is a multifunctional enzyme comprising an N-terminal helicase domain and a C-terminal low-fidelity DNA polymerase domain. These two domains have been shown to have coordinated mechanistic functions in MMEJ. The helicase domain mediates the removal of the RPA protein from the end of ssDNA and provokes annealing. The polymerase domain extends the end of the ssDNA and fills the remaining gaps. In addition, Polθ also has the function of reverse transcribing RNA and promoting DNA repair using RNA as a template. Polθ is hardly expressed in normal tissues but is highly expressed in various tumor types such as breast cancer, ovarian cancer, HNSCC, and lung cancer, the overexpression of which is associated with poor prognosis. In addition, synthetic lethal interactions between Polθ and multiple DNA repair genes have been identified, including factors involved in homologous recombination (e.g., BRCA1/2). For these reasons, Polθ inhibitors represent a promising cancer treatment strategy.

### Summary of the Invention

The technical problem to be solved by the present disclosure is that the existing Polθ inhibitors have a relatively single structure. Therefore, the present disclosure provides a DNA polymerase theta inhibitor and use thereof, which has a better inhibitory effect on Polθ.

The present disclosure provides a compound of formula II or III, or a tautomer thereof, a stereoisomer thereof, a pharmaceutically acceptable salt of any of the above (referring to the compound of formula II or III , a tautomer thereof, or a stereoisomer thereof as described above), or a solvate of any of the above (referring to the compound of formula II or III, a tautomer thereof, a stereoisomer thereof, or a pharmaceutically acceptable salt of any of the above as described above), 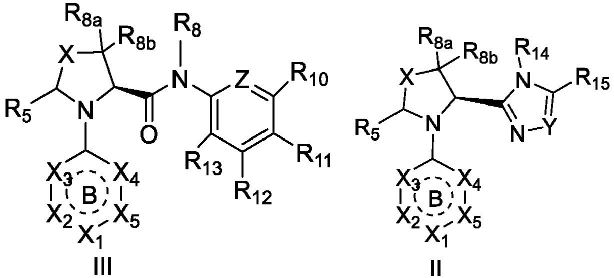
wherein Z is C(R₉) or N;
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, X₃ is C(R₃ₐ) or N, X₄ is C(R₄ₐ) or N, X₅ is C(R₃ₐ) or N, and
R₃ₐ, R₄ₐ, and R₅ₐ are each independently hydrogen, C₁₋₆ alkyl, C₁₋₆ alkoxy, halogen, C₁₋₆ alkyl substituted with one or more R^{X1}, C₁₋₆ alkoxy substituted with one or more R^{X1}, C₃₋₈ cycloalkyl substituted with one or more R^{X1}, C₃₋₈ cycloalkyl, C₂₋₆ alkenyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, X₂ is C(R₂ₐ) or N, X₃ is C(R₃ₐ) or N, X₄ is C(R₄ₐ) or N, and R₂ₐ, R₃ₐ, and R₄ₐ are each independently hydrogen, C₁₋₆ alkyl, C₁₋₆ alkoxy, halogen, C₁₋₆ alkyl substituted with one or more R^{X1}, C₃₋₈ cycloalkyl, -CN-, C₁₋₆ alkoxy substituted with one or more R^{X1}, C₃₋₈ cycloalkyl substituted with one or more R^{X1}, -SO₂-R₂ₐₐ, -SO-R₂ₐₐ, -SO(=NH)R₂ₐₐ, -PO(C₁₋₆ alkyl)₂, -SF₅, -S-R₂ₐₐ, or -NR^{x}R^{y};
or, R₄ₐ and R₅ₐ, R₄ₐ and R_{5b}, R_{4b} and R_{5b}, or R_{4b} and R₅ₐ join to form ring A, X₁ is C(R₁ₐ), X₂ is C(R₂ₐ) or N, X₃ is C(R₃ₐ) or N, and R₁ₐ, R₂ₐ, and R₃ₐ are each independently hydrogen, C₁₋₆ alkyl, C₁₋₆ alkoxy, halogen, C₁₋₆ alkyl substituted with one or more R^{X1}, C₁₋₆ alkoxy substituted with one or more R^{X1}, C₃₋₈ cycloalkyl substituted with one or more R^{X1}, C₃₋₈ cycloalkyl, -CN-, or -NR^{x}R^{y};
or, R₂ₐ and R₃ₐ, R₂ₐ and R_{3b}, R_{2b} and R_{3b}, or R_{2b} and R₃ₐ join to form ring A, X₁ is C(R₁ₐ), X₄ is C(R₄ₐ) or N, X₅ is C(R₅ₐ) or N, and R₁ₐ, R₄ₐ, and R₅ₐ are each independently hydrogen;
R₂ₐₐ is C₃₋₈ cycloalkyl, C₃₋₈ cycloalkyl substituted with one or more R^{X1}, C₁₋₆ alkyl, or C₁₋₆ alkyl substituted with one or more R^{X1};
R^{x} and R^{y} are each independently hydrogen, C₁₋₆ alkyl, C₁₋₆ alkyl substituted with 1, 2, or 3 R^{X1}, C₃₋₈ cycloalkyl, "4- to 8-membered heterocycloalkyl having 1, 2, 3, or 4 heteroatoms selected from 1, 2, 3, or 4 of N, O, S, and Se", -C(O)C₃₋₈ cycloalkyl, C₃₋₈ cycloalkyl substituted with 1, 2, or 3 R^{X2}, -C(O)C₁₋₆ alkyl substituted with 1, 2, or 3 R^{X3}, "4- to 8-membered heterocycloalkyl having 1, 2, 3, or
4 heteroatoms selected from 1, 2, 3, or 4 of N, O, S, and Se" substituted with 1, 2, or 3 R^{X4}, -C(O)C₃₋₈ cycloalkyl substituted with 1, 2, or 3 R^{X5}, or -C(O)C₁₋₆ alkyl;
R^{X1}, R^{X2}, R^{X3}, and R^{X5} are each independently halogen, hydroxyl, amino, or sulfonyl;
each R^{X4} is independently oxo (=O), hydroxyl, C₁₋₆ alkyl substituted with 1, 2, or 3 hydroxyl groups, C₁₋₆ alkoxy substituted with 1, 2, or 3 hydroxyl groups, or -C(O)C₁₋₆ alkyl;
or, R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se";
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se", or a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, halogen, nitro, amino, -NH(C₁₋₆ alkyl), -N(C₁₋₆ alkyl)₂, -SO₂(C₁₋₆ alkyl), -SCF₃, -S(C₁₋₆ alkyl), -PO(C₁₋₆ alkyl)₂, -CO(C₁₋₆ alkyl), -COOH, -CONH₂, -CONR^{x}R^{y}, -NR^{x}R^{y}, C₁₋₆ alkoxy substituted with one or more halogens, C₂₋₆ alkenyl, C₂₋₆ alkynyl, C₃₋₈ cycloalkyl substituted with one or more halogens, -SO₂(C₃₋₈ cycloalkyl), -SO(=NH)(C₁₋₆ alkyl), -SO(=NH)(C₃₋₈ cycloalkyl), -SCHF₂, -S(C₃₋₈ cycloalkyl), -SO(C₃₋₈ cycloalkyl), -CO(C₃₋₈ cycloalkyl), -COCHF₂, or "4- to 8-membered heterocycloalkyl having 1, 2, 3, or 4 heteroatoms selected from 1, 2, 3, or 4 of N, O, S, and Se";
Rs is oxo (=O), hydrogen, cyano, C₁₋₆ alkyl substituted with one or more halogens, -SO₂-C₁₋₆ alkyl, -SO₂(C₃₋₈ cycloalkyl), -P(O)(C₁₋₆ alkyl)₂, -P(O)(C₃₋₈ cycloalkyl)₂, or -CH₂-R^{z};
X is -CHR₆₋₁-, -NR₆₋₂-, -Se-, -S-, S(=O), SO₂, SO(=NH), or -O-;
R₆₋₁ is hydrogen, hydroxyl, halogen, CN, -NR^{m}Rⁿ, C₁₋₆ alkyl, C₁₋₆ alkoxy, C₁₋₆ alkyl substituted with one or more R₁₆₋₁, C₁₋₆ alkoxy substituted with one or more R₁₆₋₁, -SO₂(C₃₋₈ cycloalkyl), -SO(=NH)(C₁₋₆ alkyl), -SO(=NH)(C₃₋₈ cycloalkyl), C₃₋₈ cycloalkyl, cyano, -L-(C₆-C₁₀ aryl), -L-(C₆-C₁₀ aryl substituted with one or more R₆₋₁₋₁), -L-(4- to 8-membered heterocycloalkyl having 1, 2, 3, or 4 heteroatoms selected from 1, 2, 3, or 4 of N, O, S, and Se), -L-(4- to 8-membered heterocycloalkyl having 1, 2, 3, or 4 heteroatoms selected from 1, 2, 3, or 4 of N, O, S, and Se substituted with one or more R₆₋₁₋₂), or -SO₂-C₁₋₆ alkyl;
L represents a single link bond or C₁₋₆ alkylene optionally in which 1, 2, or 3 methylene groups are replaced by -O-;
R₆₋₁₋₁ and R₆₋₁₋₂ are each independently oxo, hydroxyl, C₁₋₆ alkyl substituted with 1, 2, or 3 hydroxyl groups, COC₁₋₆ alkyl, or - COOC₁₋₆ alkyl;
R₆₋₂ is hydrogen, hydroxyl, halogen, CN, -NR^{m}Rⁿ, C₁₋₆ alkyl, C₁₋₆ alkoxy, C₁₋₆ alkyl substituted with one or more R₁₆₋₁, C₁₋₆ alkoxy substituted with one or more R₁₆₋₁, -SO₂(C₃₋₈ cycloalkyl), -SO(=NH)(C₁₋₆ alkyl), -SO(=NH)(C₃₋₈ cycloalkyl), C₃₋₈ cycloalkyl, oxa-C₃₋₈ cycloalkyl, cyano, or -SO₂-C₁₋₆ alkyl;
R₈ₐ is hydrogen, -NR^{x}R^{y}, -CH₂-R^{z}, or hydroxyl;
or, R₆₋₁ and R₆₋₂ are each independently -OR₁₅ₐ; R₈ₐ is -OR_{15b};
R₁₅ₐ and R_{15b} together with the atom to which they are attached join to form a 3- to 8-membered saturated monocyclic, spirocyclic, or fused cyclic carbon ring, a "3- to 8-membered saturated monocyclic, spirocyclic, or fused cyclic carbon ring" substituted with one or more C₁₋₆ alkyl, or a "4- to 8-membered saturated monocyclic, spirocyclic, or fused cyclic heterocyclic ring containing one heteroatom N";
each R₁₆₋₁ is independently halogen, hydroxyl, -NR^{m}Rⁿ, oxo (=O), hydroxyl, C₁₋₆ alkyl substituted with 1, 2, or 3 hydroxyl groups, -C(O)C₁₋₆ alkyl, C₁₋₆ alkoxy, C₃₋₈ cycloalkyl, C₁₋₆ alkoxy substituted with 1, 2, or 3 hydroxyl groups, or -C(O)OC₁₋₆ alkyl;
R^{m} and Rⁿ are each independently hydrogen, C₁₋₆ alkyl, C₁₋₆ alkyl substituted with one or more halogens, C₃₋₈ cycloalkyl, C₃₋₈ cycloalkyl substituted with one or more halogens, -CO(C₁₋₆ alkyl), -CO(C₃₋₈ cycloalkyl), -CO(C₃₋₈ cycloalkyl substituted with one or more halogens), "4- to 8-membered heterocycloalkyl having 1, 2, 3, or 4 heteroatoms selected from 1, 2, 3, or 4 of N, O, S, and Se", C₁₋₆ alkyl substituted with 1, 2, or 3 R^{m-1}, -CO(C₁₋₆ alkyl) substituted with 1, 2, or 3 R^{m-2}, or "4- to 8-membered heterocycloalkyl having 1, 2, 3, or 4 heteroatoms selected from 1, 2, 3, or 4 of N, O, S, and Se" substituted with 1, 2, or 3 R^{m-3};
R^{m} and R^{m-2} are each independently hydroxyl, halogen, amino, or sulfonyl;
each R^{m-3} is independently oxo (=O), hydroxyl, -CO(C₁₋₆ alkyl), C₁₋₆ alkyl, C₁₋₆ alkoxy, C₁₋₆ alkoxy substituted with 1, 2, or 3 hydroxyl groups, or C₁₋₆ alkyl substituted with 1, 2, or 3 hydroxyl groups;
R^{z} is hydrogen, C₁₋₆ alkyl, hydroxyl, C₁₋₆ alkoxy, C₃₋₈ cycloalkyl, C₁₋₆ alkoxy substituted with 1, 2, or 3 hydroxyl groups, or C₁₋₆ alkyl substituted with 1, 2, or 3 hydroxyl groups;
R_{8b} is hydrogen or hydroxyl;
R₈ is C₁₋₆ alkyl, C₃₋₈ cycloalkyl, C₃₋₈ cycloalkyl substituted with one or more R₈₋₁, or C₁₋₆ alkyl substituted with one or more R₈₋₁; each R₈₋₁ is independently halogen or deuterium;
R₉, R₁₀, R₁₁, R₁₂, and R₁₃ are each independently hydrogen, C₁₋₆ alkyl, hydroxyl, C₁₋₆ alkoxy, C₃₋₈ cycloalkyl, -NR^{m}Rⁿ, halogen, C₁₋₆ alkyl substituted with one or more halogens, C₂₋₆ alkenyl, C₆-C₁₀ aryl, C₁₋₆ alkoxy substituted with one or more halogens, C₃₋₈ cycloalkyl substituted with one or more halogens, "8- to 10-membered heteroaryl having 1, 2, 3, or 4 heteroatoms selected from 1, 2, 3, or 4 of N, O, S, and Se", C₆-C₁₀ aryl substituted with one or more R₉₋₁, or "8- to 10-membered heteroaryl having 1, 2, 3, or 4 heteroatoms selected from 1, 2, 3, or 4 of N, O, S, and Se" substituted with one or more R₉₋₂;
R₉₋₁ and R₉₋₂ are each independently C₁₋₆ alkyl, C₁₋₆ alkoxy, C₃₋₈ cycloalkyl, halogen, -CN, -C(O)N(C₁₋₆ alkyl)₂, -CH₂-N(C₁₋₆ alkyl)₂, C₁₋₆ alkyl substituted with one or more R₉₋ₐ, or C₁₋₆ alkoxy substituted with one or more R_{9-b};
R₉₋ₐ and R_{9-b} are each independently hydroxyl or halogen;
or, two adjacent groups of the R₉, R₁₀, R₁₁, R₁₂, and R₁₃ can join to form a "4- to 8-membered saturated or unsaturated monocyclic heterocyclic ring having 1, 2, 3, or 4 heteroatoms selected from 1, 2, 3, or 4 of N, O, S, and Se";
R₁₄ is C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₁₄₋₁, or "8- to 10-membered heteroaryl having 1, 2, 3, or 4 heteroatoms selected from 1, 2, 3, or 4 of N, O, S, and Se" substituted with one or more R₁₄₋₂;
R₁₄₋₁ and R₁₄₋₂ are each independently C₁₋₆ alkyl, C₁₋₆ alkoxy, C₁₋₆ alkyl substituted with 1, 2, or 3 hydroxyl groups, C₃₋₈ cycloalkyl, halogen, -CN, C₁₋₆ alkyl substituted with one or more halogens, C₁₋₆ alkoxy substituted with one or more halogens, C₃₋₈ cycloalkyl substituted with one or more halogens, -CONR^{m}Rⁿ, or -CH₂-NR^{m}Rⁿ;
R₁₅ is hydrogen, halogen, C₁₋₆ alkyl, C₃₋₈ cycloalkyl, C₁₋₆ alkoxy, C₁₋₆ alkoxy substituted with one or more R₁₅₋₁, C₃₋₈ cycloalkyl substituted with one or more R₁₅₋₁, or C₁₋₆ alkyl substituted with one or more R₁₅₋₁;
each R₁₅₋₁ is independently halogen or deuterium;
Y is -C(R₁₆)= or -N=; and
R₁₆ is hydrogen, C₁₋₆ alkyl, C₁₋₆ alkoxy, C₃₋₈ cycloalkyl, or halogen.

The present disclosure provides a compound of formula II or III, or a tautomer thereof, a stereoisomer thereof, a pharmaceutically acceptable salt of any of the above (referring to the compound of formula II or III , a tautomer thereof, or a stereoisomer thereof as described above), or a solvate of any of the above (referring to the compound of formula II or III, a tautomer thereof, a stereoisomer thereof, or a pharmaceutically acceptable salt of any of the above as described above), 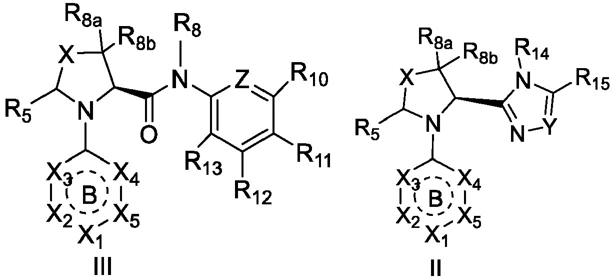
wherein Z is C(R₉) or N;
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, C₁₋₆ alkyl, C₁₋₆ alkoxy, halogen, C₁₋₆ alkyl substituted with one or more halogens, C₃₋₈ cycloalkyl, -CN-, C₁₋₆ alkoxy substituted with one or more halogens, -SF₅, -S-R₂ₐₐ, or -NR^{x}R^{y};
or, R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
R₂ₐₐ is C₁₋₆ alkyl or C₁₋₆ alkyl substituted with one or more halogens;
R^{x} and R^{y} are each independently hydrogen, C₁₋₆ alkyl, C₁₋₆ alkyl substituted with 1, 2, or 3 R^{X1}, C₃₋₈ cycloalkyl, "4- to 8-membered heterocycloalkyl having 1, 2, 3, or 4 heteroatoms selected from 1, 2, 3, or 4 of N, O, S, and Se", -C(O)C₃₋₈ cycloalkyl, C₃₋₈ cycloalkyl substituted with 1, 2, or 3 R^{X2}, -C(O)C₁₋₆ alkyl substituted with 1, 2, or 3 R^{X3}, "4- to 8-membered heterocycloalkyl having 1, 2, 3, or 4 heteroatoms selected from 1, 2, 3, or 4 of N, O, S, and Se" substituted with 1, 2, or 3 R^{X4}, -C(O)C₃₋₈ cycloalkyl substituted with 1, 2, or 3 R^{X5}, or -C(O)C₁₋₆ alkyl;
each R^{X1} is independently halogen, hydroxyl, amino, or -SO₃H; R^{X2}, R^{X3}, and R^{X5} are each independently halogen, hydroxyl, amino, or sulfonyl;
each R^{X4} is independently oxo (=O), hydroxyl, C₁₋₆ alkyl substituted with 1, 2, or 3 hydroxyl groups, or -C(O)₁₋₆ alkyl;
or, R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se";
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se", or a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, halogen, nitro, amino, -NH(C₁₋₆ alkyl), -N(C₁₋₆ alkyl)₂, -SO₂(C₁₋₆ alkyl), -SCF₃, -S(C₁₋₆ alkyl), -PO(C₁₋₆ alkyl)₂, -CO(C₁₋₆ alkyl), -COOH, -CONH₂, -CONR^{x}R^{y}, -NR^{x}R^{y}, C₁₋₆ alkoxy substituted with one or more halogens, C₂₋₆ alkenyl, C₂₋₆ alkynyl, or "4- to 8-membered heterocycloalkyl having 1, 2, 3, or 4 heteroatoms selected from 1, 2, 3, or 4 of N, O, S, and Se";
R₅ is oxo (=O), hydrogen, cyano, C₁₋₆ alkyl substituted with one or more halogens, -SO₂-C₁₋₆ alkyl, -P(O)(C₁₋₆ alkyl)₂, or -CH₂-R^{z};
X is -CHR₆₋₁-, -NR₆₋₂-, or -O-;
R₆₋₁ and R₆₋₂ are each independently hydrogen, hydroxyl, halogen, CN, -NR^{m}Rⁿ, C₁₋₆ alkyl, C₁₋₆ alkoxy, C₁₋₆ alkyl substituted with one or more R₁₆₋₁, C₃₋₈ cycloalkyl, oxa-C₃₋₈ cycloalkyl, cyano, or -SO₂-C₁₋₆ alkyl;
or, R₆₋₁ and R₆₋₂ are each independently -OR₁₅ₐ; R₈ₐ is -OR_{15b};
R₁₅ₐ and R_{15b} together with the atom to which they are attached join to form a 3- to 8-membered saturated monocyclic, spirocyclic, or fused cyclic carbon ring, or a "4- to 8-membered saturated monocyclic, spirocyclic, or fused cyclic heterocyclic ring containing one heteroatom N";
each R₁₆₋₁ is independently halogen, hydroxyl, -NR^{m}Rⁿ, oxo (=O), hydroxyl, C₁₋₆ alkyl substituted with 1, 2, or 3 hydroxyl groups, -C(O)C₁₋₆ alkyl, or -C(O)OC₁₋₆ alkyl;
R^{m} and Rⁿ are each independently hydrogen, C₁₋₆ alkyl, C₃₋₈ cycloalkyl, -CO(C₁₋₆ alkyl), "4- to 8-membered heterocycloalkyl having 1, 2, 3, or 4 heteroatoms selected from 1, 2, 3, or 4 of N, O, S, and Se", C₁₋₆ alkyl substituted with 1, 2, or 3 R^{m-1}, -CO(C₁₋₆ alkyl) substituted with 1, 2, or 3 R^{m-2}, or "4- to 8-membered heterocycloalkyl having 1, 2, 3, or 4 heteroatoms selected from 1, 2, 3, or 4 of N, O, S, and Se" substituted with 1, 2, or 3 R^{m-3};
R^{m-1} and R^{m-2} are each independently hydroxyl, halogen, amino, or sulfonyl;
each R^{m-3} is independently oxo (=O), hydroxyl, -CO(C₁₋₆ alkyl), or C₁₋₆ alkyl substituted with 1, 2, or 3 hydroxyl groups;
R₈ₐ is hydrogen, -CH₂-R^{z}, or hydroxyl;
R^{z} is hydrogen, C₁₋₆ alkyl, hydroxyl, C₁₋₆ alkoxy, or C₁₋₆ alkyl substituted with 1, 2, or 3 hydroxyl groups;
R_{8b} is hydrogen or hydroxyl;
R₈ is C₁₋₆ alkyl, C₃₋₈ cycloalkyl, or C₁₋₆ alkyl substituted with one or more R₈₋₁;
each R₈₋₁ is independently halogen or deuterium;
R₉, R₁₀, R₁₁, R₁₂, and R₁₃ are each independently hydrogen, C₁₋₆ alkyl, hydroxyl, C₁₋₆ alkoxy, C₃₋₈ cycloalkyl, -NR^{m}Rⁿ, halogen, C₁₋₆ alkyl substituted with one or more halogens, C₂₋₆ alkenyl, C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₉₋₁, or "8- to 10-membered heteroaryl having 1, 2, 3, or 4 heteroatoms selected from 1, 2, 3, or 4 of N, O, S, and Se" substituted with one or more R₉₋₂;
R₉₋₁ and R₉₋₂ are each independently C₁₋₆ alkyl, C₁₋₆ alkoxy, C₃₋₈ cycloalkyl, halogen, -CN, -C(O)N(C₁₋₆ alkyl)₂, -CH₂-N(C₁₋₆ alkyl)₂, C₁₋₆ alkyl substituted with one or more R₉₋ₐ, or C₁₋₆ alkoxy substituted with one or more R_{9-b};
R₉₋ₐ and R_{9-b} are each independently hydroxyl or halogen;
or, two adjacent groups of the R₉, R₁₀, R₁₁, R₁₂, and R₁₃ can join to form a "4- to 8-membered saturated or unsaturated monocyclic heterocyclic ring having 1, 2, 3, or 4 heteroatoms selected from 1, 2, 3, or 4 of N, O, S, and Se";
R₁₄ is C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₁₄₋₁, or "8- to 10-membered heteroaryl having 1, 2, 3, or 4 heteroatoms selected from 1, 2, 3, or 4 of N, O, S, and Se" substituted with one or more R₁₄₋₂;
R₁₄₋₁ and R₁₄₋₂ are each independently C₁₋₆ alkyl, C₁₋₆ alkoxy, C₁₋₆ alkyl substituted with 1, 2, or 3 hydroxyl groups, C₃₋₈ cycloalkyl, halogen, -CN, C₁₋₆ alkyl substituted with one or more halogens, -CONR^{m}Rⁿ, or -CH₂-NR^{m}Rⁿ;
R₁₅ is hydrogen, halogen, C₁₋₆ alkyl, C₃₋₈ cycloalkyl, or C₁₋₆ alkyl substituted with one or more R₁₅₋₁;
each R₁₅₋₁ is independently halogen or deuterium;
Y is -C(R₁₆)= or -N=; and
R₁₆ is hydrogen, C₁₋₆ alkyl, C₁₋₆ alkoxy, or halogen.

The present disclosure provides a compound of formula I or formula II, or a tautomer thereof, a stereoisomer thereof, a pharmaceutically acceptable salt of any of the above (referring to the compound of formula I or formula II, a tautomer thereof, or a stereoisomer thereof as described above), or a solvate of any of the above (referring to the compound of formula I or formula II, a tautomer thereof, a stereoisomer thereof, or a pharmaceutically acceptable salt of any of the above as described above), 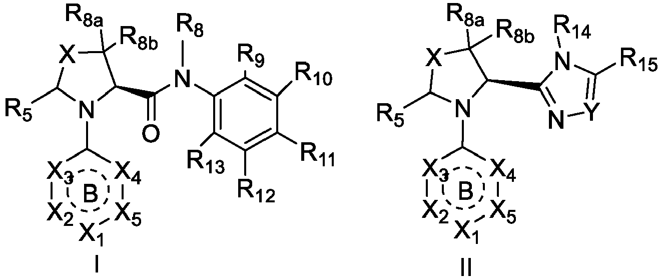 wherein
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
R^{x} and R^{y} are each independently hydrogen, C₁₋₆ alkyl, C₁₋₆ alkyl substituted with 1, 2, or 3 R^{X1}, C₃₋₈ cycloalkyl, or -C(O)C₁₋₆ alkyl; each R^{X1} is independently halogen, hydroxyl, amino, or -SO₃H;
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, or 3 heteroatoms selected from 1, 2, or 3 of N, O, and S", or a "4- to 8-membered saturated or unsaturated monocyclic, spirocyclic, or fused cyclic heterocyclic ring having 1, 2, or 3 heteroatoms selected from 1, 2, or 3 of N, O, and S" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, or halogen;
R₅ is oxo (=O) or hydrogen;
X is -CHR₆₋₁-, -NR₆₋₂-, or -O-;
R₆₋₁ and R₆₋₂ are each independently hydrogen, hydroxyl, halogen, CN, -NR^{m}Rⁿ, C₁₋₆ alkyl, C₁₋₆ alkoxy, or C₁₋₆ alkyl substituted with one or more R₁₆₋₁;
each R₁₆₋₁ is independently halogen, hydroxyl, or -NR^{m}Rⁿ;
R^{m} and Rⁿ are each independently hydrogen or C₁₋₆ alkyl;
R₈ₐ is hydrogen, -CH₂-R^{z}, or hydroxyl;
R^{z} is hydrogen, C₁₋₆ alkyl, hydroxyl, C₁₋₆ alkoxy, or C₁₋₆ alkyl substituted with 1, 2, or 3 hydroxyl groups;
R_{8b} is hydrogen or hydroxyl;
R₈ is C₁₋₆ alkyl, C₃₋₈ cycloalkyl, or C₁₋₆ alkyl substituted with one or more R₈₋₁;
each R₈₋₁ is independently halogen or deuterium;
R₉, R₁₀, R₁₁, R₁₂, and R₁₃ are each independently hydrogen, C₁₋₆ alkyl, hydroxyl, C₁₋₆ alkoxy, C₃₋₈ cycloalkyl, -NR^{m}Rⁿ, halogen, or C₁₋₆ alkyl substituted with one or more halogens;
R₁₄ is C₆-C₁₀ aryl, "8- to 10-membered heteroaryl having 1, 2, or 3 heteroatoms selected from 1, 2, or 3 of N, O, and S", C₆-C₁₀ aryl substituted with one or more R₁₄₋₁, or "8- to 10-membered heteroaryl having 1, 2, or 3 heteroatoms selected from 1, 2, or 3 of N, O, and S" substituted with one or more R₁₄₋₂;
R₁₄₋₁ and R₁₄₋₂ are each independently C₁₋₆ alkyl, C₁₋₆ alkoxy, C₁₋₆ alkyl substituted with 1, 2, or 3 hydroxyl groups, C₃₋₈ cycloalkyl, halogen, -CN, or C₁₋₆ alkyl substituted with one or more halogens;
R₁₅ is hydrogen, halogen, or C₁₋₆ alkyl;
Y is -C(R₁₆)= or -N=; and
R₁₆ is hydrogen, C₁₋₆ alkyl, C₁₋₆ alkoxy, or halogen.

In certain preferred embodiments of the present disclosure, certain groups of the compound of formula I, formula II, or formula III, or a tautomer thereof, a stereoisomer thereof, a pharmaceutically acceptable salt of any of the above (referring to the compound of formula I, formula II, or formula III, a tautomer thereof, or a stereoisomer thereof as described above), or a solvate of any of the above (referring to the compound of formula I, formula II, or formula III, a tautomer thereof, a stereoisomer thereof, or a pharmaceutically acceptable salt of any of the above as described above) are defined as follows, and groups not mentioned are as described in any of the embodiments of the present disclosure (referred to as "in some embodiments").

In some embodiments, R₂ₐ and R₃ₐ, R₂ₐ and R_{3b}, R_{2b} and R_{3b}, or R_{2b} and R₃ₐ join to form ring A, X₁ is C(R₁ₐ), X₄ is C(R₄ₐ) or N, X₅ is C(R₅ₐ) or N, and R₁ₐ, R₄ₐ, and R₅ₐ are each independently hydrogen.

In some embodiments, when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently C₁₋₆ alkoxy substituted with one or more halogens, -SF₅, or -S-R₂ₐₐ;
R₂ₐₐ is C₁₋₆ alkyl or C₁₋₆ alkyl substituted with one or more halogens.

In some embodiments, when R₁ₐ and R₂ₐ, R₁ₐ and R_{2b}, R_{1b} and R_{2b}, or R_{1b} and R₂ₐ join to form ring A, X₃ is C(R₃ₐ) or N, X₄ is C(R₄ₐ) or N, X₅ is C(R₅ₐ) or N, and R₃ₐ, R₄ₐ, and R₅ₐ are each independently C₁₋₆ alkoxy substituted with one or more R^{X1}, C₃₋₈ cycloalkyl substituted with one or more R^{X1}, or C₂₋₆ alkenyl.

In some embodiments, when R₁ₐ and R₅ₐ, R₁ₐ and R_{5b}, R_{1b} and R_{5b}, or R_{1b} and R₅ₐ join to form ring A, X₂ is C(R₂ₐ) or N, X₃ is C(R₃ₐ) or N, X₄ is C(R₄ₐ) or N, and R₂ₐ, R₃ₐ, and R₄ₐ are each independently C₃₋₈ cycloalkyl substituted with one or more R^{X1}, -SO₂-R₂ₐₐ, -SO-R₂ₐₐ, -SO(=NH)R₂ₐₐ, or -PO(C₁₋₆ alkyl)₂.

In some embodiments, when R₄ₐ and R₅ₐ, R₄ₐ and R_{5b}, R_{4b} and R_{5b}, or R_{4b} and R₅ₐ join to form ring A, X₁ is C(R₁ₐ), X₂ is C(R₂ₐ) or N, X₃ is C(R₃ₐ) or N, and R₁ₐ, R₂ₐ, and R₃ₐ are each independently C₁₋₆ alkoxy substituted with one or more R^{X1} or C₃₋₈ cycloalkyl substituted with one or more R^{X1}.

In some embodiments, when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently -SO₂-R₂ₐₐ, -SO-R₂ₐₐ, or -SO(=NH)R₂ₐₐ;
R₂ₐₐ is C₃₋₈ cycloalkyl or C₃₋₈ cycloalkyl substituted with one or more R^{X1}.

In some embodiments, R₂ₐₐ is C₃₋₈ cycloalkyl or C₃₋₈ cycloalkyl substituted with one or more R^{X1}.

In some embodiments, each R^{X1} is independently sulfonyl.

In some embodiments, each R^{X4} is independently C₁₋₆ alkoxy substituted with 1, 2, or 3 hydroxyl groups.

In some embodiments, R₁₋₁ and R₁₋₂ are each independently C₃₋₈ cycloalkyl substituted with one or more halogens, -SO₂(C₃₋₈ cycloalkyl), -SO(=NH)(C₁₋₆ alkyl), -SO(=NH)(C₃₋₈ cycloalkyl), -SCHF₂, -S(C₃₋₈ cycloalkyl), -SO(C₃₋₈ cycloalkyl), -CO(C₃₋₈ cycloalkyl), or -COCHF₂.

In some embodiments, R₅ is -SO₂(C₃₋₈ cycloalkyl) or -P(O)(C₃₋₈ cycloalkyl)₂.

In some embodiments, X is -Se-, -S-, S(=O), SO₂, or SO(=NH).

In some embodiments, when X is -S-, R₅ is oxo.

In some embodiments, R₆₋₁ is C₁₋₆ alkoxy substituted with one or more R₁₆₋₁, -SO₂(C₃₋₈ cycloalkyl), -SO(=NH)(C₁₋₆ alkyl), -SO(=NH)(C₃₋₈ cycloalkyl), -L-C₆-C₁₀ aryl, or -L-"4- to 8-membered heterocycloalkyl having 1, 2, 3, or 4 heteroatoms selected from 1, 2, 3, or 4 of N, O, S, and Se".

In some embodiments, R₆₋₂ is C₁₋₆ alkoxy substituted with one or more R₁₆₋₁, -SO₂(C₃₋₈ cycloalkyl), -SO(=NH)(C₁₋₆ alkyl), or -SO(=NH)(C₃₋₈ cycloalkyl).

In some embodiments, R₆₋₁ and R₆₋₂ are each independently -OR₁₅ₐ; R₈ₐ is -OR_{15b}; and R₁₅ₐ and R_{15b}, together with the atom to which they are attached join to form a "3- to 8-membered saturated monocyclic, spirocyclic, or fused cyclic carbon ring" substituted with one or more C₁₋₆ alkyl.

In some embodiments, each R₁₆₋₁ is independently C₁₋₆ alkoxy, C₃₋₈ cycloalkyl, or C₁₋₆ alkoxy substituted with 1, 2, or 3 hydroxyl groups.

In some embodiments, R^{m} and Rⁿ are each independently C₁₋₆ alkyl substituted with one or more halogens, C₃₋₈ cycloalkyl substituted with one or more halogens, -CO(C₃₋₈ cycloalkyl), or -CO(C₃₋₈ cycloalkyl substituted with one or more halogens).

In some embodiments, each R^{m-3} is independently C₁₋₆ alkyl, C₁₋₆ alkoxy, or C₁₋₆ alkoxy substituted with 1, 2, or 3 hydroxyl groups.

In some embodiments, R^{z} is C₃₋₈ cycloalkyl or C₁₋₆ alkoxy substituted with 1, 2, or 3 hydroxyl groups.

In some embodiments, R₈ is C₃₋₈ cycloalkyl substituted with one or more R₈₋₁.

In some embodiments, R₉, R₁₀, R₁₁, R₁₂, and R₁₃ are each independently C₁₋₆ alkoxy substituted with one or more halogens or C₃₋₈ cycloalkyl substituted with one or more halogens.

In some embodiments, R₁₄₋₁ and R₁₄₋₂ are each independently C₁₋₆ alkoxy substituted with one or more halogens or C₃₋₈ cycloalkyl substituted with one or more halogens.

In some embodiments, R₁₅ is C₁₋₆ alkoxy, C₁₋₆ alkoxy substituted with one or more R₁₅₋₁, or C₃₋₈ cycloalkyl substituted with one or more R₁₅₋₁.

In some embodiments, R₁₆ is C₃₋₈ cycloalkyl.

In some embodiments, R^{x} and R^{y} are each independently "4- to 8-membered heterocycloalkyl having 1, 2, 3, or 4 heteroatoms selected from 1, 2, 3, or 4 of N, O, S, and Se", -C(O)C₃₋₈ cycloalkyl, C₃₋₈ cycloalkyl substituted with 1, 2, or 3 R^{X2}, -C(O)C₁₋₆ alkyl substituted with 1, 2, or 3 R^{X3}, "4- to 8-membered heterocycloalkyl having 1, 2, 3, or 4 heteroatoms selected from 1, 2, 3, or 4 of N, O, S, and Se" substituted with 1, 2, or 3 R^{X4}, or -C(O)C₃₋₈ cycloalkyl substituted with 1, 2, or 3 R^{X5};
R^{X2}, R^{X3}, and R^{X5} are each independently halogen, hydroxyl, amino, or sulfonyl;
each R^{X4} is independently oxo (=O), hydroxyl, C₁₋₆ alkyl substituted with 1, 2, or 3 hydroxyl groups, or -C(O)C₁₋₆ alkyl;
or, R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se".

In some embodiments, each R^{X1} is independently sulfonyl.

In some embodiments, R₁₋₁ and R₁₋₂ are each independently nitro, amino, -NH(C₁₋₆ alkyl), -N(C₁₋₆ alkyl)₂, -SO₂(C₁₋₆ alkyl), -SCF₃, -S(C₁₋₆ alkyl), -PO(C₁₋₆ alkyl)₂, -CO(C₁₋₆ alkyl), -COOH, -CONH₂, -CONR^{x}R^{y}, -NR^{x}R^{y}, C₁₋₆ alkoxy substituted with one or more halogens, C₂₋₆ alkenyl, C₂₋₆ alkynyl, or "4- to 8-membered heterocycloalkyl having 1, 2, 3, or 4 heteroatoms selected from 1, 2, 3, or 4 of N, O, S, and Se".

In some embodiments, R₅ is cyano, C₁₋₆ alkyl substituted with one or more halogens, -SO₂-C₁₋₆ alkyl, -P(O)(C₁₋₆ alkyl)₂, or -CH₂-R^{z}. In some embodiments, when X is -NR₆₋₂- or -O-, R₅ is oxo (=O).

In some embodiments, X is -S-, -S(=O)-, -SO₂-, or -Se-.

In some embodiments, R₆₋₁ and R₆₋₂ are each independently Cs s cycloalkyl, oxa-C₃₋₈ cycloalkyl, cyano, or -SO₂-C₁₋₆ alkyl;
or, R₆₋₁ and R₆₋₂ are each independently -OR₁₅ₐ; R₈ₐ is -OR_{15b};
R₁₅ₐ and R_{15b} together with the atom to which they are attached join to form a 3- to 8-membered saturated monocyclic, spirocyclic, or fused cyclic carbon ring, or a "4- to 8-membered saturated monocyclic, spirocyclic, or fused cyclic heterocyclic ring containing one heteroatom N".

In some embodiments, each R₁₆₋₁ is independently a C₆-C₁₀ aryl substituted with one or more R₁₆₋ₐ, or "4- to 8-membered heterocycloalkyl having 1, 2, 3, or 4 heteroatoms selected from 1, 2, 3, or 4 of N, O, S, and Se" substituted with one or more R_{16-b}; R₁₆₋ₐ and R_{16-b} are each independently oxo (=O), hydroxyl, C₁₋₆ alkyl substituted with 1, 2, or 3 hydroxyl groups, -C(O)C₁₋₆ alkyl, or -C(O)OC₁₋₆ alkyl.

In some embodiments, R^{m} and R" are each independently C₃₋₈ cycloalkyl, -CO(C₁₋₆ alkyl), "4- to 8-membered heterocycloalkyl having 1, 2, 3, or 4 heteroatoms selected from 1, 2, 3, or 4 of N, O, S, and Se", C₁₋₆ alkyl substituted with 1, 2, or 3 R^{m-1}, -CO(C₁₋₆ alkyl) substituted with 1, 2, or 3 R^{m-2}, or "4- to 8-membered heterocycloalkyl having 1, 2, 3, or 4 heteroatoms selected from 1, 2, 3, or 4 of N, O, S, and Se" substituted with 1, 2, or 3 R^{m-3};
R^{m} and R^{m-2} are each independently hydroxyl, halogen, amino, or sulfonyl;
each R^{m-3} is independently oxo (=O), hydroxyl, -CO(C₁₋₆ alkyl), or C₁₋₆ alkyl substituted with 1, 2, or 3 hydroxyl groups.

In some embodiments, R₉, R₁₀, R₁₁, R₁₂, and R₁₃ are each independently C₂₋₆ alkenyl, C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₉₋₁, or "8- to 10-membered heteroaryl having 1, 2, 3, or 4 heteroatoms selected from 1, 2, 3, or 4 of N, O, S, and Se" substituted with one or more R₉₋₂;
R₉₋₁ and R₉₋₂ are each independently C₁₋₆ alkyl, C₁₋₆ alkoxy, C₃₋₈ cycloalkyl, halogen, -CN, -C(O)N(C₁₋₆ alkyl)₂, -CH₂-N(C₁₋₆ alkyl)₂, C₁₋₆ alkyl substituted with one or more R₉₋ₐ, or C₁₋₆ alkoxy substituted with one or more R_{9-b};
R₉₋ₐ and R_{9-b} are each independently hydroxyl or halogen;
or, two adjacent groups of the R₉, R₁₀, R₁₁, R₁₂, and R₁₃ can join to form a "4- to 8-membered saturated or unsaturated monocyclic heterocyclic ring having 1, 2, 3, or 4 heteroatoms selected from 1, 2, 3, or 4 of N, O, S, and Se".

In some embodiments, R₁₄₋₁ and R₁₄₋₂ are each independently -CONR^{m}Rⁿ or -CH₂-NR^{m}Rⁿ.

In some embodiments, R₁₅ is C₃₋₈ cycloalkyl or C₁₋₆ alkyl substituted with one or more R₁₅₋₁;
each R₁₅₋₁ is independently halogen or deuterium.

In some embodiments, when R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, or halogen.

In some embodiments, when R₁ₐ and R₅ₐ, R₁ₐ and R_{5b}, R_{1b} and R_{5b}, or R_{1b} and R₅ₐ jointo form ring A, R₂ₐ, R₃ₐ, and R₄ₐ are each independently hydrogen or C₁₋₆ alkyl substituted with one or more halogens.

In some embodiments, when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently C₃₋₈ cycloalkyl, -SF₅, -S-R₂ₐₐ, or -PO(C₁₋₆ alkyl)₂; R₂ₐₐ is C₁₋₆ alkyl or C₁₋₆ alkyl substituted with one or more halogens.

In some embodiments, when R₄ₐ and R₅ₐ, R₄ₐ and R_{5b}, R4b and R_{5b}, or R4b and R₅ₐ join to form ring A, R₁ₐ, R₂ₐ, and R₃ₐ are each independently hydrogen or C₁₋₆ alkyl substituted with one or more halogens.

In some embodiments, R₁₋₁ and R₁₋₂ are each independently oxo (=O), hydroxyl, -CN, C₁₋₆ alkyl, C₁₋₆ alkyl substituted with one or more halogens, or halogen.

In some embodiments, R₁₋₁ and R₁₋₂ are each independently carboxyl, amino, -CONH₂, or -CONR^{x}R^{y}.

In some embodiments, R₆₋₁ and R₆₋₂ are each independently hydrogen, hydroxyl, or C₁₋₆ alkyl substituted with one or more R₁₆₋₁.

In some embodiments, each R₁₆₋₁ is independently hydroxyl or -NR^{m}Rⁿ.

In some embodiments, R^{m} and Rⁿ are each independently C₁₋₆ alkyl.

In some embodiments, R₈ₐ is hydrogen or hydroxyl.

In some embodiments, R_{8b} is hydrogen.

In some embodiments, R₈ is C₁₋₆ alkyl or C₁₋₆ alkyl substituted with one or more R₈₋₁.

In some embodiments, R₈ is C₃₋₈ cycloalkyl.

In some embodiments, each R₈₋₁ is independently deuterium.

In some embodiments, R₉, R₁₀, R₁₁, R₁₂, and R₁₃ are each independently hydrogen or halogen.

In some embodiments, R₉, R₁₀, R₁₁, R₁₂, and R₁₃ are each independently C₁₋₆ alkyl.

In some embodiments, R₁₄ is C₆-C₁₀ aryl substituted with one or more R₁₄₋₁, or "8- to 10-membered heteroaryl having 1, 2, or 3 heteroatoms selected from 1, 2, or 3 of N, O, and S" substituted with one or more R₁₄₋₂. In some embodiments, R₁₄₋₁ and R₁₄₋₂ are each independently C₁₋₆ alkyl or halogen.

In some embodiments, R₁₅ is hydrogen.

In some embodiments, R₁₅ is methyl.

In some embodiments, Y is -C(R₁₆)=.

In some embodiments, R₁₆ is hydrogen or halogen.

In some embodiments, R₁₋₁ and R₁₋₂ are each independently oxo (=O), hydroxyl, C₁₋₆ alkyl, or halogen, preferably halogen.

In some embodiments, R₅ is oxo (=O).

In some embodiments, X is -CHR₆₋₁- or -NR₆₋₂-.

In some embodiments, R₆₋₁ and R₆₋₂ are each independently hydrogen or hydroxyl; preferably, R₆₋₁ is hydroxyl, and R₆₋₂ is hydrogen.

In some embodiments, R₈ is C₁₋₆ alkyl.

In some embodiments,

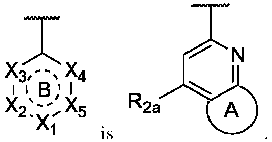

In some embodiments, 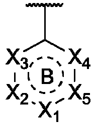 may also be

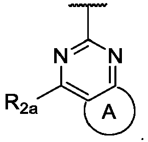

In some embodiments, 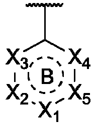 -may also be

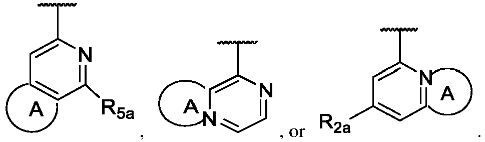

In some embodiments, R₂ₐ is halogen, C₁₋₆ alkyl, or C₁₋₆ alkyl substituted with one or more halogens.

In some embodiments, R₂ₐ is C₁₋₆ alkyl substituted with one or more halogens.

In some embodiments, R₂ₐ is C₃₋₈ cycloalkyl, C₁₋₆ alkoxy substituted with one or more halogens, -SF₅, or -S-R₂ₐₐ.

In some embodiments, ring A is a 5- to 6-membered saturated or unsaturated monocyclic carbon ring, a 5- to 6-membered saturated or unsaturated monocyclic carbon ring substituted with one or more R₁₋₁, a "5-membered saturated or unsaturated monocyclic heterocyclic ring having 1 or 2 heteroatoms selected from 1 or 2 of N, O, and S", or a "5-membered saturated or unsaturated monocyclic heterocyclic ring having 1 or 2 heteroatoms selected from 1 or 2 of N, O, and S" substituted with one or more R₁₋₂.

In some embodiments, ring A is a "6-membered saturated or unsaturated monocyclic heterocyclic ring having 1 or 2 heteroatoms selected from 1 or 2 of N, O, and S".

In some embodiments, wherein the compound of formula I has a structure of formula I-A or formula I-B:

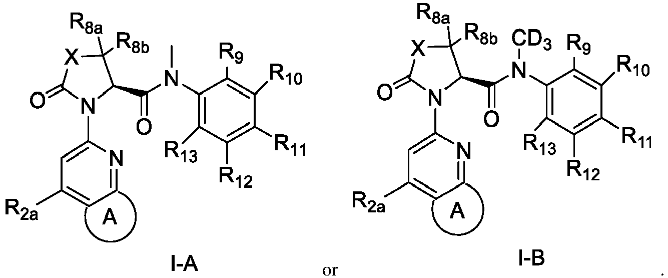

In some embodiments, 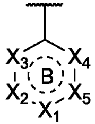 is 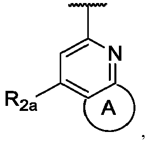 ring A is a 5-membered saturated or unsaturated monocyclic carbon ring, a 5-membered saturated or unsaturated monocyclic carbon ring substituted with one or more halogens, or a "4- to 8-membered saturated or unsaturated monocyclic heterocyclic ring having 1, 2, or 3 heteroatoms selected from 1, 2, or 3 of N, O, and S";
R₂ₐ is C₁₋₆ alkyl substituted with one or more halogens;
R₅ is oxo (=O);
X is -NH- or -CH(OH)-;
R₅ₐ is hydrogen or hydroxyl;
R_{8b} is hydrogen;
R₈ is C₁₋₆ alkyl; and
R₉, R₁₀, R₁₁, R₁₂, and R₁₃ are each independently hydrogen or halogen.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkyl" groups of the "C₁₋₆ alkyl substituted with one or more halogens" and the "C₁₋₆ alkyl" are each independently methyl, ethyl, n-propyl, isopropyl, n-butyl, isobutyl, sec-butyl, or tert-butyl, preferably methyl.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkoxy" groups are each independently methoxy, ethoxy, n-propoxy, isopropoxy, n-butoxy, isobutoxy, sec-butoxy, or tert-butoxy.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "halogen" groups of the "C₁₋₆ alkyl substituted with one or more halogens" and the "halogen" are each independently fluorine, chlorine, bromine, or iodine, preferably fluorine.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkyl" groups of the "C₁₋₆ alkyl substituted with one or more halogens" and the "C₁₋₆ alkyl" are each independently methyl, ethyl, n-propyl, isopropyl, n-butyl, isobutyl, sec-butyl, or tert-butyl, preferably methyl.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkoxy" groups are each independently methoxy, ethoxy, n-propoxy, isopropoxy, n-butoxy, isobutoxy, sec-butoxy, or tert-butoxy.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "halogen" groups of the "C₁₋₆ alkyl substituted with one or more halogens" and the "halogen" are each independently fluorine, chlorine, bromine, or iodine, preferably fluorine.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "C₁₋₆ alkyl" groups of the "C₁₋₆ alkyl substituted with one or more halogens" and the "C₁₋₆ alkyl" are each independently methyl, ethyl, n-propyl, isopropyl, n-butyl, isobutyl, sec-butyl, or tert-butyl, preferably methyl.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "C₁₋₆ alkoxy" groups are each independently methoxy, ethoxy, n-propoxy, isopropoxy, n-butoxy, isobutoxy, sec-butoxy, or tert-butoxy.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "halogen" groups of the "C₁₋₆ alkyl substituted with one or more halogens" and the "halogen" are each independently fluorine, chlorine, bromine, or iodine, preferably fluorine or chlorine.

In some embodiments, in R^{x}, R^{y}, R₁₋₁, R₁₋₂, R₆₋₁, R₆₋₂, R^{m}, Rⁿ, R^{z}, R₈, R₉, R₁₀, R₁₁, R₁₂, R₁₃, R₁₄₋₁, R₁₄₋₂, R₁₅, and R₁₆, the "C₁₋₆ alkyl" groups of the "C₁₋₆ alkyl", "C₁₋₆ alkyl substituted with 1, 2, or 3 R^{X1}", "-C(O)C₁₋₆ alkyl", "C₁₋₆ alkyl substituted with one or more halogens", "C₁₋₆ alkyl substituted with one or more R₁₆₋₁", "C₁₋₆ alkyl substituted with 1, 2, or 3 hydroxyl groups", and "C₁₋₆ alkyl substituted with one or more R₈₋₁" are each independently methyl, ethyl, n-propyl, isopropyl, n-butyl, isobutyl, sec-butyl, or tert-butyl, preferably methyl.

In some embodiments, in R^{X1}, R₁₋₁, R₁₋₂, R₆₋₁, R₆₋₂, R₁₆₋₁, R₈₋₁, R₉, R₁₀, R₁₁, R₁₂, R₁₃, R₁₄₋₁, R₁₄₋₂, R₁₅, and R₁₆, the "halogen" groups of the "halogen" and the "C₁₋₆ alkyl substituted with one or more halogens" are each independently fluorine, chlorine, bromine, or iodine, preferably fluorine.

In some embodiments, the C₁₋₆ alkoxy groups in R₁₋₁, R₁₋₂, R₆₋₁, R₆₋₂, R^{z}, R₉, R₁₀, R₁₁, R₁₂, R₁₃, R₁₄₋₁, R₁₄₋₂, and R₁₆ are each independently methoxy, ethoxy, n-propoxy, isopropoxy, n-butoxy, isobutoxy, sec-butoxy, or tert-butoxy.

In some embodiments, in R₁₄, the C₆-C₁₀ aryl groups of the "C₆-C₁₀ aryl substituted with one or more R₁₄₋₁" and the "C₆-C₁₀ aryl" are each independently phenyl.

In some embodiments, in ring A, the "3- to 8-membered saturated or unsaturated monocyclic, spirocyclic, or fused cyclic carbon ring" groups of the "3- to 8-membered saturated or unsaturated monocyclic, spirocyclic, or fused cyclic carbon ring" and the "3- to 8-membered saturated or unsaturated monocyclic, spirocyclic, or fused cyclic carbon ring substituted with one or more R₁₋₁" are each independently a 5- to 6-membered saturated or unsaturated monocyclic ring, such as 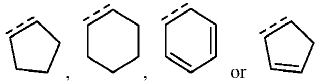 ( 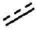 represents a bond to ring B).

In some embodiments, in ring A, the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" groups of the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" and the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se substituted with one or more R₁₋₂" are each independently a "4- to 8-membered saturated or unsaturated monocyclic, spirocyclic, or fused cyclic heterocyclic ring having 1, 2, or 3 heteroatoms selected from 1, 2, or 3 of N, O, and S", preferably a 5-membered unsaturated monocyclic ring having 1 or 2 heteroatoms selected from 1, 2, or 3 of N, O, and S, such as 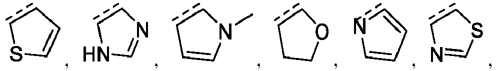 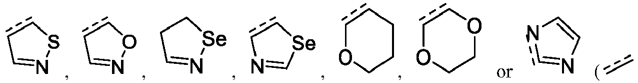 represents a bond to ring B).

In some embodiments, in R₁₋₁, R₁₋₂, R₈, R₉, R₁₀, R₁₁, R₁₂, R₁₃, R₁₄₋₁, and R₁₄₋₂, the C₃₋₈ cycloalkyl groups are each independently cyclopropyl, cyclobutyl, cyclopentyl, or cyclohexyl.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the C₃₋₈ cycloalkyl groups are each independently cyclopropyl, cyclobutyl, cyclopentyl, or cyclohexyl.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the C₃₋₈ cycloalkyl groups are each independently cyclopropyl, cyclobutyl, cyclopentyl, or cyclohexyl.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the C₃₋₈ cycloalkyl groups are each independently cyclopropyl, cyclobutyl, cyclopentyl, or cyclohexyl.

In some embodiments, in R₁₄, the C₆-C₁₀ aryl groups of the "C₆-C₁₀ aryl" and the "C₆-C₁₀ aryl substituted with one or more R₁₄₋₁" are each independently phenyl.

In some embodiments, in R₁₄, the "8- to 10-membered heteroaryl having 1, 2, 3, or 4 heteroatoms selected from 1, 2, 3, or 4 of N, O, S, and Se" groups of the "8- to 10-membered heteroaryl having 1, 2, 3, or 4 heteroatoms selected from 1, 2, 3, or 4 of N, O, S, and Se" and the "8- to 10-membered heteroaryl having 1, 2, 3, or 4 heteroatoms selected from 1, 2, 3, or 4 of N, O, S, and Se substituted with one or more R₁₄₋₂" are each independently an "8- to 10-membered heteroaryl having 1, 2, or 3 heteroatoms selected from 1, 2, or 3 of N, O, and S", preferably a heteroaryl group having 2 heteroatoms N formed by the fusion of a 5-membered heteroaromatic ring and a 5- to 6-membered heteroaromatic ring.

In some embodiments, in R₅, R₂ₐₐ, R^{x}, R^{y}, R₁₋₁, R₁₋₂, R₁₆₋ₐ, R_{16-b}, R₆₋₁, R₆₋₂, R₉₋₁, R^{m}, Rⁿ, R^{m-3}, R₉₋₂, R^{X4}, and R₁₅, the "C₁₋₆ alkyl" groups of the "C₁₋₆ alkyl", the "C₁₋₆ alkyl substituted with one or more halogens", the "-C(O)C₁₋₆ alkyl substituted with 1, 2, or 3 R^{X3}", the "C₁₋₆ alkyl substituted with 1, 2, or 3 hydroxyl groups", the "C₁₋₆ alkyl substituted with one or more R₉₋ₐ", the "-C(O)C₁₋₆ alkyl", the "-C(O)OC₁₋₆ alkyl", the "-C(O)N(C₁₋₆ alkyl)₂", the "-CH₂-N(C₁₋₆ alkyl)₂", the "-SO₂-C₁₋₆ alkyl", the "-P(O)(C₁₋₆ alkyl)₂", the "-NH(C₁₋₆ alkyl)", the "-N(C₁₋₆ alkyl)₂", the "-S(C₁₋₆ alkyl)", the "C₁₋₆ alkyl substituted with 1, 2, or 3 R^{m-1}", the "-CO(C₁₋₆ alkyl) substituted with 1, 2, or 3 R^{m-2}", and "C₁₋₆ alkyl substituted with one or more R₁₅₋₁" are each independently methyl, ethyl, n-propyl, isopropyl, n-butyl, isobutyl, sec-butyl, or tert-butyl, preferably methyl.

In some embodiments, in R₅, R₉₋₁, R₉₋₂, R₂ₐ, R₃ₐ, R₄ₐ, R₂ₐₐ, R₉₋ₐ, R_{9-b}, R^{X2}, R^{X3}, R^{X5}, R₁₋₁, R^{m-1}, R^{m-2}, R₁₋₂, and R₁₅₋₁, the "halogen" groups of the "halogen", the "C₁₋₆ alkyl substituted with one or more halogens", and the "C₁₋₆ alkoxy substituted with one or more halogens" are each independently fluorine, chlorine, bromine, or iodine, preferably fluorine or chlorine.

In some embodiments, in R₆₋₁, R₆₋₂, R₉₋₁, R₉₋₂, R^{m}, Rⁿ, R^{x}, R^{y}, and R₁₅, the "C₃₋₈ cycloalkyl" groups of the "C₃₋₈ cycloalkyl", the "-C(O)C₃₋₈ cycloalkyl", the "C₃₋₈ cycloalkyl substituted with 1, 2, or 3 R^{X2}", and the "-C(O)C₃₋₈ cycloalkyl substituted with 1, 2, or 3 R^{X5}" are each independently cyclopropyl, cyclobutyl, cyclopentyl, or cyclohexyl.

In some embodiments, in R₆₋₁ and R₆₋₂, the "oxa-C₃₋₈ cycloalkyl" groups are oxiranyl, oxetanyl, oxolanyl, or oxacyclohexyl.

In some embodiments, in R₁₆₋₁, R₉, R₁₀, R₁₁, R₁₂, and R₁₃, the "C₆-C₁₀ aryl" groups of the "C₆-C₁₀ aryl", the "C₆-C₁₀ aryl substituted with one or more R₁₆₋ₐ", and the "C₆-C₁₀ aryl substituted with one or more R₉₋₁" are phenyl.

In some embodiments, in R^{x}, R^{y}, R₁₆₋₁, R^{m}, Rⁿ, the "4- to 8-membered heterocycloalkyl having 1, 2, 3, or 4 heteroatoms selected from 1, 2, 3, or 4 of N, O, S, and Se" groups of the "4- to 8-membered heterocycloalkyl having 1, 2, 3, or 4 heteroatoms selected from 1, 2, 3, or 4 of N, O, S, and Se", the "4- to 8-membered heterocycloalkyl having 1, 2, 3, or 4 heteroatoms selected from 1, 2, 3, or 4 of N, O, S, and Se substituted with 1, 2, or 3 R^{X4}", the "4- to 8-membered heterocycloalkyl having 1, 2, 3, or 4 heteroatoms selected from 1, 2, 3, or 4 of N, O, S, and Se substituted with one or more R_{16-b}", and the "4- to 8-membered heterocycloalkyl having 1, 2, 3, or 4 heteroatoms selected from 1, 2, 3, or 4 of N, O, S, and Se substituted with 1, 2 or 3 R^{m-3}" are each independently "4- to 8-membered heterocycloalkyl having 1, 2, or 3 heteroatoms selected from 1, 2, or 3 of N, O, and S", preferably 5-membered heterocycloalkyl having 1 or 2 heteroatoms selected from N or S.

In some embodiments, in R₉, R₁₀, R₁₁, R₁₂, R₁₃, R₁₋₁, and R₁₋₂, the "C₂₋₆ alkenyl" groups are ethenyl or propenyl.

In some embodiments, in R₁₋₁ and R₁₋₂, the "C₂₋₆ alkynyl" groups are ethynyl or propynyl.

In some embodiments, in R₁₋₁, R₁₋₂, R₉₋₁, R₉₋₂, R₂ₐ, R₃ₐ, and R₄ₐ, the "C₁₋₆ alkoxy" groups of the "C₁₋₆ alkoxy", the "C₁₋₆ alkoxy substituted with one or more halogens", and the "C₁₋₆ alkoxy substituted with one or more R_{9-b}" are each independently methoxy, ethoxy, n-propoxy, isopropoxy, n-butoxy, isobutoxy, sec-butoxy, or tert-butoxy.

In some embodiments, in R₉, R₁₀, R₁₁, R₁₂, R₁₃, and R₁₄, the "8- to 10-membered heteroaryl having 1, 2, 3, or 4 heteroatoms selected from 1, 2, 3, or 4 of N, O, S, and Se" groups of the "8- to 10-membered heteroaryl having 1, 2, 3, or 4 heteroatoms selected from 1, 2, 3, or 4 of N, O, S, and Se", the "8- to 10-membered heteroaryl having 1, 2, 3, or 4 heteroatoms selected from 1, 2, 3, or 4 of N, O, S, and Se substituted with one or more R₉₋₂", and the "8- to 10-membered heteroaryl having 1, 2, 3, or 4 heteroatoms selected from 1, 2, 3, or 4 of N, O, S, and S substituted with one or more R₁₄₋₂" are each independently "8- to 10-membered heteroaryl having 1, 2, or 3 heteroatoms selected from 1, 2, or 3 of N, O, and S", preferably a heteroaryl group having 2 heteroatoms N formed by the fusion of a 5-membered heteroaromatic ring and a 5- to 6-membered heteroaromatic ring.

In some embodiments, when R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se", the heterocyclic ring is a 4- to 8-membered saturated monocyclic heterocyclic ring containing one heteroatom N.

In some embodiments, when two adjacent groups of R₉, R₁₀, R₁₁, R₁₂, and R₁₃ join to form a "4- to 8-membered saturated or unsaturated monocyclic heterocyclic ring having 1, 2, 3, or 4 heteroatoms selected from 1, 2, 3, or 4 of N, O, S, and Se", the heterocyclic ring is a "4- to 8-membered saturated or unsaturated monocyclic, spirocyclic, or fused cyclic heterocyclic ring having 1, 2, or 3 heteroatoms selected from 1, 2, or 3 of N, O, and S".

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkyl" groups of the "C₁₋₆ alkyl substituted with one or more R^{X1}" are each independently methyl, ethyl, n-propyl, isopropyl, n-butyl, isobutyl, sec-butyl, or tert-butyl.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkoxy" groups of the "C₁₋₆ alkoxy substituted with one or more R^{X1}" are each independently methoxy, ethoxy, n-propoxy, isopropoxy, n-butoxy, isobutoxy, sec-butoxy, or tert-butoxy.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkyl" groups of the "C₁₋₆ alkyl substituted with one or more R^{X1}" and the "-PO(C₁₋₆ alkyl)₂" are each independently methyl, ethyl, n-propyl, isopropyl, n-butyl, isobutyl, sec-butyl, or tert-butyl.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkoxy" groups of the "C₁₋₆ alkoxy substituted with one or more R^{X1}" are each independently methoxy, ethoxy, n-propoxy, isopropoxy, n-butoxy, isobutoxy, sec-butoxy, or tert-butoxy.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "C₁₋₆ alkyl" groups of the "C₁₋₆ alkyl substituted with one or more R^{X1}" are each independently methyl, ethyl, n-propyl, isopropyl, n-butyl, isobutyl, sec-butyl, or tert-butyl.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "C₁₋₆ alkoxy" groups of the "C₁₋₆ alkoxy substituted with one or more R^{X1}" are each independently methoxy, ethoxy, n-propoxy, isopropoxy, n-butoxy, isobutoxy, sec-butoxy, or tert-butoxy.

In some embodiments, in R₂ₐₐ, R₆₋₁, R₆₋₂, R^{m}, Rⁿ, R^{m-3}, R₆₋₁₋₁, and R₆₋₁₋₂, the "C₁₋₆ alkyl" groups of the "C₁₋₆ alkyl substituted with one or more R^{X1}", the "C₁₋₆ alkyl substituted with one or more R₁₆₋₁", the "-SO(=NH)(C₁₋₆ alkyl)", the "C₁₋₆ alkyl substituted with one or more halogens", the "C₁₆ alkyl", the "C₁₆ alkyl substituted with 1, 2, or 3 hydroxyl groups", the "-COC₁₋₆ alkyl", and the "-COOC₁₋₆ alkyl" are each independently methyl, ethyl, n-propyl, iso-propyl, n-butyl, iso-butyl, sec-butyl, or tert-butyl.

In some embodiments, in R^{X4}, R₁₆₋₁, R^{m-3}, R^{z}, R₁₅, R₆₋₁, R₆₋₂, R₉, R₁₀, R₁₁, R₁₂, R₁₃, R₁₄₋₁, and R₁₄₋₂, the C₁₋₆ alkoxy groups of the "C₁₋₆ alkoxy substituted with 1, 2, or 3 hydroxyl groups", the "C₁₋₆ alkoxy substituted with one or more R₁₆₋₁", the "C₁₋₆ alkoxy", the "C₁₋₆ alkoxy substituted with one or more halogens", and the "C₁₋₆ alkoxy substituted with one or more R₁₅₋₁" are each independently methoxy, ethoxy, n-propoxy, isopropoxy, n-butoxy, isobutoxy, sec-butoxy, or tert-butoxy.

In some embodiments, in R₆₋₁, the C₆-C₁₀ aryl groups of the "-L-C₆-C₁₀ aryl" and the "-L-(C₆-C₁₀ aryl substituted with one or more R₆₋₁₋₁)" are phenyl.

In some embodiments, in R₂ₐₐ, R₅, R₁₆₋₁, R^{z}, R₁₆, R₁₋₁, R₁₋₂, R₆₋₁, R₆₋₂, R^{m}, Rⁿ, R₈, R₉, R₁₀, R₁₁, R₁₂, R₁₃, R₁₅, R₁₄₋₁, and R₁₄₋₂, the C₃₋₈ cycloalkyl groups of the "C₃₋₈ cycloalkyl", the "C₃₋₈ cycloalkyl substituted with one or more R^{X1}", the "C₃₋₈ cycloalkyl substituted with one or more halogens", the "-SO₂(C₃₋₈ cycloalkyl)", the "-SO(=NH)(Cas cycloalkyl)", the "-S(C₃₋₈ cycloalkyl)", the "-SO(C₃₋₈ cycloalkyl)", the "-P(O)(C₃₋₈ cycloalkyl)₂", the "-CO(C₃₋₈ cycloalkyl substituted with one or more halogens)", the "C₃₋₈ cycloalkyl substituted with one or more R₈₋₁", the "C₃₋₈ cycloalkyl substituted with one or more R₁₅₋₁", and the "-C(O)C₃₋₈ cycloalkyl" are each independently cyclopropyl, cyclobutyl, cyclopentyl, or cyclohexyl.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the C₃₋₈ cycloalkyl groups of the "C₃₋₈ cycloalkyl substituted with one or more R^{X1}" are each independently cyclopropyl, cyclobutyl, cyclopentyl, or cyclohexyl.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the C₃₋₈ cycloalkyl groups of the "C₃₋₈ cycloalkyl substituted with one or more R^{X1}" are each independently cyclopropyl, cyclobutyl, cyclopentyl, or cyclohexyl.

In some embodiments, when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the C₃₋₈ cycloalkyl groups of the "C₃₋₈ cycloalkyl substituted with one or more R^{X1}" are each independently cyclopropyl, cyclobutyl, cyclopentyl, or cyclohexyl.

In some embodiments, in R₆₋₁, the "4- to 8-membered heterocycloalkyl having 1, 2, 3, or 4 heteroatoms selected from 1, 2, 3, or 4 of N, O, S, and Se" groups of the "-L-(4- to 8-membered heterocycloalkyl having 1, 2, 3, or 4 heteroatoms selected from 1, 2, 3, or 4 of N, O, S, and Se)" and the "-L-(4- to 8-membered heterocycloalkyl having 1, 2, 3, or 4 heteroatoms selected from 1, 2, 3, or 4 of N, O, S, and Se substituted with one or more R₆₋₁₋₂) are each independently "4- to 8-membered heterocycloalkyl having 1, 2, or 3 heteroatoms selected from 1, 2, or 3 of N, O, and S", preferably 5-membered heterocycloalkyl having 1 or 2 heteroatoms selected from N or S.

In some embodiments, when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₂₋₆ alkenyl" groups are ethenyl or propenyl.

In some embodiments, in L, the "C₁₋₆ alkylene" group of the "C₁₋₆ alkylene optionally substituted with an oxygen atom" is methylene or ethylene.

In some embodiments, when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the C₃₋₈ cycloalkyl groups of the "Cs s cycloalkyl" and the "Cs s cycloalkyl substituted with one or more R^{X1}" are each independently cyclopropyl.

In some embodiments, in R₂ₐₐ, R₅, R₁₆₋₁, R^{z}, R₁₆, R₁₋₁, R₁₋₂, R₈, R₉, R₁₀, R₁₁, R₁₂, R₁₃, R₁₄₋₁, R₆₋₁, R₆₋₂, R₉₋₁, R₉₋₂, R^{m}, Rⁿ, R^{x}, R^{y}, R₁₅, and R₁₄₋₂, the C₃₋₈ cycloalkyl groups of the "C₃₋₈ cycloalkyl", the "C₃₋₈ cycloalkyl substituted with one or more R^{X1}", the "C₃₋₈ cycloalkyl substituted with one or more halogens", the "-SO₂(C₃₋₈ cycloalkyl)", the "-SO(=NH)(C₃₋₈ cycloalkyl)", the "-S(C₃₋₈ cycloalkyl)", the "-SO(C₃₋₈ cycloalkyl)", the "-P(O)(C₃₋₈ cycloalkyl)₂", the "-CO(C₃₋₈ cycloalkyl substituted with one or more halogens)", the "C₃₋₈ cycloalkyl substituted with one or more R₈₋₁", the "C₃₋₈ cycloalkyl substituted with one or more R₁₅₋₁", the "-C(O)C₃₋₈ cycloalkyl", the "C₃₋₈ cycloalkyl substituted with 1, 2, or 3 R^{X2}", and the "-C(O)C₃₋₈ cycloalkyl substituted with 1, 2, or 3 R^{X5}" are each independently cyclopropyl.

In some embodiments, the heteroatoms are N, O, or S.

In some embodiments, when R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, methyl, or chlorine.

In some embodiments, when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen or trifluoromethyl.

In some embodiments, when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently cyclopropyl, -SMe, -SF5, or -P(O)(CH₃)₂.

In some embodiments, when R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen or trifluoromethyl.

In some embodiments, R₁₋₁ and R₁₋₂ are each independently oxo (=O), hydroxyl, -CN, methyl, fluorine, or trifluoromethyl, preferably fluorine.

In some embodiments, R₁₋₁ and R₁₋₂ are each independently amino, -CONH₂, or -CONHMe.

In some embodiments, 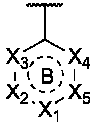 is any one of the following structural fragments:

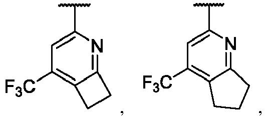

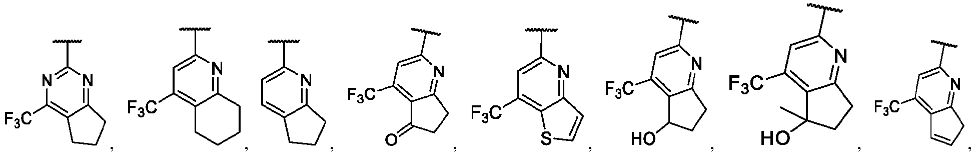

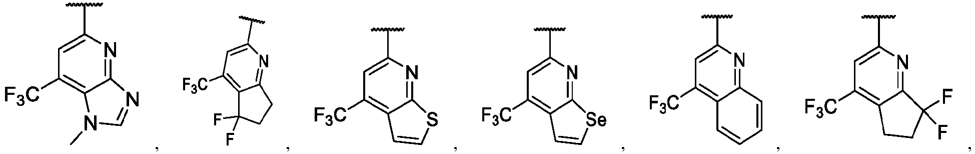

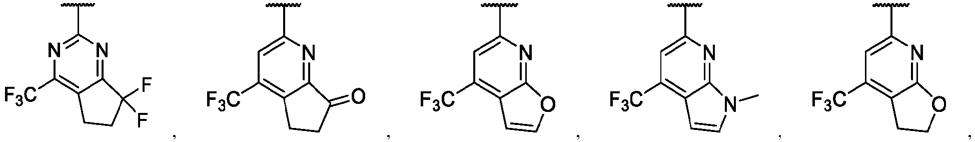

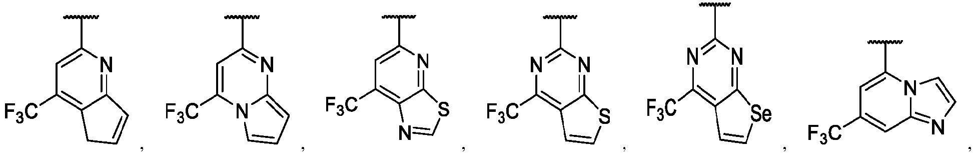

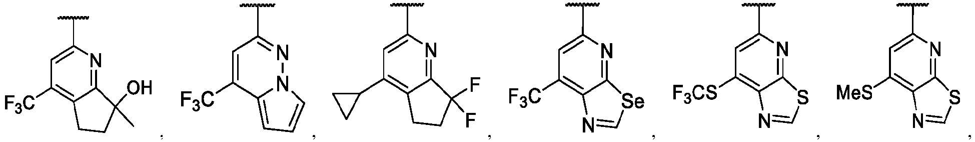

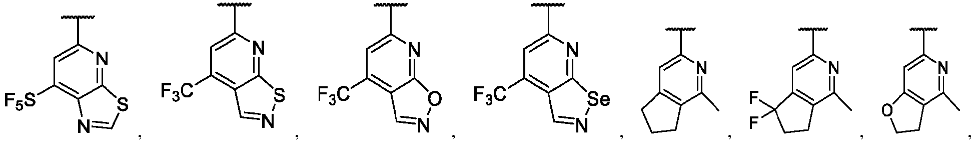

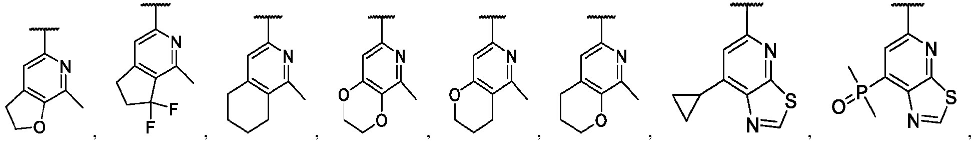

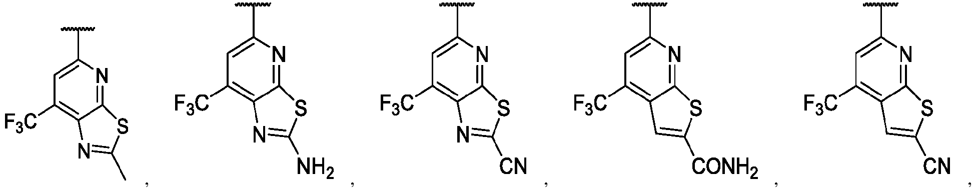

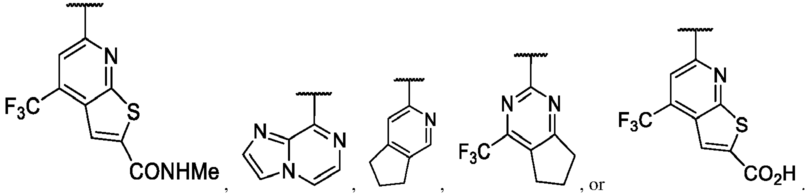

In some embodiments, R^{m} and Rⁿ are methyl.

In some embodiments, each R₁₆₋₁ is independently hydroxyl or

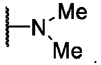

In some embodiments, X is -NH-, -CH(OH)-, -O-, or 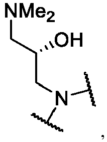 preferably O, -NH-, or -CH(OH)-.

In some embodiments, R₈ is methyl or -CD₃.

In some embodiments, R₈ is cyclopropyl.

In some embodiments, R₉, R₁₀, R₁₁, R₁₂, and R₁₃ are each independently hydrogen, fluorine, or chlorine.

In some embodiments, R₉, R₁₀, R₁₁, R₁₂, and R₁₃ are each independently methyl.

In some embodiments, R₁₄₋₁ and R₁₄₋₂ are each independently methyl, fluorine, or chlorine.

In some embodiments, R₁₄ is

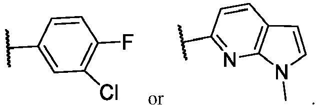

In some embodiments, R₁₅ is hydrogen.

In some embodiments, R₁₅ is methyl.

In some embodiments, Y is -C(F)=, -C(Cl)=, or -C(H)=.

In some embodiments, the compound of formula II or formula III is any one of the following:

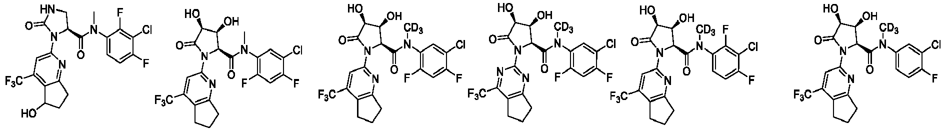

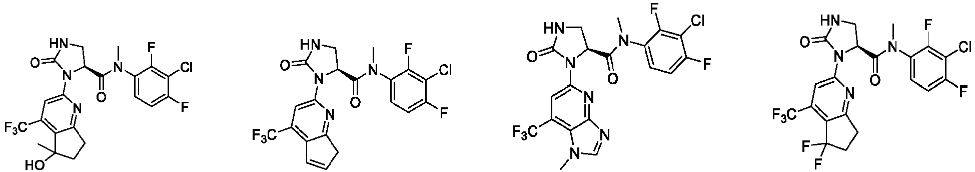

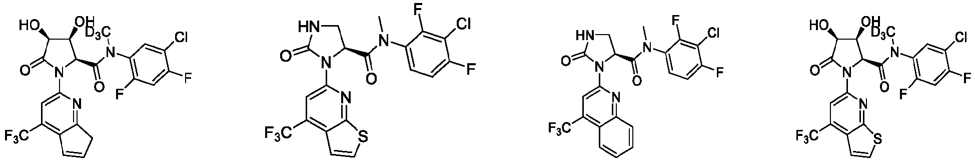

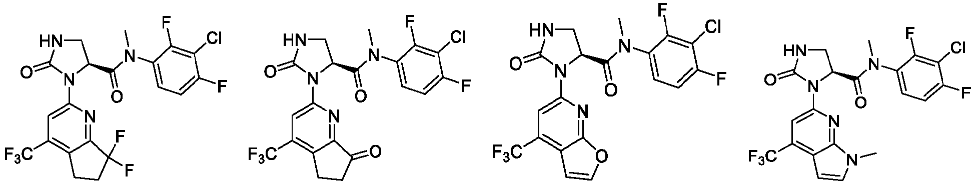

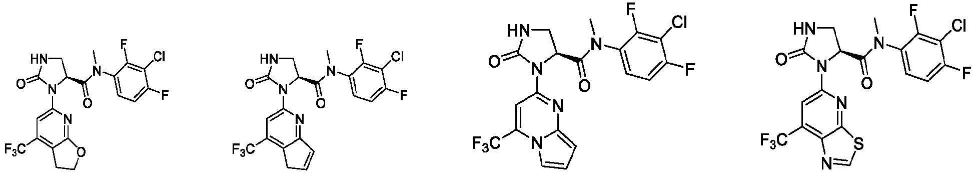

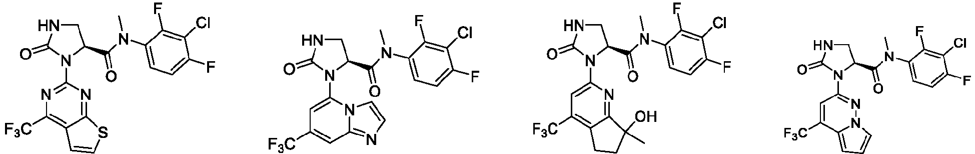

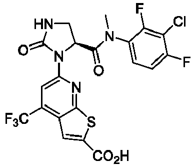

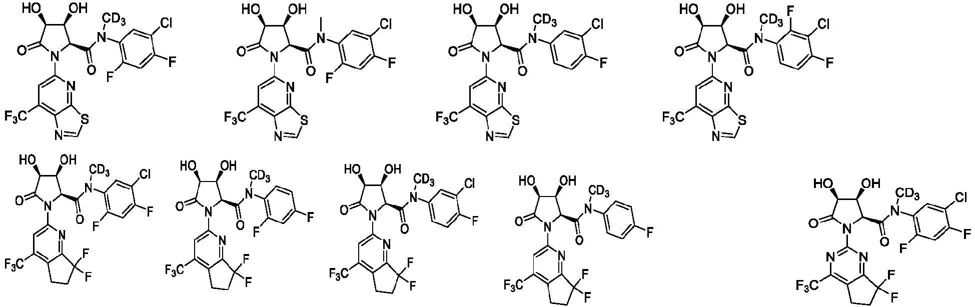

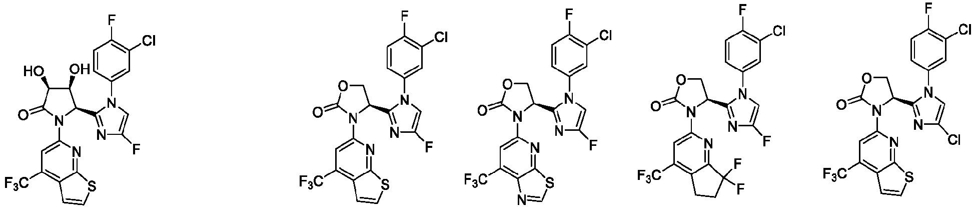

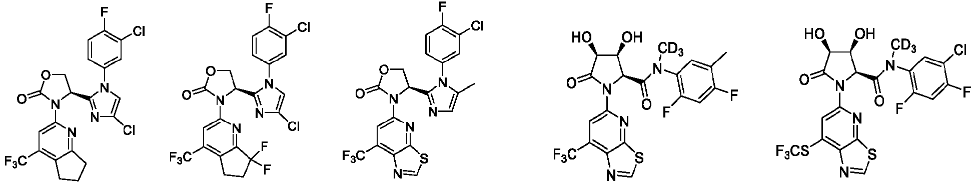

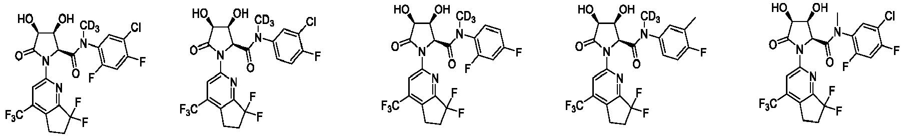

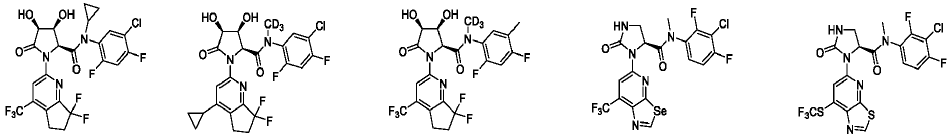

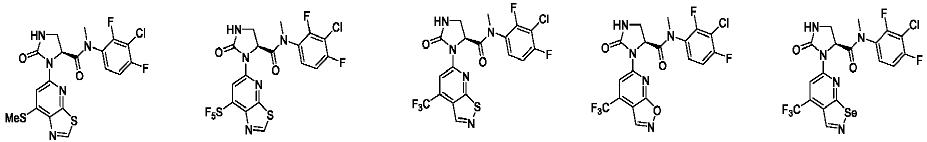

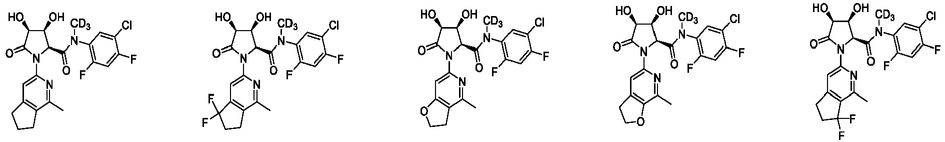

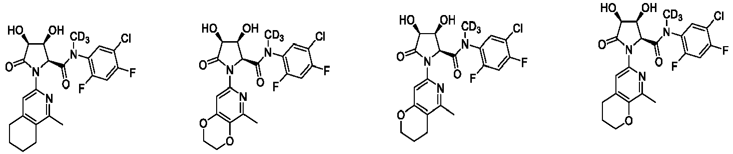

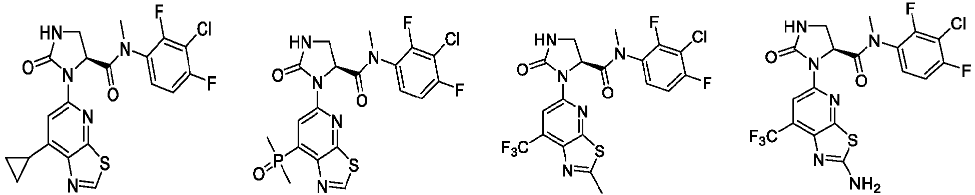

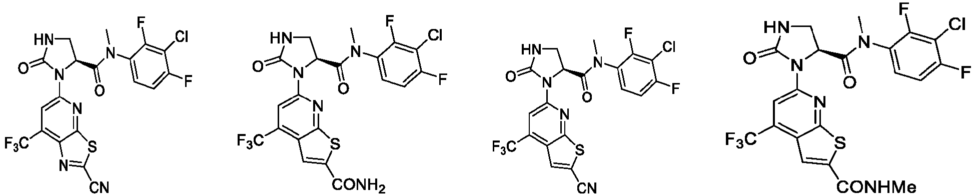

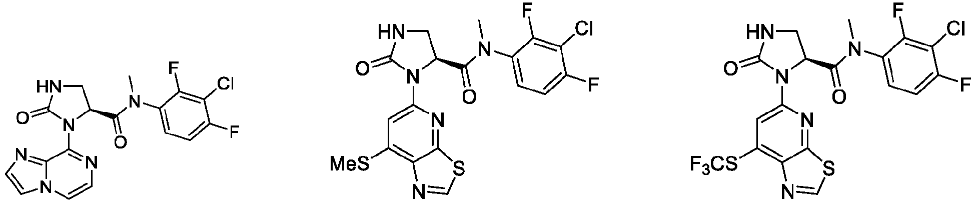

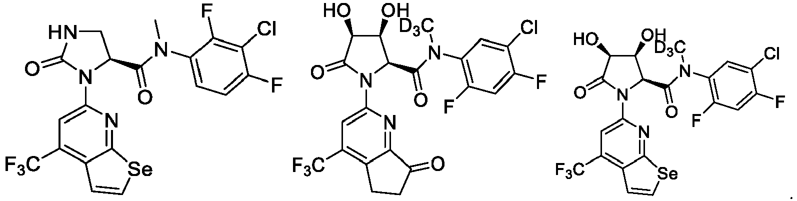

In some embodiments, the compound of formula I is as follows:
a compound with a retention time of 1.57 min under the following conditions, which is one of the stereoisomers of 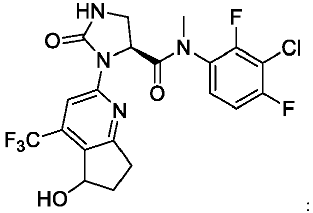 chromatographic column CHIRALPAK ID-3 4.6*50 mm, 3 um; mobile phase A: (0.1% diethylamine) n-hexane, mobile phase B: ethanol, mobile phase A:mobile phase B = 70:30; flow rate: 1.0 mL/min; temperature: 25°C;
or a compound with a retention time of 1.92 min under the following conditions, which is one of the stereoisomers of 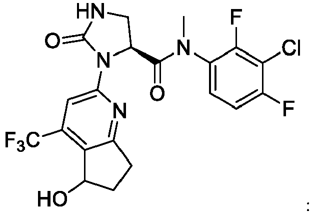 chromatographic column CHIRALPAK ID-3 4.6*50 mm, 3 um; mobile phase A: (0.1% diethylamine) n-hexane, mobile phase B: ethanol, mobile phase A:mobile phase B = 70:30; flow rate: 1.0 mL/min; temperature: 25°C;
or a compound with a retention time of 1.64 min under the following conditions, which is one of the stereoisomers of 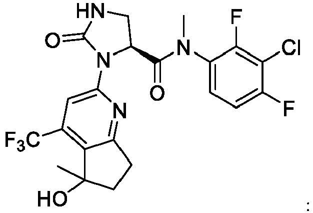 chromatographic column: CHIRALPAK IA-3 Column 4.6*50 mm, 3 um; mobile phase A: (0.1% diethylamine) n-hexane, mobile phase B: ethanol; mobile phase A:mobile phase B = 80:20; flow rate: 1 mL/min; temperature: 25°C;
or a compound with a retention time of 2.67 min under the following conditions, which is one of the stereoisomers of 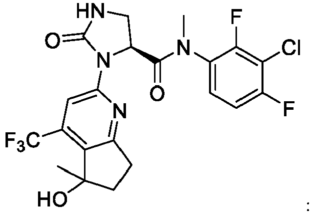 chromatographic column: CHIRALPAK IA-3 Column 4.6*50 mm, 3 um; mobile phase A: (0.1% diethylamine) n-hexane, mobile phase B: ethanol; mobile phase A:mobile phase B = 80:20; flow rate: 1 mL/min; temperature: 25°C;
or a compound having a retention time of 3.52 min under the following conditions, which is one of the rotamers of 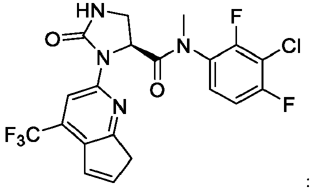 chromatographic column CHIRAL Cellulose-SB 4.6*100 mm, 3 um; mobile phase A: (0.1% formic acid) n-hexane, mobile phase B: ethanol; mobile phase A:mobile phase B = 80:20; flow rate: 1 mL/min; temperature: 25°C;
or a compound with a retention time of 3.94 min under the following conditions, which is one of the rotamers of 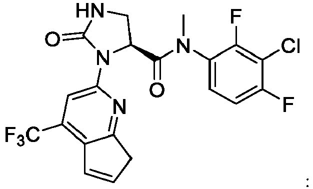 chromatographic column CHIRAL Cellulose-SB 4.6*100 mm, 3 um; mobile phase A: (0.1% formic acid) n-hexane, mobile phase B: ethanol; mobile phase A:mobile phase B = 80:20; flow rate: 1 mL/min; temperature: 25°C;
or a compound with a retention time of 6.30 min under the following conditions, which is one of the stereoisomers of 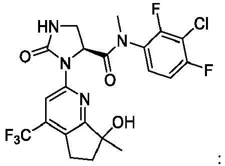 chromatographic column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 32% B to 63% B within 8 min; wavelength: 220 nm;
or a compound with a retention time of 7.58 min under the following conditions, which is one of the stereoisomers of 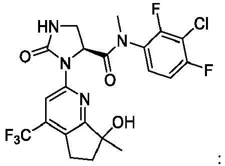 chromatographic column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 32% B to 63% B within 8 min; wavelength: 220 nm.

The test conditions for the above retention time are not limited to the compound. As long as the retention time determined by using the above test conditions is the same as recorded above or within the error range, and the compound is a stereoisomer of the above compound defined by the retention time, it falls within the scope of the present disclosure.

The present disclosure provides a pharmaceutical composition comprising:
(1) the compound of formula II or formula III, or a tautomer thereof, a stereoisomer thereof, a pharmaceutically acceptable salt of any of the above (referring to the compound of formula II or formula III , a tautomer thereof, or a stereoisomer thereof as described above), or a solvate of any of the above (referring to the compound of formula II or formula III, a tautomer thereof, a stereoisomer thereof, or a pharmaceutically acceptable salt of any of the above as described above) , and
(2) a pharmaceutically acceptable excipient.

The present disclosure further provides the use of the compound of formula II or formula III, or a tautomer thereof, a stereoisomer thereof, a pharmaceutically acceptable salt of any of the above (referring to the compound of formula II or formula III, a tautomer thereof, or a stereoisomer thereof as described above), or a solvate of any of the above (referring to the compound of formula II or formula III, a tautomer thereof, a stereoisomer thereof, or a pharmaceutically acceptable salt of any of the above as described above), in the preparation of a Polθ inhibitor.

In use, the Polθ inhibitor may be used in a mammalian organism; it may also be used in vitro, mainly for experimental purposes, such as providing a comparison as a standard or control sample or preparing kits according to conventional methods in the art to provide rapid detection for Polθ inhibition.

The present disclosure further provides the use of the compound of formula II or formula III, or a tautomer thereof, a stereoisomer thereof, a pharmaceutically acceptable salt of any of the above (referring to the compound of formula II or formula III, a tautomer thereof, or a stereoisomer thereof as described above), or a solvate of any of the above (referring to the compound of formula II or formula III, a tautomer thereof, a stereoisomer thereof, or a pharmaceutically acceptable salt of any of the above as described above), in the preparation of a medicine for the treatment and/or prevention of cancer, such as breast cancer, ovarian cancer, prostatic cancer, or pancreatic cancer.

### Definition and Description

Unless otherwise indicated, the following terms and phrases used herein are intended to have the following meanings. Unless specifically defined, a particular term or phrase should not be construed as indefinite or unclear but rather construed in a generic sense. When trade names appear herein, they are intended to refer to their corresponding goods or their active ingredients.

It will be understood by those skilled in the art that, in accordance with the conventions used in the art, 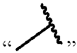 used in the formulas describing a group in the present disclosure means that the corresponding group is attached to another moiety or group in the compound via this site.

As used herein, substituents may be preceded by a single dash "-" indicating that the named substituent is attached to the parent moiety via a single bond.

In the present disclosure, the term "pharmaceutically acceptable salt" refers to a salt prepared by a compound of the present disclosure and a relatively nontoxic, pharmaceutically acceptable acid or base. When the compound of the present disclosure contains a relatively acidic functional group, a base addition salt may be obtained by contacting the neutral form of such a compound with a sufficient amount of pharmaceutically acceptable base in a pure solution or a suitable inert solvent. Pharmaceutically acceptable base addition salts include, but are not limited to, lithium salts, sodium salts, potassium salts, calcium salts, aluminum salts, magnesium salts, zinc salts, bismuth salts, ammonium salts, and diethanolamine salts. When the compound of the present disclosure contains a relatively basic functional group, an acid addition salt may be obtained by contacting the neutral form of such a compound with a sufficient amount of pharmaceutically acceptable acid in a pure solution or a suitable inert solvent. Pharmaceutically acceptable acids include inorganic acids including, but not limited to, hydrochloric acid, hydrobromic acid, hydroiodic acid, nitric acid, carbonic acid, phosphoric acid, phosphorous acid, sulfuric acid, and the like. Such pharmaceutically acceptable acids include organic acids including, but not limited to, acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, bitartaric acid, ascorbic acid, gentisic acid, fumaric acid, gluconic acid, sugar acids, formic acid, ethanesulfonic acid, pamoic acid (i.e., 4,4'-methylene-bis(3-hydroxy-2-naphthoic acid)), amino acids (e.g., glutamic acid, arginine), and the like. When the compound of the present disclosure contains relatively acidic and relatively basic functional groups, it may be converted into either a base or acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

In the present disclosure, the term "pharmaceutically acceptable excipient" refers to excipients and additives used in the production and formulation of pharmaceuticals, including all substances in a pharmaceutical formulation other than the active ingredient. See Chinese Pharmacopoeia (2015 Edition) Volume IV, or Handbook of Pharmaceutical Excipients (Raymond C Rowe, 2009 Sixth Edition).

In the present disclosure, the term "substituted" or "substituent" means that a hydrogen atom in a group is replaced by a specified group. When the substitution position is not specified, the substitution may be at any position but is only permitted when a stable or chemically feasible chemical is formed. As an example, the 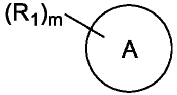 structure indicates that a hydrogen atom on ring A is substituted with m R₁ groups.

When any variable (e.g., R) occurs more than once in the composition or structure of a compound, its definition in each case is independent. Thus, for example, if a group is substituted with one or more R, the group may optionally be substituted with at least one R, and there are independent options for R in each case. In addition, a combination of a substituent and/or its variant is permissible only if such combination results in a stable compound.

In the present disclosure, the term "plurality" refers to 2, 3, 4, or 5, preferably 2 or 3.

In the present disclosure, the term "alkyl" refers to a saturated linear or branched monovalent hydrocarbon group. In the present disclosure, "C₁-C₆ alkyl" in all terms containing "C₁-C₆ alkyl" is preferably C₁-C₄ alkyl, which refers to alkyl having 1 to 4 carbon atoms, in particular methyl, ethyl, n-propyl, isopropyl, n-butyl, isobutyl, sec-butyl, or tert-butyl.

In the present disclosure, the term "C₁₋₆ alkyl substituted with one or more halogens" refers to a group formed by the substitution of one or more (e.g., 2, 3, 4, 5, or 6) hydrogen atoms in the alkyl group by halogen, in which each halogen is independently F, Cl, Br, or I.

In the present disclosure, the term "alkoxy" refers to -O-alkyl, in which the alkyl is as defined above. The C₁-C₆ alkoxy refers to -O-(C₁-C₆ alkyl), in which the C₁-C₆ alkyl is as defined above.

In the present disclosure, the term "alkenyl" refers to a linear or branched unsaturated aliphatic hydrocarbon group consisting of carbon and hydrogen atoms and having at least one double bond. The term "C₂₋₆ alkenyl" is to be understood as preferably meaning a linear or branched monovalent hydrocarbon group, which contains one or more double bonds and has 2, 3, 4, 5, or 6 carbon atoms. In the present disclosure, the term "heterocycloalkyl" refers to a cyclic group that is fully saturated and may exist as a monocyclic ring, a bridged ring, or a spiro ring. Unless otherwise indicated, the heterocycloalkyl group is typically a 4- to 8-membered ring containing 1 to 4 heteroatoms independently selected from sulfur, oxygen, nitrogen, selenium (preferably 1, 2, or 3 heteroatoms selected from nitrogen, oxygen, or sulfur). The term "4- to 8-membered heterocycloalkyl" refers to heterocycloalkyl groups having 4, 5, 6, 7, and 8 ring atoms, respectively. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothiophenyl, pyrrolidinyl, isoxazolidinyl, oxazolidinyl, isothiazolidinyl, thiazolidinyl, imidazolidinyl, tetrahydropyrazolyl. Examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, 1,4-dithianyl, and the like.

In the present disclosure, the term "aryl" refers to an all-carbon monocyclic or fused polycyclic aromatic ring radical with a conjugated pi-electron system. The term "C₆₋₁₀ aryl" is to be understood as meaning a monovalent monocyclic or bicyclic aromatic hydrocarbon ring having 6 to 10 carbon atoms, such as phenyl.

In the present disclosure, the term "cycloalkyl" refers to a monocyclic or polycyclic (e.g., monocyclic, spirocyclic, or bridged cyclic) monovalent hydrocarbon group in which each carbon atom is saturated. C₃₋₈ cycloalkyl may specifically be a 3-, 4-, 5-, 6-, 7- or 8-membered cycloalkyl group, including cyclopropyl, cyclobutyl, cyclopentyl, cyclohexyl, and the like.

In the present disclosure, the term "oxa-C₃₋₈ cycloalkyl" is a saturated 3- to 8-membered cyclic group existing as a monocyclic ring containing one oxygen atom. The term "oxa-C₃₋₈ cycloalkyl" refers to cyclic groups having 3, 4, 5, 6, 7, and 8 ring atoms, respectively. Non-limiting examples of "oxa-C₃₋₈ cycloalkyl" include, but are not limited to, oxiranyl, oxetanyl, oxolanyl, or oxacyclohexyl.

In the present disclosure, the term "halogen" refers to fluorine, chlorine, bromine, or iodine.

In the present disclosure, the term "heteroaryl" refers to an aromatic group containing heteroatoms, preferably an aromatic 8- to 10-membered bicyclic ring containing 1, 2, or 3 heteroatoms independently selected from nitrogen, oxygen, and sulfur, in which at least one ring is aromatic, such as furanyl, pyridinyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzoisothiazolyl, benzoxazolyl, benzoisoxazolyl, quinolinyl, isoquinolinyl, and the like.

In the present disclosure, the term "alkynyl" refers to a linear or branched unsaturated monovalent hydrocarbon group having the specified number of carbon atoms (e.g., C₂ to C₆) and one or more (e.g., 1, 2, or 3) carbon-carbon sp³ triple bonds. Alkynyl groups include, but are not limited to, ethynyl, 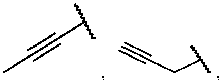 and the like.

In the present disclosure, the term "carbon ring" refers to a cyclic saturated monovalent hydrocarbon group having a specified number of carbon atoms (e.g., C₃ to C₆) and sharing two atoms and a bond with the rest of the molecule.

In the present disclosure, the term "heterocyclic ring" refers to a cyclic saturated monovalent group having the specified number of ring atoms (e.g., 4- to 8-members), the specified number of heteroatoms (e.g., 1, 2, 3, or 4), and the specified heteroatom species (one or more of N, O, S, and Se), and sharing two atoms and a bond with the rest of the molecule.

In the present disclosure, the term "solvate" refers to a substance formed by the combination of a compound and a solvent (including but not limited to water, methanol, ethanol, and the like). Solvates are divided into stoichiometric solvates and non-stoichiometric solvates. Solvates include, but are not limited to, monohydrate.

The term "solvate of a pharmaceutically acceptable salt" refers to a substance formed by the combination of a compound, a pharmaceutically acceptable acid or base, and a solvent (including but not limited to water, methanol, ethanol, and the like). Among them, the amount of the solvent may be stoichiometric or non-stoichiometric. Solvates of pharmaceutically acceptable salts include, but are not limited to, monohydrochloride monohydrate.

The preferred embodiments of the present disclosure can be obtained by any combination of the above preferred conditions on the basis of common knowledge in the art.

The reagents and starting materials used in the present disclosure are commercially available.

The positive effect of the present disclosure is that the compound of formula I or formula II, or a tautomer thereof, a stereoisomer thereof, a pharmaceutically acceptable salt of any of the above, or a solvate of any of the above, has a better inhibitory effect on Polθ.

### Detailed Description of the Invention

The present disclosure is further illustrated by way of the following examples without limiting the present disclosure to the scope of the described examples. The experimental methods in the following examples, which are not specified with specific conditions, are generally selected according to conventional methods and conditions, or according to the product manual.

### Intermediate 1: synthesis of (3aS,4S,6aS)-2,2-dimethyl-6-oxotetrahydro-4H-[1,3]dioxo[4,5-c]pyrrole-4-carboxylic acid

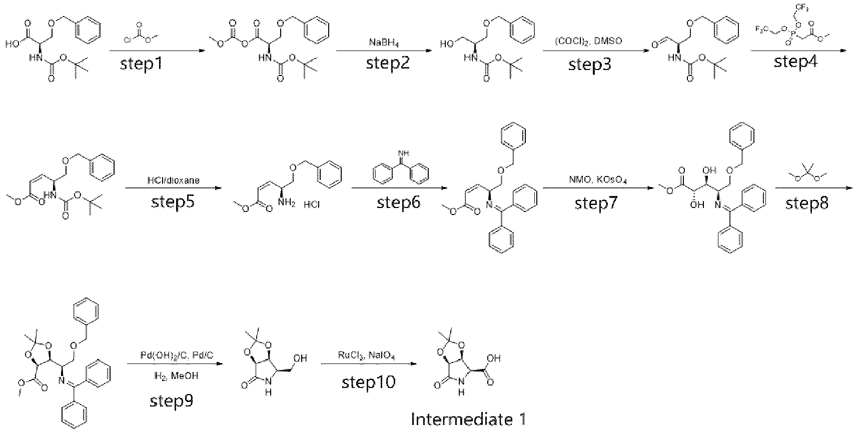

### Step 1: synthesis of (R)-3-(benzyloxy)-2-(tert-butoxycarbonyl)amino)propanoic(methyl carbonic)anhydride

O-benzyl-N-(tert-butoxycarbonyl)-D-serine (100.00 g, 338.60 mmol) and 4-methylmorpholine (3.77 g, 37.25 mmol) were dissolved in tetrahydrofuran (1000 mL) and cooled to -20°C. Methyl chloroformate (3.36 g, 35.55 mmol) was then added dropwise and stirred at -20°C for 1 h. The suspension was filtered. The filter cake was washed with tetrahydrofuran (50 mL*2), and the filtrate was used directly in the next step. LCMS (ESI) m/z: 354[M+H]+.

### Step 2: synthesis of tert-butyl (S)-(1-(benzyloxy)-3-hydroxypropan-2-yl)carbamate

To water (1000 mL) cooled to 0°C was added sodium borohydride (32.00 g, 845.89 mmol) in batches, followed by a solution of (R)-3-(benzyloxy)-2-(tert-butoxycarbonyl)amino)propanoic(methyl carbonic)anhydride in tetrahydrofuran (1100 mL) obtained in the previous step. The mixture was stirred at room temperature overnight and filtered. The filtrate was extracted with ethyl acetate (1 L*2). The combined organic phase was washed with saturated salt water (1.5 L), dried over anhydrous sodium sulfate, and concentrated to give a residue. The obtained crude product was purified on a silica gel column (ethyl acetate:petroleum ether = 33:67) to give tert-butyl (S)-(1-(benzyloxy)-3-hydroxypropan-2-yl)carbamate (55.00 g, 195.49 mmol, two-step yield: 58%) as a colorless oil. LCMS (ESI) m/z: 282[M+H]+.

### Step 3: synthesis of tert-butyl (R)-(1-(benzyloxy)-3-oxopropan-2-yl)carbamate

Oxalyl chloride (30 mL, 355.43 mmol) was dissolved in dichloromethane (750 mL) and cooled to -78°C in the presence of protective nitrogen. Dimethyl sulfoxide (5.55 g, 71.09 mmol) was then slowly added dropwise. The mixture was stirred at -78°C for half an hour. Tert-butyl (S)-(1-(benzyloxy)-3-hydroxypropan-2-yl)carbamate (50 g, 177.71 mmol) was dissolved in dichloromethane (250 mL) and then slowly added dropwise to the reaction system. The obtained mixture was stirred at -78°C for another half an hour. N,N-Diisopropylethyl amine (17.5 mL, 106.63 mmol) was added dropwise. The mixture was stirred at room temperature for 1 h, warmed to -40°C, and then stirred for another 2 h. The mixture was diluted with water (1000 mL) and extracted with dichloromethane (1000 mL*2). The combined organic phase was washed with saturated salt water (100 mL), dried over anhydrous sodium sulfate, filtered, and concentrated under reduced pressure to give the crude product tert-butyl (R)-(1-(benzyloxy)-3-oxopropan-2-yl)carbamate (32.60 g) as a colorless oil. LCMS (ESI) m/z: 280[M+H]+.

### Step 4: synthesis of methyl 5-(benzyloxy)-4-(tert-butoxycarbonyl)amino)pentyl-2-enoate

Methyl 2-(bis(2,2,2-trifluoroethoxy)phosphoryl)acetate (34.85 g, 109.55 mmol) was dissolved in tetrahydrofuran (400 mL). 18-Crown-6 (28.96 g, 109.55 mmol) was added to the mixture and then cooled to -78°C in the presence of protective nitrogen. Potassium bis(trimethylsilyl)amide (1 mol/L in tetrahydrofuran) (110 mL, 110 mmol) was then added dropwise. Finally, tert-butyl (R)-(1-(benzyloxy)-3-oxopropan-2-yl)carbamate (30.60 g, 109.55 mmol) was dissolved in tetrahydrofuran (300 mL) and added to the reaction system. The obtained mixture was stirred at -78°C for 2 h. The mixture was quenched with 1 mol/L hydrochloric acid (107 mL) and extracted with ethyl acetate (600 mL*2). The combined organic phase was washed with saturated salt water (500 mL), dried over anhydrous sodium sulfate, and concentrated to give a residue. The obtained crude product was purified on a silica gel column (ethyl acetate:petroleum ether = 25:75) to give methyl 5-(benzyloxy)-4-(tert-butoxycarbonyl)amino)pentyl-2-enoate (22.50 g, 67.08 mmol, yield: 61%) as a colorless oil. LCMS (ESI) m/z: 336[M+H]+.

### Step 5: synthesis of methyl (S,Z)-4-amino-5-(benzyloxy)pent-2-enoate hydrochloride

Methyl 5-(benzyloxy)-4-(tert-butoxycarbonyl)amino)pentyl-2-enoate (21.80 g, 65.00 mmol) was dissolved in a mol/L solution of hydrogen chloride in 1,4-dioxane (60 mL), and the mixture was stirred at room temperature for 1 h. After completion of the reaction, the mixture was concentrated under reduced pressure to give the crude product methyl (S,Z)-4-amino-5-(benzyloxy)pent -2-enoate hydrochloride (19.60 g) as a yellow solid. LCMS (ESI) m/z: 236[M+H]+.

### Step 6: synthesis of methyl 5-(benzyloxy)-4-(diphenylmethylene)amino)pentyl-2-enoate

Methyl (S,Z)-4-amino-5-(benzyloxy)pent-2-enoate hydrochloride (19.30 g, 71.02 mmol) was dissolved in dichloromethane (200 mL). Benzophenone imine (12.87 g, 71.02 mmol) was added to the mixture and stirred at room temperature overnight. After completion of the reaction, the mixture was concentrated under reduced pressure to give a residue. The obtained crude product was purified on a silica gel column (ethyl acetate:petroleum ether = 25:75) to give methyl 5-(benzyloxy)-4-(diphenylmethylene)amino)pentyl-2-enoate (23 g, 57.57 mmol, yield: 81%) as a yellow oil. LCMS (ESI) m/z: 400[M+H]+.

### Step 7: synthesis of methyl (2S,3S,4R)-5-(benzyloxy)-4-(diphenylmethylene)amino)-2,3-dihydroxyvalerate

The methyl (S,Z)-5-(benzyloxy)-4-(diphenylmethylene)amino)pentyl-2-enoate (21 g, 52.57 mmol) was dissolved in tetrahydrofuran (210 mL). Water (210 mL), potassium osmate dihydrate (0.82 g, 2.63 mmol), and N-methylmorpholine oxide (15.40 g, 131.42 mmol) were added to the mixture, heated to 35°C, and stirred for 6 h. The mixture was diluted with ethyl acetate (300 mL) and water (300 mL) and extracted with ethyl acetate (300 mL*2). The combined organic phase was washed with saturated salt water (300 mL), dried over anhydrous sodium sulfate, filtered, and concentrated under reduced pressure to give the crude product methyl (2S,3S,4R)-5-(benzyloxy)-4-(diphenylmethylene)amino)-2,3-dihydroxyvalerate (23.50 g) as a brown oil. LCMS (ESI) m/z: 434[M+H]+.

### Step 8: synthesis of methyl (4S,5S)-5-((R)-2-(benzyloxy)-1-((diphenylmethylene)amino)ethyl)-2,2-dimethyl-1,3-dioxolane-4-carboxylate

Methyl (2S,3S,4R)-5-(benzyloxy)-4-(diphenylmethylene)amino)-2,3-dihydroxypentanoate (23.50 g, 54.21 mmol) and 2,2-dimethoxypropane (28.23 g, 271.05 mmol) were dissolved in toluene (250 mL). Pyridinium p-toluenesulfonate (3.41 g, 13.55 mmol) was added to the mixture, heated to 100°C, and stirred for 12 h. The mixture was cooled to room temperature and then concentrated under reduced pressure. The obtained crude product was purified on a silica gel column (ethyl acetate:petroleum ether = 15:85) to give methyl (4S,5S)-5-((R)-2-(benzyloxy)-1-((diphenylmethylene)amino)ethyl)-2,2-dimethyl-1,3-dioxolane4-carboxylate (14.30 g, 30.20 mmol, yield: 56%) as a yellow solid. LCMS (ESI) m/z: 474[M+H]+.

### Step 9: synthesis of (3aS,6R,6aS)-6-(hydroxymethyl)-2,2-dimethyltetrahydro-4H-[1,3]dioxo[4,5-c]pyrrol-4-one

Methyl (4S,SS)-5-((R)-2-(benzyloxy)-1-((diphenylmethylene)amino)ethyl)-2,2-dimethyl-1,3-dioxolane-4-carboxylate (5 g, 10.56 mmol) was dissolved in methanol (50 mL). 20% Palladium hydroxide/carbon (0.74 g, 1.06 mmol) and 10% Hydrous Palladium/Carbon (1.12 g, 1.06 mmol) were added to the mixture. The reaction system was replaced with hydrogen three times and then stirred at 40°C under hydrogen (4 MPa) for 60 h. The mixture was filtered. The filter cake was washed with methanol (10 mL*2), and the obtained filtrate was concentrated under reduced pressure to give a residue. The residue was slurried with n-hexane (20 mL) for 30 min, filtered, and then washed with n-hexane (5 mL*2). The filter cake was dried under a vacuum to give (3aS,6R,6aS)-6-(hydroxymethyl)-2,2-dimethyltetrahydro-4H-[1,3]dioxo[4,5-c]pyrrol-4-one (1.57 g, 8.39 mmol, yield: 79%) as a white solid. LCMS (ESI) m/z: 188[M+H]+.

### Step 10: synthesis of (3aS,4S,6aS)-2,2-dimethyl-6-oxotetrahydro-4H-[1,3]dioxo[4,5-c]pyrrole-4-carboxylic acid

(3aS,6R,6aS)-6-(Hydroxymethyl)-2,2-dimethyltetrahydro-4H-[1,3]dioxo[4,5-c]pyrrol-4-one (1.00 g, 5.34 mmol) was dissolved in acetonitrile (9 mL). Carbon tetrachloride (9 mL), H₂O (13 mL), ruthenium trichloride (0.11 g, 0.53 mmol), and sodium periodate (3.43 g, 16.03 mmol) were added to the mixture and stirred in the presence of protective nitrogen for 2 h. The mixture was filtered. The filter cake was washed with methanol (10 mL*2), and the obtained filtrate was concentrated under reduced pressure to give a residue. The residue was dissolved in methanol (20 mL), filtered, and then washed with methanol (5 mL*2). The filtrate was concentrated under reduced pressure to give a crude product (560 mg). The crude product was slurried with water (2.5 mL) for 30 min, filtered, and then washed with water (0.5 mL). The filter cake was dried under a vacuum to give (3aS,4S,6aS)-2,2-dimethyl-6-oxotetrahydro-4H-[1,3]dioxo[4,5-c]pyrrole-4-carboxylic acid (0.25 g, 1.24 mmol, yield: 23%) as an off-white solid. LCMS (ESI) m/z: 202[M+H]+.

### Intermediate 2: synthesis of di-tert-butyl (S)-2-oxoimidazolidine-1,4-dicarboxylate

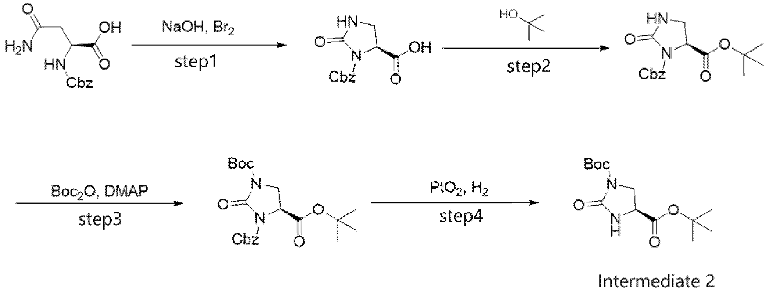

### Step 1: synthesis of (S)-3-((benzyloxy)carbonyl)-2-oxoimidazolidine-4-carboxylic acid

Sodium hydroxide (14.87 g, 371.83 mmol) was dissolved in (300 mL), and the mixture was cooled to 0°C. Bromine (18.01 g, 112.68 mmol) was then slowly added dropwise over 30 min, followed by ((benzyloxy)carbonyl)-L-asparagine (30.0 g, 112.68 mmol) in batches. The obtained colorless solution was heated to 55°C and stirred for 3 h. The mixture was cooled to room temperature and then extracted with methyl tert-butyl ether (300 mL*2). The aqueous layer was acidified to pH = 2 with 6 mol/L hydrochloric acid at 0°C. The suspension was stirred at 0°C for 2 h and filtered to give (S)-3-((benzyloxy)carbonyl)-2-oxoimidazolidine-4-carboxylic acid (21.0 g, 79.47 mmol, yield: 71%) as a white solid. LCMS (ESI) m/z: 265[M+H]+.

### Step 2: synthesis of 1-benzyl 5-(tert-butyl)(thio)-2-oxoimidazolidine-1,5-dicarboxylate

(S)-3-((Benzyloxy)carbonyl)-2-oxoimidazolidine-4-carboxylic acid (1.08 g, 4.08 mmol) was dissolved in chloroform (20 mL). Tert-butanol (1.21 g, 16.33 mmol) and pyridine (1.94 g, 24.49 mmol) were added to the mixture and cooled to -15°C. Phosphorus oxychloride (0.72 g, 4.69 mmol) was then added dropwise. The mixture was stirred at room temperature for 2 h. The mixture was washed with 1 mol/L hydrochloric acid (30 mL), saturated aqueous sodium bicarbonate solution (30 mL), and finally saturated salt water (30 mL), dried over NaSO4, filtered, and then concentrated under reduced pressure to give the crude product 1-benzyl 5-(tert-butyl)(thio)-2-oxoimidazolidine-1,5-dicarboxylate (1.3 g) as a yellow solid. LCMS (ESI) m/z: 321[M+H]+.

### Step 3: synthesis of 3-benzyl 1,4-di-tert-butyl (S)-2-oxoimidazolidine-1,3,4-tricarboxylate

A mixture of 1-benzyl 5-(tert-butyl)(S)-2-oxoimidazolidine-1,5-dicarboxylate (1.30 g, 4.06 mmol), di-tert-butyl dicarbonate (17.71 g, 81.16 mmol), and 4-dimethylaminopyridine (0.05 g, 0.41 mmol) was heated to 60°C and stirred for 2 h. The mixture was cooled to room temperature, then diluted with dichloromethane (30 mL), washed with saturated aqueous sodium bicarbonate solution (30 mL*3) and saturated salt water (30 mL), and finally dried over anhydrous sodium, and concentrated to give a residue. The obtained crude product was purified on a silica gel column (ethyl acetate:petroleum ether = 27:73) to give 3-benzyl 1,4-di-tert-butyl (S)-2-oxoimidazolidine-1,3,4-tricarboxylate (1.37 g, 3.26 mmol, yield: 80%) as an off-white solid. LCMS (ESI) m/z: 421[M+H]+.

### Step 4: synthesis of di-tert-butyl (S)-2-oxoimidazolidine-1,4-dicarboxylate

3-Benzyl 1,4-di-tert-butyl (S)-2-oxoimidazolidine-1,3,4-tricarboxylate (1.26 g, 3.00 mmol) was dissolved in tetrahydrofuran (20 mL). Platinum dioxide (0.17 g, 0.75 mmol) was added to the mixture. The reaction system was replaced with hydrogen three times and then stirred at room temperature for 1 h. The mixture was filtered through diatomaceous earth and washed with tetrahydrofuran (10 mL). The filtrate was concentrated under reduced pressure to give the crude product di-tert-butyl (S)-2-oxoimidazolidine-1,4-dicarboxylate (0.92 g) as a white solid. LCMS (ESI) m/z: 287[M+H]+.

### Intermediate 3: synthesis of (3aS,4S,6aS)-N-(5-chloro-2,4-difluorophenyl)-N,2,2-trimethyl-6-oxotetrahydro-4H-[1,3]dioxo[4,5-c]pyrrole-4-carboxamide

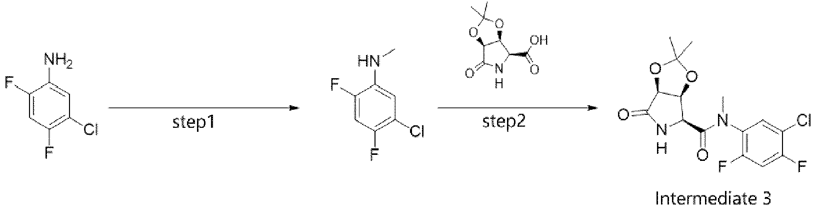

### Step 1: synthesis of 5-chloro-2,4-difluoro-N-methylaniline

Sodium methoxide (6.61 g, 122.29 mmol) was dissolved in methanol (60 mL). 5-Chloro-2,4-difluoroaniline (2.00 g, 12.23 mmol) and aqueous formaldehyde (1.36 mL, 18.34 mmol) were added to the mixture and stirred at room temperature for 16 h. The mixture was cooled to 0°C. Sodium borohydride (0.93 g, 24.46 mmol) was then slowly added in batches. The mixture was returned to room temperature and stirred for 2 h. The mixture was concentrated under reduced pressure to remove the solvent and then diluted with ethyl acetate (30 mL) and water (30 mL). The aqueous phase was extracted with ethyl acetate (30 mL*2). The combined organic phase was washed with saturated salt water (30 mL), dried over anhydrous sodium sulfate, filtered, and concentrated under reduced pressure. The obtained crude product was purified by silica gel column chromatography (ethyl acetate:petroleum ether = 20:80) to give 5-chloro-2,4-difluoro-N-methylaniline (1.37 g, 7.71 mmol, yield: 63%) as a colorless oil. LCMS (ESI) m/z: 178[M+H]+.

### Step 2: synthesis of (3aS,4S,6aS)-N-(5-chloro-2,4-difluorophenyl)-N,2,2-trimethyl-6-oxotetrahydro-4H-[1,3]dioxo[4,5-c]pyrrole-4-carboxamide

(3aS,4S,6aS)-2,2-Dimethyl-6-oxotetrahydro-4H-[1,3]dioxo[4,5-c]pyrrole-4-carboxylic acid **(Intermediate 1,** 150 mg, 0.75 mmol) was dissolved in N,N-dimethylacetamide (3 mL). 5-Chloro-2,4-difluoro-N-methylaniline (199 mg, 1.12 mmol) and pyridine (0.18 mL, 2.24 mmol) were added to the mixture and then cooled to 0°C. Phosphorus oxychloride (114 mg, 0.75 mmol) was then slowly added dropwise, and the obtained mixture was stirred at room temperature for 1 h. The mixture was directly purified by reverse phase column chromatography (acetonitrile:0.05% ammonium bicarbonate aqueous solution = 30:70) to give (3aS,4S,6As)-N-(5-chloro-2,4-difluorophenyl)-N,2,2-trimethyl-6-oxotetrahydro-4H-[1,3]dioxo[4,5-c]pyrrole-4-carboxamide (137 mg, 0.38 mmol, yield: 51%) as a white solid. LCMS (ESI) m/z: 361[M+H]+.

### Intermediate 4: synthesis of (3aS,4S,6aS)-N-(5-chloro-2,4-difluorophenyl)-2,2-dimethyl-N-(deuterated methyl)-6-oxotetrahydro-4H-[1,3]dioxo[4,5-c]pyrrole-4-carboxamide

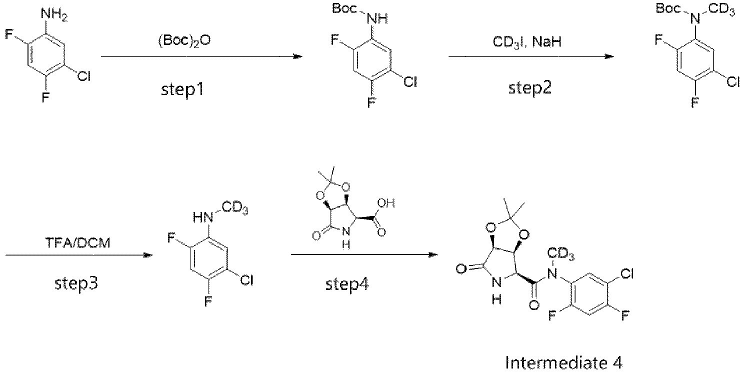

### 4Step 1: synthesis of tert-butyl (5-chloro-2,4-difluorophenyl)carbamate

5-Chloro-2,4-difluoroaniline (10 g, 61.14 mmol) was dissolved in 1,4-dioxane (50 mL). Water (50 mL), di-tert-butyl dicarbonate (40 g, 183.43 mmol), and sodium bicarbonate (30.82 g, 366.86 mmol) were added to the mixture and stirred at 40°C for 24 h. The mixture was extracted with ethyl acetate (100 mL*3). The combined organic layer was dried over anhydrous sodium sulfate, filtered, and concentrated under reduced pressure. The obtained crude product was purified by silica gel column chromatography (ethyl acetate:petroleum ether = 20:80) to give tert-butyl (5-chloro-2,4-difluorophenyl)carbamate (6.80 g, 25.79 mmol, yield: 42%) as a pink solid. LCMS (ESI) m/z: 264[M+H]+.

### Step 2: synthesis of tert-butyl (5-chloro-2,4-difluorophenyl)(deuterated methyl)carbamate

Tert-butyl (5-chloro-2,4-difluorophenyl)carbamate (900 mg, 3.41 mmol) was dissolved in N,N-dimethylformamide (10 mL) and cooled to 0°C. 60% Sodium hydride (205 mg, 5.12 mmol) was then added, and the mixture was stirred at room temperature for 1 h. The reaction mixture was cooled to 0°C again. Perdeuteroiodomethane (594 mg, 4.10 mmol) was then slowly added dropwise to the reaction system, and the obtained mixture was stirred at room temperature for 1 h. The mixture was quenched with saturated aqueous ammonium chloride solution (30 mL) and extracted with ethyl acetate (30 mL*3). The combined organic layer was dried over anhydrous sodium sulfate, filtered, and concentrated under reduced pressure. The obtained crude product was purified by silica gel column chromatography (ethyl acetate:petroleum ether = 20:80) to give tert-butyl (5-chloro-2,4-difluorophenyl)(deuterated methyl)carbamate (882 mg, 3.14 mmol, yield: 92%) as a yellow oil. LCMS (ESI) m/z: 281[M+H]+.

### Step 3: synthesis of 5-chloro-2,4-difluoro-N-(deuterated methyl)aniline

Tert-butyl (5-chloro-2,4-difluorophenyl)(deuterated methyl)carbamate (800 mg, 2.85 mmol) was dissolved in dichloromethane (5 mL). Trifluoroacetic acid (1 mL) was added to the mixture and stirred at room temperature for 1 h. The mixture was concentrated under reduced pressure to give a residue. The obtained crude product was purified by reverse phase column chromatography (acetonitrile:0.05% ammonium bicarbonate aqueous solution = 30:70) to give 5-chloro-2,4-difluoro-N-(deuterated methyl)aniline (450 mg, 2.49 mmol, yield: 87%) as a yellow oil. LCMS (ESI) m/z: 181[M+H]+.

### Step 4: synthesis of (3aS,4S,6aS)-N-(5-chloro-2,4-difluorophenyl)-2,2-dimethyl-N-(deuterated methyl)-6-oxotetrahydro-4H-[1,3]dioxo[4,5-c]pyrrole-4-carboxamide

(3aS,4S,6aS)-2,2-Dimethyl-6-oxotetrahydro-4H-[1,3]dioxo[4,5-c]pyrrole-4-carboxylic acid **(Intermediate 1,** 280 mg, 1.39 mmol) was dissolved in N,N-dimethylacetamide (5 mL). 5-Chloro-2,4-difluoro-N-(deuterated methyl)aniline (377 mg, 2.09 mmol) and pyridine (0.34 mL, 4.18 mmol) were added to the mixture and then cooled to 0°C. Phosphorus oxychloride (213 mg, 1.39 mmol) was then slowly added dropwise, and the obtained mixture was stirred at room temperature for 1 h. The mixture was directly purified by reverse phase column chromatography (acetonitrile:0.05% ammonium bicarbonate aqueous solution = 30:70) to give (3aS,4S,6As)-N-(5-chloro-2,4-difluorophenyl)-2,2-dimethyl-N-(methyl-D3)-6-oxotetrahydro-4H-[1,3]dioxo[4,5-c]pyrrole-4-carboxa mide (405 mg, 1.11 mmol, yield: 80%) as a white solid. LCMS (ESI) m/z: 364[M+H]+.

### Intermediate 5: synthesis of (3aS,4S,6aS)-N-(3-chloro-2,4-difluorophenyl)-2,2-dimethyl-N-(deuterated methyl)-6-oxotetrahydro-4H-[1,3]dioxo[4,5-c]pyrrole-4-carboxamide

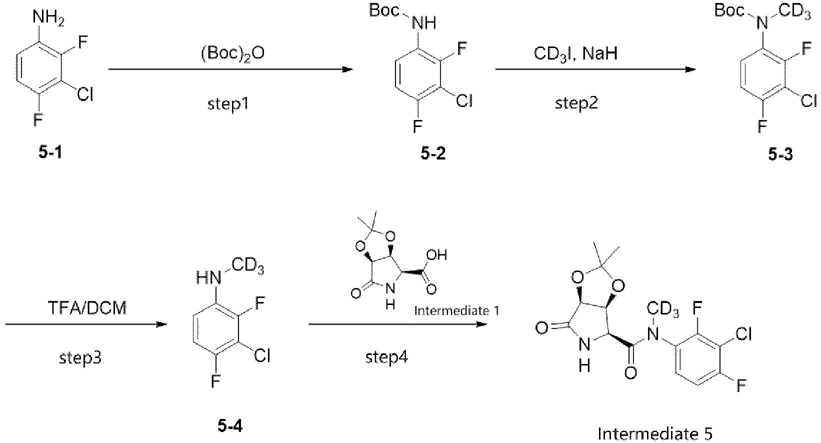

### Step 1: synthesis of tert-butyl (3-chloro-2,4-difluorophenyl)carbamate

3-Chloro-2,4-difluoroaniline (10 g, 61.14 mmol) was dissolved in 1,4-dioxane (50 mL). Water (50 mL), di-tert-butyl dicarbonate (26.69 g, 122.29 mmol), and sodium bicarbonate (20.55 g, 244.57 mmol) were added to the mixture and stirred at 40°C for 24 h. The mixture was extracted with ethyl acetate (100 mL*3). The combined organic layer was dried over anhydrous sodium sulfate, filtered, and concentrated under reduced pressure. The obtained crude product was purified by silica gel column chromatography (ethyl acetate:petroleum ether = 20:80) to give tert-butyl (3-chloro-2,4-difluorophenyl)carbamate (8.60 g, 32.62 mmol, yield: 53%) as an off-white solid. LCMS (ESI) m/z: 264[M+H]+.

### Step 2: synthesis of tert-butyl (3-chloro-2,4-difluorophenyl)(deuterated methyl)carbamate

Tert-butyl (3-chloro-2,4-difluorophenyl)carbamate (1.00 g, 3.79 mmol) was dissolved in N,N-dimethylformamide (10 mL) and cooled to 0°C. 60% Sodium hydride (0.14 g, 5.69 mmol) was then added. The mixture was stirred at room temperature for 1 h. The reaction mixture was cooled to 0°C again. Perdeuteroiodomethane (0.66 g, 4.55 mmol) was then slowly added dropwise to the reaction system, and the obtained mixture was stirred at room temperature for 1 h. The mixture was quenched with saturated aqueous ammonium chloride solution (30 mL) and extracted with ethyl acetate (30 mL*3). The combined organic layer was dried over anhydrous sodium sulfate, filtered, and concentrated under reduced pressure. The obtained crude product was purified on a silica gel column (ethyl acetate:petroleum ether = 10:90) to give tert-butyl (3-chloro-2,4-difluorophenyl)(deuterated methyl)carbamate (0.95 g, 3.38 mmol, yield: 89%) as a colorless oil. LCMS (ESI) m/z: 281[M+H]+.

### Step 3: synthesis of 3-chloro-2,4-difluoro-N-(deuterated methyl)aniline

Tert-butyl (3-chloro-2,4-difluorophenyl)(deuterated methyl)carbamate (850 mg, 3.03 mmol) was dissolved in dichloromethane (5 mL). Trifluoroacetic acid (2 mL) was added to the mixture and stirred at room temperature for 1 h. The mixture was concentrated under reduced pressure to give a residue. The obtained crude product was purified by reverse phase column chromatography (acetonitrile:0.05% ammonium bicarbonate aqueous solution = 30:70) to give 3-chloro-2,4-difluoro-N-(deuterated methyl)aniline (510 mg, 2.82 mmol, yield: 93%) as a yellow oil. LCMS (ESI) m/z: 181[M+H]+.

### Step 4: synthesis of (3aS,4S,6aS)-N-(3-chloro-2,4-difluorophenyl)-2,2-dimethyl-N-(deuterated methyl)-6-oxotetrahydro-4H-[1,3]dioxo[4,5-c]pyrrole-4-carboxamide

(3aS,4S,6aS)-2,2-Dimethyl-6-oxotetrahydro-4H-[1,3]dioxo[4,5-c]pyrrole-4-carboxylic acid (**Intermediate 1,** 130 mg, 0.65 mmol) was dissolved in N,N-dimethylacetamide (3 mL). 3-Chloro-2,4-difluoro-N-(deuterated methyl)aniline (117 mg, 0.65 mmol) and pyridine (0.16 mL, 1.94 mmol) were added to the mixture and then cooled to 0°C. Phosphorus oxychloride (99 mg, 0.65 mmol) was then slowly added dropwise, and the obtained mixture was stirred at room temperature for 1 h. The mixture was directly purified by reverse phase column chromatography (acetonitrile:0.05% ammonium bicarbonate aqueous solution = 30:70) to give (3aS,4S,6aS)-N-(3-chloro-2,4-difluorophenyl)-2,2-dimethyl-N-(methyl-D3)-6-oxotetrahydro-4H-[1,3]dioxo[4,5-c]pyrrole-4-carboxa mide (86 mg, 0.24 mmol, yield: 37%) as a white solid. LCMS (ESI) m/z: 364[M+H]+.

### Intermediate 6: synthesis of tert-butyl (S)-4-((3-chloro-2,4-difluorophenyl)(methyl)carbamoyl)-2-oxoimidazolidine-1-carboxylate

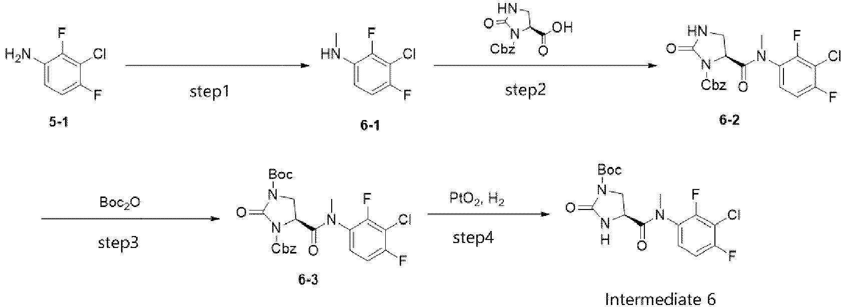

### Step 1: synthesis of 3-chloro-2,4-difluoro-N-methylaniline

Sodium methoxide (33 g, 611.43 mmol) was added to methanol (300 mL) at 0°C. 3-Chloro-2,4-difluoroaniline (10 g, 61.14 mmol) and aqueous formaldehyde (7 mL, 91.72 mmol, 37%) were then added to the system. The mixed solution was allowed to return to room temperature, stirred for 12 h, and then cooled to 0°C. Sodium borohydride (4.63 g, 122.29 mmol) was added and stirred at room temperature for 2 h. The mixture was concentrated under reduced pressure, added with water (30 mL), and extracted with ethyl acetate (30 mL*2). The organic phase was washed with saturated salt water, dried over anhydrous sodium sulfate, and filtered. The filtrate was concentrated. The obtained crude product was purified on a silica gel column (petroleum ether:ethyl acetate = 10:90) to give 3-chloro-2,4-difluoro-N-methylaniline (8.50 g, 476.91 mmol, yield: 78%) as a yellow oil. LCMS (ESI) m/z: 178[M+H]+.

### Step 2: synthesis of benzyl (S)-5-((3-chloro-2,4-difluorophenyl)(methyl)carbamoyl)-2-oxoimidazolidine-1-carboxylate

To dichloromethane (40 mL) was added (S)-3-(benzyloxy) carbonyl)-2-oxoimidazolidine-4-carboxylic acid (3.77 g, 14.27 mmol), followed by N,N-dimethylformamide (2 drops). The mixed solution was cooled to 0°C. Oxalyl chloride (2.42 mL, 28.53 mmol) was slowly added dropwise. The mixed solution was allowed to return to room temperature and stirred for 1 h. The mixture was concentrated under reduced pressure at 0°C. The residue was dissolved in dichloromethane (25 mL) and added dropwise to a solution of 3-chloro-2,4-difluoro-N-methylaniline (3.04 g, 17.12 mmol), pyridine (3.5 mL, 42.80 mmol), and DMAP (0.17 g, 1.43 mmol) in dichloromethane (25 mL) at 0°C. The mixed solution was allowed to return to room temperature and stirred for 1 h. After completion of the reaction, the mixed solution was cooled to 0°C, added dropwise with ice water (50 mL), and then extracted with dichloromethane (50 mL*3). The organic layer was collected, washed with saturated salt water, dried over anhydrous sodium sulfate, and filtered. The filtrate was concentrated. The obtained crude product was purified on a silica gel column (petroleum ether: ethyl acetate = 50:50) to give benzyl (S)-5-(3-chloro-2,4-difluorophenyl)(methyl)carbamoyl)-2-oxoimidazolidine-1-carboxylate (1.85 g, 4.42 mmol, yield: 31%) as a yellow oil. LCMS (ES, m/z): 424 [M+H]+.

### Step 3: synthesis of 3-benzyl 1-(tert-butyl) (S)-4-(3-chloro-2,4-difluorophenyl)(methyl)carbamoyl)-2-oxoimidazolidine-1,3-dicarboxylate

Benzyl (S)-5-(3-chloro-2,4-difluorophenyl)(methyl)carbamoyl)-2-oxoimidazolidine-1-carboxylate (0.64 g, 1.51 mmol), Boc-anhydride (6.57 g, 30.11 mmol), and 4-dimethylaminopyridine (0.02 g, 0.15 mmol) were added to a reaction flask and refluxed at 50°C for 2 h. After completion of the reaction, the reaction solution was cooled to room temperature and poured into water (50 mL). The aqueous phase was extracted with ethyl acetate (50 mL x 3). The organic phases were combined, washed with saturated salt water, dried over anhydrous sodium sulfate, and filtered to remove anhydrous sodium sulfate. The filtrate was concentrated to give the crude product 3-benzyl 1-(tert-butyl) (S)-4-(3-chloro-2,4-difluorophenyl)(methyl)carbamoyl)-2-oxoimidazolidine-1,3-dicarboxylate (1.45 g) as a yellow oily solid, which was used in the next step without purification. LCMS (ES, m/z):524 [M+H]+.

### Step 4: synthesis of tert-butyl (S)-4-((3-chloro-2,4-difluorophenyl)(methyl)carbamoyl)-2-oxoimidazolidine-1-carboxylate

3-Benzyl 1-(tert-butyl) (S)-4-(3-chloro-2,4-difluorophenyl)(methyl)carbamoyl)-2-oxoimidazolidine-1,3-dicarboxylate (1.30 g, 2.48 mmol) was dissolved in tetrahydrofuran (25 mL). Platinum dioxide (0.14 g, 0.62 mmol) was added to the mixture and stirred at room temperature under a hydrogen atmosphere for 1 h. The reaction solution was filtered through a Buchner funnel. The filter cake was washed with methanol (20 mL*2). The filtrate was concentrated to give the crude product tert-butyl (S)-4-((3-chloro-2,4-difluorophenyl)(methyl)carbamoyl)-2-oxoimidazolidine-1-carboxylate (0.36 g, 37%) as a white solid, which was used in the next step without purification. LCMS (ESI) m/z: 390[M+H]+.

### Intermediate 7: synthesis of 7-bromo-5-chlorothiazolo[5,4-b]pyridine

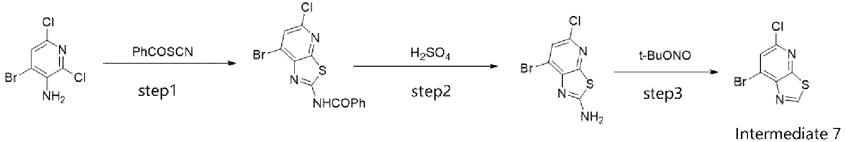

Step 1: 4-bromo-2,6-dichloropyridin-3-amine (10 g, 41.69 mmol) and phenylcyanosulfuric anhydride (10 g, 62.53 mmol) were dissolved in 1,4-dioxane (200 mL) and reacted at 100°C overnight. After completion of the reaction, the reaction solution was poured into water (30 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated to give a crude product. The crude product was purified by silica gel column chromatography (ethyl acetate:petroleum ether = 20:80) to give N-(7-bromo-5-chlorothiazolo[5,4-b]pyridin-2-yl)benzamide (10 g, 27.25 mmol, yield: 65%) as a light yellow solid. LCMS (ESI) *m*/*z*: 368 [M+H]⁺.

Step 2: N-(7-bromo-5-chlorothiazolo[5,4-b]pyridin-2-yl)benzamide (10 g, 27.25 mmol) was dissolved in 98% concentrated sulfuric acid (200 mL) and reacted at 100°C overnight. After completion of the reaction, the reaction solution was slowly dropped into an excess amount of 5% sodium hydroxide solution and suction filtered under a vacuum. The solid phase was washed with water to give 7-bromo-5-chlorothiazolo[5,4-b]pyridin-2-amine (5 g, 19.02 mmol, yield: 70%) as a white solid. LCMS (ESI) *m*/*z*: 266 [M+H]⁺. Step 3: 7-bromo-5-chlorothiazolo[5,4-b]pyridin-2-amine (5 g, 19.02 mmol) and tert-butyl nitrite (2.94 g, 28.53 mmol) were dissolved in tetrahydrofuran (100 mL) and refluxed at 70°C overnight. After completion of the reaction, the reaction solution was poured into water (30 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated to give a crude product. The crude product was purified by silica gel column chromatography (ethyl acetate:petroleum ether = 20:80) to give 7-bromo-5-chlorothiazolo[5,4-b]pyridine (2.7 g, 10.89 mmol, yield: 57%) as a light yellow solid. LCMS (ESI) *m*/*z*: 249 [M+H]⁺.

### Example 1: synthesis of (S)-N-(3-chloro-2,4-difluorophenyl)-N-methyl-2-oxo-3-(4-(trifluoromethyl)-6,7-dihydro-5H-cyclopenta[b]pyridin-2-yl)imidazo lidine-4-carboxamide (Compound 1)

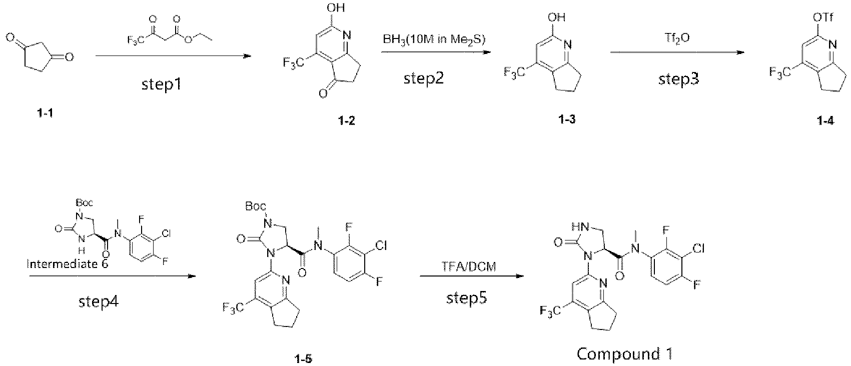

### Step 1: synthesis of 2-hydroxy-4-(trifluoromethyl)-6,7-dihydro-5H-cyclopenta[b]pyridin-5-one

Cyclopentane-1,3-dione (10.00 g, 101.94 mmol) was added to 1,2-dichloroethane (100 mL), followed by ethyl 4,4,4-trifluoro-3-oxobutanoate (28.15 g, 152.91 mmol), ammonium acetate (39.29 g, 509.68 mmol), and 4-dimethylaminopyridine (2.49 g, 20.39 mmol). The mixture was stirred at 140°C for 16 h. After completion of the reaction, the system was cooled to room temperature, poured into water (200 mL), and then extracted with dichloromethane (50 mL*3). The combined organic phase was washed with saturated salt water, dried over anhydrous sodium sulfate, filtered, and concentrated. The crude product was purified by silica gel column chromatography (dichloromethane:methanol = 90:10) to give 2-hydroxy-4-(trifluoromethyl)-6,7-dihydro-5H-cyclopenta[b]pyridin-5-one (5.00 g, 23.44 mmol, yield: 23%) as a white solid. LCMS (ESI) m/z: 218(M+H)⁺.

### Step 2: synthesis of 4-(trifluoromethyl)-6,7-dihydro-5H-cyclopenta[b]pyridin-2-ol

2-Hydroxy-4-(trifluoromethyl)-6,7-dihydro-5H-cyclopenta[b]pyridin-5-one (2.00 g, 9.21 mmol) was added to tetrahydrofuran (20 mL), followed by borane (2.76 mL, 27.63 mmol, 10 mol/L in dimethyl sulfide). The mixture was stirred in a sealed tube at 80°C for 16 h. After completion of the reaction, the system was cooled to room temperature and quenched with methanol (20 mL). The mixture was concentrated under reduced pressure. The residue was purified by reverse phase chromatography (acetonitrile:water = 40:60) to give 4-(trifluoromethyl)-6,7-dihydro-5H-cyclopenta[b]pyridin-2-ol (0.35 g, 1.75 mmol, yield: 19%) as a yellow solid. LCMS (ESI) m/z: 204(M+H)⁺.

### Step 3: synthesis of 4-(trifluoromethyl)-6,7-dihydro-5H-cyclopenta[b]pyridin-2-yl triflate

4-(Trifluoromethyl)-6,7-dihydro-5H-cyclopenta[b]pyridin-2-ol (100 mg, 0.49 mmol) was added to dichloromethane (5 mL), followed by triethylamine (149 mg, 1.48 mmol). The mixture was cooled to -45°C, and triflic anhydride (416 mg, 1.48 mmol) was added dropwise. The system was stirred at room temperature for 16 h. After completion of the reaction, the reaction solution was poured into water (20 mL) and extracted with dichloromethane (10 mL*2). The combined organic phase was washed with saturated salt water, dried over anhydrous sodium sulfate, filtered, and concentrated. The crude product was purified by silica gel column chromatography (petroleum ether:ethyl acetate = 80:20) to give 4-(trifluoromethyl)-6,7-dihydro-5H-cyclopenta[b]pyridin-2-yl triflate (40 mg, 0.12 mmol, yield: 24%) as a colorless oil. LCMS (ESI) m/z: 336(M+H)⁺.

### Step 4: synthesis of tert-butyl (S)-4-((3-chloro-2,4-difluorophenyl)(methyl)carbamoyl)-2-oxo-3-(4-(trifluoromethyl)-6,7-dihydro-5H-cyclopenta[b]pyridin-2-yl)imidazolidine-1-carboxylate

4-(Trifluoromethyl)-6,7-dihydro-5H-cyclopenta[b]pyridin-2-yl triflate (25 mg, 0.07 mmol) was added to 1,4-dioxane (2 mL), followed by tert-butyl (S)-4-((3-chloro-2,4-difluorophenyl)(methyl)carbamoyl)-2-oxoimidazolidine-1-carboxylate (**Intermediate 6**, 32 mg, 0.08 mmol), bis(dibenzylideneacetone)palladium (5 mg, 0.01 mmol), 1,1'-bis(diphenylphosphino)ferrocene (4 mg, 0.01 mmol), and potassium carbonate (21 mg, 0.15 mmol). The mixture was stirred at 100°C for 2 h. After completion of the reaction, the system was cooled to room temperature, poured into water (10 mL), and extracted with ethyl acetate (10 mL*2). The combined organic phase was washed with saturated salt water, dried over anhydrous sodium sulfate, filtered, and concentrated. The crude product was purified by silica gel column chromatography (petroleum ether:ethyl acetate = 80:20) to give tert-butyl (S)-4-((3-chloro-2,4-difluorophenyl)(methyl)carbamoyl)-2-oxo-3-(4-(trifluoromethyl)-6,7-dihydro-SH-cyclopenta[b]pyridin-2-yl)imi dazolidine-1-carboxylate (30 mg, 0.04 mmol, yield: 70%) as a yellow oil. LCMS (ESI) m/z: 575(M+H)⁺.

### Step 5: synthesis of (S)-N-(3-chloro-2,4-difluorophenyl)-N-methyl-2-oxo-3-(4-(trifluoromethyl)-6,7-dihydro-5H-cyclopenta[b]pyridin-2-yl)imidazo lidine-4-carboxamide

### Tert-butyl

(S)-4-((3-chloro-2,4-difluorophenyl)(methyl)carbamoyl)-2-oxo-3-(4-(trifluoromethyl)-6,7-dihydro-SH-cyclopenta[b]pyridin-2-yl)imi dazolidine-1-carboxylate (25 mg, 0.04 mmol) was added to dichloromethane (2 mL), followed by trifluoroacetic acid (0.4 mL). The mixture was stirred at room temperature for 1 h. After completion of the reaction, the system was concentrated under reduced pressure. The residue was purified by high-performance liquid chromatography (column type: Xsele CSH C18 OBD column 30*150 mm, 5 µm, mobile phase A: 0.05% formic acid in water, mobile phase B: acetonitrile; flow rate: 60 mL/min; elution gradient: mobile phase B from 15% to 76% within 8 min, wavelength: 220 nm, retention time: 7.02 min) to give (S)-N-(3-chloro-2,4-difluorophenyl)-N-methyl-2-oxo-3-(4-(trifluoromethyl)-6,7-dihydro-SH-cyclopenta[b]pyridin-2-yl)imidazolidine -4-carboxamide (10.95 mg, 0.02 mmol, yield: 53%) as a white solid. LCMS (ESI) m/z: 474.95(M+H) '. HNMR (400 MHz, DMSO-d6): δ ppm 8.40-8.14 (m, 1H), 7.79 - 7.60 (m, 1H), 7.59 - 7.35 (m, 2H), 5.69-4.73 (m, 1H), 3.89-3.41 (m, 2H), 3.22-3.10 (m, 3H), 3.10 - 2.76 (m, 4H), 2.19 - 2.06 (m, 2H).

### Example 2: synthesis of (S)-N-(3-chloro-2,4-difluorophenyl)-3-(6,7-dihydro-5H-cyclopenta[b]pyridin-2-yl)-N-methyl-2-oxoimidazolidine-4-carboxami de (Compound 2)

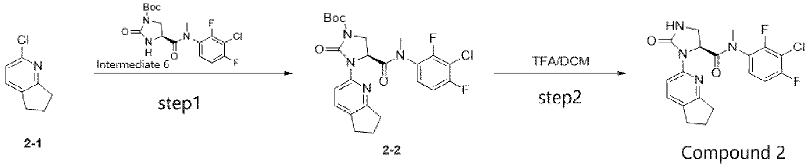

### Step 1: synthesis of tert-butyl (S)-4-((3-chloro-2,4-difluorophenyl)(methyl)carbamoyl)-3-(6,7-dihydro-5H-cyclopenta[b]pyridin-2-yl)-2-oxoimidazolidine-1-c arboxylate

Tert-butyl (S)-4-((3-chloro-2,4-difluorophenyl)(methyl)carbamoyl)-2-oxoimidazolidine-1-carboxylate (**Intermediate 6,** 100 mg, 0.26 mmol) was added to 1,4-dioxane (2.5 mL), followed by 2-chloro-6,7-dihydro-5H-cyclopenta[b]pyridine (59 mg, 0.38 mmol), palladium acetate (6 mg, 0.03 mmol), 4,5-bis(diphenylphosphine)-9,9-dimethylxanthene (15 mg, 0.03 mmol), and potassium carbonate (106 mg, 0.77 mmol). The mixture was stirred at 80°C in the presence of protective nitrogen for 2 h. After completion of the reaction, the system was cooled to room temperature, poured into water (20 mL), and then extracted with ethyl acetate (10 mL*3). The combined organic phase was washed with saturated salt water, dried over anhydrous sodium sulfate, filtered, and concentrated. The crude product was purified by silica gel column chromatography (petroleum ether:ethyl acetate = 55:45) to give tert-butyl (S)-4-((3-chloro-2,4-difluorophenyl)(methyl)carbamoyl)-3-(6,7-dihydro-5H-cyclopenta[b]pyridin-2-yl)-2-oxoimidazolidine-1-carbox ylate (82 mg, 0.16 mmol, yield: 63%) as a yellow solid. LCMS (ESI) m/z: 507(M+H)⁺.

### Step 2: synthesis of (S)-N-(3-chloro-2,4-difluorophenyl)-3-(6,7-dihydro-5H-cyclopenta[b]pyridin-2-yl)-N-methyl-2-oxoimidazolidine-4-carboxami de

### Tert-butyl

(S)-4-((3-chloro-2,4-difluorophenyl)(methyl)carbamoyl)-3-(6,7-dihydro-5H-cyclopenta[b]pyridin-2-yl)-2-oxoimidazolidine-1-carbox ylate (82 mg, 0.16 mmol) was added to dichloromethane (3 mL), followed by trifluoroacetic acid (1 mL). The mixture was stirred at room temperature for 1 h. After completion of the reaction, the system was concentrated under reduced pressure. The residue was purified by high-performance liquid chromatography (column: Xsele CSH C18 OBD column 30*150 mm, 5 µm, mobile phase A: 10 mmol/L ammonium bicarbonate aqueous solution, mobile phase B: acetonitrile; flow rate: 60 mL/min; elution gradient: mobile phase B from 30% to 70% within 8 min, wavelength: 220 nm, retention time: 7.17 min) to give (S)-N-(3-chloro-2,4-difluorophenyl)-3-(6,7-dihydro-5H-cyclopenta[b]pyridin-2-yl)-N-methyl-2-oxoimidazolidine-4-carboxamide (33.80 mg, 0.08 mmol, yield: 51%) as a white solid. LCMS (ESI) m/z: 407.10(M+H) ⁺. HNMR (400 MHz, DMSO-d6): δ ppm 7.98-7.83 (m, 1H), 7.82-7.35(m, 3H), 7.29-7.07 (m, 1H), 5.69-4.64 (m, 1H), 3.85-3.35 (m, 2H), 3.19-3.04 (m, 3H), 3.00-2.88(m, 1H), 2.87-2.72(m, 3H), 2.12-1.95(m, 2H).

### Example 3: synthesis of (S)-N-(3-chloro-2,4-difluorophenyl)-N-methyl-2-oxo-3-(5-oxo-4-(trifluoromethyl)-6,7-dihydro-5H-cyclopenta[b]pyridin-2-yl)i midazolidine-4-carboxamide (Compound 3)

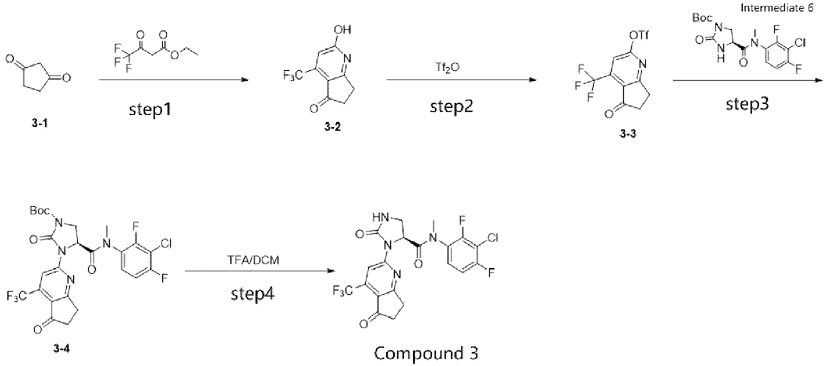

### Step 1: synthesis of 2-hydroxy-4-(trifluoromethyl)-6,7-dihydro-5H-cyclopenta[b]pyridin-5-one

Cyclopentane-1,3-dione (10 g, 101.936 mmol), ethyl 4,4,4-trifluoro-3-oxobutanoate (28.15 g, 152.904 mmol), ammonium acetate (39.29 g, 509.680 mmol), and 4-dimethylaminopyridine (2.49 g, 20.387 mmol) were dissolved in 1,2-dichloroethane (100 mL) and reacted at 140°C overnight. After completion of the reaction, the reaction solution was concentrated under reduced pressure. The residue was purified by silica gel column chromatography (petroleum ether:ethyl acetate = 80:20) to give 2-hydroxy-4-(trifluoromethyl)-6,7-dihydro-5H-cyclopenta[b]pyridin-5-one (8.50 g, 39.143 mmol, yield: 38.40%) as a pink solid. LCMS (ESI): 218 [M+H]⁺.

### Step 2: synthesis of 5-oxo-4-(trifluoromethyl)-6,7-dihydro-5H-cyclopenta[b]pyridin-2-yl triflate

2-Hydroxy-4-(trifluoromethyl)-6,7-dihydro-5H-cyclopenta[b]pyridin-5-one (200 mg, 0.921 mmol) was dissolved in dichloromethane (4 mL). Triethylamine (0.28 g, 2.763 mmol) was added at room temperature, and then triflic anhydride (0.47 mL, 2.763 mmol) was added dropwise at -45°C. The system was allowed to return to room temperature for 2 h. After completion of the reaction, the reaction solution was poured into water (10 mL) and extracted with dichloromethane (10 mL*2). The combined organic layer was washed with saturated salt water, dried over anhydrous sodium sulfate, and filtered. The filtrate was concentrated under reduced pressure. The residue was purified by silica gel column chromatography (petroleum ether:ethyl acetate = 70:30) to give 5-oxo-4-(trifluoromethyl)-6,7-dihydro-5H-cyclopentadienyl[b]pyridin-2-yl triflate (120 mg, 0.343 mmol, yield: 37.31%) as a bright yellow solid. LCMS (ESI): 350 [M+H]⁺.

### Step 3: synthesis of tert-butyl (S)-4-((3-chloro-2,4-difluorophenyl)(methyl)carbamoyl)-2-oxo-3-(5-oxo-4-(trifluoromethyl)-6,7-dihydro-5H-cyclopentadienyl[ b]pyridin-2-yl) imidazolidine-1-carboxylate

In the presence of protective nitrogen, 5-oxo-4-(trifluoromethyl)-6H,7H-cyclopenta[b]pyridin-2-yl triflate (50 mg, 0.143 mmol), tert-butyl (S)-4-((3-chloro-2,4-difluorophenyl)(methyl)carbamoyl)-2-oxoimidazolidine-1-carboxylate (**Intermediate 6,** 61 mg, 0.157 mmol), tris(dibenzylideneacetone)dipalladium (13 mg, 0.014 mmol), 1,1'-bis(diphenylphosphino)ferrocene (8 mg, 0.014 mmol), and potassium carbonate (40 mg, 0.286 mmol) were dissolved in 1,4-dioxane (3 mL) solution, and the mixture was reacted at 100°C for 2 h. After completion of the reaction, the system was cooled to room temperature. The reaction solution was poured into water (10 mL) and extracted with ethyl acetate (10 mL*2). The combined organic layer was washed with saturated salt water, dried over anhydrous sodium sulfate, and filtered. The filtrate was concentrated under reduced pressure. The residue was purified by silica gel column chromatography (petroleum ether:ethyl acetate = 50:50) to give tert-butyl (S)-4-((3-chloro-2,4-difluorophenyl)(methyl)carbamoyl)-2-oxo-3-(5-oxo-4-(trifluoromethyl)-6,7-dihydro-SH-cyclopenta[b]pyridin-2-yl)imidazolidine-1-carboxylate (50 mg, 0.085 mmol, yield: 59.29%) as a white solid. LCMS (ESI): 589 [M+H]⁺.

### Step 4: synthesis of (S)-N-(3-chloro-2,4-difluorophenyl)-N-methyl-2-oxo-3-(5-oxo-4-(trifluoromethyl)-6,7-dihydro-5H-cyclopentyl[b]pyridin-2-yl)i midazolidine-4-carboxamide

### Tert-butyl

(S)-4-((3-chloro-2,4-difluorophenyl)(methyl)carbamoyl)-2-oxo-3-(5-oxo-4-(trifluoromethyl)-6,7-dihydro-SH-cyclopentadienyl[b]pyr idin-2-yl)imidazolidine-1-carboaylate (50 mg, 0.085 mmol) and trifluoroacetic acid (63 µL, 0.850 mmol) were dissolved in dichloromethane (1 mL), and the system was stirred at room temperature in the presence of nitrogen for 1 h. After completion of the reaction, the reaction solution was concentrated under reduced pressure. The crude product was purified by high-performance liquid chromatography (column: CSH Xsele C18 OBD column 30*150 mm, 5 µm; mobile phase A: 0.05% formic acid in water, mobile phase B: acetonitrile; flow rate: 60 mL/min; gradient: 15% B to 68% B within 8 min; wavelength: 220 nm; retention time: 7.70 min) to give (S)-N-(3-chloro-2,4-difluorophenyl)-N-methyl-2-oxo-3-(5-oxo-4-(trifluoromethyl)-6,7-dihydro-5H-cyclopenta[b]pyridin-2-yl)imidaz olidine-4-carboxamide (10.35 mg, 0.021 mmol, yield: 24.94%) as a white solid. LCMS (ESI): 489.00 [M+H]⁺. HNMR (400 MHz, DMSO-d6): δ ppm 8.71-8.52 (m, 1H), 8.51-8.13 (m, 1H), 7.92-7.56 (m, 1H), 7.56-7.32 (m, 1H), 5.12-4.38 (m, 1H), 4.38-4.20 (m, 1H), 4.19-3.82 (m,2H), 3.30-3.28 (m,1H), 3.15-3.10 (s,3H), 2.75-2.66 (m,2H).

### Example 4: synthesis of (S)-N-(3-chloro-2,4-difluorophenyl)-N-methyl-2-oxo-3-(7-(trifluoromethyl)thieno[3,2-b]pyridin-5-yl)imidazoline-4-carboxami de (Compound 4)

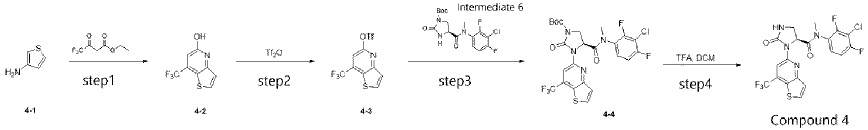

### Step 1: synthesis of 7-(trifluoromethyl)thieno[3,2-b]pyridin-5-ol

3-Aminothiophene hydrochloride (2.0 g, 14.75 mmol) was added to ethyl trifluoroacetoacetate (10.86 g, 58.99 mmol). The mixture was stirred at 130°C overnight. The mixture was concentrated, and methyl tert-butyl ether (20 mL) was added. Solid was precipitated and filtered. The filter cake was washed with methyl tert-butyl ether (20 mL*2) and dried to give 7-(trifluoromethyl)thieno[3,2-b]pyridin-5-ol (1.51 g, 6.84 mmol, yield: 34%) as a yellow solid. LCMS (ESI) m/z: 220[M+H]⁺.

### Step 2: synthesis of 7-(trifluoromethyl)thieno[3,2-b]pyridin-5-yl triflate

7-(Trifluoromethyl)thieno[3,2-b]pyridin-5-ol (550 mg, 2.51 mmol) was added to dichloromethane (5 mL), followed by triethylamine (762 mg, 7.53 mmol). The system was controlled at 0°C, and triflic anhydride (1.06 g, 3.76 mmol) was added dropwise. The mixed system was warmed to room temperature and reacted overnight. The mixture was poured into water (20 mL) and extracted with dichloromethane (10 mL*2). The combined organic phase was washed with saturated salt water, dried over anhydrous sodium sulfate, and filtered. The filtrate was concentrated. The crude product was purified by silica gel column chromatography (petroleum ether:ethyl acetate = 80:20) to give 7-(trifluoromethyl)thieno[3,2-b]pyridin-5-yl triflate (580 mg, 1.65 mmol, yield: 65%) as a yellow oil. LCMS (ESI) m/z: 352 [M+H]⁺.

### Step 3: synthesis of tert-butyl (S)-N-(3-chloro-2,4-difluorophenyl)-N-methyl-2-oxo-3-(7-(trifluoromethyl)thieno[3,2-b]pyridin-5-yl)imidazoline-4-carboxami de

Tert-butyl (S)-4-(3-chloro-2,4-difluorophenyl)methylcarbamoyl-2-oxoimidazoline-1-carboxylate **(Intermediate 6**, 50 mg, 0.13 mmol) and 7-(trifluoromethyl)thieno[3,2-b]pyridin-5-yl triflate (68 mg, 0.19 mmol) were added to dioxane (1 mL), followed by tris(dibenzylideneacetone)dipalladium (8 mg, 0.012 mmol), 1,1'-bis(diphenylphosphino)ferrocene (7 mg, 0.012 mmol), and potassium carbonate (3 mg, 0.26 mmol). The mixture was stirred at 110°C under a nitrogen atmosphere for 2 h. After completion of the reaction, the system was cooled to room temperature. The mixture was added to water (2 mL) and extracted with ethyl acetate (2 mL*2). The combined organic phase was washed with saturated salt water, dried over anhydrous sodium sulfate, and filtered. The filtrate was concentrated. The crude product was purified by silica gel column chromatography (petroleum ether:ethyl acetate = 85:15) to give tert-butyl (S)-N-(3-chloro-2,4-difluorophenyl)-N-methyl-2-oxo-3-(7-(trifluoromethyl)thieno[3,2-b]pyridin-5-yl)imidazoline-4-carboxamide (65 mg, 0.11 mmol, yield: 85%) as a yellow oil. LCMS (ESI) m/z: 591[M+H] ⁺.

### Step 4: synthesis of (S)-N-(3-chloro-2,4-difluorophenyl)-N-methyl-2-oxo-3-(7-(trifluoromethyl)thieno[3,2-b]pyridin-5-yl)imidazoline-4-carboxami de

### Tert-butyl

(S)-N-(3-chloro-2,4-difluorophenyl)-N-methyl-2-oxo-3-(7-(trifluoromethyl)thieno[3,2-b]pyridin-5-yl)imidazoline-4-carboxamide (65 mg, 0.11 mmol) was added to a mixed solution of dichloromethane (0.8 mL) and trifluoroacetic acid (0.2 mL). The mixture was stirred at room temperature for 1 h and concentrated. The crude product was purified by high-performance liquid chromatography (column: CSH C18 OBD column 30 x 150 mm, 5 µm; mobile phase A: 10 mmol/L ammonium bicarbonate aqueous solution; mobile phase B: acetonitrile; flow rate: 60 mL/min; gradient: 30% B to 90% B within 8 min) to give (S)-N-(3-chloro-2,4-difluorophenyl)-N-methyl-2-oxo-3-(7-(trifluoromethyl)thieno[3,2-b]pyridin-5-yl)imidazoline-4-carboxamide (26.66 mg, 0.054 mmol, yield: 49%) as a white solid. LCMS (ESI) m/z:490.85[M+H] ⁺. HNMR (400 MHz, DMSO-d6): δ ppm 8.76-8.67 (m, 1H), 8.41-8.29 (m, 1H), 8.01 - 7.33 (m, 4H), 5.86 - 4.84 (m, 1H), 3.96 - 3.39 (m, 2H), 3.27-3.07 (m, 3H).

### Example 5: synthesis of (S)-N-(3-chloro-2,4-difluorophenyl)-N-methyl-2-oxo-3-(4-(trifluoromethyl)-5,6,7,8-tetrahydroquinolin-2-yl)imidazolidine-4-ca rboxamide (Compound 5)

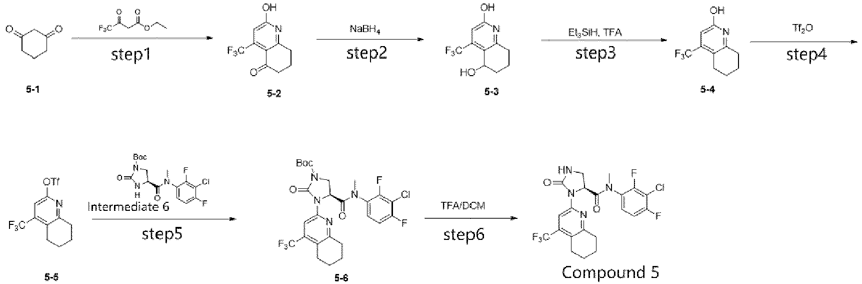

### Step 1: synthesis of 2-hydroxy-4-(trifluoromethyl)-7,8-dihydroquinolin-5(6H)-one

Cyclohexane-1,3-dione (35 g, 0.31 mol) was dissolved in 1,2-dichloroethane (700 mL). Ethyl 4,4,4-trifluoro-3-oxobutanoate (86.25 g, 0.46 mol), ammonium acetate (120.36 g, 1.55 mol), and 4-dimethylaminopyridine (7.56 g, 0.06 mol) were added to the solution, and the mixture was stirred at 140°C for 16 h. After completion of the reaction, the system was cooled to room temperature, poured into ice water, and extracted with dichloromethane (300 mL*2). The combined organic phase was washed with saturated salt water, dried over anhydrous sodium sulfate, and filtered. The filtrate was concentrated under reduced pressure. The crude product was purified by silica gel column chromatography (petroleum ether:ethyl acetate = 80:20) to give 2-hydroxy-4-(trifluoromethyl)-7,8-dihydroquinolin-5(6H)-one (12.24 g, 0.05 mol, yield: 16%) as a yellow solid. LCMS (ESI) m/z: 232[M+H]⁺.

### Step 2: synthesis of 4-(trifluoromethyl)-5,6,7,8-tetrahydroquinolin-2,5-diol

2-Hydroxy-4-(trifluoromethyl)-7,8-dihydroquinolin-5(6H)-one (12.23 g, 0.05 mol) was dissolved in methanol (200 mL), and sodium borohydride (18.91 g, 0.5 mol) was added at 0°C. The mixture was stirred at room temperature for 6 h. After completion of the reaction, the mixture was poured into ice water and extracted with dichloromethane (200 mL*2). The organic layer was washed with saturated salt water, dried over anhydrous sodium sulfate, and filtered. The filtrate was concentrated under reduced pressure to give the crude product 4-(trifluoromethyl)-5,6,7,8-tetrahydroquinolin-2,5-diol (3.43 g) as a yellow oil, which was used in the next step without purification. LCMS (ESI) m/z: 234[M+H]⁺.

### Step 3: synthesis of 4-(trifluoromethyl)-5,6,7,8-tetrahydroquinolin-2-ol

4-(Trifluoromethyl)-5,6,7,8-tetrahydroquinolin-2,5-diol (3.43 g, 14.7 mmol) was placed into a 1 L reaction flask. Trifluoroacetic acid (400 mL) was added, followed by triethylsilane (3.41 g, 29.4 mmol) at 0°C. The mixture was stirred at room temperature for 16 h. After completion of the reaction, the mixture was concentrated under reduced pressure and extracted with water (50 mL) and ethyl acetate (30 mL*2). The combined organic layer was washed with saturated salt water, dried over anhydrous sodium sulfate, and filtered. The filtrate was concentrated under reduced pressure. The crude product was purified by column chromatography (petroleum ether:ethyl acetate = 40:60) to give 4-(trifluoromethyl)-5,6,7,8-tetrahydroquinolin-2-ol (1.33 g, 5.9 mmol, yield: 40%) as a yellow solid. LCMS (ESI) m/z: 218[M+H]⁺.

### Step 4: synthesis of 4-(trifluoromethyl)-5,6,7,8-tetrahydroquinolin-2-yl triflate

4-(Trifluoromethyl)-5,6,7,8-tetrahydroquinolin-2-ol (1.31 g, 5.9 mmol) was dissolved in dichloromethane (200 mL), and triflic anhydride (3.31 g, 11.8 mmol) and triethylamine (1.78 g, 17.7 mmol) were added. The mixture was stirred at room temperature for 16 h. After completion of the reaction, the mixture was extracted with water (50 mL) and dichloromethane (50 mL*2). The combined organic layer was washed with saturated salt water, dried over anhydrous sodium sulfate, and filtered. The filtrate was concentrated under reduced pressure. The crude product was purified by silica gel column chromatography (petroleum ether:ethyl acetate = 30:70) to give 4-(trifluoromethyl)-5,6,7,8-tetrahydroquinolin-2-yl triflate (600 mg, 1.7 mmol, yield: 28%) as a yellow oil. LCMS (ESI) m/z: 218[M+H]⁺.

### Step 5: synthesis of tert-butyl (S)-4-((3-chloro-2,4-difluorophenyl)(methyl)carbamoyl)-2-oxo-3-(4-(trifluoromethyl)-5,6,7,8-tetrahydroquinolin-2-yl)imidazol idine-1-carboxylate

4-(Trifluoromethyl)-5,6,7,8-tetrahydroquinolin-2-yl triflate (600 mg, 1.7 mmol) was dissolved in dioxane (20 mL). Bis(dibenzylideneacetone)palladium (96 mg, 0.17 mmol), 1,1'-bis(diphenylphosphino)ferrocene (94 mg, 0.17 mmol), potassium carbonate (46 mg, 0.34 mmol), and tert-butyl (S)-4-((3-chloro-2,4-difluorophenyl)(methyl)carbamoyl)-2-oxoimidazolidine-1-carboxylate (**Intermediate 6**, 661 mg, 1.7 mmol) was added and stirred at 100°C in the presence of protective nitrogen for 2 h. After completion of the reaction, the mixture was extracted with water (20 mL) and ethyl acetate (20 mL*2). The combined organic layer was washed with saturated salt water, dried over anhydrous sodium sulfate, and filtered. The filtrate was concentrated under reduced pressure. The crude product was purified by silica gel column chromatography (petroleum ether:ethyl acetate = 30:70) to give tert-butyl (S)-4-((3-chloro-2,4-difluorophenyl)(methyl)carbamoyl)-2-oxo-3-(4-(trifluoromethyl)-5,6,7,8-tetrahydroquinolin-2-yl)imidazolidine-1-carboxylate (160 mg, 0.27 mmol, yield: 16%) as a yellow solid. LCMS (ESI) m/z: 589[M+H]⁺.

### Step 6: synthesis of (S)-N-(3-chloro-2,4-difluorophenyl)-N-methyl-2-oxo-3-(4-(trifluoromethyl)-5,6,7,8-tetrahydroquinolin-2-yl)imidazolidine-4-ca rboxamide

### Tert-butyl

(S)-4-((3-chloro-2,4-difluorophenyl)(methyl)carbamoyl)-2-oxo-3-(4-(trifluoromethyl)-5,6,7,8-tetrahydroquinolin-2-yl)imidazolidine-1-carboxylate (160 mg, 0.27 mmol) was placed into a 100 mL reaction flask, and a solution of hydrogen chloride in dioxane (20 mL, 4 mol/L) was added. The mixture was stirred at room temperature for 1 h. After completion of the reaction, the mixture was concentrated under reduced pressure. The crude product was purified by high-performance liquid chromatography (column: XBridge Prep OBD C18, 30 x 150 mm, 5 um, mobile phase A: 10 mmol/L ammonium bicarbonate aqueous solution, mobile phase B: acetonitrile, flow rate: 60 mL/min, elution gradient: mobile phase B increased from 15% to 45% within 8 min, detection wavelength: 220 nm, retention time: 7.90 min) to give (S)-N-(3-chloro-2,4-difluorophenyl)-N-methyl-2-oxo-3-(4-(trifluoromethyl)-5,6,7,8-tetrahydroquinolin-2-yl)imidazolidine-4-carboxa mide (8.22 mg, 0.016 mmol, yield: 5.9%) as a white solid. LCMS (ESI) m/z: 489.10[M+H]⁺. HNMR (400 MHz, DMSO-*d₆*): δ ppm 8.38-8.28(m,1H),7.83-7.33(m,3H),5.70-4.71(m,1H),3.91-3.35(m,2H),3.19-3.06(m,3H),2.96-2.85(m,1H),2.83-2.71(m,3H),1.89-1.73( m,4H).

### Examples 6a and 6b: synthesis of (S)-N-(3-chloro-2,4-difluorophenyl)-3-(R)-5-hydroxy-4-(trifluoromethyl)-6,7-dihydro-5H-cyclopentadienyl[b]pyridin-2-yl)-N-methyl-2-oxoimidazolidine-4-carboxamide and (S)-N-(3-chloro-2,4-difluorophenyl)-3-(S)-5-hydroxy-4-(trifluoromethyl)-6,7-dihydro-5H-cyclopentadienyl[b]pyridin-2-yl)-N-methyl-2-oxoimidazolidine-4-carboxamide

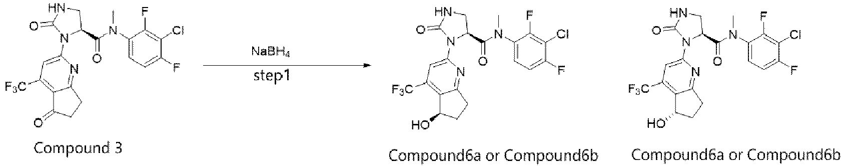

(S)-N-(3-Chloro-2,4-difluorophenyl)-N-methyl-2-oxo-3-(5-oxo-4-(trifluoromethyl)-6,7-dihydro-SH-cyclopenta[b]pyridin-2-yl)imida zolidine-4-carboxamide (**Compound 3**, 100 mg, 0.20 mmol) and sodium borohydride (16 mg, 0.41 mmol) were added to methanol (10.00 mL) and stirred at room temperature for 2 h. After completion of the reaction, the mixture was concentrated under reduced pressure. The obtained crude product was purified by high-performance liquid chromatography (column: YMC-Actus Triart C18, 30*150 mm, 5 µm; mobile phase A: 10 mmol/L ammonium bicarbonate aqueous solution; mobile phase B: acetonitrile; flow rate: 60 mL/min; gradient: 25% B to 62% B within 10 min; wavelength: 220 nm; retention time: 8.45 min and 9.28 min) to give **Compound 6a** (8.84 mg, 0.0180 mmol, yield: 8.75%) as a white solid and **Compound 6b** (11.72 mg, 0.0239 mmol, yield: 11.47%) as a white solid.

### Compound 6a: earlier peak.

LCMS (ESI) m/z: 491.15[M+H]⁺; HNMR (400 MHz, DMSO-*d₆*): δ ppm 8.38-8.28(m, 1H), 7.81-7.64(m,1H), 7.62-7.35(m,2H),5.31-5.22(m,2H), 4.87-4.74(m,1H), 3.53-3.47(m,2H), 3.19-2.96(m,4H), 2.95-2.72(m,1H), 2.42-2.32(m,1H), 1.95-1.90(m,1H). Chiral HPLC: column CHIRALPAK ID-3 4.6*50 mm, 3 um; mobile phase A: (0.1% diethylamine) n-hexane, mobile phase B: ethanol, mobile phase A:mobile phase B = 70:30; flow rate: 1.0 mL/min; temperature: 25°C; retention time: 1.57 min.

### Compound 6b: later peak.

LCMS (ESI) m/z: 491.15[M+H]⁺; 1HNMR (400 MHz, DMSO-*d₆*): δ ppm 8.38-8.28(m, 1H), 7.79-7.65(m,1H), 7.63-7.39(m,2H),5.33-5.28(m,1H), 5.21-5.17(m,1H), 4.88-4.77(m,1H), 3.53-3.48(m,1H), 3.42-3.37(m,1H), 3.29-3.07(m,4H), 2.88-2.64(m,1H), 2.36-2.27(m,1H), 2.01-1.93(m,1H). Chiral HPLC: column CHIRALPAK ID-3 4.6*50 mm, 3 um; mobile phase A: (0.1% diethylamine) n-hexane, mobile phase B: ethanol, mobile phase A:mobile phase B = 70:30; flow rate: 1.0 mL/min; temperature: 25°C; retention time: 1.92 min.

### Example 7: synthesis of (2S,3S,4S)-N-(5-chloro-2,4-difluorophenyl)-3,4-dihydroxy-N-methyl-5-oxo-1-(4-(trifluoromethyl)-6,7-dihydro-5H-cyclopentad ienyl[b]pyridin-2-yl)pyrrolidine-2-carboxamide (Compound 7)

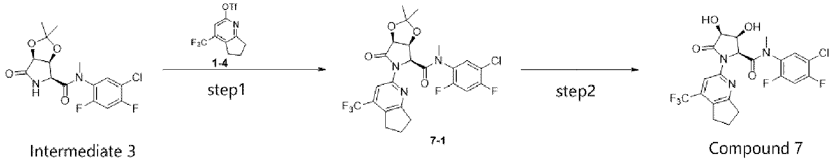

### Step 1: synthesis of (3aS,4S,6aS)-N-(5-chloro-2,4-difluorophenyl)-N,2,2-trimethyl-6-oxo-5-(4-(trifluoromethyl)-6,7-dihydro-5H-cyclopenta[b]pyri din-2-yl)tetrahydro-4H-[1,3]dioxo[4,5-c]pyrrole-4-carboxamide

4-(Trifluoromethyl)-6,7-dihydro-5H-cyclopenta[b]pyridin-2-yl triflate (40 mg, 0.12 mmol) was stirred with (3aS,4S,6aS)-N-(5-chloro-2,4-difluorophenyl)-N,2,2-trimethyl-6-oxotetrahydro-4H-[1,3]dioxo[4,5-c]pyrrole-4-carboxamide (**Intermediate 3**, 43 mg, 0.12 mmol), tris(dibenzylideneacetone)dipalladium (7 mg, 0.01 mmol), 4,5-bis(diphenylphosphino)-9,9-dimethylxanthene (14 mg, 0.02 mmol), and cesium carbonate (78 mg, 0.24 mmol) in toluene (4 mL) at 100°C for 12 h. The mixture was then filtered, concentrated, and purified by silica gel column chromatography (petroleum ether:ethyl acetate = 12:88) to give (3aS,4S,6aS)-N-(5-chloro-2,4-difluorophenyl)-N,2,2-trimethyl-6-oxo-5-(4-(trifluoromethyl)-6,7-dihydro-SH-cyclopenta[b]pyridin-2-yl)tetrahydro-4H-[1,3]dioxo[4,5-c]pyrrole-4-carboxamide (20 mg, 0.03 mmol, yield: 31%) as a yellow solid. LCMS (ESI) m/z: 546(M+H)+.

### Step 2: synthesis of (2S,3S,4S)-N-(5-chloro-2,4-difluorophenyl)-3,4-dihydroxy-N-methyl-5-oxo-1-(4-(trifluoromethyl)-6,7-dihydro-5H-cyclopenta[ b]pyridin-2-yl)pyrrolidine-2-carboxamide

A mixture of (3aS,4S,6aS)-N-(5-chloro-2,4-difluorophenyl)-N,2,2-trimethyl-6-oxo-5-(4-(trifluoromethyl)-6,7-dihydro-SH-cyclopenta[b]pyridin-2-yl)tetrahydro-4H-[1,3]dioxo[4,5-c]pyrrole-4-carboxamide (0.02 g, 0.04 mmol) in dichloromethane (2 mL) was added to boron trichloride (0.01 g 0.18 mmol). The mixture was stirred at 20°C for 2 h, poured into saturated sodium bicarbonate solution, extracted with ethyl acetate (20 mL), dried over anhydrous sodium sulfate, filtered, concentrated, and then purified by high-performance liquid chromatography (column type: Xsele CSH C18 OBD column 30*150 mm, 5 µm, mobile phase A: 0.05% formic acid in water, mobile phase B: acetonitrile; flow rate: 60 mL/min; elution gradient: mobile phase B from 15% to 76% within 8 min, wavelength: 220 nm, retention time: 7.73 min) to give (2S,3S,4S)-N-(5-chloro-2,4-difluorophenyl)-3,4-dihydroxy-N-methyl-5-oxo-1-(4-(trifluoromethyl)-6,7-dihydro-5H-cyclopenta[b]pyr idin-2-yl)pyrrolidine-2-carboxamide (1.77 mg, 0.01 mmol, yield: 9%) as a white solid. LCMS (ESI) m/z: 506.05(M+H) ⁺. 1H NMR (400 MHz, DMSO-d6) δ8.45- 8.17 (m, 1H), 7.99-7.54 (m, 1H), 5.68-5.61 (m, 1H), 5.17-5.03 (m, 1H), 4.86-4.74 (m, 2H), 4.24-4.20 (m, 1H), 4.08-4.03 (m, 1H), 3.15 - 3.01 (m, 4H), 2.95-2.85 (m, 1H), 2.25-2.08 (m, 2H).

### Examples 8a and 8b: synthesis of (S)-N-(3-chloro-2,4-difluorophenyl)-3-(S)-5-hydroxy-5-methyl-4-(trifluoromethyl)-6,7-dihydro-5H-cyclopenta[b]pyridin-2-yl)-N-methyl-2-oxoimidazolidine-4-carboxamide and (S)-N-(3-chloro-2,4-difluorophenyl)-3-(R)-5-hydroxy-5-methyl-4-(trifluoromethyl)-6,7-dihydro-5H-cyclopenta[b]pyridin-2-yl) -N-methyl-2-oxoimidazolidine-4-carboxamide

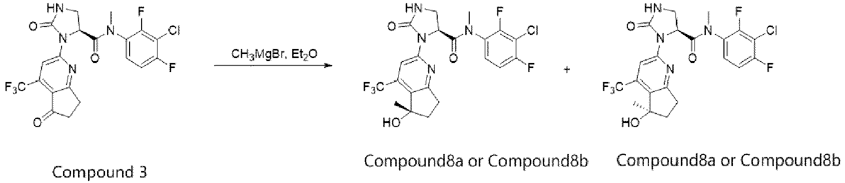

(S)-N-(3-Chloro-2,4-difluorophenyl)-N-methyl-2-oxo-3-(5-oxo-4-(trifluoromethyl)-6,7-dihydro-5H-cyclopenta[b]pyridin-2-yl)imida zolidine-4-carboxamide (**Compound 3**, 40 mg, 0.07 mmol) was dissolved in anhydrous ethyl ether (1 mL), and methylmagnesium bromide (2.8 mol/L in 2-methyltetrahydrofuran, 0.1 mL, 0.28 mmol) was added dropwise at 0°C. The mixture was stirred at 0°C for 2 h. After completion of the reaction, the mixture was quenched with ice water (10 mL) and filtered. The filtrate was extracted with ethyl acetate (5 mL*2). The organic phase was washed with saturated salt water, dried over anhydrous sodium sulfate, and filtered. The filtrate was concentrated. The obtained crude product was purified by high-performance liquid chromatography (column: Xselect CSH C18 OBD Column 30*150 mm, 5 µm; mobile phase A: 0.1% formic acid in water, mobile phase B: acetonitrile; flow rate: 60 mL/min; gradient: 20% B to 64% B within 10 min; wavelength: 220 nm; retention time 1: 7.02 min, retention time 2: 8.83 min) to give a compound earlier peak **Compound 8a** (1.91 mg, 0.003 mmol, yield: 5.38%) as a white solid and a compound later peak **Compound 8b** (2.55 mg, 0.005 mmol, yield: 7.30%) as a white solid.

### Compound 8a: earlier peak.

LCMS (ESI) m/z: 505.00[M+H]⁺ ; HNMR (400 MHz, DMSO-*d₆*): δ ppm 8.36-8.32(m,1H), 7.75-7.61(m,1H), 7.60-7.38(m,2H), 5.28-5.22(m,1H), 4.88-4.75(m,1H), 3.53-3.47(m,1H), 3.39-3.37(m,1H), 3.19-3.11(m,3H), 3.03-2.91(m, 1H), 2.87-2.83(m, 1H), 2.15-2.11(m,2H), 1.47-1.45(m,3H). Chiral HPLC: CHIRALPAK IA-3 4.6*50 mm, 3 um; mobile phase A: (0.1% diethylamine) n-hexane, mobile phase B: ethanol; mobile phase A:mobile phase B = 80:20; flow rate: 1 mL/min; temperature: 25°C; retention time: 1.64 min.

### Compound 8b: later peak.

LCMS (ESI) m/z: 505.00[M+H]⁺ ; HNMR (400 MHz, DMSO-*d₆*): δ ppm 8.36-8.33(m,1H), 7.80-7.62(m,1H), 7.60-7.36(m,2H), 5.30-5.26(m,1H), 4.85-4.75(m,1H), 3.91-3.79(m,0.5H), 3.53-3.46(m,1.5H), 3.17-3.09(m,3H), 3.05-2.90(m,1H), 2.88-2.74(m,1H), 2.21-2.12(m,2H), 1.44-1.35(m,3H). Chiral HPLC: CHIRALPAK IA-3 4.6*50 mm, 3 um; mobile phase A: (0.1% diethylamine) n-hexane, mobile phase B: ethanol; mobile phase A:mobile phase B = 80:20; flow rate: 1 mL/min; temperature: 25°C; retention time: 2.67 min.

### Examples 9a and 9b: synthesis of (S)-N-(3-chloro-2,4-difluorophenyl)-N-methyl-2-oxo-3-(4-(trifluoromethyl)-7H-cyclopenta[b]pyridin-2-yl)imidazolidine-4-car boxamide and (S)-N-(3-chloro-2,4-difluorophenyl)-N-methyl-2-oxo-3-(4-(trifluoromethyl)-7H-cyclopenta[b]pyridin-2-yl)imidazolidine carboxamide

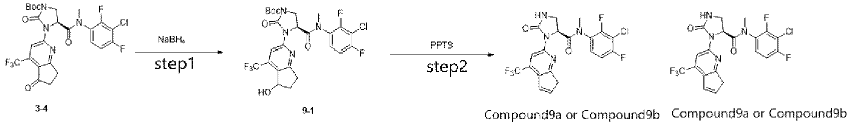

### Step 1: synthesis of tert-butyl (S)-4-((3-chloro-2,4-difluorophenyl)(methyl)carbamoyl)-2-oxo-3-(5-hydroxy-4-(trifluoromethyl)-6,7-dihydro-5H-cyclopentadi enyl[b]pyridin-2-yl)imidazolidine-1-carboxylate

### Tert-butyl

(S)-4-((3-chloro-2,4-difluorophenyl)(methyl)carbamoyl)-2-oxo-3-(5-oxo-4-(trifluoromethyl)-6,7-dihydro-SH-cyclopentadienyl[b]pyr idin-2-yl)imidazolidine-1-carboxylate (500 mg, 0.85 mmol) was dissolved in methanol (10 mL), and sodium borohydride (48 mg, 1.27 mmol) was then added. The mixture was stirred at room temperature for 1 h. After completion of the reaction, the reaction solution was poured into water (30 mL) and extracted with ethyl acetate (10 mL*2). The combined organic phase was washed with saturated salt water, dried over anhydrous sodium sulfate, and filtered. The filtrate was concentrated. The residue was purified by silica gel column chromatography (ethyl acetate:petroleum ether = 40:60) to give tert-butyl (4S)-4-((3-chloro-2,4-difluorophenyl)(methyl)carbamoyl)-3-(5-hydroxy-4-(trifluoromethyl)-6,7-dihydro-SH-cyclopenta[b]pyridin-2-yl)-2-oxoimidazolidine-1-carboxylate (360 mg, 0.60 mmol, yield: 71%) as a yellow solid. LCMS (ESI) m/z: 591[M+H]⁺ ;

### Step 2: synthesis of (S)-N-(3-chloro-2,4-difluorophenyl)-N-methyl-2-oxo-3-(4-(trifluoromethyl)-7H-cyclopenta[b]pyridin-2-yl)imidazolidine-4-car boxamide (Compound 9a) and (S)-N-(3-chloro-2,4-difluorophenyl)-N-methyl-2-oxo-3-(4-(trifluoromethyl)-7H-cyclopenta[b]pyridin-2-yl)imidazolidine-4-car boxamide (Compound 9b)

### Tert-butyl

(4S)-4-((3-chloro-2,4-difluorophenyl)(methyl)carbamoyl)-3-(5-hydroxy-4-(trifluoromethyl)-6,7-dihydro-SH-cyclopenta[b]pyridin-2-yl)-2-oxoimidazolidine-1-carboxylate (200 mg, 0.34 mmol) and pyridinium p-toluenesulfonate (170 mg, 0.38 mmol) were dissolved in 1,2-dichloroethane (10 mL) and stirred at 100°C for 6 h. After completion of the reaction, the system was cooled to room temperature. Water (20 mL) was added, and the mixture was extracted with dichloromethane (5 mL*2). The organic phase was washed with saturated salt water, dried over anhydrous sodium sulfate, and filtered. The filtrate was concentrated. The obtained crude product was purified by high-performance liquid chromatography (column: XBridge Shield RP18 OBD Column, 19*150 mm, 5 µm; mobile phase A: 10 mmol/L ammonium bicarbonate aqueous solution, mobile phase B: acetonitrile; flow rate: 60 mL/min; gradient: 30% B to 70% B within 10 min; wavelength: 220 nm; retention time: 9.12 min) to give 40 mg of a mixture of rotamers, which was subjected to chiral resolution (chiral column: CHIRAL ART Cellulose-SB, 2*25 cm, 5 µm; mobile phase A: 0.1% diethylamine in n-hexane, mobile phase B: ethanol; flow rate: 20 mL/min; gradient: 20% B to 20% B within 11 min; wavelength: 220 nm; retention time 1: 9 min, retention time 2: 10.3 min) to give **rotamer 1 (Compound 9a)** (4.95 mg, 0.01 mmol, yield: 3.09%) as a white solid and **rotamer 2 (Compound 9b)** (14.95 mg, 0.03 mmol, yield: 9.34%) as a white solid.

### Compound 9a: rotamer 1, earlier peak.

LCMS (ESI) m/z: 473.00[M+H]⁺ ; HTEM (400 MHz, DMSO-*d₆*): δ ppm: 8.42-8.25(m,1H), 8.11-7.41(m,2H), 7.40-7.14(m,2H), 7.13-6.80(m,1H), 5.44-4.72(m,1H), 3.72-3.48(m,3H), 3.39-3.14(m,4H).

Chiral HPLC: CHIRAL Cellulose-SB 4.6*100 mm, 3 um; mobile phase A: (0.1% formic acid) n-hexane, mobile phase B: ethanol; mobile phase A:mobile phase B = 80:20; flow rate: 1 mL/min; temperature: 25°C; retention time: 3.52 min.

### Compound 9b: rotamer 2, later peak.

LCMS (ESI) m/z: 472.95[M+H]⁺ ; HNMR (400 MHz, DMSO-*d₆*): δ ppm: S3S-S.24(m,1H), 7.81-7.34(m,2H), 7.33-7.04(m,2H),7.03-6.78(m,1H), 5.32-4.51(m,1H), 3.73-3.44(m,3H), 3.41-3.09(m,4H).

Chiral HPLC: CHIRAL Cellulose-SB 4.6*100 mm, 3 um; mobile phase A: (0.1% formic acid) n-hexane, mobile phase B: ethanol; mobile phase A:mobile phase B = 80:20; flow rate: 1 mL/min; temperature: 25°C; retention time: 3.94 min.

### Example 10: synthesis of (S)-N-(3-chloro-2,4-difluorophenyl)-N-methyl-3-(1-methyl-7-(trifluoromethyl)-1H-imidazo[4,5-b]pyridin-5-yl)-2-oxoimidazoli dine-4-carboxamide (Compound 10)

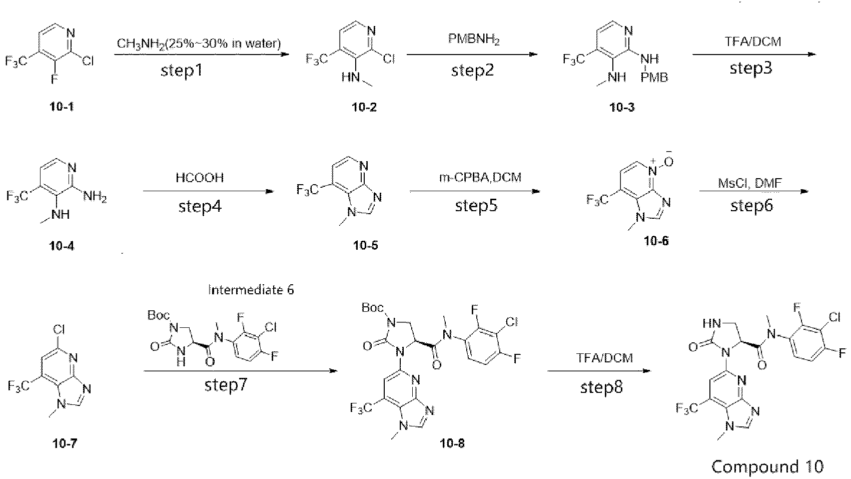

### Step 1: synthesis of 2-chloro-N-methyl-4-(trifluoromethyl)pyridin-3-amine

2-Chloro-3-fluoro-4-(trifluoromethyl)pyridine (10.00 g, 0.05 mol) was weighed into a reaction flask, and a solution of methylamine in water (20.00 mL, 25%-30% content) was added. The mixture was stirred at room temperature for 8 h. After completion of the reaction, water (100 mL) was added, and the mixture was extracted with dichloromethane (50 mL*3). The organic phases were combined, washed with saturated salt water, dried over anhydrous sodium sulfate, filtered to remove anhydrous sodium sulfate, and concentrated to give the crude product 2-chloro-N-methyl-4-(trifluoromethyl)pyridin-3-amine (6.52 g) as a yellow oil, which was used in the next step without purification. LCMS (ESI) m/z: 211[M+H]+.

### Step 2: synthesis of N²-(4-methoxybenzyl)-N³-methyl-4-(trifluoromethyl)pyridine-2,3-diamine

2-Chloro-N-methyl-4-(trifluoromethyl)pyridin-3-amine (6.32 g, 30.01 mmol) was taken into a reaction flask, and p-methoxybenzylamine (12.00 mL) was added. The mixture was stirred at 140°C overnight. After completion of the reaction, the system was cooled to room temperature. Water (100 mL) was added, and the mixture was extracted with dichloromethane (50 mL*3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 50:50) to give N²-(4-methoxybenzyl)-N³-methyl-4-(trifluoromethyl)pyridine-2,3-diamine (3.58 g, 11.47 mmol, yield: 38%) as a yellow oil. LCMS (ESI) m/z: 312[M+H]+.

### Step 3: synthesis of N³-methyl-4-(trifluoromethyl)pyridine-2,3-diamine

The N²-(4-methoxybenzyl)-N³-methyl-4-(trifluoromethyl)pyridine-2,3-diamine (2.00 g, 10.47 mmol) was dissolved in a mixed solution of trifluoroacetic acid (1.00 mL) and dichloromethane (5.00 mL), and the mixture was stirred at 60°C overnight. After completion of the reaction, the mixture was concentrated under reduced pressure to remove the solvent, added with dichloromethane (20 mL) to slurry for 30 min, and filtered. The filter cake was washed with dichloromethane (10 mL*3) and dried to give the crude product N³-methyl-4-(trifluoromethyl)pyridine-2,3-diamine (2.2 g) as a white solid, which was used in the next step without purification. LCMS (ESI) m/z: 192[M+H]+.

### Step 4: synthesis of 1-methyl-7-(trifluoromethyl)-1H-imidazo[4,5-b]pyridine

N³-Methyl-4-(trifluoromethyl)pyridine-2,3-diamine (1.35 g, 6.71 mmol) was weighed into a reaction flask, and formic acid (10.00 mL) was added for dissolution. The mixture was stirred at 100°C overnight. After completion of the reaction, the system was cooled to room temperature. Water (50 mL) was added, and the mixture was extracted with dichloromethane (20 mL*3). The organic phases were combined, washed with saturated salt water, dried over anhydrous sodium sulfate, and filtered to remove anhydrous sodium sulfate. The filtrate was concentrated under reduced pressure to give the crude product 1-methyl-7-(trifluoromethyl)-1H-imidazo[4,5-b]pyridine (1.35 g) as a yellow oil, which was used in the next step without purification. LCMS (ESI) m/z: 202[M+H]+.

### Step 5: synthesis of 1-methyl-7-(trifluoromethyl)-1H-imidazo[4,5-b]pyridine 4-oxide

1-Methyl-7-(trifluoromethyl)-1H-imidazo[4,5-b]pyridine (1.00 g, 4.97 mmol) was weighed into a reaction flask, and dichloromethane (10.00 mL) was added for dissolution. m-Chloroperoxybenzoic acid (1.72 g, 9.94 mmol) was added to the system, and the mixture was stirred at room temperature for 6 h. After completion of the reaction, the crude product was purified by silica gel column chromatography (dichloromethane:methanol = 65:35) to give 1-methyl-7-(trifluoromethyl)-1H-imidazo[4,5-b]pyridine 4-oxide (0.52 g, 2.38 mmol, yield: 48%) as a white solid. LCMS (ESI) m/z: 218[M+H]⁺.

### Step 6: synthesis of 5-chloro-1-methyl-7-(trifluoromethyl)-1H-imidazo[4,5-b]pyridine

1-Methyl-7-(trifluoromethyl)-1H-imidazo[4,5-b]pyridine 4-oxide (400 mg, 1.84 mmol) was weighed into a reaction flask. N,N-Dimethylformamide (10.00 mL) was added for dissolution, followed by methanesulfonyl chloride (211 mg, 1.84 mmol). The mixture was stirred at 80°C for 2 h. After completion of the reaction, the system was cooled to room temperature. Saturated aqueous ammonium bicarbonate solution (30 mL) was added, and the mixture was extracted with dichloromethane (10 mL*3). The organic phases were combined, washed with saturated salt water, dried over anhydrous sodium sulfate, and filtered to remove anhydrous sodium sulfate. The filtrate was concentrated under reduced pressure to give the crude product 5-chloro-1-methyl-7-(trifluoromethyl)-1H-imidazo[4,5-b]pyridine (350 mg) as a yellow oil, which was used in the next step without purification. LCMS (ESI) m/z: 236[M+H]+.

### Step 7: synthesis of tert-butyl (S)-4-((3-chloro-2,4-difluorophenyl)(methyl)carbamoyl)-3-(1-methyl-7-(trifluoromethyl)-1H-imidazo[4,5-b]pyridin-5-yl)-2-ox oimidazolidine-1-carboxylate

5-Chloro-1-methyl-7-(trifluoromethyl)-1H-imidazo[4,5-b]pyridine (100 mg, 0.42 mmol) was weighed into a reaction flask. 1,4-Dioxane (1.00 mL) was added for dissolution, followed by tert-butyl (S)-4-((3-chloro-2,4-difluorophenyl)(methyl)carbamoyl)-3-(1-methyl-7-(trifluoromethyl)-1H-imidazo[4,5-b]pyridin-5-yl)-2-oxoimid azolidine-1-carboxylate (165 mg, 0.42 mmol), tris(dibenzylideneacetone)dipalladium (51 mg, 0.08 mmol), 4,5-bis(diphenylphosphine)-9,9-dimethylxanthene (26 mg, 0.08 mmol), and cesium carbonate (211 mg, 1.84 mmol). The mixture was stirred at 80°C under a nitrogen atmosphere for 2 h. After completion of the reaction, the system was cooled to room temperature. Water (30 mL) was added, and the mixture was extracted with dichloromethane (10 mL*3). The organic phases were combined, washed with saturated salt water, dried over anhydrous sodium sulfate, and filtered to remove anhydrous sodium sulfate. The filtrate was concentrated under reduced pressure to give the crude product tert-butyl (S)-4-((3-chloro-2,4-difluorophenyl)(methyl)carbamoyl)-3-(1-methyl-7-(trifluoromethyl)-1H-imidazo[4,5-b]pyridin-5-yl)-2-oxoimid azolidine-1-carboxylate (350 mg) as a yellow oil, which was used in the next step without purification. LCMS (ESI) m/z: 589[M+H]+.

### Step 8: synthesis of (S)-N-(3-chloro-2,4-difluorophenyl)-N-methyl-3-(1-methyl-7-(trifluoromethyl)-1H-imidazo[4,5-b]pyridin-5-yl)-2-oxoimidazoli dine-4-carboxamide

### Tert-butyl

(S)-4-((3-chloro-2,4-difluorophenyl)(methyl)carbamoyl)-3-(1-methyl-7-(trifluoromethyl)-1H-imidazo[4,5-b]pyridin-5-yl)-2-oxoimid azolidine-1-carboxylate (350 mg, 0.59 mmol) was dissolved in a mixed solution of trifluoroacetic acid (1.00 mL) and dichloromethane (5.00 mL) and stirred at room temperature for 2 h. The mixture was concentrated under a vacuum to give a crude product. The crude product was purified by high-performance liquid chromatography (column: Xbridge Shield RP18 OBD Column, 19*150 mm, 5 µm, mobile phase A: 10 mmol/L formic acid in water, mobile phase B: acetonitrile, flow rate: 60 mL/min, elution gradient: mobile phase B increased from 20% to 50% within 9.33 min, detection wavelength: 220 nm, retention time: 7.58 min) to give

(S)-N-(3-chloro-2,4-difluorophenyl)-N-methyl-3-(1-methyl-7-(trifluoromethyl)-1H-imidazo[4,5-b]pyridin-5-yl)-2-oxoimidazolidine-4-carboxamide (84.70 mg, 0.173 mmol, yield: 29%) as a white solid. LCMS (ESI) m/z: 489.05[M+H]+; HNMR (400 MHz, DMSO-*d₆*): δ ppm 857(s,1H), 8.47(s,1H), 8.37 (brs,1H) 7.45(brs, 1H), 7.24(s, 1H), 5.85-4.75(m,1H), 3.92(s,3H), 3.72-3.32(m,2H), 3.31-3.12(m,3H).

### Example 11:

### (S)-N-(3-chloro-2,4-difluorophenyl)-3-(5,5-difluoro-4-(trifluoromethyl)-6,7-dihydro-5H-cyclopentadienyl[b]pyridin-2-yl)-N-m ethyl-2-oxoimidazolidine-4-carboxamide (Compound 11)

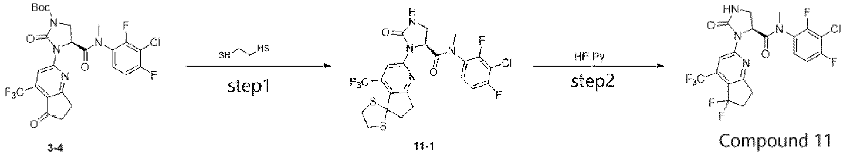

### Step 1: synthesis of (S)-N-(3-chloro-2,4-difluorophenyl)-N-methyl-2-oxo-3-(4-(trifluoromethyl)-6,7-dihydrospiro[cyclopentadienyl[b]pyridine-5, 2'-[1,3]dithiolane]-2-yl)imidazolidine-4-carboxamide

### Tert-butyl

(S)-4-((3-chloro-2,4-difluorophenyl)(methyl)carbamoyl)-2-oxo-3-(5-oxo-4-(trifluoromethyl)-6,7-dihydro-SH-cyclopentadienyl[b]pyr idin-2-yl)imidazolidine-1-carboxylate (100 mg, 0.17 mmol) was taken into a reaction flask. Acetic acid (2.00 mL) was added for dissolution, followed by ethane-1,2-dithiol (24 mg, 0.25 mmol) and aluminum trichloride (5 mg, 0.03 mmol). The mixture was stirred at 100°C overnight. After completion of the reaction, the system was cooled to room temperature. Water (20 mL) was added, and the mixture was extracted with dichloromethane (10 mL*3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 35:65) to give (S)-N-(3-chloro-2,4-difluorophenyl)-N-methyl-2-oxo-3-(4-(trifluoromethyl)-6,7-dihydrospiro[cyclopenta[b]pyridine-5,2'-[1,3]dithiol ane]-2-yl)imidazolidine-4-carboxamide (80 mg, 0.14 mmol, yield: 83%) as a yellow solid. LCMS (ESI) m/z: 565[M+H]+.

### Step 2: synthesis of (S)-N-(3-chloro-2,4-difluorophenyl)-3-(5,5-difluoro-4-(trifluoromethyl)-6,7-dihydro-5H-cyclopentadienyl[b]pyridin-2-yl)-N-m ethyl-2-oxoimidazolidine-4-carboxamide

Iodosuccinimide (35 mg, 0.18 mmol) was dissolved in dichloromethane (5.00 mL), and the mixture was cooled to -78°C. (S)-N-(3-Chloro-2,4-difluorophenyl)-N-methyl-2-oxo-3-(4-(trifluoromethyl)-6,7-dihydrospiro[cyclopenta[b]pyridine-5,2'-[1,3]dithio lane]-2-yl)imidazolidine-4-carboxamide (50 mg, 0.08 mmol) was then added, and at this temperature, pyridinium hydrofluoride (5 mg, 0.18 mmol) was added. The mixture was stirred at -78°C for 1 h. After completion of the reaction, water (10 mL) was added to the system, and the mixture was extracted with dichloromethane (5 mL*3). The organic phase was washed with saturated salt water, dried over anhydrous sodium sulfate, and filtered. The filtrate was concentrated. The residue was purified by high-performance liquid chromatography (column: XBridge Shield RP18 OBD column, 19*150 mm, 5 µm; mobile phase A: 0.05% formic acid in water, mobile phase B: acetonitrile; flow rate: 60 mL; elution gradient: mobile phase B increased from 35% to 70% within 8 min; detection wavelength: 220 nm; retention time: 7.67 min) to give (S)-N-(3-chloro-2,4-difluorophenyl)-3-(5,5-difluoro-4-(trifluoromethyl)-6,7-dihydro-5H-cyclopenta[b]pyridin-2-yl)-N-methyl-2-oxoi midazolidine-4-carboxamide (4.66 mg, 0.008 mmol, yield: 10%) as a white solid. LCMS (ESI) m/z: 511.20(M+H) ⁺; HNMR (400 MHz, DMSO-*d₆*): δ ppm 8.56(s, 1H), 7.78-7.35(m, 3H), 5.01-4.75(m, 1H), 3.62-3.41 (m, 2H), 3.33-3.11(m,5H), 2.80-2.53 (m, 2H).

### Example 12:

### (S)-N-(3-chloro-2,4-difluorophenyl)-3-(7,7-difluoro-4-(trifluoromethyl)-6,7-dihydro-5H-cyclopenta[b]pyridin-2-yl)-N-methyl-2-oxoimidazolidine-4-carboxamide (Compound 12)

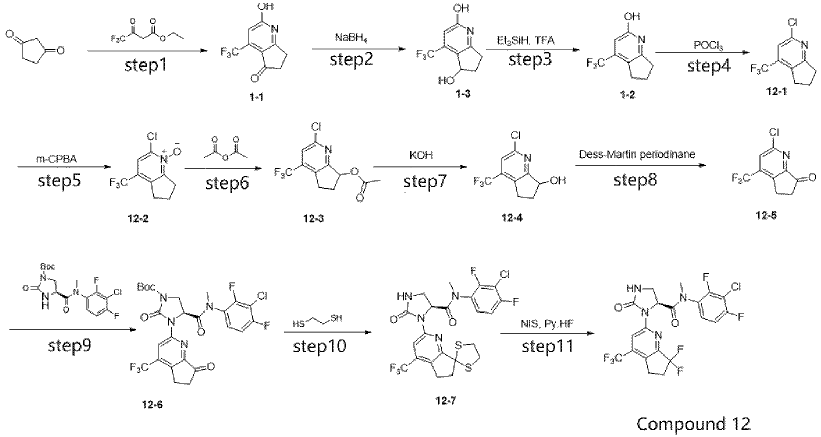

### Step 1: synthesis of 2-hydroxy-4-(trifluoromethyl)-6,7-dihydro-5H-cyclopenta[b]pyridin-5-one

1,3-Cyclopentanedione (100.00 g, 1.02 mol), ethyl 4,4,4-trifluoro-3-oxobutanoate (281.50 g, 1.53 mol), ammonium acetate (392.00 g, 5.09 mol), and 4-dimethylaminopyridine (24.91 g, 0.20 mol) were dissolved in dichloroethane (1 L) and stirred in an autoclave at 140°C overnight. After completion of the reaction, the system was cooled to room temperature. The mixture was poured into water (500 mL) and extracted with dichloromethane (500 mL*3). The organic phases were combined, dried over anhydrous sodium sulfate, and filtered. The filtrate was concentrated under reduced pressure. The crude product was purified by silica gel column chromatography (dichloromethane:methanol = 80:20) to give 2-hydroxy-4-(trifluoromethyl)-6,7-dihydro-5H-cyclopenta[b]pyridin-5-one (30.00 g, 0.14 mol, yield: 14%) as a pink solid. LCMS(ESI)m/z: **218**[M+H]⁺.

### Step 2: synthesis of 4-(trifluoromethyl)-6,7-dihydro-5H-cyclopenta[b]pyridine-2,5-diol

2-Hydroxy-4-(trifluoromethyl)-6,7-dihydro-5H-cyclopenta[b]pyridin-5-one (30.00 g, 138.15 mmol) was dissolved in methanol (600 mL) solution. Sodium borohydride (26.13 g, 690.77 mmol) was added and stirred at room temperature for 12 h. After completion of the reaction, the obtained mixture was concentrated under reduced pressure, poured into water (500 mL), and extracted with dichloromethane (500 mL*3). The organic phases were combined, dried over anhydrous sodium sulfate, and filtered. The filtrate was concentrated under reduced pressure to give the crude product 4-(trifluoromethyl)-6,7-dihydro-5H-cyclopenta[b]pyridine-2,5-diol (35.00 g) as a white solid. The crude product was directly used in the next step without purification. LCMS(ESI)m/z: **220**[M+H]⁺.

### Step 3: synthesis of 4-(trifluoromethyl)-6,7-dihydro-5H-cyclopenta[b]pyridin-2-ol

4-(Trifluoromethyl)-6,7-dihydro-5H-cyclopenta[b]pyridine-2,5-diol (30.00 g, 0.13 mol) was dissolved in a mixed solution of triethylsilane (60 mL) and trifluoroacetic acid (300 mL), and the mixture was stirred at 50°C for 2 h. After completion of the reaction, the system was cooled to room temperature. The obtained mixture was concentrated under reduced pressure to give a crude product. The crude product was purified by silica gel column chromatography (petroleum ether: ethyl acetate = 55:45) to give 4-(trifluoromethyl)-6,7-dihydro-5H-cyclopenta[b]pyridin-2-ol (15.02 g, 0.07 mmol, yield: 54%) as a yellow oil. LCMS(ESI)m/z: **204**[M+H]⁺.

### Step 4: synthesis of 2-chloro-4-(trifluoromethyl)-6,7-dihydro-5H-cyclopenta[b]pyridine

4-(Trifluoromethyl)-6,7-dihydro-5H-cyclopenta[b]pyridin-2-ol (10.00 g, 49.21 mmol) was added to phosphorus oxychloride (200 mL) solution at room temperature. The mixture was stirred at 60°C for 12 h. After completion of the reaction, the system was cooled to room temperature. The obtained mixture was concentrated under reduced pressure to give the crude product 2-chloro-4-(trifluoromethyl)-6,7-dihydro-5H-cyclopenta[b]pyridine (10.01 g) as a yellow oil. The crude product was directly used in the next step without purification. LCMS(ESI)m/z: **222**[M+H]⁺.

### Step 5: synthesis of 2-chloro-4-(trifluoromethyl)-6,7-dihydro-5H-cyclopenta[b]pyridine-1-oxide

2-Chloro-4-(trifluoromethyl)-6,7-dihydro-5H-cyclopenta[b]pyridine (1.00 g, 4.52 mmol) was dissolved in dichloromethane (20 mL) solution. m-Chloroperoxybenzoic acid (3.12 g, 18.04 mmol, 85% content) was added at room temperature. The mixture was stirred at 40°C overnight. After completion of the reaction, the system was cooled to room temperature. The mixture was poured into water (10 mL) and extracted with dichloromethane (10 mL*2). The organic phases were combined, dried over anhydrous sodium sulfate, suction filtered, and concentrated under reduced pressure to give a crude product. The crude product was purified by silica gel column chromatography (petroleum ether:ethyl acetate = 45:55) to give 2-chloro-4-(trifluoromethyl)-6,7-dihydro-5H-cyclopenta[b]pyridine-1-oxide (350 mg, 1.47 mmol, yield: 34%) as a yellow oil. LCMS(ESI)m/z: **238**[M+H]⁺.

### Step 6: synthesis of 2-chloro-4-(trifluoromethyl)-6,7-dihydro-5H-cyclopenta[b]pyridin-7-yl acetate

2-Chloro-4-(trifluoromethyl)-6,7-dihydro-5H-cyclopenta[b]pyridine-1-oxide (350 mg, 1.47 mmol) was dissolved in acetic anhydride (10 mL). The mixture was stirred at 100°C for 2 h. After completion of the reaction, the system was cooled to room temperature and directly concentrated under reduced pressure. The crude product was purified by silica gel column chromatography (petroleum ether:ethyl acetate = 70:30) to give 2-chloro-4-(trifluoromethyl)-6,7-dihydro-5H-cyclopenta[b]pyridin-7-yl acetate (350 mg, 1.25 mmol, yield: 85%) as a yellow oil. LCMS(ESI)m/z: **280**[M+H]⁺.

### Step 7: synthesis of 2-chloro-4-(trifluoromethyl)-6,7-dihydro-5H-cyclopenta[b]pyridin-7-ol

2-Chloro-4-(trifluoromethyl)-6,7-dihydro-5H-cyclopenta[b]pyridin-7-yl acetate (350 mg, 1.25 mmol) and potassium hydroxide (350 mg, 6.26 mmol) were dissolved in a solution of ethanol (10 mL) and water (1 mL), and the mixture was stirred at room temperature for 1 h. After completion of the reaction, the aqueous phase was adjusted to pH 4 with 2 mol/L aqueous hydrochloric acid solution. The mixture was extracted with dichloromethane (10 mL*2). The combined organic layer was dried over anhydrous sodium sulfate, suction filtered, and concentrated under reduced pressure. The crude product was eluted by silica gel column chromatography (petroleum ether:ethyl acetate = 90:10) to give 2-chloro-4-(trifluoromethyl)-6,7-dihydro-5H-cyclopenta[b]pyridin-7-ol (200 mg, 0.84 mmol, yield: 67%) as a yellow oil. LCMS(ESI)m/z: **238**[M+H]⁺.

### Step 8: synthesis of 2-chloro-4-(trifluoromethyl)-5,6-dihydro-7H-cyclopenta[b]pyridin-7-one

2-Chloro-4-(trifluoromethyl)-6,7-dihydro-5H-cyclopenta[b]pyridin-7-ol (200 mg, 0.84 mmol) and Dess-Martin periodinane (714 mg, 1.68 mmol) were dissolved in dichloromethane (4 mL) solution, and the mixture was stirred at room temperature for 1 h. After completion of the reaction, the reaction was quenched with saturated sodium bicarbonate solution (10 mL) and saturated sodium bisulfite solution (10 mL). The mixture was extracted with dichloromethane (10 mL*2). The organic phases were combined, dried over anhydrous sodium sulfate, filtered, and concentrated under reduced pressure. The crude product was purified by silica gel column chromatography (petroleum ether:ethyl acetate = 90:10) to give 2-chloro-4-(trifluoromethyl)-5,6-dihydro-7H-cyclopenta[b]pyridin-7-one (150 mg, 0.63 mmol, yield: 76%) as a yellow oil. LCMS(ESI)m/z: **236**[M+H]+.

### Step 9: synthesis of (S)-tert-butyl 4-((3-chloro-2,4-difluorophenyl)(methyl)carbamoyl)-2-oxo-3-(7-oxo-4-(trifluoromethyl)-6,7-dihydro-5H-cyclopenta[b]pyridin-2-yl)imidazolidine-1-carboxylate

2-Chloro-4-(trifluoromethyl)-5,6-dihydro-7H-cyclopenta[b]pyridin-7-one (150 mg, 0.64 mmol), (S)-tert-butyl 4-((3-chloro-2,4-difluorophenyl)(methyl)carbamoyl)-2-oxoimidazolidine-1-carboxylate (**Intermediate 6**, 198 mg, 0.51 mmol), palladium acetate (14 mg, 0.06 mmol), 4,5-bis(diphenylphosphine)-9,9-dimethylxanthene (14 mg, 0.06 mmol), and potassium carbonate (263 mg, 1.91 mmol) was dissolved in dioxane (3 mL) solution and reacted at 80°C for 2 h. After completion of the reaction, the system was cooled to room temperature. The reaction solution was poured into water (10 mL). The obtained mixture was extracted with ethyl acetate (10 mL x 2). The combined organic layer was washed with saturated salt water and dried over anhydrous sodium sulfate. After filtration, the filtrate was concentrated under reduced pressure. The crude product was purified by silica gel column chromatography (petroleum ether:ethyl acetate = 60:40) to give (S)-tert-butyl 4-((3-chloro-2,4-difluorophenyl)(methyl)carbamoyl)-2-oxo-3-(7-oxo-4-(trifluoromethyl)-6,7-dihydro-5H-cyclopenta[b]pyridin-2-yl)i midazolidine-1-carboxylate (140 mg, 0.23 mmol, yield: 37%) as a yellow oil. LCMS(ESI)m/z: **590**[M+H]⁺.

### Step 10: synthesis of (S)-N-(3-chloro-2,4-difluorophenyl)-N-methyl-2-oxo-3-(4-(trifluoromethyl)-5,6-dihydrospiro[cyclopenta[b]pyridine-7,2'-[1,3]d ithiolane]-2-yl)imidazolidine-4-carboxamide

### (S)-Tert-butyl

4-((3-chloro-2,4-difluorophenyl)(methyl)carbamoyl)-2-oxo-3-(7-oxo-4-(trifluoromethyl)-6,7-dihydro-5H-cyclopenta[b]pyridin-2-yl)i midazolidine-1-carboxylate (70 mg, 0.12 mmol) was dissolved in glacial acetic acid (5 mL). Ethanedithiol (33 mg, 0.30 mmol) was then added, and the reaction system was replaced with nitrogen three times. The obtained mixture was stirred at 100°C overnight. After completion of the reaction, the system was cooled to room temperature. The reaction solution was poured into water (10 mL). The obtained mixture was extracted with ethyl acetate (5 mL x 3). The combined organic layer was washed with saturated salt water and dried over anhydrous sodium sulfate. After filtration, the filtrate was concentrated under reduced pressure. The crude product was purified by silica gel column chromatography (petroleum ether:ethyl acetate = 60:40) to give (S)-N-(3-chloro-2,4-difluorophenyl)-N-methyl-2-oxo-3-(4-(trifluoromethyl)-5,6-dihydrospiro[cyclopenta[b]pyridine-7,2'-[1,3]dithiol ane]-2-yl)imidazolidine-4-carboxamide (60 mg, 0.11 mmol, yield: 89%) as a yellow solid. LCMS(ESI)m/z: **565**[M+H]⁺.

### Step 11: synthesis of (S)-N-(3-chloro-2,4-difluorophenyl)-3-(7,7-difluoro-4-(trifluoromethyl)-6,7-dihydro-5H-cyclopenta[b]pyridin-2-yl)-N-methyl-2-oxoimidazolidine-4-carboxamide

N-Bromosuccinimide (16 mg, 0.09 mmol) was dissolved in dichloromethane (2.00 mL). Pyridine hydrofluorate (5 mg, 0.05 mmol) was added, and the obtained mixture was stirred at -78°C for 0.5 h. (S)-N-(3-Chloro-2,4-difluorophenyl)-N-methyl-2-oxo-3-(4-(trifluoromethyl)-5,6-dihydrospiro[cyclopenta[b]pyridine-7,2'-[1,3]dithio lane]-2-yl)imidazolidine-4-carboxamide (25 mg, 0.04 mmol) was then added at -78°C. The obtained mixture was stirred at -40°C for 2 h. After completion of the reaction, the mixture was poured into water (10 mL) and extracted with dichloromethane (10 mL*2). The combined organic phase was dried over anhydrous sodium sulfate and suction filtered. The filtrate was spin-dried. The crude product was purified by high-performance liquid chromatography (column: Xselect CSH OBD Column 30*150 mm, 5 um; mobile phase A: 10 mmol/L ammonium bicarbonate aqueous solution, mobile phase B: acetonitrile; flow rate: 60 mL/min; elution gradient: mobile phase B increased from 40% to 60% within 10 min; detection wavelength: 220 nm; retention time: 8.93 min) to give (S)-N-(3-chloro-2,4-difluorophenyl)-3-(7,7-difluoro-4-(trifluoromethyl)-6,7-dibydro-5H-cyclopenta[b]pyridin-2-yl)-N-methyl-2-oxoi midazolidine-4-carboxamide (3.15 mg, 0.006 mmol, yield: 14%) as a white solid. HNMR(400 MHz, CDCl₃): δ ppm 8.71(s,1H), 7.94-7.88(m,1H),7.18-7.13(m,1H),4.99-4.95(m,1H),3.66-3.56(m,2H),3.26(s,3H),3.16-3.10(m, 2H), 2.74-2.63 (m,2H). LCMS(ESI)m/z:**510.80**[M+H]⁺ .

### Example 13:

### (S)-N-(3-chloro-2,4-difluorophenyl)-N-methyl-2-oxo-3-(7-oxo-4-(trifluoromethyl)-6,7-dihydro-5H-cyclopenta[b]pyridin-2-yl)i midazolidine-4-carboxamide (Compound 13)

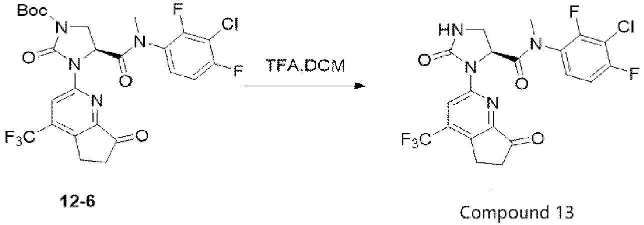

### (S)-Tert-butyl

4-((3-chloro-2,4-difluorophenyl)(methyl)carbamoyl)-2-oxo-3-(7-oxo-4-(trifluoromethyl)-6,7-dihydro-5H-cyclopenta[b]pyridin-2-yl)i midazolidine-1-carboxylate (50 mg, 0.08 mmol) was dissolved in trifluoroacetic acid (1 mL) and dichloromethane (5 mL), and the system was stirred at room temperature for 1 h. After completion of the reaction, the obtained mixture was concentrated under reduced pressure. The crude product was purified by high-performance liquid chromatography (column: XBridge Shield RP18 OBD column, 30 x 150 mm, 5 µm; mobile phase A: 10 mmol/L ammonium bicarbonate aqueous solution, mobile phase B: acetonitrile; flow rate: 60 mL/min; gradient: 25% B to 70% B within 8 min; wavelength: 220 nm; retention time: 7.53 min) to give (S)-N-(3-chloro-2,4-difluorophenyl)-N-methyl-2-oxo-3-(7-oxo-4-(trifluoromethyl)-6,7-dihydro-SH-cyclopenta[b]pyridin-2-yl)imidaz olidine-4-carboxamide (10.68 mg, 0.02 mmol, yield: 26%) as a white solid. HNMR(400 MHz, DMSO-d₆): δ ppm 8.78(s,1H), 8.59-8.53(m,1H), 7.80(s,1H),7.53-7.47(m,1H), 4.89-4.86(m,1H), 3.52-3.49(m, 1H), 3.24-3.03(m,6H), 2.81-2.67(m,2H). LCMS (ESI) m/z: **488.80** [M+H]⁺.

### Example 14:

### (S)-N-(3-chloro-2,4-difluorophenyl)-N-methyl-2-oxo-3-(4-(trifluoromethyl)thieno[2,3-b]pyridin-6-yl)imidazolidine-4-carboxa mide (Compound 14)

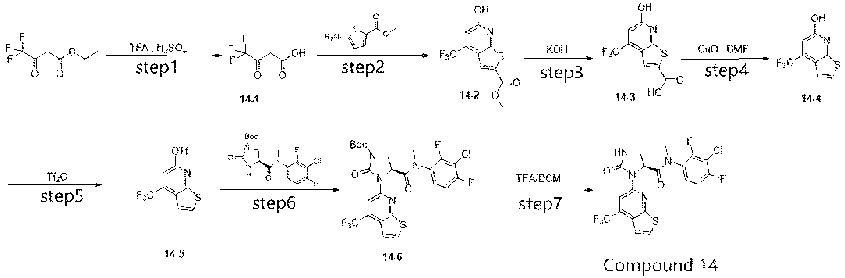

Step 1: a reaction system of ethyl 4,4,4-trifluoro-3-oxobutanoate (10.00 g, 54.32 mmol), trifluoroacetic acid (3.10 g, 27.16 mmol), and concentrated sulfuric acid (1.33 g, 13.58 mmol) was reacted at 90°C overnight in the presence of protective nitrogen. After completion of the reaction, the system was cooled to room temperature. The mixture was added dropwise to saturated aqueous sodium bicarbonate solution (50 mL) and extracted with ethyl acetate (25 mL). The aqueous phase was adjusted to pH 4 with 2 mol/L aqueous hydrochloric acid solution and extracted with ethyl acetate (25 mL*2). The organic phases were combined, washed with saturated salt water, dried over anhydrous sodium sulfate, and filtered. The filtrate was concentrated to give the crude product 4,4,4-trifluoro-3-oxobutanoic acid (5.00 g) as a colorless oil. The crude product was directly used in the next step without purification. LCMS (ESI) m/z: **157** [M+H]⁺.

Step 2: 4,4,4-trifluoro-3-oxobutanoic acid (5.00 g, 32.04 mmol) and methyl 5-aminothiophene-2-carboxylate (5.04 g, 32.04 mmol) were added to glacial acetic acid (100.00 mL) and reacted at 80°C for 2 h. After completion of the reaction, the obtained mixture was concentrated under reduced pressure. The crude product was purified by silica gel column chromatography (petroleum ether:ethyl acetate = 70:30) to give methyl 6-hydroxy-4-(trifluoromethyl)thieno[2,3-b]pyridine-2-carboxylate (1.10 g, 3.95 mmol, yield: 25%) as a yellow solid. LCMS (ESI) m/z: **278**[M+H]⁺.

Step 3: methyl 6-hydroxy-4-(trifluoromethyl)thieno[2,3-b]pyridine-2-carboxylate (1.01 g, 3.61 mmol) was dissolved in a solution of methanol (16 mL) and water (2 mL), and potassium hydroxide (606 mg, 10.83 mmol) was added. The mixture was stirred at 50°C for 2 h. After completion of the reaction, the system was cooled to room temperature. The mixture was adjusted to pH 4 with 2 mol/L aqueous hydrochloric acid solution and extracted with ethyl acetate (20 mL*3). The organic phases were combined, washed with saturated salt water, dried over anhydrous sodium sulfate, and concentrated under reduced pressure. The crude product was purified by silica gel column chromatography (dichloromethane:methanol = 80:20) to give 6-hydroxy-4-(trifluoromethyl)thieno[2,3-b]pyridine-2-carboxylic acid (900 mg, 3.4 mmol, yield: 95%) as a yellow solid. LCMS (ESI) m/z: **264** [M+H]⁺.

Step 4: 6-hydroxy-4-(trifluoromethyl)thieno[2,3-b]pyridine-2-carboxylic acid (600 mg, 2.28 mmol) and cuprous oxide (652 mg, 4.56 mmol) were dissolved in N,N-dimethylformamide (12 mL) solution, and the mixture was stirred at 140°C for 12 h. After completion of the reaction, the system was cooled to room temperature. The reaction solution was poured into water (20 mL). The mixture was extracted with ethyl acetate (20 mL*2). The combined organic phase was washed with saturated salt water, dried over anhydrous sodium sulfate, suction filtered, and concentrated under reduced pressure. The crude product was purified by silica gel column chromatography (dichloromethane:methanol = 85:15) to give 4-(trifluoromethyl)thieno[2,3-b]pyridin-6-ol (300 mg, 1.36 mmol, yield: 60%) as a light yellow solid. LCMS(ESI)m/z: **220**[M+H]+.

Step 5: 4-(trifluoromethyl)thieno[2,3-b]pyridin-6-ol (200 mg, 0.91 mmol) was dissolved in dichloromethane (5 mL), and triethylamine (808 mg, 8 mmol) was added. Triflic anhydride (772 mg, 2.74 mmol) was added to the solution at -45°C. The mixture was stirred at room temperature for 2 h. After completion of the reaction, the reaction solution was poured into water (10 mL) and extracted with dichloromethane (10 mL*2). The organic phases were combined, dried over anhydrous sodium sulfate, filtered, and concentrated under reduced pressure. The crude product was purified by silica gel column chromatography (petroleum ether:ethyl acetate = 85:15) to give 4-(trifluoromethyl)thieno[2,3-b]pyridin-6-yl triflate (240 mg, 0.68 mmol, yield: 75%) as a colorless oil. LCMS(ESI)m/z: **352**[M+H]⁺.

Step 6: 4-(trifluoromethyl)thieno[2,3-b]pyridin-6-yl triflate (100 mg, 0.28 mmol), (S)-tert-butyl 4-((3-chloro-2,4-difluorophenyl)(methyl)carbamoyl)-2-oxoimidazolidine-1-carboxylate **(Intermediate 6,** 110 mg, 0.28 mmol), tris(dibenzylideneacetone)dipalladium (26 mg, 0.03 mmol), 4,5-bis(diphenylphosphine)-9,9-dimethylxanthene (16 mg, 0.03 mmol), and potassium carbonate (117 mg, 0.85 mmol) was dissolved in toluene (3 mL), and the mixture was reacted at 80°C under a nitrogen atmosphere for 2 h. After completion of the reaction, the system was cooled to room temperature. The reaction solution was poured into water (20 mL). The obtained mixture was extracted with ethyl acetate (10 mL x 2). The combined organic layer was washed with saturated salt water and dried over anhydrous sodium sulfate. After filtration, the filtrate was concentrated under reduced pressure. The crude product was purified by silica gel column chromatography (petroleum ether:ethyl acetate = 50:50) to give (S)-tert-butyl 4-((3-chloro-2,4-difluorophenyl)(methyl)carbamoyl)-2-oxo-3-(4-(trifluoromethyl)thieno[2,3-b]pyridin-6-yl)imidazolidine-1-carboxyl ate (100 mg, 0.17 mmol, yield: 59%) as a colorless oil. LCMS (ESI) m/z: **591** [M+H]⁺.

Step 7: (S)-tert-butyl 4-((3-chloro-2,4-difluorophenyl)(methyl)carbamoyl)-2-oxo-3-(4-(trifluoromethyl)thieno[2,3-b]pyridin-6-yl)imidazolidine-1-carboxyl ate (100 mg, 0.17 mmol) was dissolved in trifluoroacetic acid (1 mL) and dichloromethane (5 mL), and the system was stirred at room temperature for 1 h. After completion of the reaction, the obtained mixture was directly concentrated under reduced pressure. The crude product was purified by high-performance liquid chromatography (column: Xselect CSH C18 OBD column 30*150 mm, 5 µm; mobile phase A: 0.05% formic acid in water, mobile phase B: acetonitrile; flow rate: 60 mL/min; gradient: 50% B to 90% B within 8 min; wavelength: 220 nm; retention time: 6.22 min) to give (S)-N-(3-chloro-2,4-difluorophenyl)-N-methyl-2-oxo-3-(4-(trifluoromethyl)thieno[2,3-b]pyridin-6-yl)imidazolidine-4-carboxamide (52.37 mg, 0.11 mmol, yield: 63%) as a white solid. HNMR (400 MHz, DMSO-d₆): δ ppm 8.71-8.66(m,1H), 7.96-7.89(m,1H), 7.82-7.65(m,2H), 7.52-7.41(m,2H), 5.82-4.67(m,1H), 3.58-3.48(m,1H), 3.42-3.37(m,1H),3.23-3.10(m,3H). LCMS (ESI)m/z: **490.95** [M+H]⁺.

### Example 15:

### (S)-N-(3-chloro-2,4-difluorophenyl)-N-methyl-2-oxo-3-(4-(trifluoromethyl)furo[2,3-b]pyridin-6-yl)imidazolidine-4-carboxami de (Compound 15)

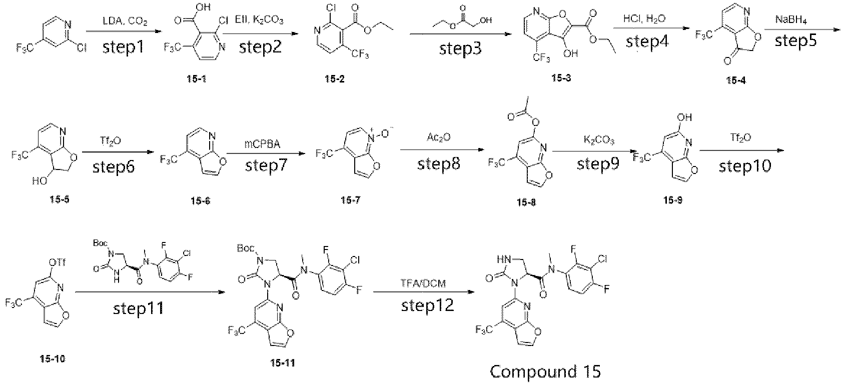

Step 1: 2-chloro-4-(trifluoromethyl)pyridine (20.00 g, 110 mmol) was dissolved in tetrahydrofuran (400 mL) and cooled to -78°C. A solution of lithium diisopropylamide in tetrahydrofuran (110 mL, 220 mmol, 2 mol/L) was added dropwise. After stirring the mixture at -78°C for 2 h, carbon dioxide gas was continuously introduced for 15 min. After completion of the reaction, saturated ammonium chloride solution (200 mL) was added, and the mixture was extracted with ethyl acetate (100 mL*2). The aqueous phase was retained, adjusted to about pH 3 with 2 mol/L aqueous hydrochloric acid solution, and extracted with ethyl acetate (50 mL*3). The organic phase was dried over anhydrous sodium sulfate and filtered. The filtrate was concentrated to give the crude product 2-chloro-4-(trifluoromethyl)nicotinic acid (19.00 g) as a white solid. LCMS (ESI) m/z: **226**[M+H]⁺.

Step 2: 2-chloro-4-(trifluoromethyl)nicotinic acid (19.00 g, 85 mmol) was dissolved in N,N-dimethylformamide (400 mL). Potassium carbonate (23.50 g, 170 mmol) and iodoethane (14.58 g, 93.5 mmol) were added and stirred at room temperature for 6 h. After completion of the reaction, water (800 mL) was added, and the mixture was extracted with ethyl acetate (400 mL*3). The combined organic phase was washed with saturated salt water, dried over anhydrous sodium sulfate, and filtered. The filtrate was concentrated. The obtained crude product was purified by silica gel column chromatography (petroleum ether:ethyl acetate = 70:30) to give ethyl 2-chloro-4-(trifluoromethyl)nicotinate (21.01 g, 83 mmol, yield: 97.6%) as a yellow solid. LCMS (ESI) m/z: **254**[M+H]⁺.

Step 3: ethyl 2-hydroxyacetate (21.61 g, 208 mmol) was dissolved in ethylene glycol dimethyl ether (400 mL) solution and cooled to 0°C. Sodium hydride (6.66 g, 166 mmol, 60% content) was added. The mixture was stirred at 0°C for 1 h and gradually warmed to room temperature. Ethyl 2-chloro-4-(trifluoromethyl)nicotinate (21.01 g, 83 mmol) was then added. The system was warmed to 75°C and stirred for 3 h. After completion of the reaction, the system was cooled to room temperature, and water (300 mL) was added. The mixture was extracted with ethyl acetate (300 mL*3). The organic phase was washed with saturated salt water, dried over anhydrous sodium sulfate, and filtered. The filtrate was concentrated. The obtained crude product was purified by silica gel column chromatography (petroleum ether:ethyl acetate = 55:45) to give ethyl 3-hydroxy-4-(trifluoromethyl)furo[2,3-b]pyridine-2-carboxylate (20.31 g, 73.9 mmol, yield: 89.1%) as a yellow solid. LCMS (ESI) m/z: **276**[M+H]⁺.

Step 4: ethyl 3-hydroxy-4-(trifluoromethyl)furo[2,3-b]pyridine-2-carboxylate (20.31 g, 73.9 mmol) was dissolved in 4 mol/L aqueous hydrochloric acid solution (200 mL). The system was warmed to 100°C and stirred for 12 h. After completion of the reaction, the system was cooled to room temperature, adjusted to pH 8 with saturated aqueous sodium bicarbonate solution, and extracted with ethyl acetate (200 mL*3). The organic phase was washed with saturated salt water, dried over anhydrous sodium sulfate, and filtered. The filtrate was concentrated. The crude product was purified by silica gel column chromatography (petroleum ether:ethyl acetate = 60:40) to give 4-(trifluoromethyl)furo[2,3-b]pyridin-3(2H)-one (4.05 g, 19.95 mmol, yield: 27.1%) as a light yellow oil. LCMS (ESI) m/z: **204**[M+H]⁺.

Step 5: 4-(trifluoromethyl)furo[2,3-b]pyridin-3(2H)-one (4.01 g, 19.7 mmol) was dissolved in methanol (80 mL), and sodium borohydride (1.52 g, 40 mmol) was added. The mixture was stirred at room temperature for 2 h. After completion of the reaction, the reaction solution was slowly added to water (100 mL) with stirring and extracted with ethyl acetate (50 mL*3). The organic phase was washed with saturated salt water, dried over anhydrous sodium sulfate, and filtered. The filtrate was concentrated to give the crude product 4-(trifluoromethyl)-2,3-dihydrofuro[2,3-b]pyridin-3-ol (4.21 g) as a white oil. The crude product was directly used in the next step without purification. LCMS (ESI) m/z: **206**[M+H]⁺.

Step 6: 4-(trifluoromethyl)-2,3-dihydrofuro[2,3-b]pyridin-3-ol (4.02 g, 19.5 mmol) was dissolved in dichloromethane (80 mL). Triflic anhydride (11.02 g, 39 mmol) was added dropwise and stirred at room temperature for 6 h. After completion of the reaction, the reaction solution was poured into water (100 mL) and extracted with dichloromethane (50 mL*2). The combined organic phase was washed with saturated salt water, dried over anhydrous sodium sulfate, and filtered. The filtrate was concentrated to give 4-(trifluoromethyl)furo[2,3-b]pyridine (1.21 g, 6.5 mmol, yield: 33%) as a yellow oil. LCMS (ESI) m/z: **188**[M+H]⁺.

Step 7: 4-(trifluoromethyl)furo[2,3-b]pyridine (0.80 g, 4.28 mmol) was dissolved in dichloromethane (10.00 mL), and m-chloroperoxybenzoic acid (1.56 g, 8.56 mmol) was added. The mixture was stirred at 40°C for 36 h. After completion of the reaction, saturated aqueous sodium bicarbonate solution (20 mL) was added, and the mixture was extracted with dichloromethane (10 mL*2). The combined organic phase was washed with saturated salt water, dried over anhydrous sodium sulfate, and filtered. The filtrate was concentrated under reduced pressure. The obtained crude product was purified by silica gel column chromatography (dichloromethane:methanol = 90:10) to give 4-(trifluoromethyl)furo[2,3-b]pyridine-7-oxide (0.40 g, yield: 46%, 1.9 mmol) as a brown solid. LCMS (ESI) m/z: **204**[M+H]⁺.

Step 8: 4-(trifluoromethyl)furo[2,3-b]pyridine-7-oxide (300 mg, 1.48 mmol) was dissolved in acetic anhydride (6.00 mL). The mixture was stirred at 100°C for 1 h. After completion of the reaction, the reaction solution was directly concentrated under reduced pressure. The obtained crude product was purified by reverse phase chromatography (10% ammonium bicarbonate aqueous solution: acetonitrile = 50:50) to give 4-(trifluoromethyl)furo[3,2-e]pyridin-6-yl acetate (120 mg, 0.48 mmol, yield: 33%) as a yellow oil. LCMS (ESI) m/z: **246**[M+H]⁺.

Step 9: 4-(trifluoromethyl)furo[3,2-e]pyridin-6-yl acetate (120 mg, 0.48 mmol) and potassium carbonate (202 mg, 1.47 mmol) were dissolved in ethanol (5 mL) and water (0.50 mL). The mixture was stirred at room temperature for 1 h. After completion of the reaction, the mixture was extracted with dichloromethane (10 mL*2). The combined organic layer was dried over anhydrous sodium sulfate and filtered. The filtrate was concentrated. The obtained crude product was purified by silica gel column chromatography (ethyl acetate:petroleum ether = 50:50) to give 4-(trifluoromethyl)furo[2,3-b]pyridin-6-ol (55 mg, 0.26 mmol, yield: 55%) as a yellow oil. LCMS (ESI) m/z: **204**[M+H]⁺.

Step 10: 4-(trifluoromethyl)furo[2,3-b]pyridin-6-ol (50 mg, 0.25 mmol) was dissolved in dichloromethane (2.00 mL) at -45°C, and triflic anhydride (104 mg, 0.37 mmol) and triethylamine (49 mg, 0.49 mmol) were added. The mixture was stirred at room temperature for 2 h. After completion of the reaction, the reaction solution was poured into water (10 mL) and extracted with dichloromethane (5 mL*3). The combined organic layer was dried over anhydrous sodium sulfate. After filtration, the filtrate was concentrated under reduced pressure. The obtained crude product was purified by silica gel column chromatography (ethyl acetate:petroleum ether = 75:25) to give 4-(trifluoromethyl)furo[2,3-e]pyridin-6-yl triflate (70 mg, 0.21 mmol, yield: 85%) as a yellow solid. LCMS (ESI) m/z: **336**[M+H]⁺.

Step 10: 4-(trifluoromethyl)furo[2,3-e]pyridin-6-yl triflate (70 mg, 0.21 mmol), (S)-tert-butyl 4-((3-chloro-2,4-difluorophenyl)(methyl)carbamoyl)-2-oxoimidazolidine-1-carboxylate **(Intermediate 6,** 65 mg, 0.17 mmol), tris(dibenzylideneacetone)dipalladium (19 mg, 0.02 mmol), 4,5-bis(diphenylphosphine)-9,9-dimethylxanthene (12 mg, 0.02 mmol), and potassium carbonate (86 mg, 0.63 mmol) was dissolved in dioxane (3 mL) solution, and the mixture was reacted at 80°C under a nitrogen atmosphere for 2 h. After completion of the reaction, the system was cooled to room temperature. The reaction solution was poured into water (10 mL) and extracted with ethyl acetate (5 mL*3). The combined organic layer was dried over anhydrous sodium sulfate and filtered. The filtrate was concentrated. The crude product was purified by silica gel column chromatography (ethyl acetate:petroleum ether = 25:75) to give (S)-tert-butyl 4-((3-chloro-2,4-difluorophenyl)(methyl)carbamoyl)-2-oxo-3-(4-(trifluoromethyl)furo[2,3-b]pyridin-6-yl)imidazolidine-1-carboxylat e (55 mg, 0.09 mmol, yield: 46%) as a yellow oil. LCMS (ESI) m/z: **575**[M+H]⁺.

Step 12: (S)-tert-butyl 4-((3-chloro-2,4-difluorophenyl)(methyl)carbamoyl)-2-oxo-3-(4-(trifluoromethyl)furo[2,3-b]pyridin-6-yl)imidazolidine-1-carboxylat e (50 mg, 0.09 mmol) was dissolved in trifluoroacetic acid (1 mL) and dichloromethane (5 mL), and the system was stirred at room temperature for 1 h. After completion of the reaction, the obtained mixture was directly concentrated under reduced pressure. The crude product was purified by high-performance liquid chromatography (column: Kinetex EVO prep C18, 30*150, 5 µm; mobile phase A: 10 mmol/L ammonium bicarbonate aqueous solution, mobile phase B: acetonitrile; flow rate: 60 mL/min; gradient: 25% B to 65% B within 10 min; wavelength: 220 nm; retention time: 9.03 min) to give (S)-N-(3-chloro-2,4-difluorophenyl)-N-methyl-2-oxo-3-(4-(trifluoromethyl)furo[2,3-b]pyridin-6-yl)imidazolidine-4-carboxamide (12.35 mg, 0.03 mmol, yield: 30%) as a white solid. HNMR (400 MHz, DMSO-d₆): δ ppm 8.64(s,1H), 8.04-8.01(m,1H), 7.68(s,1H), 7.26-7.20(m, 1H), 6.85(s, 1H), 5.04-4.90(m, 2H), 3.62-3.47 (m, 2H),3.30(s,3H). LCMS (ESI) m/z:**474.80**[M+H]⁺.

### Example 16:

### (2S,3S,4S)-N-(5-chloro-2,4-difluorophenyl)-3,4-dihydroxy-N-(methyl-D3)-5-oxo-1-(4-(trifluoromethyl)thieno[2,3-b]pyridin-6-yl)py rrolidine-2-carboxamide (Compound 16)

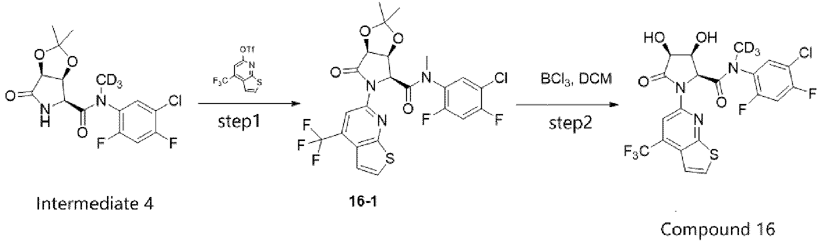

### Step 1:

(3aS,4S,6aS)-N-(5-chloro-2,4-difluorophenyl)-2,2-dimethyl-N-(methyl-D3)-6-oxotetrahydro-4H-[1,3]dioxolo[4,5-c]pyrrole-4-carbox amide (**Intermediate 4,** 90 mg, 0.24 mmol), 4-(trifluoromethyl)thieno[2,3-b]pyridin-6-yl triflate (**14-5**, 84 mg, 0.24 mmol), potassium carbonate (99 mg, 0.72 mmol), and 4,5-bis(diphenylphosphine)-9,9-dimethylxanthene (14 mg, 0.024 mmol) were dissolved in toluene (5 mL), and then tris(dibenzylideneacetone)dipalladium (22 mg, 0.024 mmol) was added. The mixture was reacted at 80°C under a nitrogen atmosphere for 2 h. After completion of the reaction, the reaction solution was poured into water (10 mL) and extracted with ethyl acetate (10 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 50:50) to give (3aS,4S,6aS)-N-(5-chloro-2,4-difluorophenyl)-N,2,2-trimethyl-6-oxo-5-(4-(trifluoromethyl)thieno[2,3-b]pyridin-6-yl)tetrahydro-4H-[1,3]dioxolo[4,5-c]pyrrole-4-carboxamide (100 mg, 0.19 mmol, yield: 79.16%) as a yellow solid. LCMS(ESI)m/z: **562**[M+H]⁺.

### Step 2:

(3aS,4S,6aS)-N-(5-chloro-2,4-difluorophenyl)-N,2,2-trimethyl-6-oxo-5-(4-(trifluoromethyl)thieno[2,3-b]pyridin-6-yl)tetrahydro-4H-[1,3]dioxolo[4,5-c]pyrrole-4-carboxamide (100 mg, 0.19 mmol) was dissolved in dichloromethane (5 mL), and then a solution of boron trichloride in dichloromethane (0.28 mL, 0.28 mmol, 1 mol/L) was added. The mixture was reacted at 0°C for 1 h. After completion of the reaction, the reaction solution was poured into saturated sodium bicarbonate solution (10 mL) and extracted with dichloromethane (10 mL x 3). The organic phases were combined, washed with saturated salt water, dried over anhydrous sodium sulfate, and filtered to remove anhydrous sodium sulfate. The filtrate was concentrated to give a crude product. The crude product was purified by high-performance liquid chromatography (column: XBridge Prep OBD C18, 30 x 150 mm, 5 um, mobile phase A: 10 mmol/L ammonium bicarbonate aqueous solution, mobile phase B: acetonitrile, flow rate: 60 mL/min, elution gradient: mobile phase B increased from 20% to 60% within 10 min, detection wavelength: 220 nm, retention time: 8.12 min) to give (2S,3S,4S)-N-(5-chloro-2,4-difluorophenyl)-3,4-dihydroxy-N-(methyl-D3)-5-oxo-1-(4-(trifluoromethyl)thieno[2,3-b]pyridin-6-yl)py rrolidine-2-carboxamide (26.36 mg, 0.05 mmol, yield: 26.31%) as a white solid. HNMR (400 MHz, DMSO-d₆): δ ppm 8.56-8.53(s ,1H), 8.11-8.04(m ,1H), 7.95-7.54(m, 2H), 7.51-7.49(m, 1H), 5.84-5.62(m, 2H), 5.30-4.93(m,1H), 4.83-4.03(m,2H). LCMS (ESI) m/z: **525.30** [M+H]⁺.

### Example 17:

### (S)-N-(3-chloro-2,4-difluorophenyl)-N-methyl-2-oxo-3-(4-(trifluoromethyl)quinolin-2-yl)imidazolidine-4-carboxamide (Compound 17)

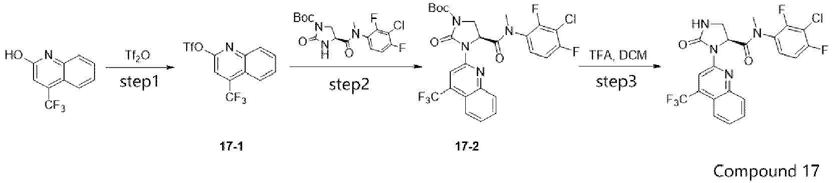

Step 1: to a solution of 4-(trifluoromethyl)quinolin-2-ol (1.00 g, 4.69 mmol) in dichloromethane (10.00 mL) was added triflic anhydride (1.59 g, 5.63 mmol), pyridine (0.74 g, 9.38 mmol), and 4-dimethylaminopyridine (0.06 g, 0.47 mmol) at 0°C. The mixture was stirred at room temperature for 3 h. After completion of the reaction, the mixture was poured into water (20 mL) and extracted with dichloromethane (10 mL x 2). The organic layers were combined, washed with saturated salt water, dried over anhydrous sodium sulfate, suction filtered, and concentrated to give a crude product. The crude product was purified by silica gel column chromatography (petroleum ether:ethyl acetate = 80:20) to give 4-(trifluoromethyl)quinolin-2-yl triflate (0.98 g, 2.8 mmol, yield: 61%) as a white solid. LCMS (ESI)m/z: **346**[M+H]⁺.

Step 2: to a solution of 4-(trifluoromethyl)quinolin-2-yl triflate (200 mg, 0.58 mmol) in dioxane (5.00 mL) was added (S)-tert-butyl 4-((3-chloro-2,4-difluorophenyl)(methyl)carbamoyl)-2-oxoimidazolidine-1-carboxylate **(Intermediate 6,** 225 mg, 0.58 mmol), potassium carbonate (159 mg, 1.16 mmol), tris(dibenzylideneacetone)dipalladium (53 mg, 0.06 mmol), and 1,1'-bis(diphenylphosphino)ferrocene (320 mg, 0.06 mmol). The mixture was stirred at 80°C under a nitrogen atmosphere for 2 h. After completion of the reaction, the mixture was poured into water (20 mL) and extracted with ethyl acetate (10 mL x 2). The organic layers were combined, washed with saturated salt water, dried over anhydrous sodium sulfate, filtered, and concentrated to give a crude product. The crude product was purified by silica gel column chromatography (petroleum ether:ethyl acetate = 60:40) to give (S)-tert-butyl 4-((3-chloro-2,4-difluorophenyl)(methyl)carbamoyl)-2-oxo-3-(4-(trifluoromethyl)quinolin-2-yl)imidazolidine-1-carboxylate (160 mg, 0.27 mmol, yield: 47%) as a white solid. LCMS(ESI)m/z: **585**[M+H]⁺.

Step 3: (S)-tert-butyl 4-((3-chloro-2,4-difluorophenyl)(methyl)carbamoyl)-2-oxo-3-(4-(trifluoromethyl)quinolin-2-yl)imidazolidine-1-carboxylate (22 mg, 0.17 mmol) was added to a mixed solution of trifluoroacetic acid (1.00 mL) and dichloromethane (5.00 mL), and the system was stirred at room temperature for 2 h. After completion of the reaction, the system was directly concentrated under reduced pressure. The crude product was purified by high-performance liquid chromatography (column: XBridge Prep OBD C18, 30 x 150 mm, 5 um, mobile phase A: 10 mmol/L ammonium bicarbonate aqueous solution, mobile phase B: acetonitrile, flow rate: 60 mL/min, elution gradient: mobile phase B increased from 30% to 60% within 10 min, detection wavelength: 220 nm, retention time: 9.21 min) to give (S)-N-(3-chloro-2,4-difluorophenyl)-N-methyl-2-oxo-3-(4-(trifluoromethyl)quinolin-2-yl)imidazolidine-4-carboxamide (22.78 mg, 0.05 mmol, yield: 28%) as a white solid. HNMR (400 MHz, DMSO-d₆): δ ppm 9.00-8.94(m ,1H), 8.05-7.81(m,4H), 7.79-7.49(m,3H), 5.86-4.97(m,1H), 3.96-3.58(m,1H), 3.56-3.38(m, 1H), 3.36-3.16(m, 3H). LCMS (ESI) m/z:**485.00** [M+H]⁺.

### Example 18:

### (S)-N-(3-chloro-2,4-difluorophenyl)-N-methyl-3-(1-methyl-4-(trifluoromethyl)-1H-pyrrolo[2,3-b]pyridin-6-yl)-2-oxoimidazoli dine-4-carboxamide (Compound 18)

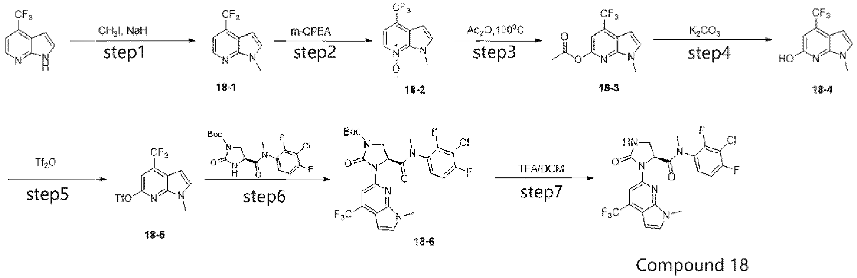

Step 1: to a solution of 4-(trifluoromethyl)-1H-pyrrolo[2,3-b]pyridine (820 mg, 4.40 mmol) in N,N-dimethylformamide (10.00 mL) was added sodium hydride (440 mg, 11.00 mmol, 60% content) and iodomethane (687 mg, 4.84 mmol) at 0°C. The mixture was stirred at room temperature for 2 h. After completion of the reaction, the mixture was poured into water (20 mL) and extracted with ethyl acetate (20 mL x 2). The organic layers were combined, washed with saturated salt water, dried over anhydrous sodium sulfate, suction filtered, and concentrated. The crude product was purified by silica gel chromatography column (petroleum ether:ethyl acetate = 80:20) to give 1-methyl-4-(trifluoromethyl)-1H-pyrrolo[2,3-b]pyridine (450 mg, 2.25 mmol, yield: 51%) as a yellow solid. LCMS (ESI)m/z:**201**[M+H]⁺.

Step 2: to a solution of the 1-methyl-4-(trifluoromethyl)-1H-pyrrolo[2,3-b]pyridine (200 mg, 1.00 mmol) in dichloromethane (5 mL) was added m-chloroperoxybenzoic acid (860 mg, 5.00 mmol). The mixture was stirred at room temperature overnight. After completion of the reaction, the mixture was quenched with saturated sodium sulfite solution (10 mL) and saturated sodium bicarbonate solution (10 mL) and extracted with dichloromethane (20 mL x 2). The organic layers were combined, washed with saturated salt water, dried over anhydrous sodium sulfate, and concentrated. The crude product was purified by silica gel column chromatography (petroleum ether:ethyl acetate = 60:40) to give 1-methyl-4-(trifluoromethyl)-1H-pyrrolo[2,3-b]pyridine 7-oxide (66 mg, 0.30 mmol, yield: 30%) as a white solid. LCMS(ESI)m/z: **217**[M+H]⁺.

Step 3: 1-methyl-4-(trifluoromethyl)pyrrolo[2,3-b]pyridin-7-one (300 mg, 1.39 mmol) was added to acetic anhydride (6.00 mL) at room temperature. The mixture was stirred at 100°C for 1 h. After completion of the reaction, the system was concentrated under reduced pressure. The obtained crude product was purified by reverse phase chromatography (acetonitrile:5% ammonium bicarbonate aqueous solution = 50:50) to give 1-methyl-4-(trifluoromethyl)-1H-pyrrolo[2,3-b]pyridin-6-yl acetate (150 mg, 0.58 mmol, yield: 42%) as a yellow oil. LCMS (ESI) m/z: **259**[M+H]+.

Step 4: 1-methyl-4-(trifluoromethyl)-1H-pyrrolo[2,3-b]pyridin-6-yl acetate (140 mg, 0.54 mmol) and potassium carbonate (224 mg, 1.63 mmol) were dissolved in ethanol (5.00 mL) and water (0.50 mL). The mixture was stirred at room temperature for 1 h. After completion of the reaction, the system was extracted with dichloromethane (10 mL*2). The combined organic layer was dried over anhydrous sodium sulfate and filtered. The filtrate was concentrated. The crude product was purified by silica gel column chromatography (ethyl acetate:petroleum ether = 50:50) to give 1-methyl-4-(trifluoromethyl)-1H-pyrrolo[2,3-b]pyridin-6-ol (110 mg, 0.50 mmol, yield: 94%) as a yellow oil. LCMS (ESI) m/z: **217**[M+H]+.

Step 5: 1-methyl-4-(trifluoromethyl)-1H-pyrrolo[2,3-b]pyridin-6-ol (110 mg, 0.51 mmol) was dissolved in dichloromethane (2 mL) at 0°C, and triflic anhydride (287 mg, 1.02 mmol) and triethylamine (103 mg, 1.02 mmol) were added. The mixture was stirred at room temperature for 2 h. After completion of the reaction, the reaction solution was poured into water (10 mL) and extracted with dichloromethane (5 mL*3). The combined organic layer was dried over anhydrous sodium sulfate and filtered. The filtrate was concentrated under reduced pressure. The obtained crude product was purified by silica gel column chromatography (ethyl acetate:petroleum ether = 20:80) to give 1-methyl-4-(trifluoromethyl)-1H-pyrrolo[2,3-b]pyridin-6-yl triflate (70 mg, 0.19 mmol, yield; 39%) as a yellow solid. LCMS (ESI) m/z: **349**[M+H]+.

Step 6: 1-methyl-4-(trifluoromethyl)-1H-pyrrolo[2,3-b]pyridin-6-yl triflate (35 mg, 0.10 mmol), (S)-4-((3-chloro-2,4-difluorophenyl)(methyl)carbamoyl)-2-oxoimidazolidine-1-carboxylate **(Intermediate 6,** 47 mg, 0.12 mmol), tris(dibenzylideneacetone)dipalladium (9 mg, 0.01 mmol), 4,5-bis(diphenylphosphine)-9,9-dimethylxanthene (5 mg, mmol), and potassium carbonate (41 mg, 0.30 mmol) were dissolved in dioxane (3 mL)solution, and the system was reacted at 80°C under a nitrogen atmosphere for 2 h. After completion of the reaction, the reaction solution was poured into water (10 mL) and extracted with ethyl acetate (3 mL*3). The combined organic layer was dried over anhydrous sodium sulfate. After filtration, the filtrate was concentrated under reduced pressure. The crude product was purified by silica gel column chromatography (ethyl acetate:petroleum ether = 50:50) to give (S)-tert-butyl 4-((3-chloro-2,4-difluorophenyl)(methyl)carbamoyl)-3-(1-methyl-4-(trifluoromethyl)-1H-pyrrolo[2,3-b]pyridin-6-yl)-2-oxoimidazoli dine-1-carboxylate (30 mg, 0.051 mmol, yield: 50%) as a yellow oil. LCMS (ESI) m/z: **588**[M+H]+.

Step 7: (S)-tert-butyl 4-((3-chloro-2,4-difluorophenyl)(methyl)carbamoyl)-3-(1-methyl-4-(trifluoromethyl)-1H-pyrrolo[2,3-b]pyridin-6-yl)-2-oxoimidazoli dine-1-carboxylate (30 mg, 0.05 mmol) was dissolved in a solution of trifluoroacetic acid (1 mL) and dichloromethane (5 mL), and the system was stirred at room temperature for 1 h. After completion of the reaction, the obtained mixture was directly concentrated under reduced pressure. The crude product was purified by high-performance liquid chromatography (column: Kinetex EVO prep C18, 30*150, 5 µm; mobile phase A: 10 mmol/L ammonium bicarbonate aqueous solution, mobile phase B: acetonitrile; flow rate: 60 mL/min; gradient: 25% B to 65% B within 10 min; wavelength: 220 nm; retention time: 9.12 min) to give (S)-N-(3-chloro-2,4-difluorophenyl)-N-methyl-3-(1-methyl-4-(trifluoromethyl)-1H-pyrrolo[2,3-b]pyridin-6-yl)-2-oxoimidazolidine-4 -carboxamide (1.79 mg, 0.003 mmol, yield: 7%) as a white solid. HNMR (400 MHz, DMSO-d6): δ ppm 8.37-8.42(s,1H), 7.60(m,1H), 7.14(s,1H), 7.05(m,1H), 6.56(s,1H), 5.22(m,1H), 4.71(m,1H), 3.83-3.87(m,3H), 3.44-3.63(m,2H), 3.27-3.30(m,3H). LCMS(ESI)m/z: **487.80**[M+H]+.

### Example 19:

### (2S,3S,4S)-N-(5-chloro-2,4-difluorophenyl)-3,4-dihydroxy-N-(methyl-D3)-5-oxo-1-(4-(trifluoromethyl)-7H-cyclopenta[b]pyrid in-2-yl)pyrrolidine-2-carboxamide (Compound 19)

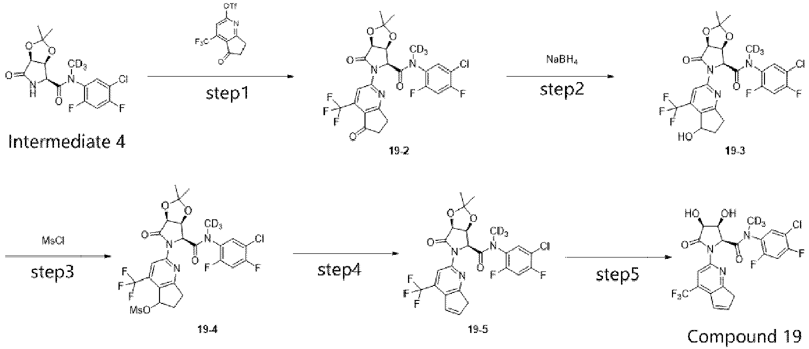

### Step 1:

(3aS,4S,6aS)-N-(5-chloro-2,4-difluorophenyl)-2,2-dimethyl-N-(methyl-D3)-6-oxotetrahydro-4H-[1,3]dioxolo[4,5-c]pyrrole-4-carbox amide (**Intermediate 4,** 100 mg, 0.27 mol), 5-oxo-4-(trifluoromethyl)-6,7-dihydro-5H-cyclopenta[b]pyridin-2-yl triflate (112 mg, 0.32 mol), potassium carbonate (75 mg, 0.54 mol), and 1,1'-bis(diphenylphosphino)ferrocene (11 mg, 0.02 mol) was dissolved in dioxane (2 mL), and then tris(dibenzylideneacetone)dipalladium (18 mg, 0.02 mol) was added. The system was replaced with nitrogen three times and reacted at 80°C for about 5 h. After completion of the reaction, the reaction solution was poured into saturated aqueous sodium chloride solution (20 mL) and extracted with ethyl acetate (10 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 50:50) to give

(3aS,4S,6aS)-N-(5-chloro-2,4-difluorophenyl)-2,2-dimethyl-N-(methyl-D3)-6-oxo-5-(5-oxo-4-(trifluoromethyl)-6,7-dihydro-5H-cycl openta[b]pyridin-2-yl)tetrahydro-4H-[1,3]dioxolo[4,5-c]pyrrole-4-carboxamide (110 mg, 0.19 mmol, yield: 70%) as a yellow solid. LCMS (ESI): 563 [M+H]⁺.

Step 2: (3aS, 4S, 6aS)-N-(5-chloro-2,4-difluorophenyl)-2,2-dimethyl-N-(methyl-D3)-6-oxo-5-(5-oxo-4-(trifluoromethyl)-6,7-dihydro-5H-cyclopenta[b ]pyridin-2-yl)tetrahydro-4H-[1,3]dioxolo[4,5-c]pyrrole-4-carboxamide (110 mg, 0.19 mmol) and sodium borohydride (7 mg, 0.19 mmol) were dissolved in anhydrous methanol solution (5 mL) and reacted at 0°C for 5 h. After completion of the reaction, the reaction solution was poured into water (5 mL) and extracted with ethyl acetate (5 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 60:40) to give
(3aS,4S,6aS)-N-(5-chloro-2,4-difluorophenyl)-5-(5-hydroxy-4-(trifluoromethyl)-6,7-dihydro-5H-cyclopenta[b]pyridin-2-yl)-2,2-dim ethyl-N-(methyl-D3)-6-oxotetrahydro-4H-[1,3]dioxolo[4,5-c]pyrrole-4-carboxamide (60 mg, 0.10 mmol, yield: 52%) as a yellow solid. LCMS (ESI): 565 [M+H]⁺.

### Step 3:

(3aS,4S,6aS)-N-(5-chloro-2,4-difluorophenyl)-5-(5-hydroxy-4-(trifluoromethyl)-6,7-dihydro-5H-cyclopenta[b]pyridin-2-yl)-2,2-dim ethyl-N-(methyl-D3)-6-oxotetrahydro-4H-[1,3]dioxolo[4,5-c]pyrrole-4-carboxamide (110 mg, 0.19 mmol), methanesulfonyl chloride (43 mg, 0.38 mmol), and triethylamine (57 mg, 0.57 mmol) were dissolved in dichloromethane solution (2 mL) and reacted at room temperature for 5 h. After completion of the reaction, the reaction solution was poured into water (10 mL) and extracted with dichloromethane (10 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 40:60) to give 2-((3aS,4S,6aS)-4-((5-chloro-2,4-difluorophenyl)(methyl-d3)carbamoyl)-2,2-dimethyl-6-oxotetrahydro-SH-[1,3]dioxolo[4,5-c]pyrrol -5-yl)-4-(trifluoromethyl)-6,7-dihydro-5H-cyclopenta[b]pyridin-5-yl methanesulfonate (45 mg, 0.07 mmol, yield: 36.8%) as a yellow solid. LCMS (ESI): 643 [M+H]⁺.

### Step 4:

2-((3aS,4S,6aS)-4-((5-chloro-2,4-difluorophenyl)(methyl-d3)carbamoyl)-2,2-dimethyl-6-oxotetrahydro-5H-[1,3]dioxolo[4,5-c]pyrrol -5-yl)-4-(trifluoromethyl)-6,7-dihydro-5H-cyclopenta[b]pyridin-5-yl methanesulfonate (45 mg, 0.07 mmol) and triethylamine (14 mg, 0.14 mmol) were dissolved in dichloromethane (2 mL) and reacted at 40°C for 2 h. After completion of the reaction, the reaction solution was poured into water (10 mL) and extracted with dichloromethane (5 mL x 3). The organic phases were combined, washed with saturated salt water, dried over anhydrous sodium sulfate, and filtered to remove anhydrous sodium sulfate. The filtrate was concentrated to give the crude product (3aS,4S,6aS)-N-(5-chloro-2,4-difluorophenyl)-5-(5-hydroxy-4-(trifluoromethyl)-6,7-dihydro-5H-cyclopenta[b]pyridin-2-yl)-2,2-dim ethyl-N-(methyl-D3)-6-oxotetrahydro-4H-[1,3]dioxolo[4,5-c]pyrrole-4-carboxamide (60 mg) as a white solid, which was directly used in the next step without purification. LCMS (ESI) m/z: 547[M+H]⁺.

### Step 5:

(3aS,4S,6aS)-N-(5-chloro-2,4-difluorophenyl)-5-(5-hydroxy-4-(trifluoromethyl)-6,7-dihydro-5H-cyclopenta[b]pyridin-2-yl)-2,2-dim ethyl-N-(methyl-D3)-6-oxotetrahydro-4H-[1,3]dioxolo[4,5-c]pyrrole-4-carboxamide (60 mg, 0.109 mmol) was dissolved in dichloromethane (5 mL) and then cooled to -20°C. Boron trichloride (0.22 mL, 0.22 mmol, 1 mol/L in dichloromethane) was added, and the reaction system was reacted at room temperature for 1 h. After completion of the reaction, the reaction solution was poured into water (10 mL) and extracted with dichloromethane (5 mL x 3). The organic phases were combined, washed with saturated salt water, dried over anhydrous sodium sulfate, and filtered to remove anhydrous sodium sulfate. The filtrate was concentrated to give a crude product. The crude product was purified by high-performance liquid chromatography (column: XBridge Prep OBD C18, 30 x 150 mm, 5 um, mobile phase A: 10 mmol/L ammonium bicarbonate aqueous solution, mobile phase B: acetonitrile, flow rate: 60 mL/min, elution gradient: mobile phase B increased from 35% to 70% within 10 min, detection wavelength: 220 nm, retention time: 9.15 min) to give (25,3 S,4S)-N-(5-chloro-2,4-difluorophenyl)-3,4-dihydroxy-N-(methyl-D3)-5-oxo-1-(4-(trifluoromethyl)-7H-cyclopenta[b]pyridin-2-yl)pyrrolidine-2-carboxamide (40 mg, 0.07 mmol, yield: 64.2%) as a white solid. LCMS (ESI) m/z: 507.05[M+H]⁺, HNMR (400 MHz, DMSO-*d₆*): δ ppm 8.33-8.14 (m, 1H), 8.11-7.50 (m, 2H), 7.31-7.12 (m, 1H), 7.11-6.83 (m, 1H), 5.80-5.48 (m, 2H),5.20-4.72 (m, 1H), 4.50-4.18 (m, 1H), 4.15-4.01 (m, 1H), 3.76-3.60 (m, 2H).

### Example 20:

### (S)-N-(3-chloro-2,4-difluorophenyl)-N-methyl-2-oxo-3-(4-(trifluoromethyl)-2,3-dihydrofuro[2,3-b]pyridin-6-yl)imidazolidine-4-carboxamide (Compound 20)

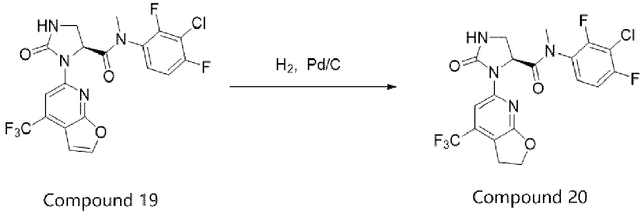

(S)-N-(3-Chloro-2,4-difluorophenyl)-N-methyl-2-oxo-3-(4-(trifluoromethyl)furo[2,3-b]pyridin-6-yl)imidazolidine-4-carboxamide **(Compound 15)** (10 mg, 0.0209 mmol) was dissolved in ethanol (3 mL), and then rhodium on carbon (25 mg) was added. The reaction system was replaced with hydrogen three times and stirred at room temperature under a hydrogen atmosphere (4 atm) for 4 h. After completion of the reaction, the mixture was filtered. The filtrate was concentrated. The crude product was purified by high-performance liquid chromatography (column: Kinetex EVO C18 column, 30*150, 5 um; mobile phase A: 10 mmol/L ammonium bicarbonate aqueous solution; mobile phase B: acetonitrile; flow rate: 60 mL/min; gradient: 20% B to 60% B within 10 min; wavelength: 220 nm; retention time: 9.48 min) to give (S)-N-(3-chloro-2,4-difluorophenyl)-N-methyl-2-oxo-3-(4-(trifluoromethyl)-2,3-dihydrofuro[2,3-b]pyridin-6-yl)imidazolidine-4-carb oxamide (4.47 mg, 0.0093 mmol, yield: 44.4%) as a white solid. LCMS (ESI) m/z: 476.75[M+H]⁺, HNMR (400 MHz, CDCl₃): δ ppm 8.06(s, 1H), 7.97-7.88(m, 1H), 7.21-7.15 (m, 1H), 4.90-4.80 (m, 2H), 4.75-4.65(m, 2H), 3.61-3.50(m, 2H), 3.41-3.34(m,2H), 3.29(s,3H).

### Example 21:

### (S)-N-(3-chloro-2,4-difluorophenyl)-N-methyl-2-oxo-3-(4-(trifluoromethyl)-5H-cyclopenta[b]pyridin-2-yl)imidazolidine-4-car boxamide (Compound 21)

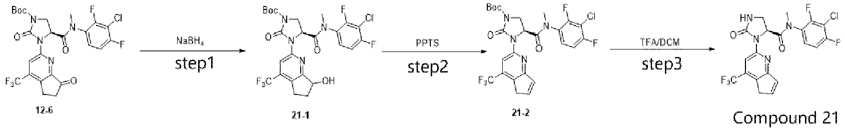

Step 1: (S)-tert-butyl 4-((3-chloro-2,4-difluorophenyl)(methyl)carbamoyl)-2-oxo-3-(7-oxo-4-(trifluoromethyl)-6,7-dihydro-5H-cyclopenta[b]pyridin-2-yl)i midazolidine-1-carboxylate (50 mg, 0.085 mmol) was dissolved in methanol (1 mL), and sodium borohydride (3.21 mg, 0.085 mmol) was added at 0°C. The reaction system was stirred at room temperature for 1 h. After completion of the reaction, the reaction was quenched with water (1 mL). The obtained mixture was concentrated under reduced pressure to give the crude product tert-butyl (4S)-4-((3-chloro-2,4-difluorophenyl)(methyl)carbamoyl)-3-(7-hydroxy-4-(trifluoromethyl)-6,7-dihydro-5H-cyclopenta[b]pyridin-2-yl)-2-oxoimidazolidine-1-carboxylate (45 mg) as a white solid. LCMS (ESI) m/z: 591[M+H] ⁺.

Step 2: tert-butyl (4S)-4-((3-chloro-2,4-difluorophenyl)(methyl)carbamoyl)-3-(7-hydroxy-4-(trifluoromethyl)-6,7-dihydro-5H-cyclopenta[b]pyridin-2-yl)-2-oxoimidazolidine-1-carboxylate (45 mg, 0.076 mmol) was dissolved in dichloroethane (2 mL), and pyridinium p-toluenesulfonate (38 mg, 0.15 mmol) was added. The reaction system was stirred at 80°C for 3 h. After completion of the reaction, the system was poured into water (10 mL) and extracted with dichloromethane (10 mL*2). The organic phases were combined, washed with saturated salt water, dried over anhydrous sodium sulfate, and filtered. The filtrate was concentrated. The residue was purified by silica gel column chromatography (petroleum ether:ethyl acetate = 75:25) to give (S)-tert-butyl 4-((3-chloro-2,4-difluorophenyl)(methyl)carbamoyl)-2-oxo-3-(4-(trifluoromethyl)-5H-cyclopenta[b]pyridin-2-yl)imidazolidine-1-car boxylate (40 mg, 0.069 mmol, yield: 90.7%) as a yellow oil. LCMS (ESI) m/z: 573[M+H] ⁺.

Step 3: (S)-tert-butyl 4-((3-chloro-2,4-difluorophenyl)(methyl)carbamoyl)-2-oxo-3-(4-(trifluoromethyl)-5H-cyclopenta[b]pyridin-2-yl)imidazolidine-1-car boxylate (40 mg, 0.069 mmol) was dissolved in trifluoroacetic acid (0.20 mL) and dichloromethane (0.50 mL). The reaction system was reacted at room temperature for 2 h. After completion of the reaction, the system was directly concentrated under reduced pressure. The crude product was purified by high-performance liquid chromatography (column: Kinetex EVO C18 column, 30*150, 5 um; mobile phase A: 10 mmol/L ammonium bicarbonate aqueous solution, mobile phase B: acetonitrile; flow rate: 60 mL/min; gradient: 35% B to 60% B within 10 min; wavelength: 220 nm; retention time: 8.55 min) to give (S)-N-(3-chloro-2,4-difluorophenyl)-N-methyl-2-oxo-3-(4-(trifluoromethyl)-5H-cyclopenta[b]pyridin-2-yl)imidazolidine-4-carboxa mide (3.34 mg, 0.0070 mmol, yield: 10.1%) as a white solid. LCMS (ESI) m/z: 472.70[M+H]⁺, HNMR (400 MHz, DMSO-d6): δ ppm 8.53-8.20(m, 1H), 8.00-7.64(m, 1H), 7.64-7.32(m, 2H), 7.32-7.09(s,1H), 7.08-6.73(m,1H), 5.75-4.40(m,1H), 3.88-3.60(m,2H), 3.55-3.50(m,1H), 3.45-3.40(m,1H), 3.22-3.10 (m,3H).

### Example 22:

### (S)-N-(3-chloro-2,4-difluorophenyl)-N-methyl-2-oxo-3-(4-(trifluoromethyl)pyrrolo[1,2-a]pyrimidin-2-yl)imidazolidine-4-carbo xamide (Compound 22)

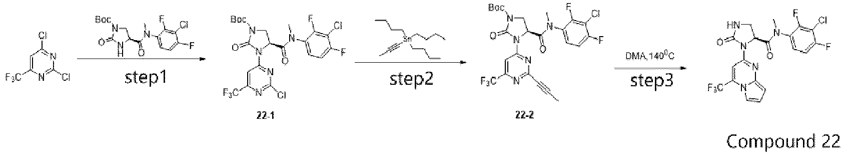

Step 1: to a solution of 2,4-dichloro-6-(trifluoromethyl) pyrimidine (200 mg, 0.92 mmol) in dichloromethane (5.00 mL) was added tert-butyl (4S)-4-[N-(3-chloro-2,4-difluorophenyl)-N-methylcarbamoyl]-2-oxoimidazolidine carboxylate (179 mg, 0.46 mmol), tris(dibenzylideneacetone)dipalladium (84 mg, 0.09 mmol), and 4,5-bis(diphenylphosphine)-9,9-dimethylxanthene (53 mg, 0.09 mmol), and the reaction system was replaced with nitrogen three times. The obtained mixture was stirred at 80°C for 1 h. The reaction showed 80% product by LC-MS on a TLC plate (Rf = 0.6, petroleum ether:ethyl acetate = 1:1). The reaction solution was poured into water (10 mL) and extracted with dichloromethane (10 mL x 2). The combined organic layer was dried over anhydrous sodium sulfate. After filtration, the filtrate was concentrated under a vacuum. The residue was purified by silica gel column chromatography (ethyl acetate:petroleum ether = 20:80) to give (S)-tert-butyl 4-((3-chloro-2,4-difluorophenyl)(methyl)carbamoyl)-3-(2-chloro-6-(trifluoromethyl)pyrimidin-4-yl)-2-oxoimidazolidine-1-carboxyla te (305 mg, yield: 58%) as a yellow solid. LCMS (ESI) m/z: **570**[M+H]⁺.

Step 2: to a solution of (S)-tert-butyl 4-((3-chloro-2,4-difluorophenyl)(methyl)carbamoyl)-3-(2-chloro-6-(trifluoromethyl)pyrimidin-4-yl)-2-oxoimidazolidine-1-carboxyla te (150 mg, 0.26 mmol) in dioxane (5.00 mL) was added bis(triphenylphosphine)palladium(II) chloride (18 mg, 0.03 mmol) and tributylstannane (173 mg, 0.53 mmol), and the obtained mixture was stirred at 80°C for 2 h. After completion of the reaction monitored by LC-MS, the reaction solution was poured into water and extracted with dichloromethane (10 mL*2). The combined organic layer was dried over anhydrous sodium sulfate. After filtration, the filtrate was concentrated under a vacuum. The residue was purified by silica gel column chromatography (petroleum ether:ethyl acetate = 70:30) to give (S)-tert-butyl 4-((3-chloro-2,4-difluorophenyl)(methyl)carbamoyl)-2-oxo-3-(2-(prop-1-yn-1-yl)-6-(trifluoromethyl)pyrimidin-4-yl)imidazolidine-1-carboxylate (90 mg, yield: 60%) as a yellow solid. LCMS (ESI) m/z: **574**[M+H]⁺.

Step 3: to a solution of tert-butyl (S)-4-((3-chloro-2,4-difluorophenyl)(methyl)carbamoyl)-2-oxo-3-(2-(prop-1-yn-1-yl)-6-(trifluoromethyl)pyrimidin-4-yl)imidazolidin e-1-carboxylate (60 mg, 0.10 mmol) in N,N-dimethylacetamide (1.00 mL) was added cuprous bromide (0.86 mg, 0.01 mmol) and triethylamine (211 mg, 2.00 mmol), and the obtained mixture was stirred at 140°C. After 12 h, after completion of the reaction monitored by LC-MS, the reaction solution was poured into water (5 mL) and extracted with ethyl acetate (5 mL*2). The combined organic phase was dried over anhydrous sodium sulfate, suction filtered, and then spin-dried. The residue was purified by high-performance liquid chromatography (column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 32% B to 65% B within 10 min; wavelength: 220 nm; retention time: 8.45 min) to give (S)-N-(3-chloro-2,4-difluorophenyl)-N-methyl-2-oxo-3-(4-(trifluoromethyl)pyrrolo[1,2-a]pyrimidin-2-yl)imidazolidine-4-carboxami de (6.66 mg, 0.014 mmol, yield: 11%) as a yellow solid.

LCMS (ESI) m/z: **473.80**[M+H]⁺, HNMR (400 MHz, DMSO-d6): δ ppm 8.30-8.25(m,1H),7.90-7.61(m,2H),7.50-7.35(m,2H),7.04-7.01 (m, 1H),6.61-6.32(m,1H),4.90-4.76(m,1H),3.55-3.50(m,1H),3.47-3.38(m,1H),3.22-3.09(m, 3H).

### Example 23:

### (S)-N-(3-chloro-2,4-difluorophenyl)-N-methyl-2-oxo-3-(7-(trifluoromethyl)thiazolo[5,4-b]pyridin-2-yl)imidazolidine-4-carbox amide (Compound 23)

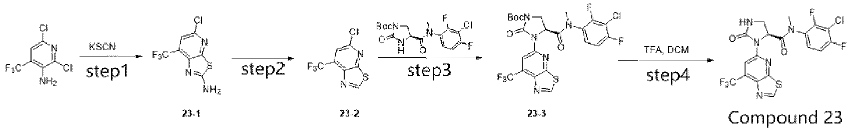

Step 1: a mixture of 2,6-dichloro-4-(trifluoromethyl)-3-pyridinamine (5.00 g, 21.65 mmol) and potassium thiocyanate (6.30 g, 64.94 mmol) in ethanol (50.00 mL) and hydrochloric acid (100.00 mL) was stirred at 100°C overnight, and the reaction was monitored by LCMS for completion. The reaction solution was poured into water (100 mL) and extracted with dichloromethane (100 mL*2). The combined organic layer was dried over anhydrous sodium sulfate. After filtration, the filtrate was concentrated under a vacuum. The concentrate was purified by silica gel column chromatography (ethyl acetate:petroleum ether = 50:50) to give 5-chloro-7-(trifluoromethyl)-1,3-thiazolo[5,4-b]pyridin-2-ylamine (2.0 g, 7.9 mmol, yield: 36%) as a yellow oil. LCMS (ESI) m/z: **253** [M+H]⁺.

Step 2: a mixture of 5-chloro-7-(trifluoromethyl)-1,3-thiazolo[5,4-b]pyridin-2-ylamine (2.00 g, 7.89 mmol) and tert-butoxy (1.87 mL, 15.77 mmol) in tetrahydrofuran (10.00 mL) was stirred at 0°C for 30 min. The reaction mixture was then stirred at 90°C, and the reaction was monitored by LC-MS. After completion of the reaction, the reaction solution was poured into water (20 mL) and extracted with ethyl acetate (20 mL*2). The combined organic layer was dried over anhydrous sodium sulfate. After filtration, the filtrate was concentrated under a vacuum. The residue was purified by silica gel column chromatography (petroleum ether:ethyl acetate = 80:20) to give 5-chloro-7-(trifluoromethyl)-1,3-thiazolo[5,4-b]pyridine (500.00 mg, 2.1 mmol, yield: 27%) as a red solid. LCMS (ESI) m/z: **238**[M+H]⁺.

Step 3: to a solution of 5-chloro-7-(trifluoromethyl)-1,3-thiazolo[5,4-b]pyridine (250 mg, 1.05 mmol) in dioxane (5.00 mL) was added (S)-tert-butyl 4-((3-chloro-2,4-difluorophenyl)(methyl)carbamoyl)-2-oxoimidazolidine-1-carboxylate (326 mg, 0.84 mmol), tris(dibenzylideneacetone)dipalladium (95 mg, 0.86 mmol), 4,5-bis(diphenylphosphino)-9,9-dimethylxanthene (60 mg, 0.10 mmol), and potassium carbonate (433 mg, 3.14 mmol), and the reaction solution was stirred at 80°C overnight. After completion of the reaction monitored by LC-MS, the reaction solution was poured into water (20 mL) and extracted with dichloromethane (10 mL*2). The combined organic layer was dried over anhydrous sodium sulfate. After filtration, the filtrate was concentrated under a vacuum. The residue was purified by silica gel column chromatography (ethyl acetate:petroleum ether = 30:70) to give (S)-tert-butyl 4-((3-chloro-2,4-difluorophenyl)(methyl)carbamoyl)-2-oxo-3-(7-(trifluoromethyl)thiazolo[5,4-b]pyridin-5-yl)imidazolidine-1-carbox ylate (220.00 mg, 0.37 mmol, yield: 35%) as a yellow oil. LCMS (ESI) m/z: **592** [M+H]⁺.

Step 4: to a solution of (S)-tert-butyl 4-((3-chloro-2,4-difluorophenyl)(methyl)carbamoyl)-2-oxo-3-(7-(trifluoromethyl)thiazolo[5,4-b]pyridin-5-yl)imidazolidine-1-carbox ylate **(Intermediate 6,** 200 mg, 0.34 mmol) in dichloromethane (5.00 mL) and trifluoroacetic acid (0.1 mL), the reaction solution was reacted at room temperature for 0.5 h and was monitored by LC-MS. After completion of the reaction, the reaction solution was spin-dried. The residue was purified by high-performance liquid chromatography (XBridge Shield RP18 OBD column, 30*150 mm, 5 um; mobile phase A: water (10 mmol/L ammonium bicarbonate aqueous solution), mobile phase B: acetonitrile; flow rate: 60 mL/min; gradient: 25% B to 62% B within 10 min, wavelength: 220 nm; peak time: 9.37 min) to give { (4S)-2-oxo-3-[7-(trifluoromethyl)(1,3-thiazolo[4,5-e]pyridin-5-yl)]imidazolidin-4-yl}-N-(3-chloro-2,4-difluorophenyl)-N-methylfor mamide (81.82 mg, 0.166 mmol, yield: 49%) as a white solid.

LCMS (ESI) m/z: **491.85** [M+H]⁺.

HNMR (400 MHz, DMSO-d₆): δ ppm 9.54-9.49(m,1H), 8.84-8.78(m,1H),7.88-7.63(m,2H), 7.51-7.36(m,1H),5.82-4.85(m,1H),3.95-3.52(m,1H),3.49-3.40 (m,1H),3.21-3.17(m,3H).

### Example 24:

### (S)-N-(3-chloro-2,4-difluorophenyl)-N-methyl-2-oxo-3-(4-(trifluoromethyl)thieno[2,3-d]pyridin-2-yl)imidazolidine-4-carboxa mide (Compound 24)

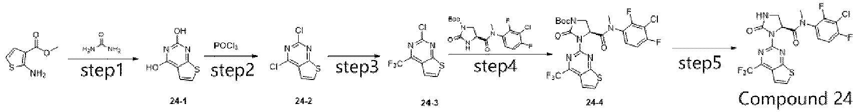

Step 1: methyl 2-aminothiophene-3-carboxylate (11.00 g, 0.07 mol) and urea (21.01 g, 0.35 mol) were reacted at 150°C for about 2.5 h. After completion of the reaction, the reaction solution was poured into saturated aqueous sodium chloride solution (30 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 50:50) to give thienopyrimidine-2,4-diol (2 g, crude product) as a yellow solid, which was directly used in the next step. LCMS(ESI): **169**[M+H]⁺.

Step 2: thienopyrimidine-2,4-diol (2 g, 0.01 mol) and phosphorus oxychloride (20 mL) were reacted at 80°C for 12 h. After completion of the reaction, phosphorus oxychloride was concentrated, and then the mixture was poured into water (20 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 40:60) to give 2,4-dichlorothieno[2,3-d]pyrimidine (1.40 g, 0.006 mol, yield: 60%) as a yellow solid. LCMS (ESI): **205**[M+H]⁺.

Step 3: 2,4-dichlorothieno[2,3-d]pyrimidine (2.10 g, 0.01 mol), [PPh4]⁺[Cu(CF3)2]⁻(10.81 g, 0.02 mol), cuprous iodide (1.91 g, 0.01 mol), and N-methylpyrrolidinone (10 mL) were reacted at 110°C for 10 h. After completion of the reaction, the reaction solution was poured into water (20 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 20:80) to give 2-chloro-4-(trifluoromethyl)thieno[2,3-d]pyrimidine (0.31 g, 0.001 mol, yield: 13%) as a white solid. LCMS (ESI): **239**[M+H]⁺.

Step 4: 2-chloro-4-(trifluoromethyl)thieno[2,3-d]pyrimidine (315 mg, 1.32 mmol), (S)-tert-butyl 4-((3-chloro-2,4-difluorophenyl)(methyl)carbamoyl)-2-oxoimidazolidine-1-carboxylate **(Intermediate 6,** 513 mg, 1.32 mmol), tris(dibenzylideneacetone)dipalladium (120 mg, 0.132 mmol), 4,5-bis(diphenylphosphine)-9,9-dimethylxanthene (76 mg, 0.132 mmol), and potassium carbonate (546 mg, 3.96 mmol) were dissolved in dioxane (5 mL) and reacted at 80°C for 12 h. After completion of the reaction, the reaction solution was poured into water (5 mL) and extracted with dichloromethane (5 mL x 3). The organic phases were combined, washed with saturated salt water, dried over anhydrous sodium sulfate, and filtered to remove anhydrous sodium sulfate. The filtrate was concentrated. The residue was purified by silica gel column chromatography (petroleum ether:ethyl acetate = 60:40) to give (S)-tert-butyl 4-((3-chloro-2,4-difluorophenyl)(methyl)carbamoyl)-2-oxo-3-(4-(trifluoromethyl)thieno[2,3-d]pyrimidin-2-yl)imidazolidine-1-carbo xylate (270 mg, 0.45 mmol, yield: 34.6%) as a white solid. LCMS (ESI) m/z: **592**[M+H]⁺.

Step 5: (S)-tert-butyl 4-((3-chloro-2,4-difluorophenyl)(methyl)carbamoyl)-2-oxo-3-(4-(trifluoromethyl)thieno[2,3-d]pyrimidin-2-yl)imidazolidine-1-carbo xylate (270 mg, 0.45 mmol) was added to a mixed solution of trifluoroacetic acid (4 mL) in dichloromethane (20 mL) and then reacted at room temperature for 10 h. After completion of the reaction, the reaction solution was poured into water (5 mL) and extracted with dichloromethane (5 mL x 3). The organic phases were combined, washed with saturated salt water, dried over anhydrous sodium sulfate, and filtered to remove anhydrous sodium sulfate. The filtrate was concentrated to give a crude product. The crude product was purified by high-performance liquid chromatography (column: XBridge Prep OBD C18, 30 x 150 mm, 5 um, mobile phase A: 10 mmol/L ammonium bicarbonate aqueous solution, mobile phase B: acetonitrile, flow rate: 60 mL/min, elution gradient: mobile phase B increased from 35% to 70% within 10 min, detection wavelength: 220 nm, retention time: 9.15 min) to give (S)-N-(3-chloro-2,4-difluorophenyl)-N-methyl-2-oxo-3-(4-(trifluoromethyl)thieno[2,3-d]pyrimidin-2-yl)imidazolidine-4-carboxamid e (114 mg, 0.23 mmol, yield: 51.1%) as a white solid.

LCMS (ESI) m/z: 491.60[M+H]+.

HNMR (400 MHz, DMSO-d6): δ ppm 8.01-7.95 (m, 1H), 7.74-7.59 (m, 1H) ,7.55-7.39 (m, 3H), 5.79-4.85(m, 1H), 3.93-3.36 (m, 2H), 3.20-3.16 (m, 3H).

### Example 25:

### ((S)-N-(3-chloro-2,4-difluorophenyl)-N-methyl-2-oxo-3-(7-(trifluoromethyl)imidazo[1,2-a]pyridin-5-yl)imidazolidine-4-carbox amide (Compound 25)

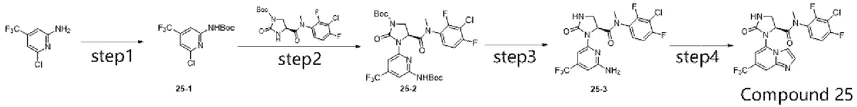

Step 1: 6-chloro-4-(trifluoromethyl)pyridin-2-amine (2.01 g, 0.01 mol), di-tert-butyl dicarbonate (6.67 g, 0.03 mol), 4-dimethylaminopyridine (0.21 g, 0.002 mol), and triethylamine (3.70 g, 0.03 mol) were dissolved in dichloromethane (10 mL) and reacted at room temperature for about 10 h. After completion of the reaction, the reaction solution was poured into saturated aqueous sodium chloride solution (20 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 20:80) to give tert-butyl (6-chloro-4-(trifluoromethyl)pyridin-2-yl)carbamate (1.91 g, 0.006 mol, yield: 60%) as a yellow solid. LCMS(ESI): **297**[M+H]⁺.

Step 2: tert-butyl (6-chloro-4-(trifluoromethyl)pyridin-2-yl)carbamate (360 mg, 1.21 mmol), (S)-tert-butyl 4-((3-chloro-2,4-difluorophenyl)(methyl)carbamoyl)-2-oxoimidazolidine-1-carboxylate **(Intermediate 6,** 513 mg, 1.32 mmol), tris(dibenzylideneacetone)dipalladium (110 mg, 0.121 mmol), 4,5-bis(diphenylphosphine)-9,9-dimethylxanthene (69.93 mg, 0.121 mmol), and potassium carbonate (500 mg, 3.63 mmol) were dissolved in dioxane solution (10 mL) and reacted at 80°C for 10 h. After completion of the reaction, the reaction solution was poured into water (5 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 50:50) to give (S)-tert-butyl 3-(6-((tert-butoxycarbonyl)amino)-4-(trifluoromethyl)pyridin-2-yl)-4-((3-chloro-2,4-difluorophenyl)(methyl)carbamoyl)-2-oxoimida zolidine-1-carboxylate (264 mg, 0.40 mmol, yield: 33%) as a yellow solid. LCMS (ESI): **650** [M+H]⁺.

Step 3: (S)-tert-butyl 3-(6-((tert-butoxycarbonyl)amino)-4-(trifluoromethyl)pyridin-2-yl)-4-((3-chloro-2,4-difluorophenyl)(methyl)carbamoyl)-2-oxoimida zolidine-1-carboxylate (259 mg, 0.39 mmol) and dichloromethane (20 mL) were dissolved in trifluoroacetic acid (4 mL) and reacted at room temperature for 10 h. After completion of the reaction, the reaction solution was poured into water (5 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 50:50) to give (S)-3-(6-amino-4-(trifluoromethyl)pyridin-2-yl)-N-(3-chloro-2,4-difluorophenyl)-N-methyl-2-oxoimidazolidine-4-carboxamide (45 mg, 0.07 mmol, yield: 36.8%) as a yellow solid. LCMS (ESI): **450** [M+H]⁺.

### Step 4:

(S)-3-(6-amino-4-(trifluoromethyl)pyridin-2-yl)-N-(3-chloro-2,4-difluorophenyl)-N-methyl-2-oxoimidazolidine-4-carboxamide (60 mg, 0.13 mmol) and chloroacetaldehyde (10.14 mg, 0.13 mmol) were dissolved in anhydrous ethanol (2 mL) and reacted at 90°C for 16 h. After completion of the reaction, the reaction solution was poured into water (10 mL) and extracted with ethyl acetate (15 mL x 3). The organic phases were combined, washed with saturated salt water, dried over anhydrous sodium sulfate, and filtered to remove anhydrous sodium sulfate. The filtrate was concentrated to give (S)-N-(3-chloro-2,4-difluorophenyl)-N-methyl-2-oxo-3-(7-(trifluoromethyl)imidazo[1,2-a]pyridin-5-yl)imidazolidine-4-carboxamide (4.16 mg, 0.008 mmol, yield: 6%) as a white solid.

LCMS (ESI) m/z: **473.80** [M+H]⁺.

HNMR (400 MHz, DMSO-d6): δ ppm 8.05-8.01 (m, 1H), 7.98-7.93 (m, 1H) ,7.79-7.78(m, 1H) ,7.77-7.62(m, 1H), 7.49-7.27(m, 2H), 7.16-7.08 (m, 1H), 5.21-5.11 (m, 1H),4.17-3.52 (m, 1H), 3.47-3.38 (m, 1H), 3.21-2.94(m, 3H).

### Example 26:

### (2S,3S,4S)-N-(5-chloro-2,4-difluorophenyl)-3,4-dihydroxy-N-(methyl-D3)-5-oxo-1-(7-(trifluoromethyl)thiazolo[5,4-b]pyridin-5-yl)p yrrolidine-2-carboxamide (Compound 26)

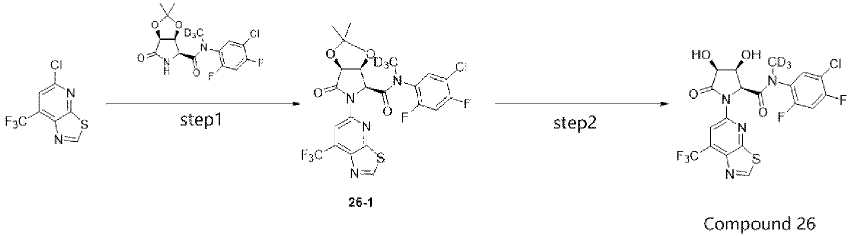

The same synthetic method as **Compound 16** using 5-chloro-7-(trifluoromethyl)-1,3-thiazolo[5,4-b]pyridine (**Intermediate 23-2**) and **Intermediate 4** as the starting materials and high-performance liquid chromatography (column: XBridge Shield RP18 OBD column, 30*150 mm, 5 µm; mobile phase A: 10 mmol/L ammonium bicarbonate aqueous solution, mobile phase B: acetonitrile; flow rate: 60 mL/min; gradient: 25% B to 50% B within 8 min; wavelength: 220 nm; retention time: 7.83 min) were performed to give (2S,3S,4S)-N-(5-chloro-2,4-difluorophenyl)-3,4-dihydroxy-N-(methyl-D3)-5-oxo-1-(7-(trifluoromethyl)thiazolo[5,4-b]pyridin-5-yl)p yrrolidine-2-carboxamide (4.67 mg, yield: 10%, 0.008 mmol) as a white solid.

LCMS (ESI): 525.80[M+H]⁺.

HNMR (400 MHz, DMSO-d6): δ ppm 9.66-9.62(m, 1H), 8.68-8.64 (m, 1H), 7.96-7.90 (m, 1H), 7.87-7.56(m, 1H), 5.85-5.32(m, 2H), 5.13-4.80(m, 1H), 4.50-4.29(m,1H), 4.10-4.05(m,1H).

### Example 27:

### (S)-N-(3-chloro-2,4-difluorophenyl)-N-methyl-2-oxo-3-(4-(trifluoromethyl)pyrrolo[1,2-b]pyridazin-2-yl)imidazolidine-4-carboxami de (Compound 27)

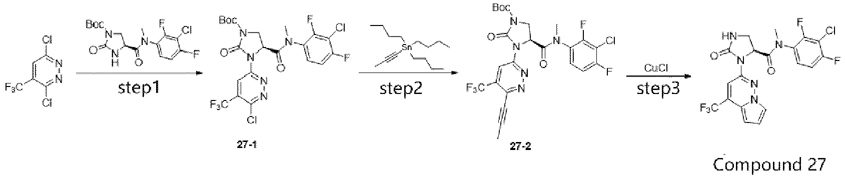

Step 1: 3,6-dichloro-4-(trifluoromethyl)pyridazine (1.01 g, 4.61 mmol), (S)-tert-butyl 4-((3-chloro-2,4-difluorophenyl)(methyl)carbamoyl)-2-oxoimidazolidine-1-carboxylate **(Intermediate 6,** 538 mg, 1.38 mmol), palladium acetate (103.24 mg, 0.46 mmol), 4,5-bis(diphenylphosphine)-9,9-dimethylxanthene (266 mg, 0.46 mmol), and potassium carbonate (1.91 g, 13.83 mmol) were dissolved in 1,4-dioxane (20 mL) solution and reacted at 80°C for 2 h. The desired product can be detected by LCMS. The obtained mixture was extracted with dichloromethane (3 x 5 mL). The combined organic layer was washed with salt water (3 x 3 mL) and dried over anhydrous sodium sulfate. After filtration, the filtrate was concentrated under reduced pressure. The residue was purified by silica gel column chromatography (50% ethyl acetate in petroleum ether) to give (S)-tert-butyl
4-((3-chloro-2,4-difluorophenyl)(methyl)carbamoyl)-3-(6-chloro-5-(trifluoromethyl)pyridazin-3-yl)-2-oxoimidazolidine-1-carboxylat e (500 mg, 0.87 mmol, yield: 19%) as a yellow solid. LCMS (ESI): 570 [M+H]⁺.

Step 2: (S)-tert-butyl 4-((3-chloro-2,4-difluorophenyl)(methyl)carbamoyl)-3-(6-chloro-5-(trifluoromethyl)pyridazin-3-yl)-2-oxoimidazolidine-1-carboxylat e (450 mg, 0.79 mmol) was dissolved in 1,4-dioxane (10 mL), and 1,1'-bis(diphenylphosphino)ferrocene]dichloropalladium (55 mg, 0.08 mmol) and tributylstannane (520 mg, 1.58 mmol) were added at room temperature. The system was then warmed to 80°C and reacted for 4 h. The desired product can be detected by LCMS. The obtained mixture was extracted with dichloromethane (3 x 10 mL). The combined organic layer was washed with saturated salt water (3 x 5 mL) and dried over anhydrous sodium sulfate. After filtration, the filtrate was concentrated under reduced pressure and purified by silica gel column chromatography (30% ethyl acetate in petroleum ether) to give (S)-tert-butyl 4-((3-chloro-2,4-difluorophenyl)(methyl)carbamoyl)-2-oxo-3-(6-(prop-1-yn-1-yl)-5-(trifluoromethyl)pyridazin-3-yl)imidazolidine-1-carboxylate (300 mg, 0.56 mmol, yield: 60%) as a yellow oil. LCMS (ESI): 574 [M+H]⁺.

Step 3: (S)-tert-butyl 4-((3-chloro-2,4-difluorophenyl)(methyl)carbamoyl)-2-oxo-3-(6-(prop-1-yn-1-yl)-5-(trifluoromethyl)pyridazin-3-yl)imidazolidine-1-carboxylate (150 mg, 0.26 mmol), cuprous chloride (25 mg, 0.26 mmol), and triethylamine (726 µL, 5.23 mmol) were dissolved in N,N-dimethylacetamide (5 mL) and reacted at 140°C overnight. The desired product can be detected by LCMS. The obtained mixture was extracted with ethyl acetate (3 x 5 mL). The combined organic layer was washed with salt water (3 x 3 mL) and dried over anhydrous sodium sulfate. After filtration, the filtrate was concentrated under reduced pressure. After concentration, the concentrate was purified on a column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 39% to 65% B within 10 min; wavelength: 220 nm; retention time: 9.5 min, to give (S)-N-(3-chloro-2,4-difluorophenyl)-N-methyl-2-oxo-3-(4-(trifluoromethyl)pyrrolo[1,2-b]pyridazin-2-yl)imidazolidine-4-carboxami de (16.38 mg, 0.034 mmol, yield: 13%) as a light yellow solid.

LCMS (ESI): 473.75[M+H]⁺.

HNMR (400 MHz, DMSO-d6): δ ppm 8.25-8.22(m, 1H), 8.06-7.85 (m, 1H), 7.78-7.62 (m, 2H), 7.60-7.38(m, 1H), 6.95-6.91(m, 1H), 6.68-6.62 (m, 1H), 5.68-4.75(m,1H), 3.54-3.50(m,1H),3.48-3.40(m,1H), 3.24-3.11(m,3H).

### Example 28a and Example 28b:

(S)-N-(3-chloro-2,4-difluorophenyl)-3-((R)-7-hydroxy-7-methyl-4-(trifluoromethyl)-6,7-dihydro-SH-cyclopenta[b]pyridin-2-yl)-N-m ethyl-2-oxoimidazolidine-4-carboxamide and (R)-N-(3-chloro-2,4-difluorophenyl)-3-((R)-7-hydroxy-7-methyl-4-(trifluoromethyl)-6,7-dihydro-SH-cyclopenta[b]pyridin-2-yl)-N-methyl-2-oxoimidazolidine-4-carboxamide

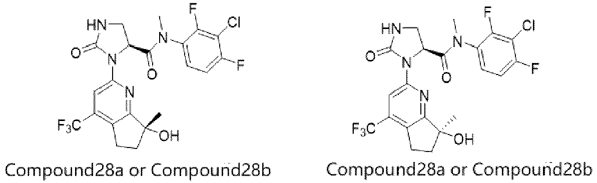

The same synthetic method as **Compound 8a** using **Compound 13** as the starting material and high-performance liquid chromatography (column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 32% B to 63% B within 8 min; wavelength: 220 nm) were performed to give a earlier peak with peak time of 6.30 min as a yellow solid and a later peak with peak time of 7.58 min as a yellow solid.

### Compound 28a: earlier peak.

LCMS (ESI): 504.75[M+H]⁺.

HNMR (400 MHz, DMSO-d6): δ ppm 8.56-8.39 (m, 1H), 8.34-7.49 (m, 2H), 7.41-7.21 (m, 1H), 5.80-4.77 (m, 2H), 3.93-3.51 (m, 2H), 3.18-3.09 (m, 3H), 3.05-2.97(m, 1H), 2.87-2.83 (m, 1H), 2.25-2.12(m, 2H), 1.48-1.36 (m, 3H).

### Compound 28b: later peak.

LCMS (ESI): 504.80[M+H]⁺.

HNMR (400 MHz, DMSO-d6): δ ppm 8.58-8.52 (m, 1H), 8.42-8.38 (m, 1H), 7.70-7.58 (m, 1H), 7.52-7.28 (m, 1H), 5.75-4.77 (m, 2H), 3.93-3.45 (m, 2H), 3.18-3.09 (m, 3H), 3.05-2.94(m, 1H), 2.83-2.78 (m, 1H),2.25-2.12 (m, 2H), 1.48-1.36 (m, 3H).

### Example 29:

### (S)-6-(5-((3-chloro-2,4-difluorophenyl)(methyl)carbamoyl)-2-oxoimidazolidin-1-yl)-4-(trifluoromethyl)thieno[2,3-b]pyridine-2-carb oxylic acid (Compound 29)

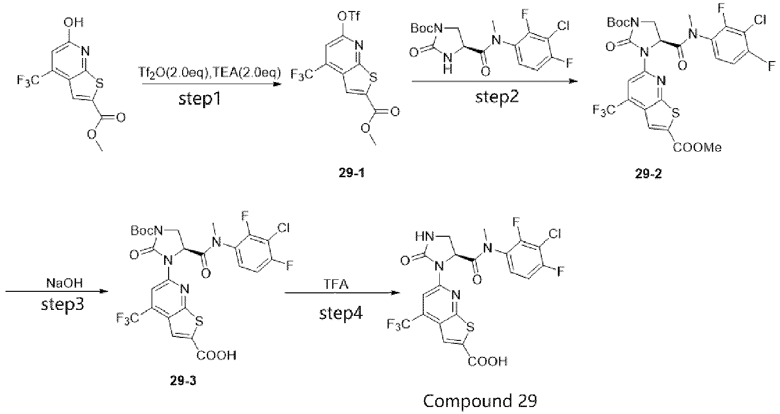

Step 1: triflic anhydride (1.22 g, 4.33 mmol) was added to a mixture of methyl 6-hydroxy-4-(trifluoromethyl)thieno[2,3-b]pyridine-2-carboxylate (1.01 g, 3.61 mmol) and triethylamine (721 mg, 7.21 mmol) in dichloromethane (10.00 mL) at 0°C and reacted at room temperature for 2 h. After completion of the reaction, the mixture was poured into water and extracted with ethyl acetate (10 mL). The organic phase was dried over anhydrous sodium sulfate, suction filtered, spin-dried, and purified by silica gel column chromatography (20% ethyl acetate in petroleum ether) to give methyl 4-(trifluoromethyl)-6-(((trifluoromethyl)sulfonyl)oxy)thieno[2,3-b]pyridine-2-carboxylate (1.00 g, 1.8 mmol, yield: 68%). LCMS (ESI): 410[M+H]⁺.

Step 2: methyl 4-(trifluoromethyl)-6-[(trifluoromethyl)sulfonyloxy]thieno[2,3-b]pyridine-2-carboxylate (0.20 g, 0.49 mmol), tert-butyl (4S)-4-[N-(3-chloro-2,4-difluorophenyl)-N-methylcarbamoyl]-2-oxoimidazolidine carboxylate (**Intermediate 6**, 0.19 g, 0.49 mmol), tris(dibenzylideneacetone)dipalladium (0.04 g, 0.05 mmol), 4,5-bis(diphenylphosphino)-9,9-dimethylxanthene (0.03 g, 0.06 mmol), and potassium carbonate (0.13 g, 0.98 mmol) were stirred in dioxane (5.00 mL) at 80°C for 12 h. After completion of the reaction, the mixture was poured into water, extracted with ethyl acetate (10 mL), dried over anhydrous sodium sulfate, filtered, concentrated, and purified by silica gel column chromatography (30% ethyl acetate in petroleum ether) to give (S)-6-(3-(tert-butoxycarbonyl)-5-((3-chloro-2,4-difluorophenyl)(methyl)carbamoyl)-2-oxoimidazolidin-1-yl)-4-(trifluoromethyl)thien o[2,3-b]pyridine-2-carboxylate (0.16 g, 0.24 mmol, yield:50%) as a white solid, LCMS (ESI): 649[M+H]⁺.

Step 3: a mixture of (S)-methyl 6-(3-(tert-butoxycarbonyl)-5-((3-chloro-2,4-difluorophenyl)(methyl)carbamoyl)-2-oxoimidazolidin-1-yl)-4-(trifluoromethyl)thieno[2, 3-b]pyridine-2-carboxylate (200 mg, 0.31 mmol) and sodium hydroxide (24.65 mg, 0.62 mmol) in methanol (2.00 mL) was stirred at room temperature for 2 h. After completion of the reaction, the mixture was spin-dried to give 6-{ (5 S)-3-[(tert-butyl)oxycarbonyl]-5-[N-(3-chloro-2,4-difluorophenyl)-N-methylcarbamoyl]-2-oxoimidazolidinyl}-4-(trifluorometh yl)thieno[2,3-b]pyridine-2-carboxylic acid (120 mg, yield: 61%, 0.192 mmol) as a white solid. LCMS (ESI): 635[M+H]⁺.

### Step 4:

(S)-6-(3-(tert-butoxycarbonyl)-5-((3-chloro-2,4-difluorophenyl)(methyl)carbamoyl)-2-oxoimidazolidin-1-yl)-4-(trifluoromethyl)thien o[2,3-b]pyridine-2-carboxylic acid (100 mg, 0.16 mmol) was dissolved in dichloromethane (10.00 mL), and then trifluoroacetic acid (2.00 mL) was added dropwise to the reaction. The reaction mixture was stirred at room temperature for 2 h. After completion of the reaction, the organic phase was spin-dried and subjected to high-performance liquid chromatography (column: Xselect CSH C18 OBD column 30 x 150 mm, 5 µm; mobile phase A: 10 mmol/L ammonium bicarbonate aqueous solution, mobile phase B: acetonitrile; flow rate: 60 mL/min; gradient: 45% B to 75% B within 8 min; wavelength: 220 nm; retention time: 6.93 min) to give (S)-6-(5-((3-chloro-2,4-difluorophenyl)(methyl)carbamoyl)-2-oxoimidazolidin-1-yl)-4-(trifluoromethyl)thieno[2,3-b]pyridine-2-carb oxylic acid (17.92 mg, 21%, 0.033 mmol) as a white solid.

LCMS (ESI): 535.25[M+H].

HNMR (400 MHz, DMSO-d6): δ ppm 9.66-9.62(m, 1H), 8.89-8.66 (m, 3H), 7.51-7.40 (m, 1H), 5.00-4.86(m, 1H), 3.56-3.42(m, 2H), 3.18-3.09(m, 3H).

### Example 30:

### (R)-4-(1-(3-chloro-4-fluorophenyl)-4-fluoro-1H-imidazol-2-yl)-3-(4-(trifluoromethyl)thieno[2,3-b]pyridin-6-yl)oxazolidin-2-one (Compound 30)

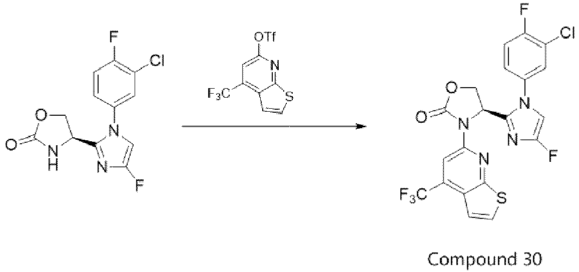

A mixture of (R)-4-(1-(3-chloro-4-fluorophenyl)-4-fluoro-1H-imidazol-2-yl)oxazolidin-2-one (synthesized according to the method reported in the patent WO2021123785) (70 mg, 0.23 mmol), 4-(trifluoromethyl)thieno[2,3-b]pyridin-6-yl triflate (98 mg, 0.28 mmol), potassium carbonate (96 mg, 0.70 mmol), tris(dibenzylideneacetone)dipalladium (21 mg, 0.02 mmol), and 4,5-bis(diphenylphosphine)-9,9-dimethylxanthene (13 mg, 0.02 mol) in dioxane (5 mL) was stirred at 80°C under a nitrogen atmosphere for 4 h. After completion of the reaction, the reaction mixture was diluted with water and extracted with ethyl acetate (3 x 5 mL). The combined organic layer was washed with salt water, dried over anhydrous sodium sulfate, filtered, and concentrated under a vacuum to give a crude product. The crude product was purified by high-performance liquid chromatography (column: Xselect CSH C18 OBD column 30*150 mm, 5 µm, mobile phase A: water (0.05% formic acid), mobile phase B: acetonitrile; flow rate: 60 mL/min; gradient: 50% B to 80% B within 8 min, wavelength: 220 nm; retention time: 7.55 min) to give (R)-4-(1-(3-chloro-4-fluorophenyl)-4-fluoro-1H-imidazol-2-yl)-3-(4-(trifluoromethyl)thieno[2,3-b]pyridin-6-yl)oxazolidin-2-one (19.76 mg, yield: 17%, 0.039 mmol) as a white solid.

LCMS (ESI): 501.15[M+H]⁺.

HNMR (400 MHz, DMSO-d6): δ ppm 8.45(s, 1H), 8.04 (d, J = 4 Hz, 1H), 7.99 (dd, J = 6.4Hz, 1H), 7.80- 7.71 (m, 2H), 7.47-7.45 (m, 1H), 7.25 (d, J = 8 Hz, 1H), 5.82-5.79 (m, 1H), 4.82 (t, J = 8.4 Hz, 1H), 4.69-4.66 (m, 1H).

### Example 31: [(4S)-3-(7-cyclopropyl (1,3-thiazolo[4,5-e]pyridin-5-yl))-2-oxoimidazolidin-4-yl]-N-(3-chloro-2,4-difluorophenyl)-N-methylformamide (Compound 31)

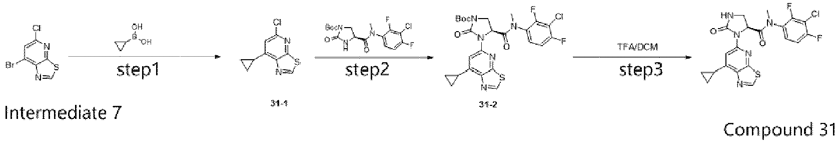

Step 1: 7-bromo-5-chloro-1,3-thiazolo[5,4-b]pyridine **(Intermediate 7**, 500.00 mg, 2.00 mmol), cyclopropylboronic acid (688.52 mg, 8.02 mmol), [1,1'-bis(diphenylphosphino)ferrocene]dichloropalladium (145.41 mg, 0.20 mmol), and potassium carbonate (553.90 mg, 4.01 mmol) were reacted in 1,4-dioxane (1.00 mL) at 140°C for 12 h. The mixture was poured into water with stirring, extracted with ethyl acetate (5 mL), dried over sodium sulfate, filtered, concentrated, and purified by column chromatography (petroleum ether:ethyl acetate = 60:40) to give 5-chloro-7-cyclopropyl-1,3-thiazolo[5,4-b]pyridine (300.00 mg, 2 mmol, yield: 71%) as a colorless oil. LCMS (ESI): 210[M+H]+.

Step 2: 5-chloro-7-cyclopropyl-1,3-thiazolo[5,4-b]pyridine (0.10 g, 0.47 mmol) was reacted with tert-butyl 4-[N-(3-chloro-2,4-difluorophenyl)-N-methylcarbamoyl]-2-oxoimidazolidine carboxylate **(Intermediate 6,** 0.15 g, 0.38 mmol), tris(dibenzylideneacetone)dipalladium ((0.03 g, 0.05 mmol), 4,5-bis(diphenylphosphino)-9,9-dimethylxanthene (0.01 g, 0.06 mmol), and potassium carbonate (0.20 g, 1.42 mmol) in 1,4-dioxane (10 mL) at 100°C for 2 h. After completion of the reaction, the mixture was poured into water with stirring, extracted with ethyl acetate (5 mL), dried over sodium sulfate, filtered, concentrated, and purified by column chromatography (petroleum ether:ethyl acetate = 80:20) to give 5-chloro-7-cyclopropyl-1,3-thiazolo[5,4-b]pyridine (300 mg, 2 mmol, yield: 71%) as a colorless oil, LCMS (ESI): 564[M+H]+.

Step 3: a mixture of tert-butyl (4S)-4-[N-(3-chloro-2-fluoro-4-methylphenyl)-N-methylcarbamoyl]-3-(7-cyclopropyl (1,3-thiazolo[4,5-e]pyridin-5-yl))-2-oxoimidazolidine carboxylate (85 mg, 0.15 mmol) in trifluoroacetic acid (0.2 mL) and dichloromethane (1.00 mL) was stirred at room temperature for 2 h. After completion of the reaction, the mixture was spin-dried and purified by high-performance liquid chromatography (column: XBridge Shield RP18 OBD column, 30*150 mm, 5 µm; mobile phase A: water (10 mmol/L NH4HCO3), mobile phase B: ACN; flow rate: 60 mL/min; gradient: 30% B to 60% B within 10 min; wavelength: 220 nm; retention time (min): 8.62) to give [(4S)-3-(7-cyclopropyl (1,3-thiazolo[4,5-e]pyridin-5-yl))-2-oxoimidazolidin-4-yl]-N-(3-chloro-2,4-difluorophenyl)-N-methylformamide (14.25 mg, 0.15 mmol, yield: 14%) as a white solid.

LCMS (ESI): 464.05[M+H]⁺.

HNMR (400 MHz, DMSO-d6): δ ppm 9.28-9.22(m, 1H), 8.00-7.93(m, 1H),7.74-7.64(m, 1H), 7.54-7.39 (m, 2H), 5.72-4.78(m, 1H),3.84-3.38(m, 2H), 3.15-3.13(m,3H),1.22-1.69(m,1H),1.01-0.99(m,2H).

### Example 32: {(4S)-3-[7-(dimethylcarbonyl) (1,3-thiazolo[4,5-e]pyridin-5-yl)]-2-oxoimidazolidin-4-yl}-N-(3-chloro-2,4-difluorophenyl)-N-methylformamide (Compound 32)

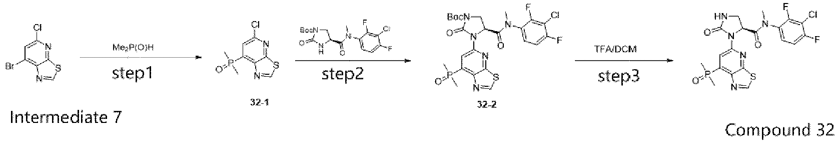

Step 1: a solution of 7-bromo-5-chloro-1,3-thiazolo[5,4-b]pyridine (200 mg, 0.80 mmol), tris(dibenzylideneacetone)dipalladium (57 mg, 0.08 mmol), 4,5-bis(diphenylphosphino)-9,9-dimethylxanthene (46 mg, 0.08 mmol), potassium phosphate (255 mg, 1.20 mmol), and dimethylphosphine oxide (7 mg, 0.96 mmol) in dioxane (5 mL) was reacted at 100°C for 4 h. After completion of the reaction, the system was cooled to room temperature, and saturated aqueous sodium bicarbonate solution (15 mL) was added. The mixture was extracted with ethyl acetate (25 mL). The aqueous phases were combined, acidified with 2M aqueous hydrochloric acid solution (5 mL), and extracted 5 times with 5 mL of ethyl acetate each. The organic phases were combined, washed with saturated salt water, dried over anhydrous sodium sulfate, concentrated under reduced pressure, and purified by silica gel column chromatography (petroleum ether:ethyl acetate = 70:30) to give 1,4(5-chloro(1,3-thiazolo[5,4-b]pyridin-7-yl))dimethylphosphin-1-one (150 mg, 0.8 mmol, yield: 76%) as a colorless oil. LCMS (ESI): 246 [M+H]⁺.

Step 2: 5-chloro-7-cyclopropyl-1,3-thiazolo[5,4-b]pyridine (0.10 g, 0.47 mmol), tert-butyl 4-[N-(3-chloro-2,4-difluorophenyl)-N-methylcarbamoyl]-2-oxoimidazolidine carboxylate **(Intermediate 6,** 0.3 g, 0.76 mmol), tris(dibenzylideneacetone)dipalladium (53 mg, 0.08 mmol), 4,5-bis(diphenylphosphino)-9,9-dimethylxanthene (43 mg, 0.08 mmol), cesium carbonate (733 mg, 2.25 mmol), and 5-chloro(1,3-thiazolo[5,4-b]pyridin-7-yl))dimethylphosphin-1-one (185 mg, 0.75 mmol) were refluxed in dioxane (5 mL) for 2-4 h. After completion of the reaction, the obtained mixture was concentrated under reduced pressure and purified by silica gel column chromatography (petroleum ether/ethyl acetate = 80:20) to give tert-butyl 6(4S)-3-[7-(dimethylcarbonyl)(1,3-thiazolo[4,5-e]pyridin-5-yl)]-4-[N-(3-chloro-2,4-difluorophenyl)-N-methylcarbamoyl]-2-oxoimid azolidine carboxylate (40 mg, 0.75 mmol, yield: 9%) as a yellow solid. LCMS (ESI): 600 [M+H]+.

Step 3: a mixture of tert-butyl (4S)-3-[7-(dimethylcarbonyl)(1,3-thiazolo[4,5-e]pyridin-5-yl)]-4-[N-(3-chloro-2,4-difluorophenyl)-N-methylcarbamoyl]-2-oxoimida zolidine carboxylate (80 mg, 0.13 mmol) in trifluoroacetic acid (0.5 mL) and dichloromethane (2.00 mL) was stirred at room temperature for 0.5 h. After completion of the reaction, the combined organic layer was washed with salt water, dried over anhydrous sodium sulfate, filtered, concentrated under a vacuum, and purified by high-performance liquid chromatography (column: XBridge Prep OBD C18 column, 30*150 mm, 5 µm; mobile phase A: water (10 mmol/L NH4HCO3), mobile phase B: ACN; flow rate: 60 mL/min; gradient: 37% B to 67% B within 10 min; wavelength: 220 nm; retention time (min): 8.53) to give { (4S)-3-[7-(dimethylcarbonyl)(1,3-thiazolo[4,5-e]pyridin-5-yl)]-2-oxoimidazolidin-4-yl}-N-(3-chloro-2,4-difluorophenyl)-N-methylf ormamide (2.70 mg, 0.13 mmol, yield: 4%) as a white solid.

LCMS (ESI): 500.20 [M+H]⁺.

HNMR (400 MHz, DMSO-d6): δ ppm 9.48-9.42(m, 1H), 8.90-8.78(m, 1H),7.79-7.64(m, 2H), 7.49-7.42 (m, 1H), 5.79-4.23(m, 2H),3.92-3.91(m, 1H), 3.16(s,3H),1.87(s,6H).

### Example 33:

### (S)-N-(3-chloro-2,4-difluorophenyl)-N-methyl-2-oxo-3-(4-(trifluoromethyl)selenobenzo[2,3-b]pyridin-6-yl)imidazolidine-4-carboxa mide (Compound 33)

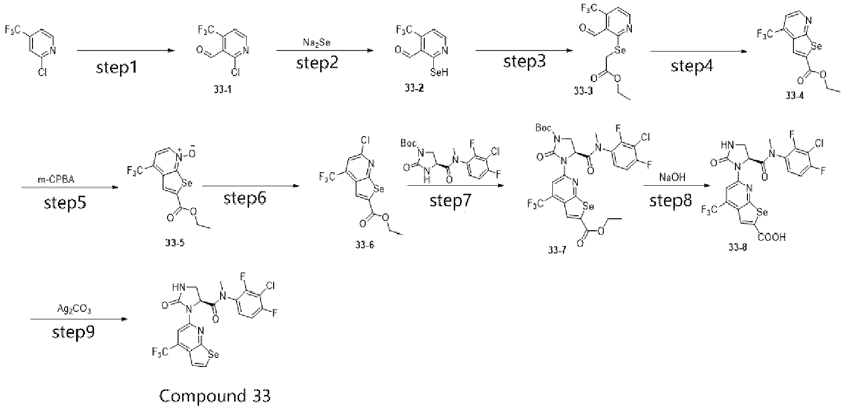

Step 1: 2-chloro-4-(trifluoromethyl)pyridine (20.00 g, 110 mmol) was dissolved in tetrahydrofuran (200 mL) and cooled to -78°C. A solution of lithium diisopropylamide in tetrahydrofuran (110 mL, 220 mmol, 2 mol/L) was added dropwise. The mixture was stirred at room temperature for 30 min and then cooled again to -78°C. Anhydrous N,N-dimethylformamide (10.96 g, 150 mmol) was added in one portion. After completion of the reaction, saturated ammonium chloride solution (100 mL) was added, and the mixture was extracted with ethyl acetate (100 mL*2). The organic phases were combined, dried over anhydrous sodium sulfate, and filtered. The filtrate was concentrated and purified by silica gel column chromatography (petroleum ether:ethyl acetate = 70:30) to give 2-chloro-4-(trifluoromethyl)nicotinaldehyde (6.50 g, yield: 28%, 31 mmol) as a yellow oil. LCMS (ESI) m/z: 210[M+H]⁺, ¹H NMR (400 MHz, DMSO-d6) δ 10.42 (q, J = 1.8 Hz, 1H), 8.89-8.88 (m, 1H), 8.00 (d, J = 5.2 Hz, 1H).

Step 2: 2-chloro-4-(trifluoromethyl)nicotinaldehyde (6.00 g, 29 mmol) was dissolved in N,N-dimethylformamide (60 mL). Sodium selenide (3.58 g, 29 mmol) was added and stirred at room temperature for 2 h. After completion of the reaction, the crude reaction solution was directly used in the next step without post-processing. LCMS(ESI) 256[M+H]⁺.

Step 3: ethyl 2-chloroacetate (7.10 g, 58 mmol) and sodium acetate (5.68 g, 58 mmol) were added to the above crude reaction solution and stirred at room temperature for 2 h. After completion of the reaction, water (30 mL) was added, and the mixture was extracted with ethyl acetate (40 mL*3). The combined organic phase was washed with saturated salt water, dried over anhydrous sodium sulfate, and filtered. The filtrate was concentrated to give the crude product ethyl 2-((3-formyl-4-(trifluoromethyl)pyridin-2-yl)seleno)acetate (7.00 g) as a yellow oil, which was directly used in the next step without further purification. LCMS (ESI) m/z: 342[M+H]⁺.

Step 4: the crude product ethyl 2-((3-formyl-4-(trifluoromethyl)pyridin-2-yl)seleno)acetate (7.00 g) was dissolved in anhydrous N,N-dimethylformamide (50 mL). Potassium carbonate (8.28 g, 60 mmol) was added and stirred at room temperature for 2 h. After completion of the reaction, the mixture was filtered to remove the potassium carbonate solid. The filtrate was added with water (50 mL) and extracted with ethyl acetate (60 mL*3). The combined organic phase was washed with saturated salt water, dried over anhydrous sodium sulfate, and filtered. The filtrate was concentrated. The obtained crude product was purified by silica gel column chromatography (petroleum ether:ethyl acetate = 70:30) to give ethyl 4-(trifluoromethyl)selenobenzo[2,3-b]pyridine-2-carboxylate (3.50 g, 11 mmol, yield: 55%) as a light yellow solid. LCMS(ESI) 324[M+H]⁺, 1H NMR (400 MHz, DMSO-d6) δ 8.23 (t, J = 1.8 Hz, 1H), 8.14 (d, J = 0.8 Hz, 1H), 4.41 (q, J = 7.2 Hz, 2H), 1.35 (t, J = 7.2 Hz,3H).

Step 5: ethyl 4-(trifluoromethyl)selenobenzo[2,3-b]pyridine-2-carboxylate (2.00 g, 6 mmol) was dissolved in acetic acid (20 mL), and 30% aqueous hydrogen peroxide solution (4 mL, 30 mmol) was added. The system was warmed to 80°C and stirred for 2 h. After completion of the reaction, the reaction solution was concentrated to give the crude product 2-(ethoxycarbonyl)-4-(trifluoromethyl)selenobenzo[2,3-b]pyridine 7-oxide (2.00 g) as a light yellow solid, which was used in the next step without purification. LCMS (ESI) m/z: 340[M+H]⁺.

Step 6: the crude product 2-(ethoxycarbonyl)-4-(trifluoromethyl)selenobenzo[2,3-b]pyridine 7-oxide (2.00 g) was added to phosphorus oxychloride (20 mL) solution at room temperature. The mixture was stirred at 120 °C for 2 h. After completion of the reaction, the system was cooled to room temperature. The obtained mixture was concentrated under reduced pressure. The obtained crude product was purified by silica gel column chromatography (petroleum ether:ethyl acetate = 70:30) to give ethyl 6-chloro-4-(trifluoromethyl)selenobenzo[2,3-b]pyridine-2-carboxylate (1.80 g, 5 mmol, yield: 84.75%) as a light yellow solid. LCMS (ESI) m/z: 358[M+H]⁺.

Step 7: ethyl 6-chloro-4-(trifluoromethyl)selenobenzo[2,3-b]pyridine-2-carboxylate (150 mg, 0.42 mmol), (S)-tert-butyl 4-((3-chloro-2,4-difluorophenyl)(methyl)carbamoyl)-2-oxoimidazolidine-1-carboxylate (**Intermediate 6,** 163 mg, 0.42 mmol), tris(dibenzylideneacetone)dipalladium (37 mg, 0.04 mmol), 4,5-bis(diphenylphosphine)-9,9-dimethylxanthene (23 mg, 0.04 mmol), and potassium carbonate (174 mg, 1.26 mmol) were dissolved in dioxane (15 mL) solution and reacted at 80°C for 1 h. After completion of the reaction, the system was cooled to room temperature. The reaction solution was poured into water (10 mL). The obtained mixture was extracted with ethyl acetate (20 mL x 2). The combined organic layer was washed with saturated salt water and dried over anhydrous sodium sulfate. After filtration, the filtrate was concentrated under reduced pressure. The crude product was purified by silica gel column chromatography (petroleum ether:ethyl acetate = 60:40) to give (S)-ethyl 6-(3-(tert-butoxycarbonyl)-5-((3-chloro-2,4-difluorophenyl)(methyl)carbamoyl)-2-oxoimidazolidin-1-yl)-4-(trifluoromethyl)selenobe nzo[2,3-b]pyridine-2-carboxylate (130 mg, 0.18 mmol, yield: 44%) as a light yellow solid. LCMS (ESI) m/z: 711[M+H]⁺.

Step 8: ethyl (S)-6-(3-(tert-butoxycarbonyl)-5-((3-chloro-2,4-difluorophenyl)(methyl)carbamoyl)-2-oxoimidazolidin-1-yl)-4-(trifluoromethyl)sele nobenzo[2,3-b]pyridine-2-carboxylate (130 mg, 0.18 mmol) was dissolved in methanol (3 mL) solution. Sodium hydroxide (72 mg, 1.8 mmol) was dissolved in water (1 mL) and added to the above methanol solution. The system was warmed to 70°C and stirred for 1 h. After the completion of the reaction, the system was cooled to room temperature. The reaction solution was poured into water (5 mL). The obtained mixture was extracted with ethyl acetate (20 mL x 2). The aqueous phase was retained, adjusted to about pH 3 with 2 mol/L aqueous hydrochloric acid solution, and extracted with ethyl acetate (20 mL*3). The organic phase was dried over anhydrous sodium sulfate and filtered. The filtrate was concentrated to give the crude product (S)-6-(5-((3-chloro-2,4-difluorophenyl)(methyl)carbamoyl)-2-oxoimidazolidin-1-yl)-4-(trifluoromethyl)selenobenzo[2,3-b]pyridine-2-carboxylic acid (90 mg) as a white solid. LCMS (ESI) m/z: 583[M+H]⁺.

Step 9: the crude product (S)-6-(5-((3-chloro-2,4-difluorophenyl)(methyl)carbamoyl)-2-oxoimidazolidin-1-yl)-4-(trifluoromethyl)selenobenzo[2,3-b]pyridine-2-carboxylic acid (90 mg, 15.46 mmol), silver carbonate (1.7 mg, 0.006 mmol), and acetic acid (0.7 mg, 0.012 mmol) were dissolved in dimethyl sulfoxide (4 mL) solution. The system was warmed to 100°C and stirred for 4 h. After completion of the reaction, the obtained mixture was directly filtered to give a crude product. The crude product was purified by high-performance liquid chromatography (column: Kinetex EVO prep C18, 30*150, 5 µm; mobile phase A: 10 mmol/L ammonium bicarbonate aqueous solution, mobile phase B: acetonitrile; flow rate: 60 mL/min; gradient: 37% B to 67% B within 10 min; wavelength: 220 nm; retention time: 8.53 min) to give (S)-N-(3-chloro-2,4-difluorophenyl)-N-methyl-2-oxo-3-(4-(trifluoromethyl)selenobenzo[2,3-b]pyridin-6-yl)imidazolidine-4-carboxa mide (3.88 mg, 0.007 mmol, yield: 4.8%) as a white solid.

LCMS(ESI) 538.85[M+H]⁺.

HNMR (400 MHz, DMSO-d6): δ ppm 8.67-8.64(m, 1H), 8.45-8.36(m, 1H), 7.83-7.70(m, 1H), 7.66-7.37 (m,3H), 5.82-4.86 (m,1H), 5.81-4.86(m,1H), 3.95-3.65(m,2H), 3.25-3.12(m,3H).

### Example 34:

### (S)-N-(3-chloro-2,4-difluorophenyl)-N-methyl-3-(2-methyl-7-(trifluoromethyl)thiazolo[5,4-b]pyridin-5-yl)-2-oxoimidazolidine-4-car boxamide (Compound 34)

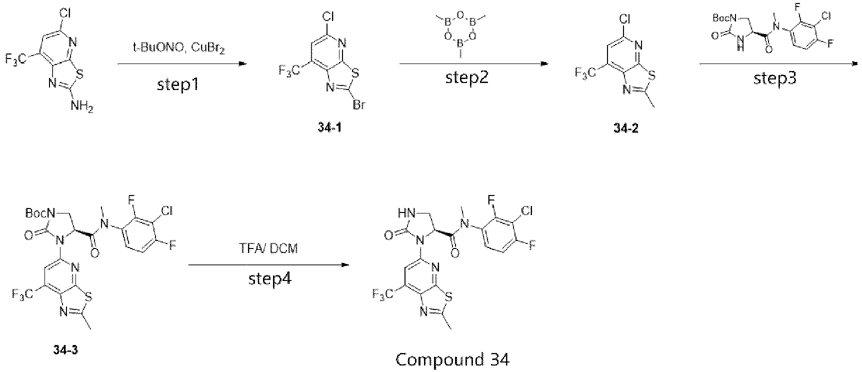

Step 1: 5-chloro-7-(trifluoromethyl)thiazolo[5,4-b]pyridin-2-amine (500 mg, 1.97 mmol) was dissolved in acetonitrile (20 mL) and cooled to 0°C. Tert-butyl nitrite (0.35 mL) was added at 0°C, and the system was reacted at 0°C for 10 min. Copper bromide (660.46 mg, 2.96 mmol) was then added, and the system was reacted at 0°C for 30 min and then at room temperature for 2 h. The desired product can be detected by LCMS. The obtained mixture was extracted with dichloromethane (3 x 10 mL). The combined organic layer was washed with salt water (3 x 5 mL) and dried over anhydrous sodium sulfate. After filtration, the filtrate was concentrated under reduced pressure. The residue was purified by silica gel column chromatography and eluted with petroleum ether/ethyl acetate (5:1) to give 2-bromo-5-chloro-7-(trifluoromethyl)thiazolo[5,4-b]pyridine (435 mg, yield: 70%) as a yellow solid. LCMS (ESI): 317 [M+H]+.

Step 2: 2-bromo-5-chloro-7-(trifluoromethyl)thiazolo[5,4-b]pyridine (300 mg, 0.94 mmol) was dissolved in 1,4-dioxane (10 mL) solution. Trimethylboroxine (0.32 mL, 1.13 mmol) and potassium carbonate (195.58 mg, 1.42 mmol) were added at room temperature, followed by tetrakis(triphenylphosphine)palladium (109.13 mg, 0.09 mmol) under a nitrogen atmosphere. The reaction was reacted at 100°C for 4 h. The desired product can be detected by LCMS. The obtained mixture was extracted with dichloromethane (3 x 10 mL). The combined organic layer was washed with saturated salt water (5 mL x 3) and dried over anhydrous sodium sulfate. After filtration, the filtrate was concentrated under reduced pressure. The residue was purified by silica gel column chromatography and eluted with petroleum ether/ethyl acetate (3:1) to give 5-chloro-2-methyl-7-(trifluoromethyl)thiazolo[5,4-b]pyridine (145 mg, yield: 61%) as a yellow solid. LCMS (ESI): 253 [M+H]+.

Step 3: 5-chloro-2-methyl-7-(trifluoromethyl)thiazolo[5,4-b]pyridine (130 mg, 0.51 mmol), (S)-tert-butyl 4-((3-chloro-2,4-difluorophenyl)(methyl)carbamoyl)-2-oxoimidazolidine-1-carboxylate (**Intermediate 6,** 160.45 mg, 0.41 mmol), tris(dibenzylideneacetone)dipalladium (47.08 mg, 0.05 mmol), 4,5-bis(diphenylphosphine)-9,9-dimethylxanthene (29.74 mg, 0.05 mmol), and potassium carbonate (213.00 mg, 1.54 mmol) were dissolved in 1,4-dioxane (5 mL) solution and reacted at 80°C for 2 h. The desired product can be detected by LCMS. The obtained mixture was extracted with dichloromethane (3 x 5 mL). The combined organic layer was washed with salt water (3 x 1 mL) and dried over anhydrous sodium sulfate. After filtration, the filtrate was concentrated under reduced pressure. The residue was purified by silica gel column chromatography and eluted with petroleum ether/ethyl acetate (1:1) to give (S)-tert-butyl 4-((3-chloro-2,4-difluorophenyl)(methyl)carbamoyl)-3-(2-methyl-7-(trifluoromethyl)thiazolo[5,4-b]pyridin-5-yl)-2-oxoimidazolidine -1-carboxylate (200 mg, yield: 64%) as a yellow solid. LCMS (ESI): 606 [M+H]+.

Step 4: (S)-tert-butyl 4-((3-chloro-2,4-difluorophenyl)(methyl)carbamoyl)-3-(2-methyl-7-(trifluoromethyl)thiazolo[5,4-b]pyridin-5-yl)-2-oxoimidazolidine -1-carboxylate (180 mg, 0.30 mmol) was dissolved in 5 mL of trifluoroacetic acid:dichloromethane (1:5) and reacted at room temperature for 0.5 h. The desired product can be detected by LCMS. After distillation under reduced pressure, the mixture was passed through a column: XBridge Prep OBD C18 column, 30*150 mm, 5 µm; mobile phase A: water (10 mmol/L ammonium bicarbonate), mobile phase B: acetonitrile; flow rate: 60 mL/min; gradient: 33% B to 65% B within 8 min; wavelength: 220 nm; retention time 7.42 min; run times: 0, to give (S)-N-(3-chloro-2,4-difluorophenyl)-N-methyl-3-(2-methyl-7-(trifluoromethyl)thiazolo[5,4-b]pyridin-5-yl)-2-oxoimidazolidine-4-car boxamide (101.44 mg, yield: 68%) as a white solid.

LCMS (ESI): 505.80 [M+H]+.

HNMR (400 MHz, DMSO-d6): δ ppm 8.70-8.69(s, 1H), 7.85-7.73(m, 1H),7.73-7.63(m, 1H), 7.52-7.39 (m, 1H), 5.80-4.83 (m, 1H),3.90-3.41(m, 2H), 3.24-3.11(s,3H),2.90-2.93(s,3H).

### Example 35:

### { (4S)-3-[2-amino-7-(trifluoromethyl)(1,3-thiazolo[4,5-e]pyridin-5-yl)]-2-oxoimidazolidin-4-yl}-N-(3-chloro-2,4-difluorophenyl)-N-methylformamide (Compound 35)

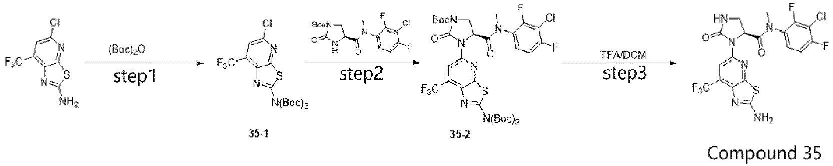

Step 1: a mixture of 5-chloro-7-(trifluoromethyl)-1,3-thiazolo[5,4-b]pyridin-2-ylamine (100.00 mg, 0.39 mmol), di-tert-butyl dicarbonate (257.86 mg, 1.18 mmol), and dimethylaminopyridine (0.12 mg, 0.00 mmol) in dichloromethane (3.00 mL) was stirred at room temperature for 12 h. After completion of the reaction, the reaction mixture was diluted with water and extracted with ethyl acetate. The combined organic layer was washed with salt water, dried over anhydrous sodium sulfate, filtered, and concentrated under a vacuum. The crude product was purified by column chromatography (petroleum ether:ethyl acetate = 90:10) to give the product tert-butyl fbis(tert-butoxy)-N-[5-chloro-7-(trifluoromethyl)(1,3-thiazolo[5,4-b]pyridin-2-yl)]carbonylaminol carboxylate (170 mg, 0.37 mmol, yield: 60%) as a colorless oil. LCMS (ESI): 454 [M+H]⁺.

Step 2: tert-butyl fbis(tert-butoxy)-N-[5-chloro-7-(trifluoromethyl)(1,3-thiazolo[5,4-b]pyridin-2-yl)]carbonylaminol carboxylate (100.00 mg, 0.22 mmol), (S)-tert-butyl 4-((3-chloro-2,4-difluorophenyl)(methyl)carbamoyl)-2-oxoimidazolidine-1-carboxylate (**Intermediate 6**, 85.88 mg, 0.22 mmol), tris(dibenzylideneacetone)dipalladium (20.18 mg, 0.02 mmol), 4,5-bis(diphenylphosphino)-9,9-dimethylxanthene (12.76 mg, 0.02 mmol), and potassium carbonate (91.22 mg, 0.66 mmol) were reacted in dioxane (10.00 mL) at 100°C for 1 h. After completion of the reaction, the reaction mixture was diluted with water and extracted with ethyl acetate. The combined organic layer was washed with salt water, dried over anhydrous sodium sulfate, filtered, and concentrated under a vacuum. The crude product was purified by column chromatography (petroleum ether:ethyl acetate = 70:30) to give the product { (4S)-tris(tert-butoxy)-3-[2-amino-7-(trifluoromethyl)(1,3-thiazolo[4,5-e]pyridin-5-yl)]-2-oxoimidazolidin-4-yl }-N-(3-chloro-2,4-dif luorophenyl)-N-methylformamide (200 mg, 0.24 mmol, yield: 80%) as a yellow solid. LCMS (ESI): 807[M+H]⁺.

Step 3: a mixture of { (4S)-tris(tert-butoxy)-3-[2-amino-7-(trifluoromethyl)(1,3-thiazolo[4,5-e]pyridin-5-yl)]-2-oxoimidazolidin-4-yl }-N-(3-chloro-2,4-dif luorophenyl)-N-methylformamide (200.00 mg, 0.25 mmol) and trifluoroacetic acid (2.00 mL) in dichloromethane (10.00 mL) was stirred at room temperature for 1 h. After completion of the reaction, the reaction was quenched with saturated salt and extracted with ethyl acetate. The combined organic layer was washed with salt water, dried over anhydrous sodium sulfate, filtered, and concentrated under a vacuum. The crude product was purified by high-performance liquid chromatography (column: Xselect CSH C18 OBD column 30*150 mm, 5 µm; mobile phase A: water (0.05% formic acid), mobile phase B: acetonitrile; flow rate: 60 mL/min; gradient: 25% B to 50% B within 8 min; wavelength: 220 nm; retention time: 7.15 min) to give { (4S)-3-[2-amino-7-(trifluoromethyl)(1,3-thiazolo[4,5-e]pyridin-5-yl)]-2-oxoimidazolidin-4-yl}-N-(3-chloro-2,4-difluorophenyl)-N-methylformamide (20.22 mg, yield: 16%) as a white solid.

LCMS (ESI): 507.25[M+H],

HNMR (400 MHz, DMSO-d6): δ ppm 8.40-8.34(s, 1H), 8.13-8.06(m,2H),7.68-7.57(m, 1H), 7.55-7.38 (m, 2H), 5.70-4.75(m, 1H),3.86-3.45(m, 2H), 3.22-3.16(m,3H).

### Example 36:

### (S)-N-(3-chloro-2,4-difluorophenyl)-3-(2-cyano-7-(trifluoromethyl)thiazolo[5,4-b]pyridin-5-yl)-N-methyl-2-oxoimidazolidine-4-carb oxamide (Compound 36)

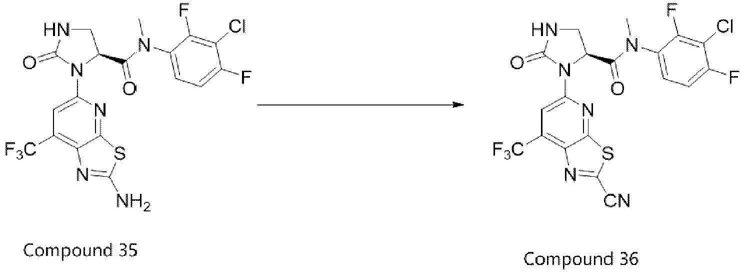

A solution of cuprous cyanide (26.64 mg, 0.30 mmol) and tert-butyl nitrite (30.48 mg, 0.30 mmol) in acetonitrile (10.00 mL) was stirred at 50°C for 10 min. Then, {(4S)-3-[2-amino-7-(trifluoromethyl) (1,3-thiazolo[4,5-e]pyridin-5-yl)]-2-oxoimidazolidin-4-yl}-N-(3-chloro-2,4-difluorophenyl)-N-methylformamide (**Compound 35**, 100.00 mg, 0.20 mmol) in acetonitrile (5.00 mL) was added to the mixture at the same temperature. The reaction solution was concentrated. The crude product was purified by prep-HPLC: column: XBridge Prep OBD C18 column, 30*150 mm, 5 µm; mobile phase A: water (10 mmol/L NH4HCO3), mobile phase B: ACN; flow rate: 60 mL/min; gradient: 37% B to 67% B within 10 min; wavelength: 220 nm; RT1 (min): 8.53, to give (S)-N-(3-chloro-2,4-difluorophenyl)-3-(2-cyano-7-(trifluoromethyl)thiazolo[5,4-b]pyridin-5-yl)-N-methyl-2-oxoimidazolidine-4-carb oxamide (3.57 mg, yield: 3%) as a yellow solid.

LCMS (ESI): 517.20[M+H]+.

HNMR (400 MHz, DMSO-d6): δ ppm 9.03-8.97(m, 1H), 8.13-8.05(m, 1H),7.74-7.68(m, 1H), 7.52-7.48 (m, 1H), 5.07-4.86(m, 1H),3.62-3.58(m, 1H), 3.51-3.49(m,1H),3.22-3.17(m,3H).

### Example 37:

### { (25,3 S,4S)-1-[7,7-difluoro-4-(trifluoromethyl)(5,6,7-trihydrocyclopenta[1,2-e]pyridin-2-yl)]-3,4-dihydroxy-5-oxopyrrolidin-2-yl }-N-(5-chloro-2,4-difluorophenyl)-N-methylformamide (Compound 37)

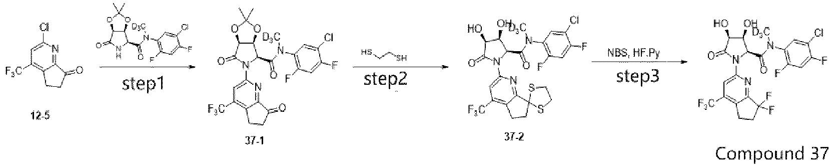

Step 1: a solution of ((1S,2S,5S)-7,7-dimethyl-4-oxo-6,8-dioxa-3-azabicyclo[3.3.0]octan-2-yl)-N-(5-chloro-2,4-difluorophenyl)-N-methylformamide (**Intermediate 4,** 400.00 mg, 1.10 mmol), 2-chloro-4-(trifluoromethyl)-5,6-dihydrocyclopenta[2,1-b]pyridin-7-one (**Compound 12-5,** 388.59 mg, 1.65 mmol), potassium carbonate (455.25 mg, 3.30 mmol), tris(dibenzylideneacetone)dipalladium (100.70 mg, 0.0.11 mmol), and 4,5-bis(diphenylphosphino)-9,9-dimethylxanthene (63.67 mg, 0.11 mmol) in dioxane (20.00 mL) was stirred at 80°C for 2 h. After completion of the reaction, the system was cooled to room temperature, and saturated aqueous sodium bicarbonate solution (15 mL) was added. The mixture was extracted with ethyl acetate (25 mL). The aqueous phases were combined and extracted 5 times with 5 mL of ethyl acetate each. The organic phases were combined, washed with saturated salt water, dried over anhydrous sodium sulfate, and concentrated under reduced pressure to give {(1S,2S,5S)-7,7-dimethyl-4-oxo-3-[7-oxo-4-(trifluoromethyl)(5,6-dihydrocyclopenta[1,2-e]pyridin-2-yl)]-6,8-dioxa-3-azabicyclo[3.3 .0]octan-2-yl}-N-(5-chloro-2,4-difluorophenyl)-N-methylformamide (140.00 mg, 1.09 mmol, yield: 23%) as a colorless oil. LCMS (ESI): 563[M+H]⁺.

Step 2: to a solution of {(1S,2S,5S)-7,7-dimethyl-4-oxo-3-[7-oxo-4-(trifluoromethyl)(5,6-dihydrocyclopenta[1,2-e]pyridin-2-yl)]-6,8-dioxa-3-azabicyclo[3.3 .0]octan-2-yl}-N-(5-chloro-2,4-difluorophenyl)-N-methylformamide (140.00 mg, 0.25 mmol) in acetic acid (8.00 mL) was added ethane-1,2-dithiol (70.29 mg, 0.75 mmol) and aluminum trichloride (13.27 mg, 0.10 mmol). The reaction system was then replaced with nitrogen three times. The obtained mixture was stirred at 80°C overnight for 2 h. After completion, the reaction mixture was diluted with water and extracted with ethyl acetate. The combined organic layer was washed with salt water, dried over anhydrous sodium sulfate, filtered, and concentrated under a vacuum. The crude product was purified by reverse phase low-pressure chromatography (5% to 60% ammonium bicarbonate aqueous solution/acetonitrile, yield: 30%) to give {(25,3S,4S)-3,4-dihydroxy-5-oxo-1-[9-(trifluoromethyl)spiro[1,3-dithiolane-2,7'-5,6,7-trihydrocyclopenta[2,1-e]pyridin]-7-yl]pyrrol idin-2-yl}-N-(5-chloro-2,4-difluorophenyl)-N-methylformamide (60.00 mg, 0.24 mmol, yield: 40%), LCMS (ESI): 599 [M+H]+. Step 3: to a solution of N-bromosuccinimide (35.66 mg, 0.20 mmol) in dichloromethane (6.00 mL) was added pyridine hydrofluoride (0.60 mL) at -78°C. The mixture was stirred for 0.5 h. {(25,3S,4S)-3,4-Dihydroxy-5-oxo-1-[9-(trifluoromethyl)spiro[1,3-dithiolane-2,7'-5,6,7-trihydrocyclopenta[1,2-e]pyridin]-7-yl]pyrrol idin-2-yl}-N-(5-chloro-2,4-difluorophenyl)-N-methylformamide (90.00 mg, 0.02 mmol) in dichloromethane (0.5 mL) was then added. After completion, the reaction mixture was diluted with ice water and extracted with dichloromethane. The combined organic layer was washed with salt water, dried over anhydrous sodium sulfate, filtered, and concentrated under a vacuum. The reaction solution was concentrated. The residue was purified by reverse phase low-pressure chromatography (5% to 60% ammonium bicarbonate aqueous solution/acetonitrile, yield: 45%) to give a crude product (60 mg). The crude product was purified by high-performance liquid phase: column: Xselect CSH C18 OBD column 30*150 mm 5 µm; mobile phase A: water (0.05% FA), mobile phase B: acetonitrile; flow rate: 60 mL/min; gradient: 25% B to 50% B within 8 min; wavelength: 220 nm; retention time: 7.15 min, to give { (25,3 S,4S)-1-[7,7-difluoro-4-(trifluoromethyl)(5,6,7-trihydrocyclopenta[1,2-e]pyridin-2-yl)]-3,4-dihydroxy-5-oxopyrrolidin-2-yl }-N-(5-chloro-2,4-difluorophenyl)-N-methylformamide (11.18 mg, 0.1 mmol, yield: 20%) as a white solid.

LCMS (ESI): 545.00[M+H]⁺.

HNMR (400 MHz, DMSO-d6): δ ppm 8.60-8.52(m, 1H), 7.94-7.43(m, 2H), 5.74-5.25(m, 2H),5.04-4.79(m, 1H), 4.48-4.33(m,1H),4.09-3.98(m,1H),3.20-3.19(m,2H), 2.79-2.74(m,2H).

### Example 38:

### 6-1(5S)-5-[N-(3-chloro-2,4-difluorophenyl)-N-methylcarbamoyl]-2-oxoimidazolidinyl]-4-(trifluoromethyl)thieno[2,3-b]pyridine-2-c arboxamide (Compound 38)

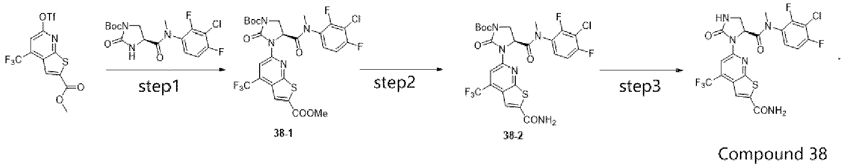

Step 1: methyl 4-(trifluoromethyl)-6-(((trifluoromethyl)sulfonyl)oxy)thieno[2,3-b]pyridine-2-carboxylate (800 mg, 1.96 mmol) and (S)-tert-butyl 4-((3-chloro-2,4-difluorophenyl)(methyl)carbamoyl)-2-oxoimidazolidine-1-carboxylate (**Intermediate 6,** 768 mg, 1.96 mmol) were dissolved in 1,4-dioxane (50 mL). Tris(dibenzylideneacetone)dipalladium (44 mg, 0.20 mmol), 4,5-bis(diphenylphosphino)-9,9-dimethylxanthene (0.14 g, 0.20 mmol), and anhydrous potassium carbonate (4.08 g, 3.88 mmol) were then added. The mixture was reacted at 80°C for 1 h. After completion of the reaction, the reaction solution was poured into saturated aqueous sodium bicarbonate solution (60 mL) and extracted with ethyl acetate (60 mL x 3). The organic phases were combined, washed with saturated salt water, dried over anhydrous sodium sulfate, and filtered to remove anhydrous sodium sulfate. The filtrate was concentrated to give a crude product. The crude product was purified by silica gel column chromatography (ethyl acetate:petroleum ether = 20:80) to give (S)-methyl 6-(3-(tert-butoxycarbonyl)-5-((3-chloro-2,4-difluorophenyl)(methyl)carbamoyl)-2-oxoimidazolidin-1-yl)-4-(trifluoromethyl)thieno[2, 3-b]pyridine-2-carboxylate (780 mg, 1.20 mmol, yield: 61%) as a light yellow solid powder. LCMS (ESI) *m*/*z*: 649 [M+H]⁺.

Step 2: methyl (S)-6-(3-(tert-butoxycarbonyl)-5-((3-chloro-2,4-difluorophenyl)(methyl)carbamoyl)-2-oxoimidazolidin-1-yl)-4-(trifluoromethyl)thien o[2,3-b]pyridine-2-carboxylate (350 mg, 0.54 mmol) was dissolved in ammonia-methanol (7M, 30 mL) solution and reacted at 80°C for 2 h. After completion of the reaction, the reaction solution was poured into water (30 mL) and extracted with ethyl acetate (30 mL x 3). The organic phases were combined, washed with saturated salt water, dried over anhydrous sodium sulfate, and filtered to remove anhydrous sodium sulfate. The filtrate was concentrated to give a crude product. The crude product was purified by silica gel column chromatography (ethyl acetate:petroleum ether = 30:70) to give (S)-tert-butyl 3-(2-carbamoyl-4-(trifluoromethyl)thieno[2,3-b]pyridin-6-yl)-4-((3-chloro-2,4-difluorophenyl)(methyl)carbamoyl)-2-oxoimidazolidi ne-1-carboxylate (250 mg, 0.40 mmol, yield: 73%) as a light yellow solid powder. LCMS (ESI) *m*/*z*: 634 [M+H]⁺.

Step 3: (S)-tert-butyl 3-(2-carbamoyl-4-(trifluoromethyl)thieno[2,3-b]pyridin-6-yl)-4-((3-chloro-2,4-difluorophenyl)(methyl)carbamoyl)-2-oxoimidazolidi ne-1-carboxylate (125 mg, 0.20 mmol) was dissolved in trifluoroacetic acid (5 mL) and dichloromethane (25 mL). The mixture was reacted at room temperature for 1 h. After completion of the reaction, the reaction solution was concentrated to give a crude product. The crude product was purified by preparative liquid chromatography (column: Kinetex EVO prep C18, 30*150, 5 µm; mobile phase A: water (10 mmol/L NH4HCO3), mobile phase B: ACN; flow rate: 60 mL/min; gradient: 20% B to 50% B within 8 min; wavelength: 220 nm; RT1 (min): 7.97; run times: 0) to give 29.86 mg of 6-{(5S)-5-[N-(3-chloro-2,4-difhjorophenyl)-N-methylcarbamoyl]-2-oxoimidazolidinyl}-4-(trifhioromethyl)thieno[2,3-b]pyridine-2-c arboxamide as a white solid.

LCMS(ESI) 534.20 [M+H]⁺.

¹H NMR (400 MHz, DMSO-d6) δ 8.77 - 8.70 (m, 1H), 8.45-8.42 (m, 1H), 8.19 - 8.16 (m, 1H), 7.90-7.82 (m, 1H), 7.77-7.73 (m, 1H), 7.72-7.67 (m, 1H), 7.51 - 7.47 (m, 1H), 5.65 - 4.66 (m, 1H), 3.60 - 3.41 (m, 2H), 3.23-3.20 (m, 3H).

### Example 39:

### (S)-N-(3-chloro-2,4-difluorophenyl)-3-(2-cyano-4-(trifluoromethyl)thieno[2,3-b]pyridin-6-yl)-N-methyl-2-oxoimidazolidine-4-carbo xamide (Compound 39)

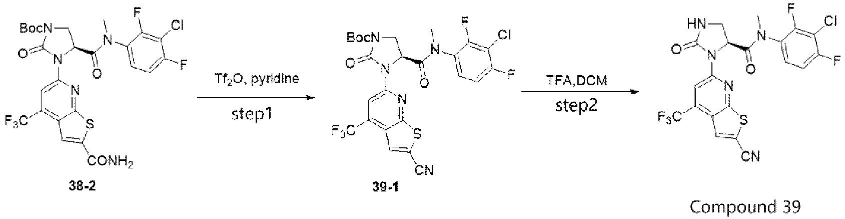

Step 1: a mixture of tert-butyl (4S)-3-[2-carbamoyl-4-(trifluoromethyl) thieno [3, 2-e] pyridin -6-yl]-4-[N-(3-chloro-2, 4-difluorophenyl)-N-methylcarbamoyl]-2-oxoimidazolidine carboxylate (500.00 mg, 0.79 mmol), pyridine (7.5 mg, 9.46 mmol), and trifluoroacetic anhydride (198.77 mg, 0.95 mmol) in dichloromethane (0.50 mL) were stirred at room temperature for 12 h. The mixture was then concentrated and purified by column chromatography (petroleum ether:ethyl acetate = 70:30) to give (S)-tert-butyl 4-((3-chloro-2,4-difluorophenyl)(methyl)carbamoyl)-3-(2-cyano-4-(trifluoromethyl)thieno[2,3-b]pyridin-6-yl)-2-oxoimidazolidine-1-carboxylate (200 mg, yield: 59%) as a colorless oil. LCMS (ESI): 616 [M+H]⁺.

Step 2: (S)-tert-butyl 4-((3-chloro-2,4-difluorophenyl)(methyl)carbamoyl)-3-(2-cyano-4-(trifluoromethyl)thieno[2,3-b]pyridin-6-yl)-2-oxoimidazolidine-1-carboxylate (100 mg, 0.17 mmol) was dissolved in trifluoroacetic acid (1 mL) and dichloromethane (5 mL), and the system was stirred at room temperature for 1 h. After completion of the reaction, the obtained mixture was concentrated under reduced pressure. The crude product (50 mg) was purified by preparative high performance liquid chromatography under the following conditions: column: Xselect CSH C18 OBD column 30*150 mm, 5 µm; mobile phase A: water (0.05% trifluoroacetic acid), mobile phase B: acetonitrile; flow rate: 60 mL/min; gradient: 50% B to 90% B within 8 min; wavelength: 220 nm; RT1 (min): 6.22; run times: 0, to give
(S)-N-(3-chloro-2,4-difluorophenyl)-3-(2-cyano-4-(trifluoromethyl)thieno[2,3-b]pyridin-6-yl)-N-methyl-2-oxoimidazolidine-4-carbo xamide (4.94 mg, 0.009 mmol, yield: 63%) as a white solid.

LCMS (ESI): 516.25 [M+H]+.

HNMR (400 MHz, DMSO-d6): δ ppm8.87-8.81(m, 1H), 8.45-8.42(m, 1H), 8.05-7.96(m, 1H), 7.76-7.69 (m,1H), 7.47-7.39(m,1H), 5.82-4.86(m, 1H), 3.61-3.55(m, 1H), 3.42-3.40(m,1H),3.25-3.18(m,3H).

### Example 40:

### (6-{ (5 S)-5-[N-(3-chloro-2,4-difluorophenyl)-N-methylcarbamoyl]-2-oxoimidazolidinyl }-4-(trifluoromethyl)thieno [2,3-b]pyridin-2-y 1)-N-methylformamide (Compound 40)

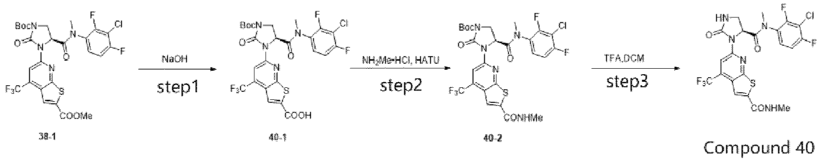

Step 1: a mixture of methyl 6-{ (5 S)-3-[(tert-butyl)oxycarbonyl]-5-[N-(3-chloro-2,4-difluorophenyl)-N-methylcarbamoyl]-2-oxoimidazolidinyl}-4-(trifluorometh yl)thieno[2,3-b]pyridine-2-carboxylate (500 mg, 0.77 mmol) and sodium hydroxide (61 mg, 1.54 mmol) in methanol (10.00 mL) was stirred at room temperature for 5h, adjusted to acidic (pH = 3) with dilute hydrochloric acid, and then filtered to give 6-{ (5 S)-3-[(tert-butyl)oxycarbonyl]-5-[N-(3-chloro-2,4-difluorophenyl)-N-methylcarbamoyl]-2-oxoimidazolidinyl}-4-(trifluorometh yl)thieno[2,3-b]pyridine-2-carboxylic acid (300 mg, 0.77 mmol, yield: 61%) as a colorless oil. LCMS (ESI):634 [M+H]⁺.

Step 2: (S)-6-(3-(tert-butoxycarbonyl)-5-((3-chloro-2,4-difluorophenyl)(methyl)carbamoyl)-2-oxoimidazolidin-1-yl)-4-(trifluoromethyl)thien o[2,3-b]pyridine-2-carboxylic acid (300 mg, 0.47 mmol), methylamine (27 mg, 0.94 mmol), 2-(7-azabenzotriazole)-N,N,N',N'-tetramethyluronium hexafluorophosphate (303 mg, 0.94 mmol), and N,N-diisopropylethyl amine (182 mg, 1.42 mmol) were stirred in dimethylformamide (5.00 mL) for 2 h. The mixture was then poured into water (10 mL), extracted with ethyl acetate (10 mL), dried over anhydrous sodium sulfate, filtered, and concentrated to give a crude product. The crude product was then purified by column chromatography (petroleum ether:ethyl acetate = 70:30) to give tert-butyl (S)-4-((3-chloro-2,4-difluorophenyl)(methyl)carbamoyl)-3-(2-(methylcarbamoyl)-4-(trifluoromethyl)thieno[2,3-b]pyridin-6-yl 2-oxoimidazolidine-1-carboxylate (200.00 mg, 0.47 mmol, yield: 65%) as a yellow solid. LCMS (ESI): 648[M+H]+.

Step 3: a mixture of tert-butyl (S)-4-((3-chloro-2,4-difluorophenyl)(methyl)carbamoyl)-3-(2-(methylcarbamoyl)-4-(trifluoromethyl)thieno[2,3-b]pyridin-6-yl 2-oxoimidazolidine-1-carboxylate (250 mg, 0.39 mmol) in trifluoroacetic acid (2 mL) and dichloromethane (6 mL) was stirred at room temperature for 2 h. The mixture was spin-dried and purified by high-performance liquid chromatography (column: XBridge Shield RP18 OBD column, 30 x 150 mm, 5 µm; mobile phase A: water (10 mmol/L ammonium bicarbonate), mobile phase B: acetonitrile; flow rate: 60 mL/min; gradient: 30% B to 60% B within 10 min; wavelength: 220 nm; retention time (min): 8.62) to give (6-{ (5 S)-5-[N-(3-chloro-2,4-difluorophenyl)-N-methylcarbamoyl]-2-oxoimidazolidinyl }-4-(trifluoromethyl)thieno [2,3-b]pyridin-2-y l)-N-methylformamide (30.73 mg, 0.38 mmol, yield: 14%) as a white solid.

LCMS (ESI): 548.25 [M+H]⁺.

HNMR (400 MHz, DMSO-d6): δ ppm 8.96-8.92(m, 1H), 8.76-8.72(m, 1H),8.14-8.10(m, 1H), 7.90-7.75 (m, 1H), 7.74-7.68 (m, 1H), 7.51-7.39(m, 1H), 5.81-4.86(m, 1H),3.58-3.48(m,2H), 3.27-3.23(m,3H),2.83-2.82(m,3H).

### Example 41: (S)-N-(3-chloro-2,4-difluorophenyl)-3-(imidazo[1,2-a]pyrazin-8-yl)-N-methyl-2-oxoimidazolidine-4-carboxamide (Compound 41)

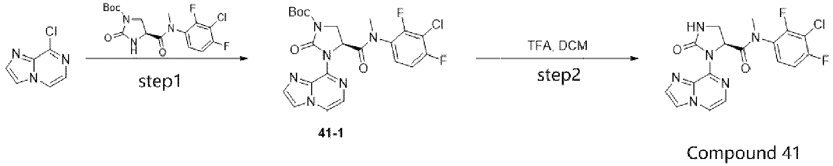

Step 1: a solution of 8-chloro-4-hydroimidazo[1,2-a]pyrazine (100 mg, 0.65 mmol), tert-butyl (4S)-4-[N-(3-chloro-2,4-difluorophenyl)-N-methylcarbamoyl]-2-oxoimidazolidine carboxylate (**Intermediate 6,** 253 mg, 0.65 mmol), potassium carbonate (269 mg, 1.95 mmol), palladium acetate (14 mg, 0.07 mmol), and 4,5-bis(diphenylphosphine)-9,9-dimethylxanthene (37 mg, 0.07 mmol) in dioxane (10 mL) was reacted at 80°C for 12 h. After completion of the reaction, the mixture was poured into water (5 mL), extracted with ethyl acetate (5 mL), dried, concentrated, and then purified by column chromatography (petroleum ether:ethyl acetate = 70:30) to give (S)-tert-butyl 4-((3-chloro-2,4-difluorophenyl)(methyl)carbamoyl)-3-(imidazo[1,2-a]pyrazin-8-yl)-2-oxoimidazolidine-1-carboxylate (60 mg, 0.118 mmol, yield: 18%) as a colorless oil. LCMS (ESI): 506 [M+H]+.

Step 2: (S)-tert-butyl 4-((3-chloro-2,4-difluorophenyl)(methyl)carbamoyl)-3-(imidazo[1,2-a]pyrazin-8-yl)-2-oxoimidazolidine-1-carboxylate (60 mg, 0.11 mmol) was dissolved in trifluoroacetic acid (1 mL) and dichloromethane (5 mL), and the system was stirred at room temperature for 1 h. After completion of the reaction, the obtained mixture was concentrated under reduced pressure. The crude product (50 mg) was purified by preparative high performance liquid chromatography under the following conditions: column: Xselect CSH C18 OBD column 30*150 mm, 5 µm; mobile phase A: water (10 mmol/L ammonium bicarbonate), mobile phase B: acetonitrile; flow rate: 60 mL/min; gradient: 15% B to 40% B within 8 min; wavelength: 220 nm; retention time (min): 7.23 min, to give (S)-N-(3-chloro-2,4-difluorophenyl)-3-(imidazo[1,2-a]pyrazin-8-yl)-N-methyl-2-oxoimidazolidine-4-carboxamide (59 mg, 0.10 mmol, yield: 63%) as a white solid.

LCMS (ESI): 406.80 [M+H]⁺.

HNMR (400 MHz, DMSO-d6): δ ppm 8.40-8.30(m, 1H), 8.10-8.05(m, 1H),7.80-7.70(m, 1H), 7.68-7.50 (m, 2H), 7.48-7.28(m,2H), 6.28-5.35(m,1H), 3.93-3.42(m,2H),3.20-3.05(m,3H).

### Example 42:

### (S)-N-(3-chloro-2,4-difluorophenyl)-N-methyl-2-oxo-3-(4-(trifluoromethyl)isoxazolo[5,4-b]pyridin-6-yl)imidazolidine-4-carboxami de (Compound 42)

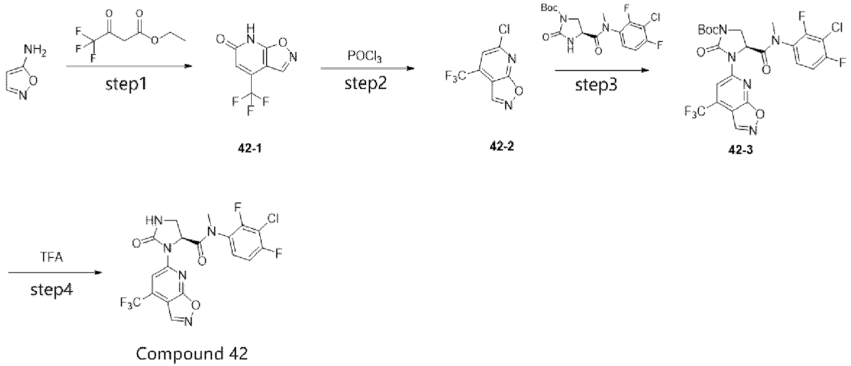

Step 1: a mixture of isoxazol-5-ylamine (50.00 mg, 0.59 mmol) and ethyl 4,4,4-trifluoro-3-oxobutanoate (109.49 mg, 0.59 mmol) in acetic acid (1.00 mL) was stirred at 120°C for 12 h. After completion of the reaction, the reaction solution was poured into water (50 mL) and extracted with ethyl acetate (50 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 80:20) to give 4-(trifluoromethyl)-7-hydroisoxazolo[5,4-b]pyridin-6-one (30.00 mg, yield: 25%, 0.146 mmol) as a yellow oil. LCMS (ESI): 205 [M+H]⁺.

Step 2: a solution of 4-(trifluoromethyl)-7-hydroisoxazolo[5,4-b]pyridin-6-one (300 mg, 1.46 mmol) in phosphorus oxychloride (2 mL) was stirred at 150°C under microwave for 2 h. After the reaction, the reaction solution was concentrated, poured into water, extracted with ethyl acetate (30 mL), dried over anhydrous sodium sulfate, filtered, and concentrated to give a crude product. The crude product was then concentrated and purified by column chromatography (petroleum ether:ethyl acetate = 50:50) to give 6-chloro-4-(trifluoromethyl)isoxazolo[5,4-b]pyridine (300 mg, yield: 55%, 1.4 mmol) as a white solid. LCMS (ESI): 223 [M+H]⁺.

Step 3: a solution of 6-chloro-4-(trifluoromethyl)isoxazolo[5,4-b]pyridine (40 mg, 0.18 mmol), (S)-tert-butyl 4-((3-chloro-2,4-difluorophenyl)(methyl)carbamoyl)-2-oxoimidazolidine-1-carboxylate (70 mg, 0.18 mmol), potassium carbonate (70 mg, 0.54 mmol), tris(dibenzylideneacetone)dipalladium (20 mg, 0.02 mmol), and 4,5-bis(diphenylphosphino)-9,9-dimethylxanthene (10 mg, 0.02 mmol) in dioxane (4.00 mL) was stirred at 80°C for 12 h. The mixture was poured into water (5 mL), extracted with ethyl acetate (10 mL*3), dried over anhydrous sodium sulfate, filtered, and concentrated. The crude product was purified by column chromatography (dichloromethane:methanol = 80:20) to give (S)-tert-butyl 4-((3-chloro-2,4-difluorophenyl)(methyl)carbamoyl)-2-oxo-3-(4-(trifluoromethyl)isoxazolo[5,4-b]pyridin-6-yl)imidazolidine-1-carbo xylate (50 mg, yield: 48%, 0.086 mmol) as a yellow oil. LCMS (ESI): 576 [M+H]⁺.

Step 4: to a solution of (S)-tert-butyl 4-((3-chloro-2,4-difluorophenyl)(methyl)carbamoyl)-2-oxo-3-(4-(trifluoromethyl)isoxazolo[5,4-b]pyridin-6-yl)imidazolidine-1-carbo xylate (210.00 mg, 0.36 mmol) in dichloromethane (10 mL) was added trifluoroacetic acid (5 mL). The mixture was stirred at room temperature for 1 h. The reaction solution was concentrated. The crude product was purified by chiral_HPLC: column: CHIRALPAK-IA 2*25 cm, 5 m; mobile phase A: n-hexane (0.1% formic acid), mobile phase B: ethanol:dichloromethane = 1:1; flow rate: 20 mL/min; gradient: isocratic 20; wavelength: 254/220 nm; RT1 (min): 7.44; sample solvent: ethanol; injection volume: 2.0 mL; run times: 3 times, to give (S)-N-(3-chloro-2,4-difluorophenyl)-N-methyl-2-oxo-3-(4-(trifluoromethyl)isoxazolo[5,4-b]pyridin-6-yl)imidazolidine-4-carboxami de (18.53 mg, yield: 10%, 0.038 mmol) as a white solid.

LCMS (ESI): 475.90 [M+H]⁺.

1H NMR (400 MHz, DMSO-d6) δ ppm 8.78-8.76 (m, 1H), 7.41-7.39 (m, 2H), 7.20-6.95 (m, 2H), 5.55-4.67 (m, 1H), 3.21-3.20 (m, 1H), 3.11 (s, 3H), 3.01-2.92 (m, 1H).

### Example 43:

### (S)-N-(3-chloro-2,4-difluorophenyl)-N-methyl-3-(7-(methylthio)thiazolo[5,4-b]pyridin-5-yl)-2-oxoimidazolidine-4-carboxamide (Compound 43)

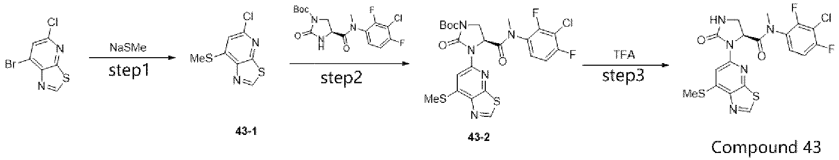

Step 1: 7-bromo-5-chlorothiazolo[5,4-b]pyridine (500 mg, 2.02 mmol) was dissolved in tetrahydrofuran (50 mL), and sodium methanethiolate (141 mg, 2.02 mmol) was then added. The system was reacted at 60°C for 2 h. After completion of the reaction, the reaction solution was poured into water (50 mL) and extracted with ethyl acetate (50 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 75:25) to give 5-chloro-7-(methylthio)thiazolo[5,4-b]pyridine (350 mg, 1.62 mmol, yield: 80%) as a white solid. LCMS (ESI): 217 [M+H]⁺.

Step 2: 5-chloro-7-(methylthio)thiazolo[5,4-b]pyridine (350 mg, 1.62 mmol) was dissolved in 1,4-dioxane (30 mL) and added to tert-butyl (S)-4-((3-chloro-2,4-difluorophenyl)(methyl)carbamoyl)-2-oxoimidazolidine-1-carboxylate (535 mg, 1.37 mmol). Tris(dibenzylideneacetone)dipalladium (126 mg, 0.14 mmol), 4,5-bis(diphenylphosphino)-9,9-dimethylxanthene (79 mg, 0.14 mmol), and anhydrous potassium carbonate (569 mg, 4.12 mmol) were then added and stirred at 80°C overnight. After completion of the reaction, the filtrate was concentrated. The crude product was purified by silica gel column chromatography (dichloromethane:methanol = 85:15) to give (S)-tert-butyl 4-((3-chloro-2,4-difluorophenyl)(methyl)carbamoyl)-3-(7-(methylthio)thiazolo[5,4-b]pyridin-5-yl)-2-oxoimidazolidine-1-carboxylate (210 mg, 0.37 mmol, yield: 24.67%) as a white solid. LCMS (ESI): 570 [M+H]⁺.

Step 3: (S)-tert-butyl 4-((3-chloro-2,4-difluorophenyl)(methyl)carbamoyl)-3-(7-(methylthio)thiazolo[5,4-b]pyridin-5-yl)-2-oxoimidazolidine-1-carboxylate (210 mg, 0.37 mmol) was dissolved in dichloromethane:trifluoroacetic acid (10 mL:2 mL) and reacted at room temperature for 2 h. After completion of the reaction, the mixture was spin-dried. The crude product was purified by high-performance liquid chromatography (column: ultimate µXB-C18; mobile phase A: water (10 mmol/L ammonium bicarbonate aqueous solution), mobile phase B: acetonitrile; flow rate: 100 mL/min; gradient: 30% B to 60% B within 20 min; wavelength: 254 nm/220 nm; retention time: 19 min) to give (S)-N-(3-chloro-2,4-difluorophenyl)-N-methyl-3-(7-(methylthio)thiazolo[5,4-b]pyridin-5-yl)-2-oxoimidazolidine-4-carboxamide (73 mg, 0.16 mmol, yield: 43%) as a white solid.

LCMS (ESI): 470.20 [M+H]⁺.

1H NMR (400 MHz, DMSO-d6) δ ppm 9.24-9.18 (m, 1H), 8.32-8.28 (m, 1H), 7.65-7.60 (m, 1H), 7.52 (s, 1H), 7.47-7.42 (m, 1H), 5.72-4.92 (m, 1H), 3.87- 3.45 (m, 2H), 3.14-3.11 (m, 3H), 2.53-2.48 (m, 3H).

### Example 44:

### (S)-N-(3-chloro-2,4-difluorophenyl)-N-methyl-2-oxo-3-(7-((trifluoromethyl)thio)thiazolo[5,4-b]pyridin-5-yl)imidazolidine-4-carbox amide (Compound 44)

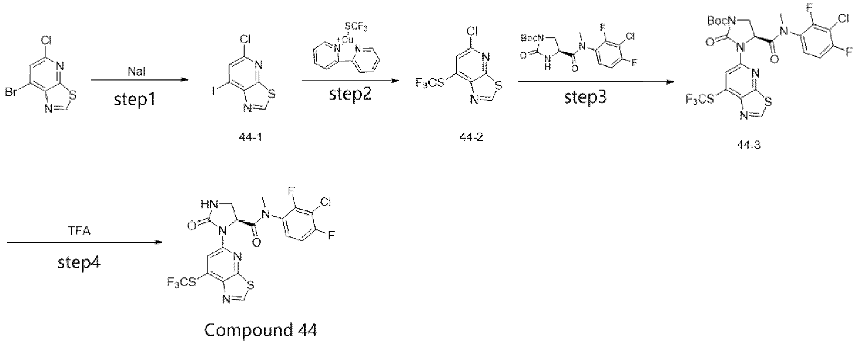

Step 1: a mixture of trimethylsilyl chloride (43 mg, 0.4 mmol), sodium iodide (44 mg, 0.4 mmol), and 7-bromo-5-chloro-1,3-thiazolo[5,4-b]pyridine (50 mg, 0.2 mmol) in acetonitrile (1.00 mL) was stirred at 80°C for 3 h. After completion of the reaction, the reaction solution was poured into water (50 mL) and extracted with ethyl acetate (50 mL x 3). The organic phases were combined, washed with saturated salt water, dried over anhydrous sodium sulfate, and filtered to remove anhydrous sodium sulfate. The filtrate was concentrated. The crude product was purified by silica gel column chromatography (ethyl acetate:petroleum ether = 15:75) to give 7-iodo-5-chloro-1,3-thiazolo[5,4-b]pyridine (20 mg, yield: 40%, 0.058 mmol) as a yellow oil. LCMS (ESI): 342 [M+H]⁺.

Step 2: a mixture of thiotrifluoromethyl copper salt (64 mg, 0.2 mmol) and 7-iodo-5-chloro-1,3-thiazolo[5,4-b]pyridine (50 mg, 0.2 mmol) in dioxane (1.00 mL) was stirred at 100°C overnight. After the reaction, the reaction solution was concentrated, poured into water, extracted with ethyl acetate (30 mL), dried over anhydrous sodium sulfate, filtered, and concentrated to give a crude product. The crude product was then concentrated and purified by column chromatography (petroleum ether:ethyl acetate = 30%) to give 5-chloro-7-(trifluoromethylthio)-1,3-thiazolo[5,4-b]pyridine (27 mg, 0.1 mmol, yield: 50%) as a white solid. LCMS (ESI): 272 [M+H]⁺.

Step 3: 5-chloro-7-(trifluoromethylthio)-1,3-thiazolo[5,4-b]pyridine (200 mg, 0.74 mmol), tert-butyl (4S)-4-[N-(3-chloro-2,4-difluorophenyl)-N-methylcarbamoyl]-2-oxoimidazolidine carboxylate (290 mg, 0.74 mmol), potassium carbonate (310 mg, 2.22 mmol), tris(dibenzylideneacetone)dipalladium (30 mg, 0.07 mmol), and 4,5-bis(diphenylphosphino)-9,9-dimethylxanthene (20 mg, 0.07 mmol) were reacted in dioxane (1.00 mL) at 80°C for 12 h. The mixture was poured into water (2 mL), extracted with ethyl acetate (5 mL*3), dried over anhydrous sodium sulfate, filtered, and concentrated. The crude product was purified by column chromatography (ethyl acetate:petroleum ether = 45:55) to give tert-butyl (4S)-4-[N-(3-chloro-2,4-difluorophenyl)-N-methylcarbamoyl]-2-oxo-3-[7-(trifluoromethylthio)(1,3-thiazolo[4,5-e]pyridin-5-yl)]imid azolidine carboxylate (50 mg, yield: 48%, 0.086 mmol) as a yellow oil. LCMS (ESI): 624 [M+H]⁺.

Step 4: to a solution of (S)-tert-butyl 4-((3-chloro-2,4-difluorophenyl)(methyl)carbamoyl)-2-oxo-3-(7-((trifluoromethyl)thio)thiazolo[5,4-b]pyridin-5-yl)imidazolidine-1-c arboxylate (50 mg, 0.086 mmol) in dichloromethane (10 mL) was added trifluoroacetic acid (5 mL). The mixture was stirred at room temperature for 1 h. The reaction solution was concentrated and purified by high-performance liquid chromatography (column: µXB-C18; mobile phase A: water (10 mmol/L ammonium bicarbonate aqueous solution), mobile phase B: acetonitrile; flow rate: 100 mL/min; gradient: 30% B to 60% B within 20 min; wavelength: 254 nm/220 nm; retention time: 16 min) to give (S)-N-(3-chloro-2,4-difluorophenyl)-N-methyl-2-oxo-3-(7-((trifluoromethyl)thio)thiazolo[5,4-b]pyridin-5-yl)imidazolidine-4-carbox amide (1.77 mg, yield: 4, 0.003 mmol) as a white solid.

LCMS (ESI): 524 [M+H]⁺.

HNMR (400 MHz, DMSO-d6): δ ppm 9.46-9.34 (m, 1H), 8.85-8.73 (m, 1H), 7.80-7.63 (m, 2H), 7.51-7.39 (m, 1H), 5.78-4.83 (m, 1H), 4.12-3.52 (m, 2H), 3.18-2.97 (m, 3H).

### Example 45:

### (2S,3S,4S)-N-(5-chloro-2,4-difluorophenyl)-3,4-dihydroxy-N-(methyl-D3)-5-oxo-1-(7-oxo-4-(trifluoromethyl)-6,7-dihydro-SH-cycl openta[b]pyridin-2-yl)pyrrolidine-2-carboxamide (Compound 45)

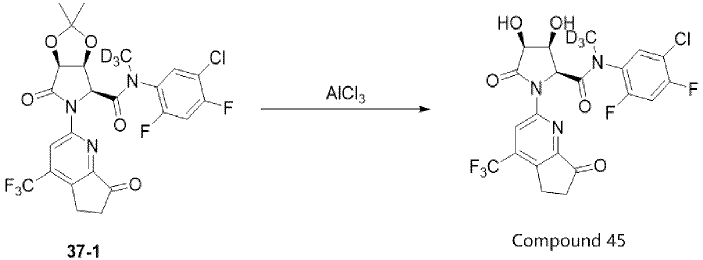

To a solution of (3aS,4S,6aS)-N-(5-chloro-2,4-difluorophenyl)-2,2-dimethyl-N-(methyl-D3)-6-oxo-5-(7-oxo-4-(trifluoromethyl)-6,7-dihydro-5H-cycl openta[b]pyridin-2-yl)tetrahydro-4H-[1,3]dioxolo[4,5-c]pyrrole-4-carboxamide (80 mg, 0.142 mmol) in acetic acid (8.00 mL) was added anhydrous aluminum chloride (8 mg, 0.056 mmol), and the mixture was stirred at 80°C for 2 h. The reaction solution was concentrated. The crude product was purified by high-performance liquid chromatography: column: Kinetex 5 m EVO C18, 30 mm*150 mm; mobile phase A: water (10 mmol/L ammonium bicarbonate), mobile phase B: 20 mm sodium hydroxide + 10% acetonitrile; flow: 60 mL/min; gradient: 20% B to 50% B within 8 min; wavelength: 254 nm/220 nm; retention time (min): 7.55, to give

(2S,3S,4S)-N-(5-chloro-2,4-difluorophenyl)-3,4-dihydroxy-N-(methyl-D3)-5-oxo-1-(7-oxo-4-(trifluoromethyl)-6,7-dihydro-SH-cycl openta[b]pyridin-2-yl)pyrrolidine-2-carboxamide (10 mg, yield: 13%, 0.019 mmol) as a white solid.

LCMS (ESI): 523.30 [M+H]⁺.

1H NMR (400 MHz, DMSO-d6) δ ppm 8.73-8.69 (m, 1H), 8.67 (s, 1H), 7.89-7.83 (m, 1H), 5.79-5.56 (m, 2H), 5.24-5.23 (m, 1H), 5.00-4.97 (m, 1H), 4.31-4.26 (m, 1H), 4.11-4.05 (m, 1H), 3.23-3.17 (m, 2H), 2.83-2.82 (m, 1H).

### Example 46:

### (2S,3S,4S)-N-(5-chloro-2,4-difluorophenyl)-3,4-dihydroxy-N-(methyl-D3)-5-oxo-1-(4-(trifluoromethyl)selenophenyl[2,3-b]pyridin-6-yl)pyrrolidine-2-carboxamide (Compound 46)

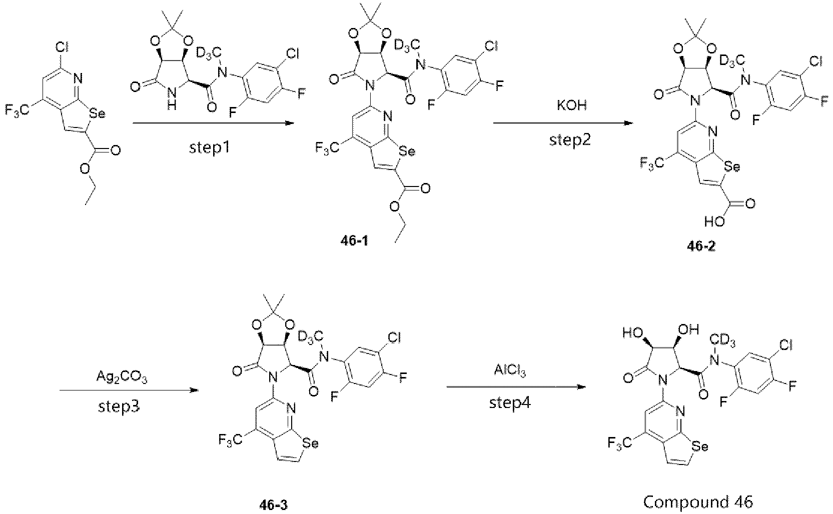

Step 1: ethyl 6-chloro-4-(trifluoromethyl)selenobenzo[2,3-b]pyridine-2-carboxylate (200 mg, 0.56 mmol), (3aS,4S,6aS)-N-(5-chloro-2,4-difluorophenyl)-2,2-dimethyl-N-(methyl-D3)-6-oxotetrahydro-4H-[1,3]dioxolo[4,5-clpyrrole-4-carbox amide (203 mg, 0.56 mmol), tris(dibenzylideneacetone)dipalladium (55 mg, 0.06 mmol), 4,5-bis(diphenylphosphino)-9,9-dimethylxanthene (35 mg, 0.06 mmol), and potassium carbonate (232 mg, 1.68 mmol) were dissolved in dioxane (15 mL) and reacted at 80°C for 1 h. After completion of the reaction, the system was cooled to room temperature. The reaction solution was poured into water (10 mL). The obtained mixture was extracted with ethyl acetate (20 mL x 2). The combined organic layer was washed with saturated salt water and dried over anhydrous sodium sulfate. After filtration, the filtrate was concentrated under reduced pressure. The crude product was purified by silica gel column chromatography (petroleum ether:ethyl acetate = 60:40) to give ethyl 6-((3aS,4S,6aS)-4-((5-chloro-2,4-difluorophenyl)(methyl-d3)carbamoyl)-2,2-dimethyl-6-oxotetrahydro-SH-[1,3]dioxolo[4,5-c]pyrrol -5-yl)-4-(trifluoromethyl)selenobenzo[2,3-b]pyridine-2-carboxylate (250 mg, 0.37 mmol, yield: 65%) as a light yellow solid. LCMS (ESI) m/z: **685** [M+H]⁺.

Step 2: ethyl 6-((3aS,4S,6aS)-4-((5-chloro-2,4-difluorophenyl)(methyl-d3)carbamoyl)-2,2-dimethyl-6-oxotetrahydro-SH-[1,3]dioxolo[4,5-c]pyrrol -5-yl)-4-(trifluoromethyl)selenobenzo[2,3-b]pyridine-2-carboxylate (250 mg, 0.37 mmol) was dissolved in methanol (3 mL) and tetrahydrofuran (3 mL). Potassium hydroxide (414 mg, 7.4 mmol) was dissolved in water (3 mL) and added to the above solution at 0°C. The system was warmed to room temperature and stirred for 2 h. After completion of the reaction, the reaction solution was poured into water (10 mL). The obtained mixture was extracted with ethyl acetate (20 mL x 2). The aqueous phase was retained, adjusted to about pH 5 with formic acid, and extracted with ethyl acetate (20 mL*3). The organic phase was dried over anhydrous sodium sulfate and filtered. The filtrate was concentrated to give the crude product 6-((3aS,4S,6aS)-4-((5-chloro-2,4-difluorophenyl)(methyl-d3)carbamoyl)-2,2-dimethyl-6-oxotetrahydro-5H-[1,3]dioxolo[4,5-c]pyrrol -5-yl)-4-(trifluoromethyl)selenobenzo[2,3-b]pyridine-2-carboxylic acid (200 mg) as a white solid. LCMS (ESI) m/z: **657** [M+H]⁺.

Step 3: the crude product 6-((3aS,4S,6aS)-4-((5-chloro-2,4-difluorophenyl)(methyl-d3)carbamoyl)-2,2-dimethyl-6-oxotetrahydro-5H-[l,3]dioxolo[4,5-c]pyrrol -5-yl)-4-(trifluoromethyl)selenobenzo[2,3-b]pyridine-2-carboxylic acid (200 mg), silver carbonate (2.8 mg, 0.01 mmol), and acetic acid (1.2 mg, 0.02 mmol) were dissolved in dimethyl sulfoxide (4 mL) solution. The system was warmed to 100°C and reacted overnight. After completion of the reaction, the reaction solution was poured into water (4 mL). The obtained mixture was extracted with ethyl acetate (10 mL x 3). The combined organic layer was washed with saturated salt water (5 mL x 3) and dried over anhydrous sodium sulfate. After filtration, the filtrate was concentrated under reduced pressure to give the crude product (3aS,4S,6aS)-N-(5-chloro-2,4-difluorophenyl)-2,2-dimethyl-N-(methyl-D3)-6-oxo-5-(4-(trifluoromethyl)selenophenyl[2,3-b]pyridin-6-yl)tetrahydro-4H-[1,3]dioxolo[4,5-c]pyrrole-4-carboxamide (150 mg) as a black solid. LCMS (ESI) m/z: **613** [M+H]⁺.

Step 4: the crude product (3aS,4S,6aS)-N-(5-chloro-2,4-difluorophenyl)-2,2-dimethyl-N-(methyl-D3)-6-oxo-5-(4-(trifluoromethyl)selenophenyl[2,3-b]pyridin-6-yl)tetrahydro-4H-[1,3]dioxolo[4,5-c]pyrrole-4-carboxamide (150 mg) and anhydrous aluminum chloride (13.2 mg, 0.1 mmol) was dissolved in acetic acid. The system was warmed to 80°C and reacted overnight. Part of the solvent was removed by concentration under reduced pressure to give a crude product. The crude product was purified by high-performance liquid chromatography (column: Kinetex EVO prep C18, 30*150, 5 µm; mobile phase A: 10 mmol/L ammonium bicarbonate aqueous solution, mobile phase B: acetonitrile; flow rate: 60 mL/min; gradient: 25% B to 60% B within 10 min; wavelength: 254 nm/220 nm; retention time: 8.87 min) to give (2S,3S,4S)-N-(5-chloro-2,4-difluorophenyl)-3,4-dihydroxy-N-(methyl-D3)-5-oxo-1-(4-(trifluoromethyl)selenophenyl[2,3-b]pyridin-6-yl)pyrrolidine-2-carboxamide (24.93 mg, 0.04 mmol, yield: 16.3%) as a light pink solid.

LCMS (ESI) m/z: **573** [M+H]⁺.

HNMR (400 MHz, DMSO-d6): δ ppm 8.58-8.52 (m, 2H), 7.98-7.68 (m, 2H), 7.67-7.55 (m, 1H), 5.75-5.63 (m, 2H), 5.29-4.92 (m, 1H), 4.83-4.05 (m, 2H).

### Effect Example 1

### Polθ polymerase activity assay

The ability of compounds to inhibit Polθ polymerase activity was determined by the PicoGreen method in vitro.

The His-TEV-SUMO-tagged Polθ protein (amino acids 1792-2590) expressed in E.coli was purified and stored in aliquots at -80°C. The composition of the assay buffer is 25 mM Tris HCl pH 7.5, 12.5 mM NaCl, 0.5 mM MgCl₂, 5% glycerol, 0.01% Triton X-100, 0.01% BSA, and 1 mM DTT.

Test compounds were diluted in 100% DMSO and diluted three-fold into 10 concentration points in a dilution plate (Greiner-781280) using Bravo (Agilent) according to concentration requirements. The compounds diluted in DMSO were then diluted 20-fold in the assay buffer using Bravo. Further, 2 µl of diluted compounds were transferred to the assay plate (Corning-4512) using Bravo. The purified Polθ enzyme and primers ((primer strand: 5'-GCGGCTGTCATAAG-3'):(template strand: 5'-GCTACATTGACAATGGCATCAAATCTCAGATTGCGTCTTATGACAGCCGCG-3') = 1:1.1) were prepared at a working concentration of 2.5x (1.5 nM Polθ and 50 nM PTD) in the assay buffer, transferred to the assay plate at 4 µl per well using an E1-CLIPTIP 12-channel pipette (Thermo, 1-30 µl), and incubated at room temperature for 30 min. dNTP (Sigma-D7295) was diluted with the assay buffer to a working concentration of 2.5x (40 uM dNTP), transferred to the assay plate at 4 µl per well (final DMSO concentration of 1%) using an E1-CLIPTIP 12-channel pipette (Thermo, 1-30 µl), and incubated at room temperature for 60 min. A mixture containing 10 mM EDTA, 25 mM Tris pH 7.5, and a 1:200 diluted PicoGreen dye (Invitrogen-P7581) was added to the assay plate at 6 µl per well to stop the reaction. After 30 min of reaction at room temperature in the dark, fluorescence values were read on EnVision 2105 (PerkinElmer) using the Ex480nm Em520nm program, and the raw data was analyzed using XLfit to generate IC50 values. The results are shown in the table below.

| Compound No. | Polθ IC₅₀ |
|---|---|
| 1 | A |
| 2 | C |
| 3 | D |
| 4 | B |
| 5 | C |
| 6a | C |
| 7 | A |
| 8a | C |
| 9a | A |
| 9b | A |
| 10 | D |
| 11 | B |
| 12 | A |
| 13 | A |
| 14 | A |
| 15 | B |
| 16 | A |
| 17 | B |
| 18 | B |
| 19 | A |
| 20 | B |
| 21 | A |
| 22 | A |
| 23 | A |
| 24 | A |
| 26 | A |
| 27 | A |
| 30 | A |
| 31 | A |
| 33 | A |
| 37 | A |
| 41 | D |
| 43 | B |
| 44 | B |
| 45 | A |
| 46 | A |

| | |
|---|---|
| A: IC₅₀ ≤ 50 nM; B: 50 nM < IC₅₀ ≤ 500 nM; C: 500 nM < IC₅₀ ≤ 5000 nM, D: IC₅₀ > 5000 nM. | |

## Claims

1. A compound of formula II or III, or a tautomer thereof, a stereoisomer thereof, a pharmaceutically acceptable salt of any of the above, or a solvate of any of the above, 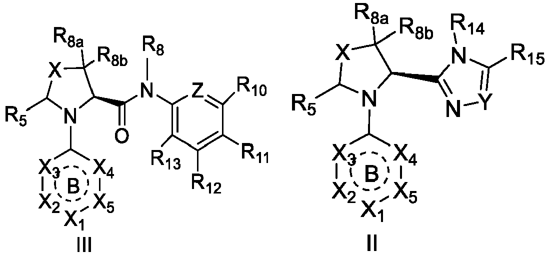
wherein Z is C(R₉) or N;
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, X₃ is C(R₃ₐ) or N, X₄ is C(R₄ₐ) or N, X₅ is C(R₅ₐ) or N, and
R₃ₐ, R₄ₐ, and R₅ₐ are each independently hydrogen, C₁₋₆ alkyl, C₁₋₆ alkoxy, halogen, C₁₋₆ alkyl substituted with one or more R^{X1}, C₁₋₆ alkoxy substituted with one or more R^{X1}, C₃₋₈ cycloalkyl substituted with one or more R^{X1}, C₃₋₈ cycloalkyl, C₂₋₆ alkenyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, X₂ is C(R₂ₐ) or N, X₃ is C(R₃ₐ) or N, X₄ is C(R₄ₐ) or N, and R₂ₐ, R₃ₐ, and R₄ₐ are each independently hydrogen, C₁₋₆ alkyl, C₁₋₆ alkoxy, halogen, C₁₋₆ alkyl substituted with one or more R^{X1}, C₃₋₈ cycloalkyl, -CN-, C₁₋₆ alkoxy substituted with one or more R^{X1}, C₃₋₈ cycloalkyl substituted with one or more R^{X1}, -SO₂-R₂ₐₐ, -SO-R₂ₐₐ, -SO(=NH)R₂ₐₐ, -PO(C₁₋₆ alkyl)₂, -SF₅, -S-R₂ₐₐ, or -NR^{x}R^{y};
or, R₄ₐ and R₅ₐ, R₄ₐ and R_{5b}, R_{4b} and R_{5b}, or R_{4b} and R₅ₐ join to form ring A, X₁ is C(R₁ₐ), X₂ is C(R₂ₐ) or N, X₃ is C(R₃ₐ) or N, and
R₁ₐ, R₂ₐ, and R₃ₐ are each independently hydrogen, C₁₋₆ alkyl, C₁₋₆ alkoxy, halogen, C₁₋₆ alkyl substituted with one or more R^{X1}, C₁₋₆ alkoxy substituted with one or more R^{X1}, C₃₋₈ cycloalkyl substituted with one or more R^{X1}, C₃₋₈ cycloalkyl, -CN-, or -NR^{x}R^{y};
or, R₂ₐ and R₃ₐ, R₂ₐ and R_{3b}, R_{2b} and R_{3b}, or R_{2b} and R₃ₐ join to form ring A, X₁ is C(R₁ₐ), X₄ is C(R₄ₐ) or N, X₅ is C(R₅ₐ) or N, and
R₁ₐ, R₄ₐ, and R₅ₐ are each independently hydrogen;
R₂ₐₐ is C₃₋₈ cycloalkyl, C₃₋₈ cycloalkyl substituted with one or more R^{X1}, C₁₋₆ alkyl, or C₁₋₆ alkyl substituted with one or more R^{X1};
R^{x} and R^{y} are each independently hydrogen, C₁₋₆ alkyl, C₁₋₆ alkyl substituted with 1, 2, or 3 R^{X1}, C₃₋₈ cycloalkyl, "4- to 8-membered heterocycloalkyl having 1, 2, 3, or 4 heteroatoms selected from 1, 2, 3, or 4 of N, O, S, and Se", -C(O)C₃₋₈ cycloalkyl, C₃₋₈ cycloalkyl substituted with 1, 2, or 3 R^{X2}, -C(O)C₁₋₆ alkyl substituted with 1, 2, or 3 R^{X3}, "4- to 8-membered heterocycloalkyl having 1, 2, 3, or 4 heteroatoms selected from 1, 2, 3, or 4 of N, O, S, and Se" substituted with 1, 2, or 3 R^{X4}, -C(O)C₃₋₈ cycloalkyl substituted with 1, 2, or 3 R^{X5}, or -C(O)C₁₋₆ alkyl;
R^{X1}, R^{X2}, R^{X3}, and R^{X5} are each independently halogen, hydroxyl, amino, or sulfonyl;
each R^{X4} is independently oxo (=O), hydroxyl, C₁₋₆ alkyl substituted with 1, 2, or 3 hydroxyl groups, C₁₋₆ alkoxy substituted with 1, 2, or 3 hydroxyl groups, or -C(O)C₁₋₆ alkyl;
or, R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se";
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se", or a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, halogen, nitro, amino, -NH(C₁₋₆ alkyl), -N(C₁₋₆ alkyl)₂, -SO₂(C₁₋₆ alkyl), -SCF₃, -S(C₁₋₆ alkyl), -PO(C₁₋₆ alkyl)₂, -CO(C₁₋₆ alkyl), -COOH, -CONH₂, -CONR^{x}R^{y}, -NR^{x}R^{y}, C₁₋₆ alkoxy substituted with one or more halogens, C₂₋₆ alkenyl, C₂₋₆ alkynyl, C₃₋₈ cycloalkyl substituted with one or more halogens, -SO₂(C₃₋₈ cycloalkyl), -SO(= NH)(C₁₋₆ alkyl), -SO(=NH)(C₃₋₈ cycloalkyl), -SCHF₂, -S(C₃₋₈ cycloalkyl), -SO(C₃₋₈ cycloalkyl), -CO(C₃₋₈ cycloalkyl), -COCHF₂, or "4- to 8-membered heterocycloalkyl having 1, 2, 3, or 4 heteroatoms selected from 1, 2, 3, or 4 of N, O, S, and Se";
R₅ is oxo (=O), hydrogen, cyano, C₁₋₆ alkyl substituted with one or more halogens, -SO₂-C₁₋₆ alkyl, -SO₂(C₃₋₈ cycloalkyl), -P(O)(C₁₋₆ alkyl)₂, -P(O)(C₃₋₈ cycloalkyl)₂, or -CH₂-R^{z};
X is -CHR₆₋₁-, -NR₆₋₂-, -Se-, -S-, S(=O), SO₂, SO(=NH), or -O-;
R₆₋₁ is hydrogen, hydroxyl, halogen, CN, -NR^{m}Rⁿ, C₁₋₆ alkyl, C₁₋₆ alkoxy, C₁₋₆ alkyl substituted with one or more R₁₆₋₁, C₁₋₆ alkoxy substituted with one or more R₁₆₋₁, -SO₂(C₃₋₈ cycloalkyl), -SO(=NH)(C₁₋₆ alkyl), -SO(=NH)(C₃₋₈ cycloalkyl), C₃₋₈ cycloalkyl, cyano, -L-(C₆-C₁₀ aryl), -L-(C₆-C₁₀ aryl substituted with one or more R₆₋₁₋₁), -L-"4- to 8-membered heterocycloalkyl having 1, 2, 3, or 4 heteroatoms selected from 1, 2, 3, or 4 of N, O, S, and Se", -L-"4- to 8-membered heterocycloalkyl having 1, 2, 3, or 4 heteroatoms selected from 1, 2, 3, or 4 of N, O, S, and Se substituted with one or more R₆₋₁₋₂", or -SO₂-C₁₋₆ alkyl;
L represents a single link bond or C₁₋₆ alkylene optionally in which 1, 2, or 3 methylene groups are replaced by -O-;
R₆₋₁₋₁ and R₆₋₁₋₂ are each independently oxo, hydroxyl, C₁₋₆ alkyl substituted with 1, 2, or 3 hydroxyl groups, -COC₁₋₆ alkyl, or -
COOC₁₋₆ alkyl;
R₆₋₂ is hydrogen, hydroxyl, halogen, CN, -NR^{m}Rⁿ, C₁₋₆ alkyl, C₁₋₆ alkoxy, C₁₋₆ alkyl substituted with one or more R₁₆₋₁, C₁₋₆ alkoxy substituted with one or more R₁₆₋₁, -SO₂(C₃₋₈ cycloalkyl), -SO(=NH)(C₁₋₆ alkyl), -SO(=NH)(C₃₋₈ cycloalkyl), C₃₋₈ cycloalkyl, oxa-C₃₋₈ cycloalkyl, cyano, or -SO₂-C₁₋₆ alkyl;
R₅ₐ is hydrogen, -NR^{x}R^{y}, -CH₂-R^{z}, or hydroxyl;
or, R₆₋₁ and R₆₋₂ are each independently -OR₁₅ₐ; R₈ₐ is -OR_{15b};
R₁₅ₐ and R_{15b} together with the atom to which they are attached join to form a 3- to 8-membered saturated monocyclic, spirocyclic, or
fused cyclic carbon ring, a "3- to 8-membered saturated monocyclic, spirocyclic, or fused cyclic carbon ring" substituted with one or more C₁₋₆ alkyl, or a "4- to 8-membered saturated monocyclic, spirocyclic, or fused cyclic heterocyclic ring containing one heteroatom N";
each R₁₆₋₁ is independently halogen, hydroxyl, -NR^{m}Rⁿ, oxo (=O), hydroxyl, C₁₋₆ alkyl substituted with 1, 2, or 3 hydroxyl groups, -C(O)C₁₋₆ alkyl, C₁₋₆ alkoxy, C₃₋₈ cycloalkyl, C₁₋₆ alkoxy substituted with 1, 2, or 3 hydroxyl groups, or -C(O)OC₁₋₆ alkyl;
R^{m} and Rⁿ are each independently hydrogen, C₁₋₆ alkyl, C₁₋₆ alkyl substituted with one or more halogens, C₃₋₈ cycloalkyl, C₃₋₈ cycloalkyl substituted with one or more halogens, -CO(C₁₋₆ alkyl), -CO(C₃₋₈ cycloalkyl), -CO(C₃₋₈ cycloalkyl substituted with one or more halogens), "4- to 8-membered heterocycloalkyl having 1, 2, 3, or 4 heteroatoms selected from 1, 2, 3, or 4 of N, O, S, and Se", C₁₋₆ alkyl substituted with 1, 2, or 3 R^{m-1}, -CO(C₁₋₆ alkyl) substituted with 1, 2, or 3 R^{m-2}, or "4- to 8-membered heterocycloalkyl having 1, 2, 3, or 4 heteroatoms selected from 1, 2, 3, or 4 of N, O, S, and Se" substituted with 1, 2, or 3 R^{m-3};
R^{m-1} and R^{m-2} are each independently hydroxyl, halogen, amino, or sulfonyl;
each R^{m-3} is independently oxo (=O), hydroxyl, -CO(Ci e alkyl), C₁₋₆ alkyl, C₁₋₆ alkoxy, C₁₋₆ alkoxy substituted with 1, 2, or 3 hydroxyl groups, or C₁₋₆ alkyl substituted with 1, 2, or 3 hydroxyl groups;
R^{z} is hydrogen, C₁₋₆ alkyl, hydroxyl, C₁₋₆ alkoxy, C₃₋₈ cycloalkyl, C₁₋₆ alkoxy substituted with 1, 2, or 3 hydroxyl groups, or C₁₋₆ alkyl substituted with 1, 2, or 3 hydroxyl groups;
R_{8b} is hydrogen or hydroxyl;
R₈ is C₁₋₆ alkyl, C₃₋₈ cycloalkyl, C₃₋₈ cycloalkyl substituted with one or more R₈₋₁, or C₁₋₆ alkyl substituted with one or more R₈₋₁; each R₈₋₁ is independently halogen or deuterium;
R₉, R₁₀, R₁₁, R₁₂, and R₁₃ are each independently hydrogen, C₁₋₆ alkyl, hydroxyl, C₁₋₆ alkoxy, C₃₋₈ cycloalkyl, -NR^{m}Rⁿ, halogen, C₁₋₆ alkyl substituted with one or more halogens, C₂₋₆ alkenyl, C₆-C₁₀ aryl, C₁₋₆ alkoxy substituted with one or more halogens, C₃₋₈ cycloalkyl substituted with one or more halogens, "8- to 10-membered heteroaryl having 1, 2, 3, or 4 heteroatoms selected from 1, 2, 3, or 4 of N, O, S, and Se", C₆-C₁₀ aryl substituted with one or more R₉₋₁, or "8- to 10-membered heteroaryl having 1, 2, 3, or 4 heteroatoms selected from 1, 2, 3, or 4 of N, O, S, and Se" substituted with one or more R₉₋₂;
R₉₋₁ and R₉₋₂ are each independently C₁₋₆ alkyl, C₁₋₆ alkoxy, C₃₋₈ cycloalkyl, halogen, -CN, -C(O)N(C₁₋₆ alkyl)₂, -CH₂-N(C₁₋₆ alkyl)₂, C₁₋₆ alkyl substituted with one or more R₉₋ₐ, or C₁₋₆ alkoxy substituted with one or more R_{9-b};
R₉₋ₐ and R_{9-b} are each independently hydroxyl or halogen;
or, two adjacent groups of the R₉, R₁₀, R₁₁, R₁₂, and R₁₃ join to form a "4- to 8-membered saturated or unsaturated monocyclic heterocyclic ring having 1, 2, 3, or 4 heteroatoms selected from 1, 2, 3, or 4 of N, O, S, and Se";
R₁₄ is C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₁₄₋₁, or "8- to 10-membered heteroaryl having 1, 2, 3, or 4 heteroatoms selected from 1, 2, 3, or 4 of N, O, S, and Se" substituted with one or more R₁₄₋₂;
R₁₄₋₁ and R₁₄₋₂ are each independently C₁₋₆ alkyl, C₁₋₆ alkoxy, C₁₋₆ alkyl substituted with 1, 2, or 3 hydroxyl groups, C₃₋₈ cycloalkyl, halogen, -CN, C₁₋₆ alkyl substituted with one or more halogens, C₁₋₆ alkoxy substituted with one or more halogens, C₃₋₈ cycloalkyl substituted with one or more halogens, -CONR^{m}Rⁿ, or -CH₂-NR^{m}Rⁿ;
R₁₅ is hydrogen, halogen, C₁₋₆ alkyl, C₃₋₈ cycloalkyl, C₁₋₆ alkoxy, C₁₋₆ alkoxy substituted with one or more R₁₅₋₁, C₃₋₈ cycloalkyl substituted with one or more R₁₅₋₁, or C₁₋₆ alkyl substituted with one or more R₁₅₋₁;
each R₁₅₋₁ is independently halogen or deuterium;
Y is -C(R₁₆)= or -N=; and
R₁₆ is hydrogen, C₁₋₆ alkyl, C₁₋₆ alkoxy, C₃₋₈ cycloalkyl, or halogen.

2. The compound of formula II or III, or a tautomer thereof, a stereoisomer thereof, a pharmaceutically acceptable salt of any of the above, or a solvate of any of the above according to claim 1, wherein:
Z is C(R₉) or N;
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, C₁₋₆ alkyl, C₁₋₆ alkoxy, halogen, C₁₋₆ alkyl substituted with one or more halogens, C₃₋₈ cycloalkyl, -CN-, C₁₋₆ alkoxy substituted with one or more halogens, -SF₅, -S-R₂ₐₐ, or -NR^{x}R^{y};
or, R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
R₂ₐₐ is C₁₋₆ alkyl or C₁₋₆ alkyl substituted with one or more halogens;
R^{x} and R^{y} are each independently hydrogen, C₁₋₆ alkyl, C₁₋₆ alkyl substituted with 1, 2, or 3 R^{X1}, C₃₋₈ cycloalkyl, "4- to 8-membered heterocycloalkyl having 1, 2, 3, or 4 heteroatoms selected from 1, 2, 3, or 4 of N, O, S, and Se", -C(O)C₃₋₈ cycloalkyl, C₃₋₈ cycloalkyl substituted with 1, 2, or 3 R^{X2}, -C(O)C₁₋₆ alkyl substituted with 1, 2, or 3 R^{X3}, "4- to 8-membered heterocycloalkyl having 1, 2, 3, or 4 heteroatoms selected from 1, 2, 3, or 4 of N, O, S, and Se" substituted with 1, 2, or 3 R^{X4}, -C(O)C₃₋₈ cycloalkyl substituted with 1, 2, or 3 R^{X5}, or -C(O)C₁₋₆ alkyl;
each R^{X1} is independently halogen, hydroxyl, amino, or -SO₃H;
R^{X2}, R^{X3}, and R^{X5} are each independently halogen, hydroxyl, amino, or sulfonyl;
each R^{X4} is independently oxo (=O), hydroxyl, C₁₋₆ alkyl substituted with 1, 2, or 3 hydroxyl groups, or -C(O)C₁₋₆ alkyl;
or, R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se";
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se", or a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, halogen, nitro, amino, -NH(C₁₋₆ alkyl), -N(C₁₋₆ alkyl)₂, -SO₂(C₁₋₆ alkyl), -SCF₃, -S(C₁₋₆ alkyl), -PO(C₁₋₆ alkyl)₂, -CO(C₁₋₆ alkyl), -COOH, -CONH₂, -CONR^{x}R^{y}, -NR^{x}R^{y}, C₁₋₆ alkoxy substituted with one or more halogens, C₂₋₆ alkenyl, C₂₋₆ alkynyl, or "4- to 8-membered heterocycloalkyl having 1, 2, 3, or 4 heteroatoms selected from 1, 2, 3, or 4 of N, O, S, and Se";
R₅ is oxo (=O), hydrogen, cyano, C₁₋₆ alkyl substituted with one or more halogens, -SO₂-C₁₋₆ alkyl, -P(O)(C₁₋₆ alkyl)₂, or -CH₂-R^{z};
X is -CHR₆₋₁-, -NR₆₋₂-, or -O-;
R₆₋₁ and R₆₋₂ are each independently hydrogen, hydroxyl, halogen, CN, -NR^{m}Rⁿ, C₁₋₆ alkyl, C₁₋₆ alkoxy, C₁₋₆ alkyl substituted with one or more R₁₆₋₁, C₃₋₈ cycloalkyl, oxa-Cs-s cycloalkyl, cyano, or -SO₂-C₁₋₆ alkyl;
or, R₆₋₁ and R₆₋₂ are each independently -OR₁₅ₐ; R₈ₐ is -OR_{15b};
R₁₅ₐ and R_{15b} together with the atom to which they are attached join to form a 3- to 8-membered saturated monocyclic, spirocyclic, or
fused cyclic carbon ring, or a "4- to 8-membered saturated monocyclic, spirocyclic, or fused cyclic heterocyclic ring containing one heteroatom N";
each R₁₆₋₁ is independently halogen, hydroxyl, -NR^{m}Rⁿ, oxo (=O), hydroxyl, C₁₋₆ alkyl substituted with 1, 2, or 3 hydroxyl groups, -C(O)C₁₋₆ alkyl, or -C(O)OC₁₋₆ alkyl;
R^{m} and Rⁿ are each independently hydrogen, C₁₋₆ alkyl, C₃₋₈ cycloalkyl, -CO(Ci e alkyl), "4- to 8-membered heterocycloalkyl having 1, 2, 3, or 4 heteroatoms selected from 1, 2, 3, or 4 of N, O, S, and Se", C₁₋₆ alkyl substituted with 1, 2, or 3 R^{m-1}, -CO(C₁₋₆ alkyl) substituted with 1, 2, or 3 R^{m-2}, or "4- to 8-membered heterocycloalkyl having 1, 2, 3, or 4 heteroatoms selected from 1, 2, 3, or 4 of N, O, S, and Se" substituted with 1, 2, or 3 R^{m-3};
R^{m-1} and R^{m-2} are each independently hydroxyl, halogen, amino, or sulfonyl;
each R^{m-3} is independently oxo (=O), hydroxyl, -CO(C₁₋₆ alkyl), or C₁₋₆ alkyl substituted with 1, 2, or 3 hydroxyl groups;
R₈ₐ is hydrogen, -CH₂-R^{z}, or hydroxyl;
R^{z} is hydrogen, C₁₋₆ alkyl, hydroxyl, C₁₋₆ alkoxy, or C₁₋₆ alkyl substituted with 1, 2, or 3 hydroxyl groups;
R_{8b} is hydrogen or hydroxyl;
R₈ is C₁₋₆ alkyl, C₃₋₈ cycloalkyl, or C₁₋₆ alkyl substituted with one or more R₈₋₁;
each R₈₋₁ is independently halogen or deuterium;
R₉, R₁₀, R₁₁, R₁₂, and R₁₃ are each independently hydrogen, C₁₋₆ alkyl, hydroxyl, C₁₋₆ alkoxy, C₃₋₈ cycloalkyl, -NR^{m}Rⁿ, halogen, C₁₋₆ alkyl substituted with one or more halogens, C₂₋₆ alkenyl, C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₉₋₁, or "8- to 10-membered heteroaryl having 1, 2, 3, or 4 heteroatoms selected from 1, 2, 3, or 4 of N, O, S, and Se" substituted with one or more R₉₋₂;
R₉₋₁ and R₉₋₂ are each independently C₁₋₆ alkyl, C₁₋₆ alkoxy, C₃₋₈ cycloalkyl, halogen, -CN, -C(O)N(C₁₋₆ alkyl)₂, -CH₂-N(C₁₋₆ alkyl)₂,
C₁₋₆ alkyl substituted with one or more R₉₋ₐ, or C₁₋₆ alkoxy substituted with one or more R_{9-b};
R₉₋ₐ and R_{9-b} are each independently hydroxyl or halogen;
or, two adjacent groups of the R₉, R₁₀, R₁₁, R₁₂, and R₁₃ join to form a "4- to 8-membered saturated or unsaturated monocyclic heterocyclic ring having 1, 2, 3, or 4 heteroatoms selected from 1, 2, 3, or 4 of N, O, S, and Se";
R₁₄ is C₆-C₁₀ aryl, "8- to 10-membered heteroaryl having 1, 2, 3, or 4 heteroatoms selected from 1, 2, 3, or 4 of N, O, S, and Se",
C₆-C₁₀ aryl substituted with one or more R₁₄₋₁, or "8- to 10-membered heteroaryl having 1, 2, 3, or 4 heteroatoms selected from 1, 2, 3, or 4 of N, O, S, and Se" substituted with one or more R₁₄₋₂;
R₁₄₋₁ and R₁₄₋₂ are each independently C₁₋₆ alkyl, C₁₋₆ alkoxy, C₁₋₆ alkyl substituted with 1, 2, or 3 hydroxyl groups, C₃₋₈ cycloalkyl, halogen, -CN, C₁₋₆ alkyl substituted with one or more halogens, -CONR^{m}Rⁿ, or -CH₂-NR^{m}Rⁿ;
R₁₅ is hydrogen, halogen, C₁₋₆ alkyl, C₃₋₈ cycloalkyl, or C₁₋₆ alkyl substituted with one or more R₁₅₋₁;
each R₁₅₋₁ is independently halogen or deuterium;
Y is -C(R₁₆)= or -N=; and
R₁₆ is hydrogen, C₁₋₆ alkyl, C₁₋₆ alkoxy, or halogen.

3. The compound of formula II or III, or a tautomer thereof, a stereoisomer thereof, a pharmaceutically acceptable salt of any of the above, or a solvate of any of the above according to claim 2, wherein the compound of formula II or III, or a tautomer thereof, a stereoisomer thereof, a pharmaceutically acceptable salt of any of the above, or a solvate of any of the above, satisfies one or more of the following conditions:
(1) the compound of formula III is a compound of formula I as follows, 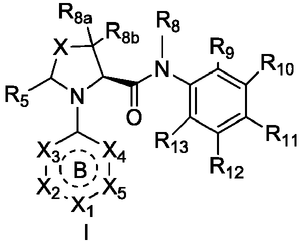 in the formula I,
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
R^{x} and R^{y} are each independently hydrogen, C₁₋₆ alkyl, C₁₋₆ alkyl substituted with 1, 2, or 3 R^{X1}, C₃₋₈ cycloalkyl, or -C(O)C₁₋₆ alkyl; each R^{X1} is independently halogen, hydroxyl, amino, or -SO₃H;
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, or 3 heteroatoms selected from 1, 2, or 3 of N, O, and S", or a "4- to 8-membered saturated or unsaturated monocyclic, spirocyclic, or fused cyclic heterocyclic ring having 1, 2, or 3 heteroatoms selected from 1, 2, or 3 of N, O, and S" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, or halogen;
R₅ is oxo (=O) or hydrogen;
X is -CHR₆₋₁-, -NR₆₋₂-, or -O-;
R₆₋₁ and R₆₋₂ are each independently hydrogen, hydroxyl, halogen, CN, -NR^{m}Rⁿ, C₁₋₆ alkyl, C₁₋₆ alkoxy, or C₁₋₆ alkyl substituted with one or more R₁₆₋₁;
each R₁₆₋₁ is independently halogen, hydroxyl, or -NR^{m}Rⁿ;
R^{m} and Rⁿ are each independently hydrogen or C₁₋₆ alkyl;
R₈ₐ is hydrogen, -CH₂-R^{z}, or hydroxyl;
R^{z} is hydrogen, C₁₋₆ alkyl, hydroxyl, C₁₋₆ alkoxy, or C₁₋₆ alkyl substituted with 1, 2, or 3 hydroxyl groups;
R_{8b} is hydrogen or hydroxyl;
R₈ is C₁₋₆ alkyl, C₃₋₈ cycloalkyl, or C₁₋₆ alkyl substituted with one or more R₈₋₁;
each R₈₋₁ is independently halogen or deuterium; and
R₉, R₁₀, R₁₁, R₁₂, and R₁₃ are each independently hydrogen, C₁₋₆ alkyl, hydroxyl, C₁₋₆ alkoxy, C₃₋₈ cycloalkyl, -NR^{m}Rⁿ, halogen, or C₁₋₆ alkyl substituted with one or more halogens;
(2) in the formula II,
ring B is a 6-membered heteroaromatic ring containing at least one N;
X₁ is C(R₁ₐ) or N(R_{1b});
X₂ is C(R₂ₐ), N(R_{2b}), or N;
X₃ is C(R₃ₐ), N(R_{3b}), or N;
X₄ is C(R₄ₐ), N(R_{4b}), or N;
X₅ is C(R₅ₐ), N(R_{5b}), or N;
R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
or, R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen, C₁₋₆ alkyl, C₁₋₆ alkoxy, halogen, C₁₋₆ alkyl substituted with one or more halogens, C₃₋₈ cycloalkyl, -CN-, or -NR^{x}R^{y};
R^{x} and R^{y} are each independently hydrogen, C₁₋₆ alkyl, C₁₋₆ alkyl substituted with 1, 2, or 3 R^{X1}, C₃₋₈ cycloalkyl, or -C(O)C₁₋₆ alkyl; each R^{X1} is independently halogen, hydroxyl, amino, or -SO₃H;
ring A is a 3- to 8-membered saturated or unsaturated monocyclic, spirocyclic, or fused cyclic carbon ring, a 3- to 8-membered saturated or unsaturated monocyclic, spirocyclic, or fused cyclic carbon ring substituted with one or more R₁₋₁, a "4- to 8-membered saturated or unsaturated monocyclic, spirocyclic, or fused cyclic heterocyclic ring having 1, 2, or 3 heteroatoms selected from 1, 2, or 3 of N, O, and S", or a "4- to 8-membered saturated or unsaturated monocyclic, spirocyclic, or fused cyclic heterocyclic ring having 1, 2, or 3 heteroatoms selected from 1, 2, or 3 of N, O, and S" substituted with one or more R₁₋₂;
R₁₋₁ and R₁₋₂ are each independently oxo (=O), hydroxyl, -CN, C₁₋₆ alkyl, C₁₋₆ alkoxy, C₁₋₆ alkyl substituted with one or more halogens, C₃₋₈ cycloalkyl, or halogen;
R₅ is oxo (=O) or hydrogen;
X is -CHR₆₋₁-, -NR₆₋₂-, or -O-;
R₆₋₁ and R₆₋₂ are each independently hydrogen, hydroxyl, halogen, CN, -NR^{m}Rⁿ, C₁₋₆ alkyl, C₁₋₆ alkoxy, or C₁₋₆ alkyl substituted with one or more R₁₆₋₁;
each R₁₆₋₁ is independently halogen, hydroxyl, or -NR^{m}Rⁿ;
R^{m} and Rⁿ are each independently hydrogen or C₁₋₆ alkyl;
R₈ₐ is hydrogen, -CH₂-R^{z}, or hydroxyl;
R^{z} is hydrogen, C₁₋₆ alkyl, hydroxyl, C₁₋₆ alkoxy, or C₁₋₆ alkyl substituted with 1, 2, or 3 hydroxyl groups;
R_{8b} is hydrogen or hydroxyl;
R₁₄ is C₆-C₁₀ aryl, "8- to 10-membered heteroaryl having 1, 2, or 3 heteroatoms selected from 1, 2, or 3 of N, O, and S", C₆₋C₁₀ aryl substituted with one or more R₁₄₋₁, or "8- to 10-membered heteroaryl having 1, 2, or 3 heteroatoms selected from 1, 2, or 3 of N, O, and S" substituted with one or more R₁₄₋₂;
R₁₄₋₁ and R₁₄₋₂ are each independently C₁₋₆ alkyl, C₁₋₆ alkoxy, C₁₋₆ alkyl substituted with 1, 2, or 3 hydroxyl groups, C₃₋₈ cycloalkyl, halogen, -CN, or C₁₋₆ alkyl substituted with one or more halogens;
R₁₅ is hydrogen, halogen, or C₁₋₆ alkyl;
Y is -C(R₁₆)= or -N=; and
R₁₆ is hydrogen, C₁₋₆ alkyl, C₁₋₆ alkoxy, or halogen.

4. The compound of formula II or formula III, or a tautomer thereof, a stereoisomer thereof, a pharmaceutically acceptable salt of any of the above, or a solvate of any of the above according to claim 2, wherein the compound of formula II or formula III, or a tautomer thereof, a stereoisomer thereof, a pharmaceutically acceptable salt of any of the above, or a solvate of any of the above, satisfies one or more of the following conditions:
(1) when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently C₁₋₆ alkoxy substituted with one or more halogens, -SF₅, or -S-R₂ₐₐ;
R₂ₐₐ is C₁₋₆ alkyl or C₁₋₆ alkyl substituted with one or more halogens;
(2) R^{x} and R^{y} are each independently "4- to 8-membered heterocycloalkyl having 1, 2, 3, or 4 heteroatoms selected from 1, 2, 3, or 4 of N, O, S, and Se", -C(O)C₃₋₈ cycloalkyl, C₃₋₈ cycloalkyl substituted with 1, 2, or 3 R^{X2}, -C(O)C₁₋₆ alkyl substituted with 1, 2, or 3 R^{X3}, "4- to 8-membered heterocycloalkyl having 1, 2, 3, or 4 heteroatoms selected from 1, 2, 3, or 4 of N, O, S, and Se" substituted with 1, 2, or 3 R^{X4}, or -C(O)C₃₋₈ cycloalkyl substituted with 1, 2, or 3 R^{X5};
R^{X2}, R^{X3}, and R^{X5} are each independently halogen, hydroxyl, amino, or sulfonyl;
each R^{X4} is independently oxo (=O), hydroxyl, C₁₋₆ alkyl substituted with 1, 2, or 3 hydroxyl groups, or -C(O)C₁₋₆ alkyl;
or, R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se";
(3) each R^{X1} is independently sulfonyl;
(4) R₁₋₁ and R₁₋₂ are each independently nitro, amino, -NH(C₁₋₆ alkyl), -N(C₁₋₆ alkyl)₂, -SO₂(C₁₋₆ alkyl), -SCF₃, -S(C₁₋₆ alkyl), -PO(C₁₋₆ alkyl)₂, -CO(C₁₋₆ alkyl), -COOH, -CONH₂, -CONR^{x}R^{y}, -NR^{x}R^{y}, C₁₋₆ alkoxy substituted with one or more halogens, C₂₋₆ alkenyl, C₂₋₆ alkynyl, or "4- to 8-membered heterocycloalkyl having 1, 2, 3, or 4 heteroatoms selected from 1, 2, 3, or 4 of N, O, S, and Se";
(5) R₅ is cyano, C₁₋₆ alkyl substituted with one or more halogens, -SO₂-C₁₋₆ alkyl, -P(O)(C₁₋₆ alkyl)₂, or -CH₂-R^{z};
(6) X is -S-, -S(=O)-, -SO₂-, or -Se;
(7) R₆₋₁ and R₆₋₂ are each independently C₃₋₈ cycloalkyl, oxa-C₃₋₈ cycloalkyl, cyano, or -SO₂-C₁₋₆ alkyl;
or, R₆₋₁ and R₆₋₂ are each independently -OR₁₅ₐ; R₈ₐ is -OR_{15b}; R₁₅ₐ and R_{15b}, together with the atom to which they are attached, join to form a 3- to 8-membered saturated monocyclic, spirocyclic, or fused cyclic carbon ring, or a "4- to 8-membered saturated monocyclic, spirocyclic, or fused cyclic heterocyclic ring containing one heteroatom N";
(8) each R₁₆₋₁ is independently C₆-C₁₀ aryl substituted with one or more R₁₆₋ₐ, or "4- to 8-membered heterocycloalkyl having 1, 2, 3, or 4 heteroatoms selected from 1, 2, 3, or 4 of N, O, S, and Se" substituted with one or more R_{16-b};
R₁₆₋ₐ and R_{16-b} are each independently oxo (=O), hydroxyl, C₁₋₆ alkyl substituted with 1, 2, or 3 hydroxyl groups, -C(O)C₁₋₆ alkyl, or -C(O)OC₁₋₆ alkyl;
(9) R^{m} and Rⁿ are each independently C₃₋₈ cycloalkyl, -CO(C₁₋₆ alkyl), "4- to 8-membered heterocycloalkyl having 1, 2, 3, or 4 heteroatoms selected from 1, 2, 3, or 4 of N, O, S, and Se", C₁₋₆ alkyl substituted with 1, 2, or 3 R^{m-1}, -CO(C₁₋₆ alkyl) substituted with 1, 2, or 3 R^{m-2}, or "4- to 8-membered heterocycloalkyl having 1, 2, 3, or 4 heteroatoms selected from 1, 2, 3, or 4 of N, O, S, and Se" substituted with 1, 2, or 3 R^{m-3};
R^{m} and R^{m-2} are each independently hydroxyl, halogen, amino, or sulfonyl;
each R^{m-3} is independently oxo (=O), hydroxyl, -CO(C₁₋₆ alkyl), or C₁₋₆ alkyl substituted with 1, 2, or 3 hydroxyl groups;
(10) R₉, R₁₀, R₁₁, R₁₂, and R₁₃ are each independently C₂₋₆ alkenyl, C₆-C₁₀ aryl, "8- to 10-membered heteroaryl having 1, 2, 3, or 4 heteroatoms selected from 1, 2, 3, or 4 of N, O, S, and Se", C₆-C₁₀ aryl substituted with one or more R₉₋₁, or "8- to 10-membered heteroaryl having 1, 2, 3, or 4 heteroatoms selected from 1, 2, 3, or 4 of N, O, S, and Se" substituted with one or more R₉₋₂;
R₉₋₁ and R₉₋₂ are each independently C₁₋₆ alkyl, C₁₋₆ alkoxy, C₃₋₈ cycloalkyl, halogen, -CN, -C(O)N(C₁₋₆ alkyl)₂, -CH₂-N(C₁₋₆ alkyl)₂, C₁₋₆ alkyl substituted with one or more R₉₋ₐ, or C₁₋₆ alkoxy substituted with one or more R_{9-b};
R₉₋ₐ and R_{9-b} are each independently hydroxyl or halogen;
or, two adjacent groups of the R₉, R₁₀, R₁₁, R₁₂, and R₁₃ join to form a "4- to 8-membered saturated or unsaturated monocyclic heterocyclic ring having 1, 2, 3, or 4 heteroatoms selected from 1, 2, 3, or 4 of N, O, S, and Se";
(11) R₁₄₋₁ and R₁₄₋₂ are each independently -CONR^{m}Rⁿ or -CH₂-NR^{m}Rⁿ; and
(12) R₁₅ is C₃₋₈ cycloalkyl or C₁₋₆ alkyl substituted with one or more R₁₅₋₁;
each R₁₅₋₁ is independently halogen or deuterium.

5. The compound of formula II or formula III, or a tautomer thereof, a stereoisomer thereof, a pharmaceutically acceptable salt of any of the above, or a solvate of any of the above according to claim 1, wherein the compound of formula II or formula III, or a tautomer thereof, a stereoisomer thereof, a pharmaceutically acceptable salt of any of the above, or a solvate of any of the above, satisfies one or more of the following conditions:
(1) R₂ₐ and R₃ₐ, R₂ₐ and R_{3b}, R_{2b} and R_{3b}, or R_{2b} and R₃ₐ join to form ring A, X₁ is C(R₁ₐ), X₄ is C(R₄ₐ) or N, X₅ is C(R₅ₐ) or N, and R₁ₐ, R₄ₐ, and R₅ₐ are each independently hydrogen;
(2) when R₁ₐ and R₂ₐ, R₁ₐ and R_{2b}, R_{1b} and R_{2b}, or R_{1b} and R₂ₐ join to form ring A, X₃ is C(R₃ₐ) or N, X₄ is C(R₄ₐ) or N, X₅ is C(R₅ₐ) or N, and R₃ₐ, R₄ₐ, and R₅ₐ are each independently C₁₋₆ alkoxy substituted with one or more R^{X1}, C₃₋₈ cycloalkyl substituted with one or more R^{X1}, or C₂₋₆ alkenyl;
(3) when R₁ₐ and R₅ₐ, R₁ₐ and R_{5b}, R_{1b} and R_{5b}, or R_{1b} and R₅ₐ join to form ring A, X₂ is C(R₂ₐ) or N, X₃ is C(R₃ₐ) or N, X₄ is C(R₄ₐ) or N, and R₂ₐ, R₃ₐ, and R₄ₐ are each independently C₃₋₈ cycloalkyl substituted with one or more R^{X1}, -SO₂-R₂ₐₐ, -SO-R₂ₐₐ, -SO(=NH)R₂ₐₐ, or -PO(C₁₋₆ alkyl)₂.
(4) when R₄ₐ and R₅ₐ, R₄ₐ and R_{5b}, R_{4b} and R_{5b}, or R_{4b} and R₅ₐ join to form ring A, X₁ is C(R₁ₐ), X₂ is C(R₂ₐ) or N, X₃ is C(R₃ₐ) or N, and R₁ₐ, R₂ₐ, and R₃ₐ are each independently C₁₋₆ alkoxy substituted with one or more R^{X1} or C₃₋₈ cycloalkyl substituted with one or more R^{X1};
(5) when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently -SO₂-R₂ₐₐ, -SO-R₂ₐₐ, or -SO(=NH)R₂ₐₐ;
R₂ₐₐ is C₃₋₈ cycloalkyl or C₃₋₈ cycloalkyl substituted with one or more R^{X1};
(6) R₂ₐₐ is C₃₋₈ cycloalkyl or C₃₋₈ cycloalkyl substituted with one or more R^{X1};
(7) each R^{X1} is independently sulfonyl;
(8) each R^{X4} is independently C₁₋₆ alkoxy substituted with 1, 2, or 3 hydroxyl groups;
(9) R₁₋₁ and R₁₋₂ are each independently C₃₋₈ cycloalkyl substituted with one or more halogens, -SO₂(C₃₋₈ cycloalkyl), -SO(=NH)(C₁₋₆ alkyl), -SO(=NH)(C₃₋₈ cycloalkyl), -SCHF₂, -S(C₃₋₈ cycloalkyl), -SO(C₃₋₈ cycloalkyl), -CO(C₃₋₈ cycloalkyl), or -COCHF₂;
(10) R₅ is -SO₂(C₃₋₈ cycloalkyl) or -P(O)(C₃₋₈ cycloalkyl)₂;
(11) X is -Se-, -S-, S(=O), SO₂, or SO(=NH);
(12) when X is -S-, R₅ is oxo;
(13) R₆₋₁ is C₁₋₆ alkoxy substituted with one or more R₁₆₋₁, -SO₂(C₃₋₈ cycloalkyl), -SO(=NH)(C₁₋₆ alkyl), -SO(=NH)(C₃₋₈ cycloalkyl), -L-C₆-C₁₀ aryl, or -L-"4- to 8-membered heterocycloalkyl having 1, 2, 3, or 4 heteroatoms selected from 1, 2, 3, or 4 of N, O, S, and Se";
L is a single link bond or C₁₋₆ alkylene optionally substituted with an oxygen atom;
(14) R₆₋₂ is C₁₋₆ alkoxy substituted with one or more R₁₆₋₁, -SO₂(C₃₋₈ cycloalkyl), -SO(=NH)(C₁₋₆ alkyl), or -SO(=NH)(C₃₋₈ cycloalkyl);
(15) R₆₋₁ and R₆₋₂ are each independently -OR₁₅ₐ; R₈ₐ is -OR_{15b}; and R₁₅ₐ and R_{15b}, together with the atom to which they are attached, join to form a "3- to 8-membered saturated monocyclic, spirocyclic, or fused cyclic carbon ring" substituted with one or more C₁₋₆ alkyl;
(16) each R₁₆₋₁ is independently C₁₋₆ alkoxy, C₃₋₈ cycloalkyl, or C₁₋₆ alkoxy substituted with 1, 2, or 3 hydroxyl groups;
(17) R^{m} and Rⁿ are each independently C₁₋₆ alkyl substituted with one or more halogens, C₃₋₈ cycloalkyl substituted with one or more halogens, -CO(C₃₋₈ cycloalkyl), or -CO(C₃₋₈ cycloalkyl substituted with one or more halogens);
(18) each R^{m-3} is independently C₁₋₆ alkyl, C₁₋₆ alkoxy, or C₁₋₆ alkoxy substituted with 1, 2, or 3 hydroxyl groups;
(19) R^{z} is C₃₋₈ cycloalkyl or C₁₋₆ alkoxy substituted with 1, 2, or 3 hydroxyl groups;
(20) R₈ is C₃₋₈ cycloalkyl substituted with one or more R₈₋₁;
(21) R₉, R₁₀, R₁₁, R₁₂, and R₁₃ are each independently C₁₋₆ alkoxy substituted with one or more halogens or C₃₋₈ cycloalkyl substituted with one or more halogens;
(22) R₁₄₋₁ and R₁₄₋₂ are each independently C₁₋₆ alkoxy substituted with one or more halogens or C₃₋₈ cycloalkyl substituted with one or more halogens;
(23) R₁₅ is C₁₋₆ alkoxy, C₁₋₆ alkoxy substituted with one or more R₁₅₋₁, or C₃₋₈ cycloalkyl substituted with one or more R₁₅₋₁; and
(24) R₁₆ is C₃₋₈ cycloalkyl.

6. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) when R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, C₁₋₆ alkyl, or halogen;
(2) when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, C₃₋₈ cycloalkyl, -SF₅, -S-R₂ₐₐ, -PO(C₁₋₆ alkyl)₂, or C₁₋₆ alkyl substituted with one or more halogens; R₂ₐₐ is C₁₋₆ alkyl or C₁₋₆ alkyl substituted with one or more halogens;
(3) when R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen or C₁₋₆ alkyl substituted with one or more halogens;
(4) R₁₋₁ and R₁₋₂ are each independently oxo (=O), hydroxyl, -CN, C₁₋₆ alkyl, C₁₋₆ alkyl substituted with one or more halogens, carboxyl, amino, -CONH₂, -CONR^{x}R^{y}, or halogen;
(5) R₆₋₁ and R₆₋₂ are each independently hydrogen, hydroxyl, or C₁₋₆ alkyl substituted with one or more R₁₆₋₁;
(6) each R₁₆₋₁ is independently hydroxyl or -NR^{m}Rⁿ;
(7) R^{m} and Rⁿ are each independently C₁₋₆ alkyl;
(8) R₈ₐ is hydrogen or hydroxyl;
(9) R_{8b} is hydrogen;
(10) R₈ is C₁₋₆ alkyl, C₃₋₈ cycloalkyl, or C₁₋₆ alkyl substituted with one or more R₈₋₁;
(11) each R₈₋₁ is independently deuterium;
(12) R₉, R₁₀, R₁₁, R₁₂, and R₁₃ are each independently hydrogen, C₁₋₆ alkyl, or halogen;
(13) R₁₄ is C₆-C₁₀ aryl substituted with one or more R₁₄₋₁, or "8- to 10-membered heteroaryl having 1, 2, or 3 heteroatoms selected from 1, 2, or 3 of N, O, and S" substituted with one or more R₁₄₋₂;
(14) R₁₄₋₁ and R₁₄₋₂ are each independently C₁₋₆ alkyl or halogen;
(15) R₁₅ is hydrogen or methyl;
(16) Y is -C(R₁₆)=;
(17) R₁₆ is hydrogen or halogen;
(18) ring A is a 5- to 6-membered saturated or unsaturated monocyclic carbon ring, a 5- to 6-membered saturated or unsaturated monocyclic carbon ring substituted with one or more R₁₋₁, a "5-membered saturated or unsaturated monocyclic heterocyclic ring having 1 or 2 heteroatoms selected from 1 or 2 of N, O, and S", a "5-membered saturated or unsaturated monocyclic heterocyclic ring having 1 or 2 heteroatoms selected from 1 or 2 of N, O, and S" substituted with one or more R₁₋₂, or a "6-membered saturated or unsaturated monocyclic heterocyclic ring having 1 or 2 heteroatoms selected from 1 or 2 of N, O, and S"; and
(19) when X is -NR₆₋₂- or -O-, R₅ is oxo (=O).

7. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) R₁₋₁ and R₁₋₂ are each independently oxo (=O), hydroxyl, C₁₋₆ alkyl, or halogen;
(2) R₅ is oxo (=O);
(3) X is -CHR₆₋₁- or -NR₆₋₂-;
(4) R₆₋₁ and R₆₋₂ are each independently hydrogen or hydroxyl;
(5) R₈ is C₁₋₆ alkyl; 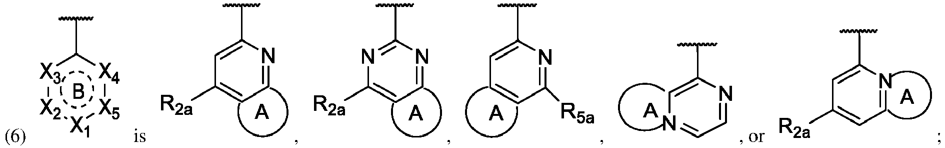 and
(7) R₂ₐ is halogen, C₃₋₈ cycloalkyl, C₁₋₆ alkoxy substituted with one or more halogens, -SF₅, -S-R₂ₐₐ, C₁₋₆ alkyl, or C₁₋₆ alkyl substituted with one or more halogens.

8. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) R₁₋₁ and R₁₋₂ are each independently halogen;
(2) R₆₋₁ is hydroxyl, and R₆₋₂ is hydrogen;
(3) R₂ₐ is C₁₋₆ alkyl substituted with one or more halogens; and
(4) the compound of formula I has a structure of formula I-A or formula I-B:

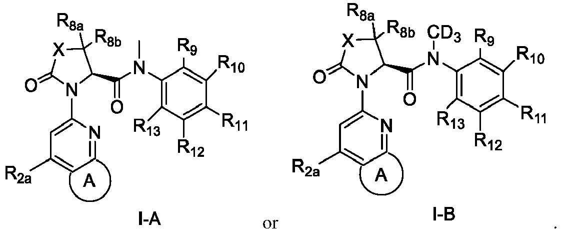

9. The compound of formula II or formula III, or a tautomer thereof, a stereoisomer thereof, a pharmaceutically acceptable salt of any of the above, or a solvate of any of the above according to any one of claims 1-3, wherein 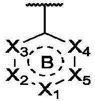 is 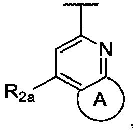 ring A is a 5-membered saturated or unsaturated monocyclic carbon ring, a 5-membered saturated or unsaturated monocyclic carbon ring substituted with one or more halogens, or a "4- to 8-membered saturated or unsaturated monocyclic heterocyclic ring having 1, 2, or 3 heteroatoms selected from 1, 2, or 3 of N, O, and S";
R₂ₐ is C₁₋₆ alkyl substituted with one or more halogens;
R₅ is oxo (=O);
X is -NH- or -CH(OH)-;
R₈ₐ is hydrogen or hydroxyl;
R_{8b} is hydrogen;
R₈ is C₁₋₆ alkyl; and
R₉, R₁₀, R₁₁, R₁₂, and R₁₃ are each independently hydrogen or halogen.

10. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkyl" groups of the "C₁₋₆ alkyl substituted with one or more halogens", the "C₁₋₆ alkyl substituted with one or more R^{X1}", and the "C₁₋₆ alkyl" are each independently methyl, ethyl, n-propyl, isopropyl, n-butyl, isobutyl, sec-butyl, or tert-butyl;
(2) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkoxy" groups of the "C₁₋₆ alkoxy" and the "C₁₋₆ alkoxy substituted with one or more R^{X1}" are each independently methoxy, ethoxy, n-propoxy, isopropoxy, n-butoxy, isobutoxy, sec-butoxy, or tert-butoxy;
(3) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "halogen" groups of the "C₁₋₆ alkyl substituted with one or more halogens" and the "halogen" are each independently fluorine, chlorine, bromine, or iodine;
(4) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkyl" groups of the "C₁₋₆ alkyl substituted with one or more halogens", the "C₁₋₆ alkyl substituted with one or more R^{X1}", the "-PO(C₁₋₆ alkyl)₂", and the "C₁₋₆ alkyl" are each independently methyl, ethyl, n-propyl, isopropyl, n-butyl, isobutyl, sec-butyl, or tert-butyl;
(5) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkoxy" groups of the "C₁₋₆ alkoxy" and the "C₁₋₆ alkoxy substituted with one or more R^{X1}" are each independently methoxy, ethoxy, n-propoxy, isopropoxy, n-butoxy, isobutoxy, sec-butoxy, or tert-butoxy;
(6) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "halogen" groups of the "C₁₋₆ alkyl substituted with one or more halogens" and the "halogen" are each independently fluorine, chlorine, bromine, or iodine;
(7) when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "C₁₋₆ alkyl" groups of the "C₁₋₆ alkyl substituted with one or more halogens", the "C₁₋₆ alkyl substituted with one or more R^{X1}", and the "C₁₋₆ alkyl" are each independently methyl, ethyl, n-propyl, isopropyl, n-butyl, isobutyl, sec-butyl, or tert-butyl;
(8) when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "C₁₋₆ alkoxy" groups of the "C₁₋₆ alkoxy" and the "C₁₋₆ alkoxy substituted with one or more R^{X1}" are each independently methoxy, ethoxy, n-propoxy, isopropoxy, n-butoxy, isobutoxy, sec-butoxy, or tert-butoxy;
(9) when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "halogen" groups of the "C₁₋₆ alkyl substituted with one or more halogens" and the "halogen" are each independently fluorine, chlorine, bromine, or iodine;
(10) in R^{x}, R^{y}, R₁₋₁, R₁₋₂, R₆₋₁, R₆₋₂, R^{m}, Rⁿ, R^{z}, R₈, R₉, R₁₀, R₁₁, R₁₂, R₁₃, R₁₄₋₁, R₁₄₋₂, R₁₅, R₅, R₂ₐₐ, R₁₆₋ₐ, R_{16-b}, R₉₋₁, R^{m-3}, R₉₋₂, R^{X4}, R₁₆, R₆₋₁₋₁, and R₆₋₁₋₂, the "C₁₋₆ alkyl" groups of the "C₁₋₆ alkyl", the "C₁₋₆ alkyl substituted with 1, 2, or 3 R^{X1}", the "-C(O)C₁₋₆ alkyl", the "C₁₋₆ alkyl substituted with one or more halogens", the "C₁₋₆ alkyl substituted with one or more R^{X1}", the "C₁₋₆ alkyl substituted with one or more R₁₆₋₁", the "C₁₋₆ alkyl substituted with 1, 2, or 3 hydroxyl groups", the "C₁₋₆ alkyl substituted with one or more R₈₋₁", the "-SO(=NH)(C₁₋₆ alkyl)", the "-C(O)C₁₋₆ alkyl substituted with 1, 2, or 3 R^{X3}", the "C₁₋₆ alkyl substituted with one or more R₉₋ₐ", the "-C(O)OC₁₋₆ alkyl", the "-C(O)N(C₁₋₆ alkyl)₂", the "-CH₂-N(C₁₋₆ alkyl)₂", the "-SO₂-C₁₋₆ alkyl", the "-P(O)(C₁₋₆ alkyl)₂", the "-NH(C₁₋₆ alkyl)", the "-N(C₁₋₆ alkyl)₂", the "-S(C₁₋₆ alkyl)", the "C₁₋₆ alkyl substituted with 1, 2, or 3 R^{m-1}", the "-CO(C₁₋₆ alkyl) substituted with 1, 2, or 3 R^{m-2}", and the "C₁₋₆ alkyl substituted with one or more R₁₅₋₁" are each independently methyl, ethyl, n-propyl, isopropyl, n-butyl, isobutyl, sec-butyl, or tert-butyl;
(11) in R^{X1}, R₁₋₁, R₁₋₂, R₆₋₁, R₆₋₂, R₁₆₋₁, R₈₋₁, R₉, R₁₀, R₁₁, R₁₂, R₁₃, R₁₄₋₁, R₁₄₋₂, R₁₅, R₅, R₉₋₁, R₉₋₂, R₂ₐ, R₃ₐ, R₄ₐ, R₂ₐₐ, R₉₋ₐ, R_{9-b}, R^{X2}, R^{X3}, R^{X5}, R^{m-1}, R^{m-2}, R₁₅₋₁, and R₁₆, the "halogen" groups of the "halogen", the "C₁₋₆ alkoxy substituted with one or more halogens", and the "C₁₋₆ alkyl substituted with one or more halogens" are each independently fluorine, chlorine, bromine, or iodine;
(12) in R^{X4}, R₁₆₋₁, R^{m-3}, R^{z}, R₁₅, R₁₋₁, R₁₋₂, R₆₋₁, R₆₋₂, R^{z}, R₉, R₁₀, R₁₁, R₁₂, R₁₃, R₁₄₋₁, R₁₄₋₂, R₉₋₁, R₉₋₂, R₂ₐ, R₃ₐ, R₄ₐ, and R₁₆, the C₁₋₆ alkoxy groups of the "C₁₋₆ alkoxy", the "C₁₋₆ alkoxy substituted with one or more halogens", the "C₁₋₆ alkoxy substituted with 1, 2, or 3 hydroxyl groups", the "C₁₋₆ alkoxy substituted with one or more R₁₆₋₁", the "C₁₋₆ alkoxy substituted with one or more R₁₅₋₁", and the "C₁₋₆ alkoxy substituted with one or more R_{9-b}" are each independently methoxy, ethoxy, n-propoxy, isopropoxy, n-butoxy, isobutoxy, sec-butoxy, or tert-butoxy;
(13) in R₆₋₁, R₁₄, R₁₆₋₁, R₉, R₁₀, R₁₁, R₁₂, and R₁₃, the C₆-C₁₀ aryl groups of the "C₆-C₁₀ aryl substituted with one or more R₁₄₋₁", the "C₆-C₁₀ aryl substituted with one or more R₁₆₋ₐ", the "C₆-C₁₀ aryl substituted with one or more R₉₋₁", the "-L-C₆-C₁₀ aryl", the "-L-(C₆-C₁₀ aryl substituted with one or more R₆₋₁₋₁)", and the "C₆-C₁₀ aryl" are each independently phenyl;
(14) in ring A, the "3- to 8-membered saturated or unsaturated monocyclic, spirocyclic, or fused cyclic carbon ring" groups of the "3-to 8-membered saturated or unsaturated monocyclic, spirocyclic, or fused cyclic carbon ring" and the "3- to 8-membered saturated or unsaturated monocyclic, spirocyclic, or fused cyclic carbon ring substituted with one or more R₁₋₁" are each independently a 5- to 6-membered saturated or unsaturated monocyclic ring;
(15) in ring A, the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" groups of the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" and the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se substituted with one or more R₁₋₂" are each independently a "4- to 8-membered saturated or unsaturated monocyclic, spirocyclic, or fused cyclic heterocyclic ring having 1, 2, or 3 heteroatoms selected from 1, 2, or 3 of N, O, and S", preferably a 5-membered unsaturated monocyclic ring having 1 or 2 heteroatoms selected from 1, 2, or 3 of N, O, and S;
(16) in R₂ₐₐ, R₅, R₁₆₋₁, R^{z}, R₁₆, R₁₋₁, R₁₋₂, R₈, R₉, R₁₀, R₁₁, R₁₂, R₁₃, R₁₄₋₁, R₆₋₁, R₆₋₂, R₉₋₁, R₉₋₂, R^{m}, Rⁿ, R^{x}, R^{y}, R₁₅, and R₁₄₋₂, the C₃₋₈ cycloalkyl groups of the "C₃₋₈ cycloalkyl", the "C₃₋₈ cycloalkyl substituted with one or more R^{X1}", the "C₃₋₈ cycloalkyl substituted with one or more halogens", the "-SO₂(C₃₋₈ cycloalkyl)", the "-SO(=NH)(C₃₋₈ cycloalkyl)", the "-S(C₃₋₈ cycloalkyl)", the "-SO(C₃₋₈ cycloalkyl)", the "-P(O)(C₃₋₈ cycloalkyl)₂", the "-CO(C₃₋₈ cycloalkyl substituted with one or more halogens)", the "C₃₋₈ cycloalkyl substituted with one or more R₈₋₁", the "C₃₋₈ cycloalkyl substituted with one or more R₁₅₋₁", the "-C(O)C₃₋₈ cycloalkyl", the "C₃₋₈ cycloalkyl substituted with 1, 2, or 3 R^{X2}", and the "C₃₋₈ cycloalkyl substituted with 1, 2, or 3 R^{X5}" are each independently cyclopropyl, cyclobutyl, cyclopentyl, or cyclohexyl;
(17) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the C₃₋₈ cycloalkyl groups of the "C₃₋₈ cycloalkyl" and the "C₃₋₈ cycloalkyl substituted with one or more R^{X1}" are each independently cyclopropyl, cyclobutyl, cyclopentyl, or cyclohexyl;
(18) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the C₃₋₈ cycloalkyl groups of the "C₃₋₈ cycloalkyl" and the "C₃₋₈ cycloalkyl substituted with one or more R^{X1}" are each independently cyclopropyl, cyclobutyl, cyclopentyl, or cyclohexyl;
(19) when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the C₃₋₈ cycloalkyl groups of the "C₃₋₈ cycloalkyl" and the "C₃₋₈ cycloalkyl substituted with one or more R^{X1}" are each independently cyclopropyl, cyclobutyl, cyclopentyl, or cyclohexyl;
(20) in R₉, R₁₀, R₁₁, R₁₂, R₁₃, and R₁₄, the "8- to 10-membered heteroaryl having 1, 2, 3, or 4 heteroatoms selected from 1, 2, 3, or 4 of N, O, S, and Se" groups of the "8- to 10-membered heteroaryl having 1, 2, 3, or 4 heteroatoms selected from 1, 2, 3, or 4 of N, O, S, and Se", the "8- to 10-membered heteroaryl having 1, 2, 3, or 4 heteroatoms selected from 1, 2, 3, or 4 of N, O, S, and Se substituted with one or more R₉₋₂", and the "8- to 10-membered heteroaryl having 1, 2, 3, or 4 heteroatoms selected from 1, 2, 3, or 4 of N, O, S, and S substituted with one or more R₁₄₋₂" are each independently "8- to 10-membered heteroaryl having 1, 2, or 3 heteroatoms selected from 1, 2, or 3 of N, O, and S", preferably a heteroaryl group having 2 heteroatoms N formed by the fusion of a 5-membered heteroaromatic ring and a 5- to 6-membered heteroaromatic ring;
(21) in R₆₋₁ and R₆₋₂, the "oxa-C₃₋₈ cycloalkyl" groups are oxiranyl, oxetanyl, oxolanyl, or oxacyclohexyl;
(22) in R₆₋₁, R^{x}, R^{y}, R₁₆₋₁, R^{m}, and Rⁿ, the "-L-(4- to 8-membered heterocycloalkyl having 1, 2, 3, or 4 heteroatoms selected from 1, 2, 3, or 4 of N, O, S, and Se)" groups of the "-L-(4- to 8-membered heterocycloalkyl having 1, 2, 3, or 4 heteroatoms selected from 1, 2, 3, or 4 of N, O, S, and Se)", the "-L-(4- to 8-membered heterocycloalkyl having 1, 2, 3, or 4 heteroatoms selected from 1, 2, 3, or 4 of N, O, S, and Se substituted with one or more R₆₋₁₋₂)", the "4- to 8-membered heterocycloalkyl having 1, 2, 3, or 4 heteroatoms selected from 1, 2, 3, or 4 of N, O, S, and Se", the "4- to 8-membered heterocycloalkyl having 1, 2, 3, or 4 heteroatoms selected from 1, 2, 3, or 4 of N, O, S, and Se substituted with 1, 2, or 3 R^{X4}", the "4- to 8-membered heterocycloalkyl having 1, 2, 3, or 4 heteroatoms selected from 1, 2, 3, or 4 of N, O, S, and Se substituted with one or more R_{16-b}", and the "4- to 8-membered heterocycloalkyl having 1, 2, 3, or 4 heteroatoms selected from 1, 2, 3, or 4 of N, O, S, and Se substituted with 1, 2, or 3 R^{m-3}" are each independently "4- to 8-membered heterocycloalkyl having 1, 2, or 3 heteroatoms selected from 1, 2, or 3 of N, O, and S", preferably 5-membered heterocycloalkyl having 1 or 2 heteroatoms selected from N or S;
(23) in R₉, R₁₀, R₁₁, R₁₂, R₁₃, R₁₋₁, and R₁₋₂, the "C₂₋₆ alkenyl" groups are ethenyl or propenyl;
(24) in R₁₋₁ and R₁₋₂, the "C₂₋₆ alkynyl" groups are ethynyl or propynyl;
(25) when R^{x} and R^{y} together with the N atom to which they are attached join to form a "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se", the heterocyclic ring is a 4- to 8-membered saturated monocyclic heterocyclic ring containing one heteroatom N;
(26) when two adjacent groups of R₉, R₁₀, R₁₁, R₁₂, and R₁₃ join to form a "4- to 8-membered saturated or unsaturated monocyclic heterocyclic ring having 1, 2, 3, or 4 heteroatoms selected from 1, 2, 3, or 4 of N, O, S, and Se", the heterocyclic ring is a "4- to 8-membered saturated or unsaturated monocyclic, spirocyclic, or fused cyclic heterocyclic ring having 1, 2, or 3 heteroatoms selected from 1, 2, or 3 of N, O, and S";
(27) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₂₋₆ alkenyl" groups are ethenyl or propenyl; and
(28) in L, the "C₁₋₆ alkylene" group of the "C₁₋₆ alkylene optionally substituted with an oxygen atom" is methylene or ethylene.

11. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "C₁₋₆ alkyl" groups of the "C₁₋₆ alkyl substituted with one or more halogens" and the "C₁₋₆ alkyl" are methyl;
(2) when R₁ₐ and R₂ₐ, R₁ₐ and R_{2b}, R_{1b} and R_{2b}, or R_{1b} and R₂ₐ join to form ring A, in R₃ₐ, R₄ₐ, and R₅ₐ, the "halogen" groups of the "C₁₋₆ alkyl substituted with one or more halogens" and the "halogen" are fluorine;
(3) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "C₁₋₆ alkyl" groups of the "C₁₋₆ alkyl substituted with one or more halogens" and the "C₁₋₆ alkyl" are methyl;
(4) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the "halogen" groups of the "C₁₋₆ alkyl substituted with one or more halogens" and the "halogen" are fluorine;
(5) when R₄ₐ and R₅ₐ, R₄ₐ and R_{5b}, R_{4b} and R_{5b} or R_{4b} and R₅ₐ join to form ring A, in R₁ₐ, R₂ₐ, and R₃ₐ, the "C₁₋₆ alkyl" groups of the "C₁₋₆ alkyl substituted with one or more halogens" and the "C₁₋₆ alkyl" are methyl;
(6) when R₄ₐ and R₅ₐ, R₄ₐ and R_{5b}, R_{4b} and R_{5b}, or R_{4b} and R₅ₐ join to form ring A, in R₁ₐ, R₂ₐ, and R₃ₐ, the "halogen" groups of the "C₁₋₆ alkyl substituted with one or more halogens" and the "halogen" are each independently fluorine or chlorine;
(7) in R^{x}, R^{y}, R₁₋₁, R₁₋₂, R₆₋₁, R₆₋₂, R^{m}, Rⁿ, R^{z}, R₈, R₉, R₁₀, R₁₁, R₁₂, R₁₃, R₁₄₋₁, R₁₄₋₂, R₁₅, R₅, R₂ₐₐ, R₁₆₋ₐ, R_{16-b}, R₉₋₁, R^{m-3}, R₉₋₂, R^{X4}, and R₁₆, the "C₁₋₆ alkyl" groups of the "C₁₋₆ alkyl", "C₁₋₆ alkyl substituted with 1, 2, or 3 R^{X1}", "-C(O)C₁₋₆ alkyl", "C₁₋₆ alkyl substituted with one or more halogens", "C₁₋₆ alkyl substituted with one or more R₁₆₋₁", "C₁₋₆ alkyl substituted with 1, 2, or 3 hydroxyl groups", "C₁₋₆ alkyl substituted with one or more R₈₋₁", the "-C(O)C₁₋₆alkyl substituted with 1, 2, or 3 R^{X3}", the "C₁₋₆ alkyl substituted with one or more R₉₋ₐ", the "-C(O)OC₁₋₆ alkyl", the "-C(O)N(C₁₋₆ alkyl)₂", the "-CH₂-N(C₁₋₆ alkyl)₂", the "-SO₂-C₁₋₆ alkyl", the "-P(O)(C₁₋₆ alkyl)₂", the "-NH(C₁₋₆ alkyl)", the "-N(C₁₋₆ alkyl)₂", the "-S(C₁₋₆ alkyl)", the "C₁₋₆ alkyl substituted with 1, 2, or 3 R^{m-1}", the "-CO(C₁₋₆ alkyl) substituted with 1, 2, or 3 R^{m-2}", and the "C₁₋₆ alkyl substituted with one or more R₁₅₋₁" are methyl;
(8) in R^{X1}, R₁₋₁, R₁₋₂, R₆₋₁, R₆₋₂, R₁₆₋₁, R₈₋₁, R₉, R₁₀, R₁₁, R₁₂, R₁₃, R₁₄₋₁, R₁₄₋₂, R₁₅, R₅, R₉₋₁, R₉₋₂, R₂ₐ, R₃ₐ, R₄ₐ, R₂ₐₐ, R₉₋ₐ, R_{9-b}, R^{X2}, R^{X3}, R^{X5}, R^{m-1}, R^{m-2}, R₁₅₋₁, and R₁₆, the "halogen" groups of the "halogen", the "C₁₋₆ alkoxy substituted with one or more halogens", and the "C₁₋₆ alkyl substituted with one or more halogens" are fluorine;
(9) in ring A, the "3- to 8-membered saturated or unsaturated monocyclic, spirocyclic, or fused cyclic carbon ring" groups of the "3-to 8-membered saturated or unsaturated monocyclic, spirocyclic, or fused cyclic carbon ring" and the "3- to 8-membered saturated or unsaturated monocyclic, spirocyclic, or fused cyclic carbon ring substituted with one or more R₁₋₁" are each independently 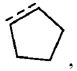 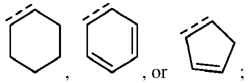
(10) in ring A, the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" groups of the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se" and the "4- to 8-membered saturated or unsaturated monocyclic, spirocyclic, or fused cyclic heterocyclic ring having 1, 2, 3, or 4 heteroatoms selected from 1, 2, 3, or 4 of N, O, S, and Se substituted with one or more R₁₋₂" are each independently 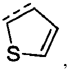 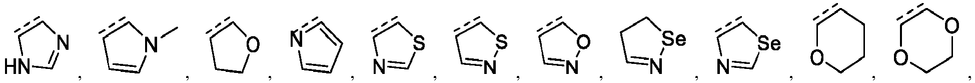 or 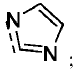
(11) when R₁ₐ and R₅ₐ, R₁ₐ and R_{5b}, R_{1b} and R_{5b}, or R_{1b} and R₅ₐ join to form ring A, in R₂ₐ, R₃ₐ, and R₄ₐ, the C₃₋₈ cycloalkyl groups of the "C₃₋₈ cycloalkyl" and the "C₃₋₈ cycloalkyl substituted with one or more R^{X1}" are each independently cyclopropyl; and
(12) in R₂ₐₐ, R₅, R₁₆₋₁, R^{z}, R₁₆, R₁₋₁, R₁₋₂, R₈, R₉, R₁₀, R₁₁, R₁₂, R₁₃, R₁₄₋₁, R₆₋₁, R₆₋₂, R₉₋₁, R₉₋₂, R^{m}, Rⁿ, R^{x}, R^{y}, R₁₅, and R₁₄₋₂, the C₃₋₈ cycloalkyl groups of the "C₃₋₈ cycloalkyl", the "C₃₋₈ cycloalkyl substituted with one or more R^{X1}", the "C₃₋₈ cycloalkyl substituted with one or more halogens", the "-SO₂(C₃₋₈ cycloalkyl)", the "-SO(=NH)(C₃₋₈ cycloalkyl)", the "-S(C₃₋₈ cycloalkyl)", the "-SO(C₃₋₈ cycloalkyl)", the "-P(O)(C₃₋₈ cycloalkyl)₂", the "-CO(C₃₋₈ cycloalkyl substituted with one or more halogens)", the "C₃₋₈ cycloalkyl substituted with one or more R₈₋₁", the "C₃₋₈ cycloalkyl substituted with one or more R₁₅₋₁", the "-C(O)C₃₋₈ cycloalkyl", the "C₃₋₈ cycloalkyl substituted with 1, 2, or 3 R^{X2}", and the "-C(O)C₃₋₈ cycloalkyl substituted with 1, 2, or 3 R^{X5}" are each independently cyclopropyl.

12. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) when R₁ₐ and R₂ₐ, R₁ₐ and R_{2b}, R_{1b} and R_{2b}, or R_{1b} and R₂ₐ join to form ring A, R₃ₐ, R₄ₐ, and R₅ₐ are each independently hydrogen, methyl, or chlorine;
(2) when R₁ₐ and R₅ₐ, R₁ₐ and R_{5b}, R_{1b} and R_{5b}, or R_{1b} and R₅ₐ join to form ring A, R₂ₐ, R₃ₐ, and R₄ₐ are each independently hydrogen, trifluoromethyl, cyclopropyl, -SMe, -SF₅, or -P(O)(CH₃)₂;
(3) when R₄ₐ and R₅ₐ, R₄ₐ and R_{5b}, R_{4b} and R_{5b}, or R_{4b} and R₅ₐ join to form ring A, R₁ₐ, R₂ₐ, and R₃ₐ are each independently hydrogen or trifluoromethyl;
(4) R₁₋₁ and R₁₋₂ are each independently oxo (=O), hydroxyl, amino, -CONH₂, -CONHMe, -CN, methyl, fluorine, or trifluoromethyl;
(5) R^{m} and Rⁿ are methyl;
(6) X is -NH-, -CH(OH)-, -O- or 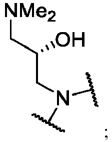
(7) R₈ is methyl, cyclopropyl, or -CD₃;
(8) R₉, R₁₀, R₁₁, R₁₂, and R₁₃ are each independently hydrogen, fluorine, methyl, or chlorine;
(9) R₁₄₋₁ and R₁₄₋₂ are each independently methyl, fluorine, or chlorine;
(10) R₁₅ is hydrogen or methyl; and
(11) Y is -C(F)=, -C(Cl)=, or -C(H)=.

13. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III, or a tautomer thereof, a stereoisomer thereof, a pharmaceutically acceptable salt of any of the above, or a solvate of any of the above, satisfies one or more of the following conditions:
(1) R₁₋₁ and R₁₋₂ are each independently fluorine;
(2) X is -NH- or -CH(OH)-;
(3) each R₁₆₋₁ is independently hydroxyl or 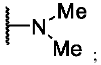
(4) R₈ is methyl; and
(5) R₁₄ is

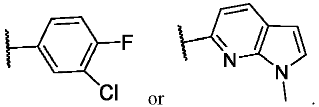

14. The compound of formula II or formula III, or a tautomer thereof, a stereoisomer thereof, a pharmaceutically acceptable salt of any of the above, or a solvate of any of the above according to any one of claims 1-3, wherein 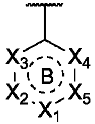 is any one of the following structural fragments: 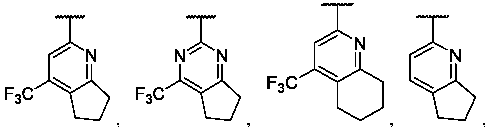 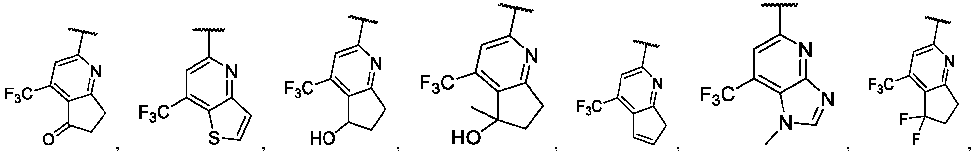 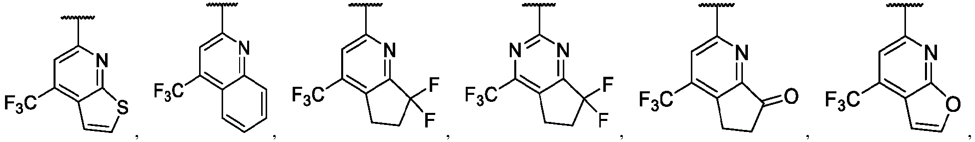 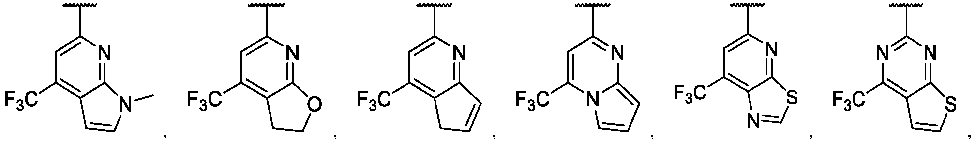 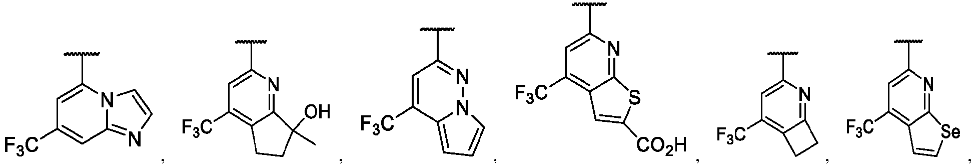 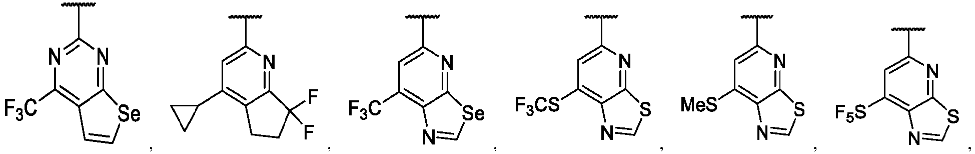 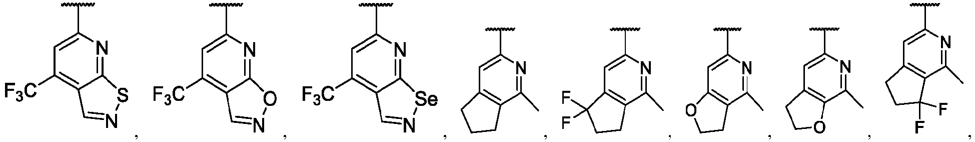 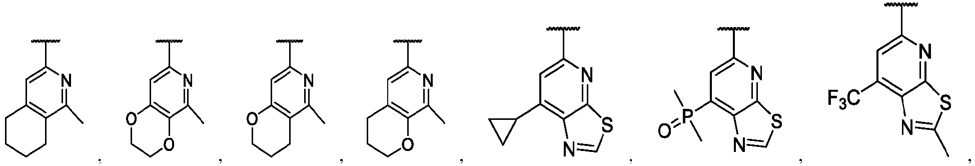 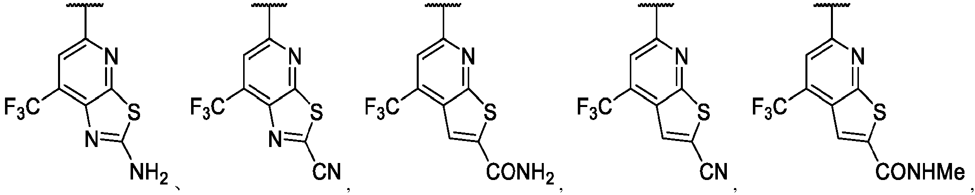 or

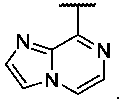

15. The compound of formula II or formula III, or a tautomer thereof, a stereoisomer thereof, a pharmaceutically acceptable salt of any of the above, or a solvate of any of the above according to any one of claims 1-3, wherein the compound of formula II or formula III is any one of the following:

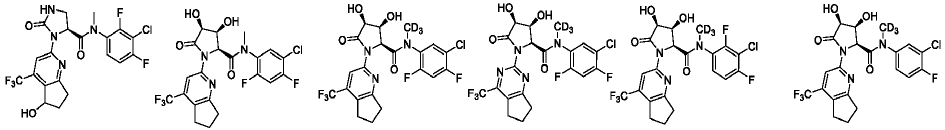

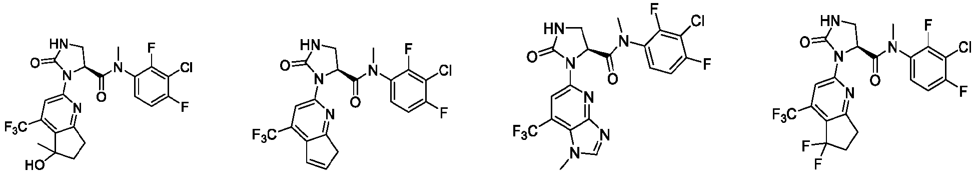

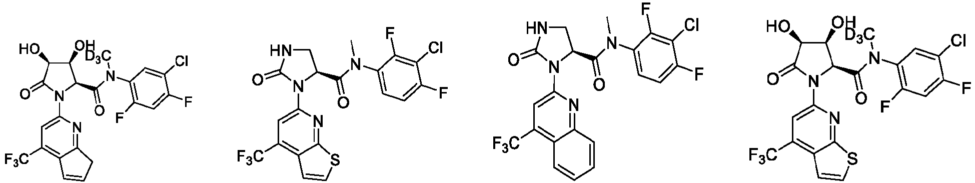

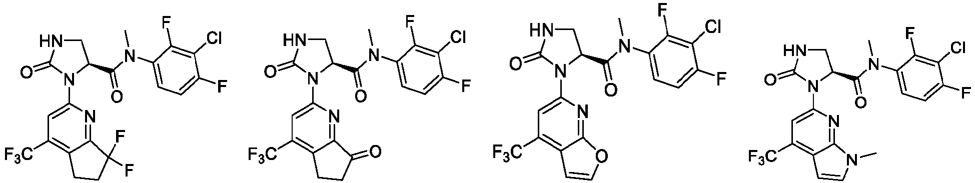

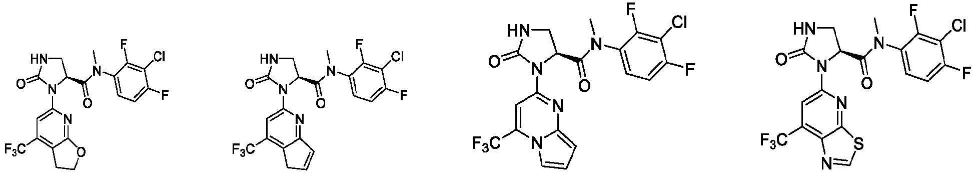

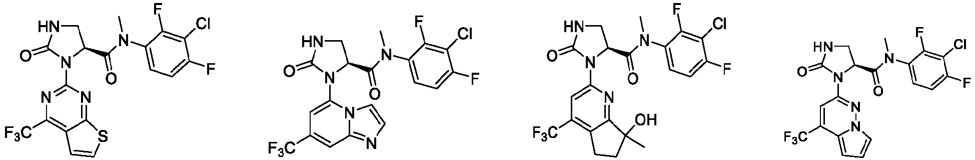

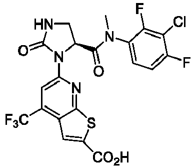

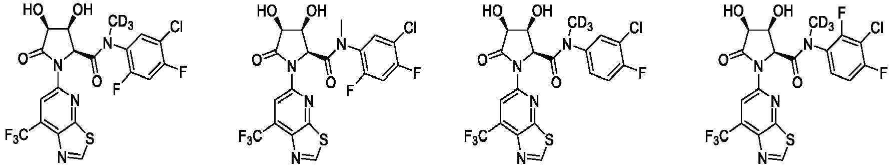

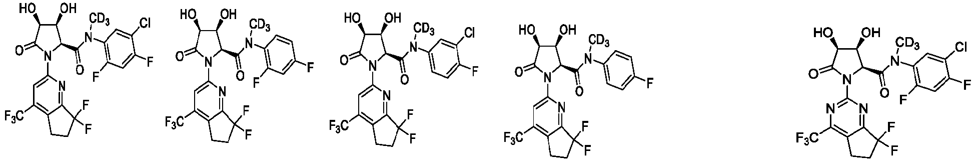

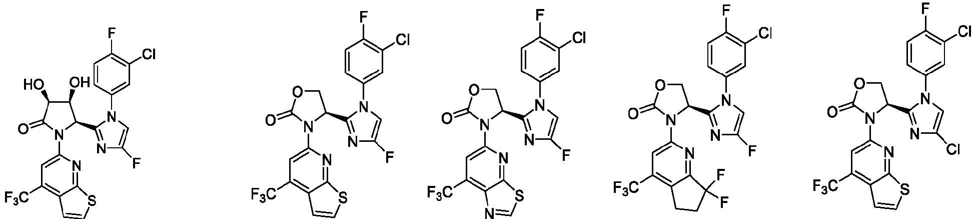

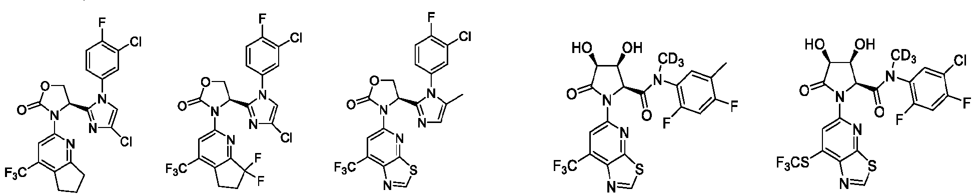

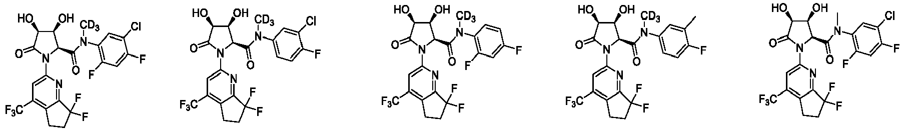

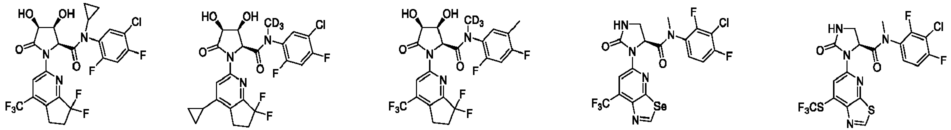

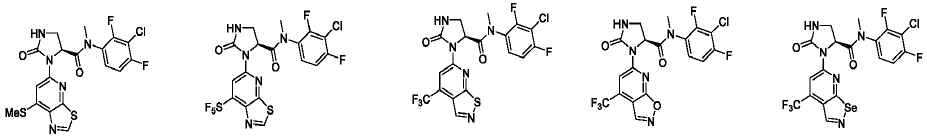

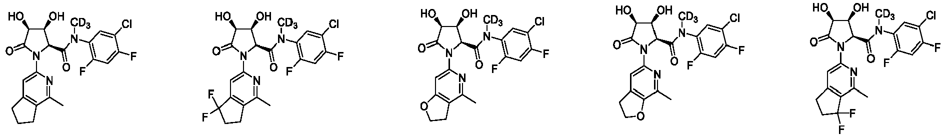

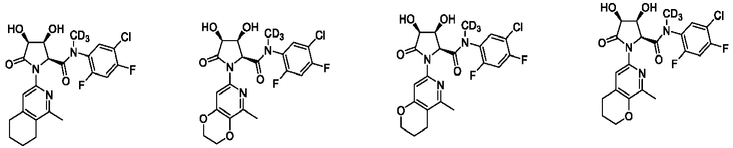

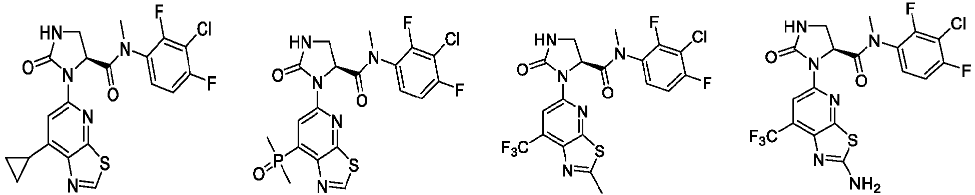

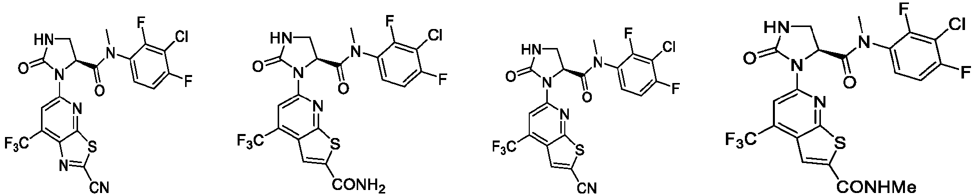

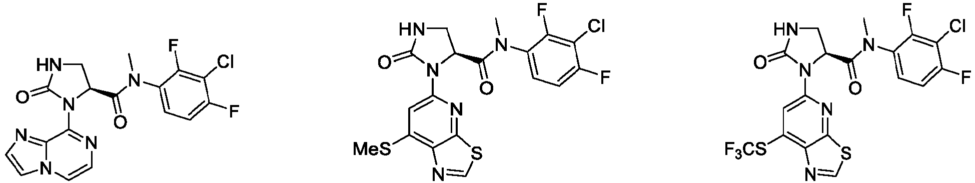

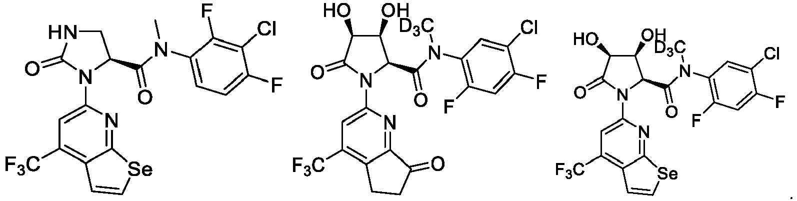

16. The compound of formula II or formula III, or a tautomer thereof, a stereoisomer thereof, a pharmaceutically acceptable salt of any of the above, or a solvate of any of the above according to any one of claims 1-3, wherein the compound of formula III is any one of the following:
a compound having a retention time of 1.57 min under the following conditions, which is one of the stereoisomers of 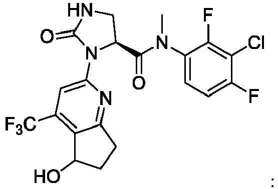 chromatographic column CHIRALPAK ID-3 4.6*50 mm, 3 um; mobile phase A: (0.1% diethylamine) n-hexane, mobile phase B: ethanol, mobile phase A:mobile phase B = 70:30; flow rate: 1.0 mL/min; temperature: 25°C;
a compound having a retention time of 1.92 min under the following conditions, which is one of the stereoisomers of 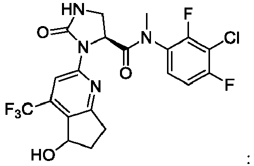 chromatographic column CHIRALPAK ID-3 4.6*50 mm, 3 um; mobile phase A: (0.1% diethylamine) n-hexane, mobile phase B: ethanol, mobile phase A:mobile phase B = 70:30; flow rate: 1.0 mL/min; temperature: 25°C;
a compound having a retention time of 1.64 min under the following conditions, which is one of the stereoisomers of 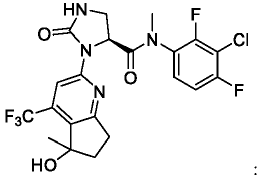 chromatographic column: CHIRALPAK IA-3 Column 4.6*50 mm, 3 um; mobile phase A: (0.1% diethylamine) n-hexane, mobile phase B: ethanol; mobile phase A:mobile phase B = 80:20; flow rate: 1 mL/min; temperature: 25°C;
a compound having a retention time of 2.67 min under the following conditions, which is one of the stereoisomers of 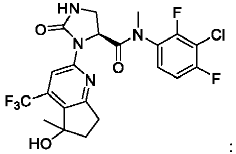 chromatographic column: CHIRALPAK IA-3 Column 4.6*50 mm, 3 um; mobile phase A: (0.1% diethylamine) n-hexane, mobile phase B: ethanol; mobile phase A:mobile phase B = 80:20; flow rate: 1 mL/min; temperature: 25°C;
a compound having a retention time of 3.52 min under the following conditions, which is one of the rotamers of 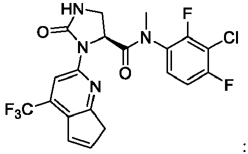 chromatographic column CHIRAL Cellulose-SB 4.6*100 mm, 3 um; mobile phase A: (0.1% formic acid) n-hexane, mobile phase B: ethanol; mobile phase A:mobile phase B = 80:20; flow rate: 1 mL/min; temperature: 25°C;
a compound having a retention time of 3.94 min under the following conditions, which is one of the rotamers of 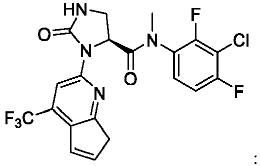 chromatographic column CHIRAL Cellulose-SB 4.6*100 mm, 3 um; mobile phase A: (0.1% formic acid) n-hexane, mobile phase B: ethanol; mobile phase A:mobile phase B = 80:20; flow rate: 1 mL/min; temperature: 25°C;
a compound having a retention time of 6.30 min under the following conditions, which is one of the stereoisomers of 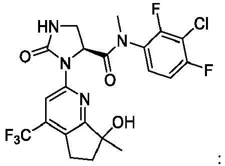 chromatographic column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 32% B to 63% B within 8 min; wavelength: 220 nm; and
a compound having a retention time of 7.58 min under the following conditions, which is one of the stereoisomers of 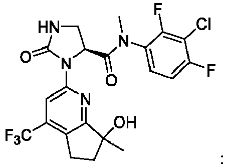 chromatographic column: XBridge Prep OBD C18 column, 30*150 mm, 5 µm; mobile phase A: 10 mmol/L ammonium bicarbonate aqueous solution, mobile phase B: acetonitrile; flow rate: 60 mL/min; gradient: 32% B to 63% B within 8 min; wavelength: 220 nm.

17. A pharmaceutical composition comprising: (1) the compound of formula II or formula III, or a tautomer thereof, a stereoisomer thereof, a pharmaceutically acceptable salt of any of the above, or a solvate of any of the above according to any one of claims 1-16, and (2) a pharmaceutically acceptable excipient.

18. Use of the compound of formula II or formula III, or a tautomer thereof, a stereoisomer thereof, a pharmaceutically acceptable salt of any of the above, or a solvate of any of the above according to any one of claims 1-16, in the preparation of a Polθ inhibitor.

19. The use according to claim 18, wherein the Polθ inhibitor is used in a mammalian organism and/or in vitro, mainly for experimental purposes.

20. Use of the compound of formula II or formula III, or a tautomer thereof, a stereoisomer thereof, a pharmaceutically acceptable salt of any of the above, or a solvate of any of the above according to any one of claims 1-16, in the preparation of a medicine for the treatment and/or prevention of cancer.

21. The use according to claim 20, wherein the cancer is breast cancer, ovarian cancer, prostatic cancer, or pancreatic cancer.
